# EUROPÄISCHE PATENTANMELDUNG

(11) **EP 3 828 159 A1**
(43) Veröffentlichungstag der Anmeldung: **02.06.2021**
(21) Anmeldenummer: 19212189.5
(22) Anmeldetag: 28.11.2019
(51) Int. Cl.: C07C 51/50, C07C 57/04, C07C 67/62, C07C 69/54, C07C 7/20, C07C 15/46

(54) **LAGER- UND TRANSPORTSTABILISATOREN FÜR POLYMERISATIONSFÄHIGE VERBINDUNGEN**

(71) Anmelder: BASF SE, 67056 Ludwigshafen am Rhein (DE)
(72) Erfinder: HAREMZA, Sylke, 69151 Neckargemuend (DE); MARTIN, Friedrich-Georg, 67056 Ludwigshafen (DE); KREMZOW-GRAW, Doris, 67056 Ludwigshafen (DE); SURE, Rebecca, 67056 Ludwigshafen (DE); ROMEIS, Juergen, 67056 Ludwigshafen (DE); SCHELKLE, Korwin, 400705 Navi Mumbai (IN); FLEISCHHAKER, Friederike, 67056 Ludwigshafen (DE); GORGES, Jan Niclas, 67056 Ludwigshafen (DE)
(74) Vertreter: BASF IP Association

(57) **Zusammenfassung**

Mischung enthaltend eine oder mehrere Verbindungen der allgemeinen Formel (I) 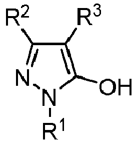 wobei
R¹ H, C₁- bis C₂₀-Alkyl, Alkanoyl mit C₁- bis C₈-Alkyl oder ein aromatischer Rest ist,
R² H, C₁- bis C₂₀-Alkyl, Carboxylester mit C₁- bis C₈-Alkyl, Alkanoyl mit C₁- bis C₈-Alkyl oder ein aromatischer Rest ist,
R³ H, C₁- bis C₂₀-Alkyl, Carboxylester mit C₁- bis C₈-Alkyl, Alkanoyl mit C₁- bis C₈-Alkyl, ein aromatischer Rest oder -CH₂-CHR⁴-C(O)OR⁵ ist, wobei R⁴ H oder Methyl ist und R⁵ H, C₁-bis C₂₀ Alkyl oder ein aromatischer Rest ist,
wobei R² und R³ nicht gleichzeitig Methyl sind, wenn R¹ Phenyl ist,
und/oder eine oder mehrere Verbindungen der allgemeinen Formel (II) 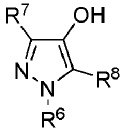 wobei
R⁶ H, C₁- bis C₂₀-Alkyl, Alkanoyl mit C₁- bis C₈-Alkyl oder ein aromatischer Rest ist,
R⁷ H, C₁- bis C₂₀-Alkyl, Carboxylester mit C₁- bis C₈-Alkyl, Alkanoyl mit C₁- bis C₈-Alkyl oder ein aromatischer Rest ist,
R⁸ H, C₁- bis C₂₀-Alkyl, Carboxylester mit C₁- bis C₈-Alkyl, Alkanoyl mit C₁- bis C₈-Alkyl, ein aromatischer Rest oder-CH₂-CHR^{4I}-C(O)OR^{5I} ist, wobei R^{4I} H oder Methyl ist und R^{5I} H, C₁-bis C₂₀-Alkyl oder ein aromatischer Rest ist,

wobei
R⁶ und R⁷ nicht gleichzeitig Phenyl sind, wenn R⁸ Phenyl oder Methyl ist,
R⁶ und R⁸ nicht gleichzeitig Phenyl sind, wenn R⁷ H oder Methyl ist,
R⁶ nicht Phenyl und R⁸ nicht Methyl ist, wenn R⁷ H oder Methyl ist,
und/oder eine oder mehrere Verbindungen der allgemeinen Formel (III) 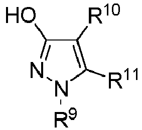 wobei
R⁹ H, C₁- bis C₂₀-Alkyl, Alkanoyl mit C₁- bis C₈-Alkyl oder ein aromatischer Rest ist,
R¹⁰ H, C₁- bis C₂₀-Alkyl, Alkanoyl mit C₁- bis C₈-Alkyl, ein aromatischer Rest oder -C(O)O-R^{5II} ist, wobei R^{5II} H, C₁-bis C₂₀ Alkyl oder ein aromatischer Rest ist,
R¹¹ H, C₁-bis C₂₀-Alkyl, Alkanoyl mit C₁- bis C₈-Alkyl, ein aromatischer Rest oder -C(O)O-R^{5III} ist, wobei R^{5III} H, C₁- bis C₂₀-Alkyl oder ein aromatischer Rest ist,
und/oder eine oder mehrere Verbindungen der allgemeinen Formel (IV) 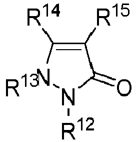 wobei
R¹² H, C₁- bis C₂₀-Alkyl, Alkanoyl mit C₁- bis C₈-Alkyl oder ein aromatischer Rest ist,
R¹³ H, C₁- bis C₂₀-Alkyl, Alkanoyl mit C₁- bis C₈-Alkyl, ein aromatischer Rest oder -C(O)O-R^{5IV} ist, wobei R^{5IV} H, C₁- bis C₂₀-Alkyl oder ein aromatischer Rest ist.
R¹⁴ H, C₁- bis C₂₀-Alkyl, Alkanoyl mit C₁- bis C₈-Alkyl, Caroxylester mit C₁- bis C₈-Alkyl oder ein aromatischer Rest ist,
R¹⁵ H, NH₂, NH(CO)R¹⁶, C₁- bis C₂₀-Alkyl, Alkanoyl mit C₁- bis C₈-Alkyl, Carboxylester mit C₁- bis C₈-Alkyl oder ein aromatischer Rest ist, wobei R¹⁶ H, C₁- bis C₂₀-Alkyl oder ein aromatischer Rest ist

und eine oder mehrere polymerisationsfähige Verbindungen.

## Beschreibung

Chemische Verbindungen, die eine oder mehrere ethylenisch ungesättigte Gruppen aufweisen, haben eine ausgeprägte Neigung zur radikalischen Polymerisation. Nachfolgend werden solche Verbindungen daher auch als polymerisationsfähige Verbindungen bezeichnet. Aufgrund ihrer Neigung zur radikalischen Polymerisation werden diese Verbindungen als Monomere zur Herstellung von Polymeren, z.B. durch gewollte radikalische Polymerisation, verwendet. Die ausgeprägte Neigung zur radikalischen Polymerisation dieser Verbindungen ist jedoch insofern von Nachteil, da es sowohl bei der Lagerung und beim Transport als auch bei der chemischen und/oder physikalischen Bearbeitung, z.B. durch Destillation oder Rektifikation, insbesondere unter der Einwirkung von Energie, z.B. Wärme und/oder Licht, zur unerwünschten, spontanen radikalischen Polymerisation kommen kann. Derartig unkontrollierte Polymerisationen können zur allmählichen Bildung von Polymerisatbelägen, z.B. auf erwärmten Oberflächen führen, was eine Entfernung der Polymerisatbeläge notwendig macht und damit oft zu einer Reduktion der Betriebszeiten führt, oder sogar explosionsartig verlaufen.

Es ist daher üblich, ethylenisch ungesättigten Verbindungen, die eine Neigung zur radikalischen Polymerisation haben, beziehungsweise Gemischen, die solche Verbindungen enthalten, sowohl bei der Lagerung und beim Transport als auch bei der chemischen und/oder physikalischen Bearbeitung Substanzen zuzusetzen, die eine unerwünschte, spontane radikalische Polymerisation unterbinden, beziehungsweise verlangsamen. Derartige Substanzen werden im Allgemeinen und im Folgenden als Polymerisationsinhibitoren oder Polymerisationsstabilisatoren bezeichnet.

Polymerisationsstabilisatoren können als chemische Einzelverbindungen oder als Gemische von Verbindungen eingesetzt werden. Je nach Einsatzgebiet des Polymerisationsstabilisators sind bestimmte Anforderungen an diesen gerichtet. Für die Eignung eines Polymerisationsstabilisators als Transport- und/oder Lagerstabilisator von ethylenisch ungesättigten Verbindungen ist es z. B. wichtig, dass die Effizienz des Polymerisationsstabilisators, d.h. das Ausmaß der Polymerisationsstabilisierung bzw. Polymerisationsinhibierung, reguliert werden kann. Unter den Bedingungen der Lagerung und/oder dem Transport der ethylenisch ungesättigten Verbindungen soll der Polymerisationsstabilisator die unerwünschte, spontane radikalische Polymerisation ausreichend unterbinden, beziehungsweise verlangsamen, wohingegen die gewollte radikalische Polymerisation der ethylenisch ungesättigten Verbindungen unter entsprechenden Polymerisationsbedingungen möglich sein soll, ohne die Notwendigkeit, den bei der Lagerung und/oder dem Transport verwendeten Polymerisationsstabilisator zuvor abtrennen zu müssen. Wird der bei der Lagerung und/oder Transport verwendete Stabilisator bei der gewollten Polymerisation nicht abgetrennt, ist es wichtig, dass dieser während der gewollten Polymerisation diese nicht nachteilig beeinflusst, bspw. ungewollt als Regler wirkt (zu Polymerisationsregler siehe beispielsweise Ullmann's Encyclopedia of Industrial Chemistry, Polymerization Processes, 1. Fundamentals, 6 Free-Radical Polymerization, DOI 10.1002/14356007.21_305.pub3 oder Ullmann's Encyclopedia of Industrial Chemistry, Polyacrylates, 4.1.1 Starting Materials, DOI 10.1002/14356007.a21_157.pub2).

Gemessen am weltweiten Produktionsvolumen ist Acrylsäure sicherlich eine der bedeutsamsten ethylenisch ungesättigten Verbindungen. Standardmäßig wird Acrylsäure während der Lagerung und/oder dem Transport mit 180 bis 220 Gewichts-ppm Hydrochinonmonomethylether (MEHQ), bezogen auf die Menge Acrylsäure, gegen unerwünschte, spontane radikalische Polymerisation stabilisiert. Für eine ausreichende Stabilisierung der Acrylsäure gegen unerwünschte, spontane radikalische Polymerisation mittels MEHQ ist es notwendig, dass Sauerstoff in ausreichenden Mengen in der Acrylsäure eingelöst ist. Ausreichende Mengen von Sauerstoff sind in der Regel dann in der Acrylsäure eingelöst, wenn Acrylsäure unter einer Atmosphäre mit 5 bis 21 vol.% Sauerstoff gelagert und/oder transportiert wird. Die Stabilisierung von Acrylsäure durch MEHQ bei Lagerung und/oder Transport ist beispielsweise in Acrylic Acid, A Summary of Safety and Handling, 4th Edition 2013, 6.1 Instability and Reactivity - Polymerisation, und 7.1. Bulk Storage Facilities and Accesories - General Considerations beschreiben. Bei der gewollten Polymerisation von Acrylsäure, beispielsweise bei der Herstellung von Polyacrylaten, wird der in der Acrylsäure eingelöste Sauerstoffgehalt verringert, wodurch die Effizienz von MEHQ zur Polymerisationsstabilisierung derart reduziert wird, dass Acrylsäure in Gegenwart von MEHQ polymerisiert werden kann.

Neben der Verwendung als Polymerisationsstabilisator bei der Lagerung und/oder Transport von Acrylsäure findet MEHQ auch Verwendung als Polymerisationsstabilisator bei der Lagerung und/oder Transport von Methacrylsäure, Acrylsäureestern, Methacrylsäureestern oder Gemischen, die eine oder mehr der zuvor genannten Verbindungen enthalten. Acrylsäure oder Methacrylsäure werden im Folgenden als (Meth)acrylsäure bezeichnet, deren Ester werden im Folgenden als (Meth)acrylsäureester bezeichnet.

Aufgrund der breiten Verwendung stellt MEHQ einen der wichtigsten Lager- und/oder Transportstabilisatoren für polymerisationsfähige Verbindungen im Allgemeinen und für (Meth)acrylsäure, bzw. (Meth)acrylsäureester im Speziellen dar.

Die Lagerung von polymerisationsfähigen Verbindungen erfolgt in dafür geeigneten, fest installierten Gebinden wie Lagertanks oder Lagerbehälter (siehe beispielsweise Acrylic Acid, A Summary of Safety and Handlung, 4th Edition 2013, 7 Bulk Storage Facilities and Accessories). Es ist bevorzugt, dass sich bei der Lagerung die polymerisationsfähigen Verbindungen für 1 Minute oder mehr, weiter bevorzugt für 10 Minuten oder mehr und besonders bevorzugt für 60 Minuten oder mehr in den jeweiligen Gebinden befinden. Obwohl die Dauer der Lagerung unter entsprechenden Bedingungen theoretisch unbeschränkt ist, wird die Lagerungsdauer in der Regel aus wirtschaftlichen Gründen auf ein Minimum reduziert. Es ist bevorzugt, dass sich bei der Lagerung die polymerisationsfähigen Verbindungen für maximal 180 Tage, weiter bevorzugt für maximal 90 Tage und besonders bevorzugt für maximal 30 Tage in den jeweiligen Gebinden befinden. Besonders bevorzugte Bereiche ergeben sich aus der beliebigen Kombination der zuvor genannten unteren und oberen Grenzen.

Der Transport von polymerisationsfähigen Verbindungen erfolgt üblicherweise in dafür geeigneten, transportablen Gebinden wie Tanks oder Behälter per Schiff, Eisenbahnwagen und/oder Lastwagen (siehe beispielsweise Acrylic Acid, A Summary of Safety and Handlung, 4th Edition 2013, 9 Safe Transport of Acrylic Acid). Natürlich können die transportablen Gebinde auch fest auf dem entsprechenden Transportmittel installiert sein, bspw. Schifftanks oder Bahnkesselwagen. Natürlich ist es auch möglich, dass die Gebinde für eine gewisse Zeit an einer bestimmten Stelle gelagert werden, bevor diese an eine andere Stelle transportiert werden. Der Transport von polymerisationsfähigen Verbindungen kann auch in Rohrleitungen oder Schläuchen erfolgen, beispielsweise nach Aufreinigung der polymerisationsfähigen Verbindungen zum Lagertank, bei der Verladung vom Lagertank in ein transportables Gebinde und/oder bei der Verladung von einem transportablen Gebinde in ein anderes. Es ist bevorzugt, dass sich beim Transport die polymerisationsfähigen Verbindungen für eine Dauer von 10 Sekunden oder mehr, weiter bevorzugt für 1 Minute oder mehr, besonders bevorzugt für 10 Minuten oder mehr und insbesonders bevorzugt für 60 Minuten oder mehr in den jeweiligen Gebinden befinden. Obwohl die Dauer unter entsprechenden Bedingungen theoretisch unbeschränkt ist, wird die Dauer in der Regel aus wirtschaftlichen und sicherheitstechnischen Gründen auf ein Minimum reduziert. Es ist bevorzugt, dass sich beim Transport die polymerisationsfähigen Verbindungen für maximal 180 Tage, weiter bevorzugt für maximal 90 Tage und besonders bevorzugt für maximal 30 Tage in den jeweiligen Gebinden befinden. Besonders bevorzugte Bereiche ergeben sich aus der beliebigen Kombination der zuvor genannten unteren und oberen Grenzen.

Um die Abhängigkeit von MEHQ zu reduzieren, war es die Aufgabe der vorliegenden Erfindung, Mischungen zur Verfügung zu stellen, die eine oder mehrere polymerisationsfähige Verbindungen, insbesondere (Meth)acrylsäure und/oder (Meth)acrylsäureester und einen oder mehrere Lager- und/oder Transportstabilisatoren enthalten. Die Lager - und/oder Transportstabilisatoren sollen eine ausreichende Stabilisierung der polymerisationsfähigen Verbindungen gegen ungewollte radikalische Polymerisation gewährleisten. Bei der gewollten radikalischen Polymerisation soll jedoch keine Notwendigkeit bestehen, die Lager- und/oder Transportstabilisatoren vor der Polymerisation aus der Mischung abzutrennen. Die Lager- und oder Transportstabilisatoren sollen daher bei der gewollten radikalischen Polymerisation nicht als Regler und/oder Inhibitoren wirken.

Im Rahmen der vorliegenden Erfindung ist eine Verbindung dann als Lager- und/oder Transportstabilisator von polymerisationsfähigen Verbindungen geeignet und wirkt nicht als Regler und/oder Inhibitor bei der gewollten radikalischen Polymerisation, wenn diese Verbindung unter vergleichbaren Bedingungen
a) die spontane radikalische Polymerisation von flüssiger Acrylsäure, die unter einer Atmosphäre mit 5 bis 30 vol. % Sauerstoff gelagert wird mit einer Effizienz von 75 oder mehr Prozent bezogen auf die Effizienz einer äquimolaren Menge MEHQ inhibiert und
b) die radikalische Polymerisation von flüssiger Acrylsäure, die unter einer Atmosphäre mit 96 bis 100 vol.% Stickstoff gelagert wird und mit 1000 bis 15000 Gew. ppm Polymerisationsstarter versetzt ist, mit einer Effizienz von 130 oder weniger Prozent, insbesondere mit einer Effizienz von 100 oder weniger Prozent, bezogen auf die Effizienz einer äquimolaren Menge MEHQ inhibiert und nach der Polymerisation vergleichbare bzw. ähnliche Polymere erhalten werden. Bevorzugt beträgt der Stickstoffgehalt der Atmosphäre 99 bis 100 vol. %.

MEHQ wird in a) und b) jeweils in Mengen von 10 bis 250 Gew. ppm eingesetzt, bevorzugt in einer Menge von 35 bis 45 Gew. ppm.
Angaben in Gew. ppm beziehen sich auf die Menge an eingesetzter flüssiger Acrylsäure.

Vergleichbare bzw. ähnliche Polymere werden dann erhalten, wenn die Polymere im Wesentlichen identische mittlere Gewichtsmittel (gemessen nach der gleichen GPC-Methode) aufweisen. Im wesentlich identische Gewichtsmittel liegen dann vor, wenn die Gewichtsmittel um 20 Prozent oder weniger auseinander liegen.

Als Polymerisationsstarter eignen sich die dem Fachmann bekannten Polymerisationsstarter, die üblicherweise zur Polymerisation von (Meth)acrylsäure eingesetzt werden (siehe beispielsweise The Chemistry of Radical Polymerisation, 2nd Edition, 2005, Seite 49 bis 166).

Die Aufgabe wird gelöst durch eine Mischung enthaltend eine oder mehrere Verbindungen der allgemeinen Formel (I) 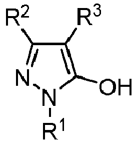 wobei
R¹ H, C₁- bis C₂₀-Alkyl, Alkanoyl mit C₁- bis C₈-Alkyl oder ein aromatischer Rest ist,
R² H, C₁- bis C₂₀-Alkyl, Carboxylester mit C₁- bis C₈-Alkyl, Alkanoyl mit C₁- bis C₈-Alkyl oder ein aromatischer Rest ist,
R³ H, C₁- bis C₂₀-Alkyl, Carboxylester mit C₁- bis C₈-Alkyl, Alkanoyl mit C₁- bis C₈-Alkyl, ein aromatischer Rest oder-CH₂-CHR⁴-C(O)OR⁵ ist, wobei R⁴ H oder Methyl ist und R⁵ H, C₁-bis C₂₀ Alkyl oder ein aromatischer Rest ist,
wobei R² und R³ nicht gleichzeitig Methyl sind, wenn R¹ Phenyl ist,
und/oder eine oder mehrere Verbindungen der allgemeinen Formel (II) 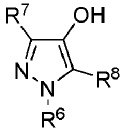
wobei
R⁶ H, C₁- bis C₂₀-Alkyl, Alkanoyl mit C₁- bis C₈-Alkyl oder ein aromatischer Rest ist,
R⁷ H, C₁- bis C₂₀-Alkyl, Carboxylester mit C₁- bis C₈-Alkyl, Alkanoyl mit C₁- bis C₈-Alkyl oder ein aromatischer Rest ist,
R⁸ H, C₁- bis C₂₀-Alkyl, Carboxylester mit C₁- bis C₈-Alkyl, Alkanoyl mit C₁- bis C₈-Alkyl, ein aromatischer Rest oder -CH₂-CHR^{4I}-C(O)OR^{5I} ist, wobei R^{4I} H oder Methyl ist und R^{5I} H, C₁-bis C₂₀-Alkyl oder ein aromatischer Rest ist,
wobei
R⁶ und R⁷ nicht gleichzeitig Phenyl sind, wenn R⁸ Phenyl oder Methyl ist,
R⁶ und R⁸ nicht gleichzeitig Phenyl sind, wenn R⁷ H oder Methyl ist,
R⁶ nicht Phenyl und R⁸ nicht Methyl ist, wenn R⁷ H oder Methyl ist,
und/oder eine oder mehrere Verbindungen der allgemeinen Formel (III) 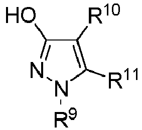
wobei
R⁹ H, C₁- bis C₂₀-Alkyl, Alkanoyl mit C₁- bis C₈-Alkyl, oder ein aromatischer Rest ist,
R¹⁰ H, C₁- bis C₂₀-Alkyl, Alkanoyl mit C₁- bis C₈-Alkyl, ein aromatischer Rest oder -C(O)O-R^{5II} ist, wobei R^{5II} H, C₁-bis C₂₀ Alkyl oder ein aromatischer Rest ist,
R¹¹ H, C₁-bis C₂₀-Alkyl, Alkanoyl mit C₁- bis C₈-Alkyl, ein aromatischer Rest oder -C(O)O-R^{5III} ist, wobei R^{5III} H, C₁- bis C₂₀-Alkyl oder ein aromatischer Rest ist,
und/oder eine oder mehrere Verbindungen der allgemeinen Formel (IV) 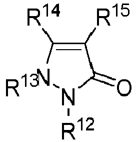
wobei
R¹² H, C₁- bis C₂₀-Alkyl, Alkanoyl mit C₁- bis C₈-Alkyl, oder ein aromatischer Rest ist,
R¹³ H, C₁- bis C₂₀-Alkyl, Alkanoyl mit C₁- bis C₈-Alkyl, ein aromatischer Rest oder -C(O)O-R^{5IV} ist, wobei R^{5IV} H, C₁- bis C₂₀-Alkyl oder ein aromatischer Rest ist.
R¹⁴ H, C₁- bis C₂₀-Alkyl, Carboxylester mit C₁- bis C₈-Alkyl, Alkanoyl mit C₁- bis C₈-Alkyl oder ein aromatischer Rest ist,
R¹⁵ H, C₁- bis C₂₀-Alkyl, NH₂, NH(CO)R¹⁶, Carboxylester mit C₁- bis C₈-Alkyl, Alkanoyl mit C₁-bis C₈-Alkyl, oder ein aromatischer Rest ist, wobei R¹⁶ H, C₁- bis C₂₀-Alkyl oder ein aromatischer Rest ist
und eine oder mehrere polymerisationsfähige Verbindungen.

Ein weiterer Gegenstand der vorliegenden Erfindung ist die Verwendung einer oder mehrerer Verbindungen der allgemeinen Formel (I), (II), (III), (IV) oder einer beliebigen Mischung dieser Verbindungen als Lager- und/oder Transportstabilisator von einer oder mehrerer polymerisationsfähigen Verbindungen.

Weiterhin ist ein Verfahren zur Stabilisierung von einer oder mehrerer polymerisationsfähigen Verbindungen bei der Lagerung und/oder Transport unter Verwendung einer oder mehrerer Verbindungen der allgemeinen Formel (I), (II), (III), (IV) oder einer beliebigen Mischung dieser Verbindungen Gegenstand der vorliegenden Erfindung.

Ebenfalls Gegenstand der Erfindung ist ein Verfahren zur Polymerisation einer oder mehrerer polymerisationsfähigen Verbindungen in Gegenwart einer oder mehrerer Verbindungen der allgemeinen Formel (I), (II), (III), (IV) oder einer beliebigen Mischung dieser.

Die erfindungsgemäße Mischung ist unter Standardbedingungen flüssig.

Der Test, ob sich eine Verbindung als Lager- und/oder Transportstabilisator eignet, wird im Folgenden beispielhaft dargestellt.

Die flüssige Acrylsäure, die für den Test eingesetzt wurde, wurde frisch destilliert.

### Zusammensetzung Acrylsäure:

>99,5 Gew. % Acrylsäure, 0,0726 Gew. % Essigsäure, 0,0497Gew. % Diacrylsäure, 0,0184Gew. % Propionsäure. Gewichtsprozentangaben beziehen sich auf die Gesamtmenge der eingesetzten Acrylsäure incl. der darin enthaltenen Nebenkomponenten.

Als Starterlösung wurde eine frisch angesetzte Toluol-Lösung von 2,2'-Azobis(4-methoxy-2,4-dimehtylvalerontril) verwendet. Der Gehalt an 2,2'-Azobis(4-methoxy-2,4-dimehtylvalerontril) in der Starterlösung beträgt 6420 Gew. ppm bezogen auf die Masse an Toluol in der Starterlösung.

### Teil 1: Inhibierung unter Luft oder Stickstoff

Unter Luft wurden 2.2 g Acrylsäurelösung enthaltend 0,000715 mmol des jeweiligen Polymerisationsstabilisators unter Raumtemperatur vorgelegt. Die vorgelegte Acrylsäurelösung wurde mit 0,22 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß.

Das Reaktionsgefäß enthaltend die Mischung wurde anschließend in einem auf 40°C erwärmten Heizblock platziert.

Nachdem die Mischung die Temperatur von 40°C erreicht hat wurde der Temperaturverlauf über die Zeit gemessen. Der Zeitraum von Erreichen der 40°C bis zum Erreichen der Maximaltemperatur der Mischung wird als Bezugswert für die Inhibierungsleistung des jeweiligen Polymerisationsstabilisators herangezogen.

Für eine Mischung mit MEHQ als Polymerisationsstabilisator wurde ein 42 Sekunden dauernder Temperaturanstieg um 145°C auf eine maximale Temperatur von 185°C gemessen. Die maximale Temperatur lag nach 9188 Sekunden vor, nachdem die Mischung auf 40°C temperiert war. Zur Berechnung der Effizienz der Polymerisationsstabilisatoren in Bezug auf MEHQ entspricht der Wert von 9188 Sekunden einer Effizienz von 100 Prozent.

Für eine erfindungsgemäße Mischung mit 5-Methyl-2-phenyl-4H-pyrazol-3-on als Polymerisationsstabilisator wurde ein 48 Sekunden dauernder Temperaturanstieg um 125°C auf eine maximale Temperatur von 165°C beobachtet. Die maximale Temperatur lag nach 7874 Sekunden vor, nachdem die Mischung auf 40°C temperiert war.

Im Vergleich zu MEHQ weist 5-Methyl-2-phenyl-4H-pyrazol-3-on als Polymerisationsstabilisator somit eine Effizienz von 86 Prozent auf.

### Tests zur Polymerisationsinhibierung unter Stickstoff

Unter Luft wurden 2.2 g Acrylsäurelösung enthaltend 0,000715 mmol des jeweiligen Polymerisationsstabilisators vorgelegt. Die vorgelegte Acrylsäurelösung wurde mit 0,22 g Starterlösung versetzt und homogenisiert. 1,9 ml der so erhaltenen Mischung wurden in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß. In das Reaktionsgefäß wurde oberhalb der vorgelegten Mischung Stickstoff unter Druckausgleich eingeleitet. Der Stickstoffstrom beträgt 3 bis 4 l/h. Die Einleitdauer beträgt 20 Sekunden. Der Stickstoff weist eine Reinheit von größer 99,9 % auf.

Das Reaktionsgefäß enthaltend die Mischung wurde in einem Heizblock platziert. Die Mischung wurde anschließend im Heizblock innerhalb von 4 Minuten auf 40°C erwärmt. Nachdem die Mischung die Temperatur von 40°C erreicht hat, wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsstabilisators herangezogen.

Für eine Mischung mit MEHQ als Polymerisationsstabilisator wurde ein 54 Sekunden dauernder Temperaturanstieg um 133°C auf eine maximale Temperatur von 173°C gemessen. Die maximale Temperatur lag nach 3978 Sekunden vor, nachdem die Mischung auf 40°C temperiert war. Zur Berechnung der Effizienz der Polymerisationsstabilisatoren in Bezug auf MEHQ entspricht der Wert von 3978 Sekunden einer Effizienz von 100 Prozent.

Für eine erfindungsgemäße Mischung mit 5-Methyl-2-phenyl-4H-pyrazol-3-on als Polymerisationsstabilisator wurde ein 66 Sekunden dauernder Temperaturanstieg um 119°C auf eine maximale Temperatur von 159°C beobachtet. Die maximale Temperatur lag nach 1056 Sekunden vor, nachdem die Mischung auf 40°C temperiert war. Im Vergleich zu MEHQ weist 5-Methyl-2-phenyl-4H-pyrazol-3-on als Polymerisationsstabilisator somit eine Effizienz von 27 Prozent auf.

### Teil 2: Synthese und Analyse von Polymeren

Unter einer Stickstoffatmosphäre wurden in einem Reaktionsgefäß 450 g deionisiertes Wasser vorgelegt. Die vorgelegte Reaktionsmischung wurde unter Rühren auf 95°C erhitzt. Nach Erreichen der Temperatur von 95°C erfolgte die Zuführung von drei Strömen unter Rühren und unter Beibehaltung der Temperatur.
Strom 1: Zuführung über 5 h, 500 g Acrylsäure
Strom 2: Zuführung über 4,75 h, 15 g Natriumhypophosphit in 35 g deionisiertem Wasser
Strom 3: Zuführung über 5,25 h, 5 g Natriumpersulfat in 66,4 g deionisiertem Wasser

Nach Zugabe der drei Ströme wurde die Reaktionsmischung für eine weitere Stunde bei 95°C gerührt. Die Reaktionsmischung wurde anschließend auf Raumtemperatur abgekühlt und mit 80 g deionisiertem Wasser versetzt.

Erfindungsgemäß war die Acrylsäure von Strom 1 mit 280 Gew. ppm Edaravon bezogen auf die Gesamtmasse der eingesetzten Acrylsäure incl. der darin enthaltenen Nebenkomponenten stabilisiert. Im Vergleichsversuch war die Acrylsäure von Strom 1 mit 200 Gew. ppm MEHQ bezogen auf die Gesamtmasse der eingesetzten Acrylsäure incl. der darin enthaltenen Nebenkomponenten stabilisiert.

Die erhaltenen Polymere wurden mittels GPC analysiert (Kalibrierung mit Na-PAA-Standard, Elutionsmittel 0,01 mol/l Phosphatpuffer, pH=7,4 in dest. Wasser, mit 0,01 M NaN₃).

| | Mw (g/mol) |
|---|---|
| Polymer (Edaravon / 5-Methyl-2-phenyl-4H-pyrazol-3-on) | 9560 |
| Polymer (MEHQ) | 9400 |

### Beschreibung:

C₁- bis C₂₀-Alkyl umfasst gerade oder verzweigte C₁- bis C₂₀-Alkylgruppen oder cyclische C₃-bis C₂₀-Alkylgruppen. Bei C₁- bis C₂₀-Alkyl handelt es sich beispielsweise um Methyl, Ethyl, n-Propyl, iso-Propyl, n-Butyl, sec-Butyl, iso-Butyl, tert.-Butyl, n-Pentyl, 2-Methylbutyl, 3-Methylbutyl, Cyclopentyl, n-Hexyl, Cyclohexyl, 2-Methylpentyl, 3-Methylpentyl, 4-Methylpentyl, 3,3-Dimethylbutyl, 2-Ethylbutyl, n-Heptyl, 2-Methylhexyl, 2-Ethylpentyl, 4-MethylCyclohexyl, n-Octyl, iso-Octyl, 2-Ethylhexyl, n-Nonyl, iso-Nonyl, 2-Propylhexyl, n-Decyl, iso-Decyl, 2-Propylheptyl, n-Undecyl, Iso-Undecyl, n-Dodecyl, Iso-Dodecyl, n-Tridecyl oder Iso-Tridecyl.

C₁- bis C₈-Alkyl umfasst gerade oder verzweigte C₁- bis C₈-Alkylgruppen oder cyclische C₃-bis C₈-Alkylgruppen. Bei C₁- bis C₈-Alkyl handelt es sich beispielsweise um Methyl, Ethyl, n-Propyl, iso-Propyl, n-Butyl, sec-Butyl, iso-Butyl, tert.-Butyl, n-Pentyl, 2-Methylbutyl, 3-Methylbutyl, Cyclopentyl, n-Hexyl, Cyclohexyl, 2-Methylpentyl, 3-Methylpentyl, 4-Methylpentyl, 3,3-Dimethylbutyl, 2-Ethylbutyl, n-Heptyl, 2-Methylhexyl, 2-Ethylpentyl, 4-MethylCyclohexyl, n-Octyl, iso-Octyl oder 2-Ethylhexyl.

C₁- bis C₆-Alkyl umfasst gerade oder verzweigte C₁- bis C₆-Alkylgruppen oder cyclische C₃-bis C₆-Alkylgruppen. Bei C₁- bis C₆-Alkyl handelt es sich beispielsweise um Methyl, Ethyl, n-Propyl, iso-Propyl, n-Butyl, sec-Butyl, iso-Butyl, tert.-Butyl, n-Pentyl, 2-Methylbutyl, 3-Methylbutyl, Cyclopentyl, n-Hexyl, Cyclohexyl, 2-Methylpentyl, 3-Methylpentyl, 4-Methylpentyl, 3,3-Dimethylbutyl oder 2-Ethylbutyl.

Bei einem aromatischen Rest handelt es sich um einen anellierten aromatischen Rest, um einen aromatischen Substituenten, einen anellierten heteroaromatischen Rest oder um einen heteroaromatischen Substituenten. Der aromatische oder heteroaromatische Substituent ist direkt über eine Einfachbindung oder über eine CH₂-Gruppe oder über ein verbrückendes Heteroatom wie Sauerstoff, Stickstoff oder Schwefel mit dem 5 Ring der Verbindungen der allgemeinen Formel (I), (II), (III) oder (IV) verbunden.

Ein anellierter aromatischer Rest weist einen oder mehrere annellierte aromatische Kerne auf. Es ist bevorzugt, dass der annellierte aromatische Rest einen aromatischen Kern aufweist. Der anellierte aromatische Rest kann einen oder mehrere von H unterschiedliche Substituenten aufweisen. Die Substituenten sind ausgewählt aus der Gruppe bestehend aus C₁-bis C₂₀-Alkyl, Alkoxysubstituenten mit C₁- bis C₂₀-Alkyl, bevorzugt C₁- bis C₈-Alkyl, die über das Sauerstoffatom mit dem anellierten aromatischen Rest verbunden sind, Aminosubstituenten der Formel NR¹⁷R¹⁸, wobei R¹⁷ und R¹⁸ unabhängig voneinander H oder C₁- bis C₂₀-Alkyl sind oder einen Zyklus mit 4 bis 5 Kohlenstoffatomen im Zyklus bilden und die über das Stickstoffatom mit dem anellierten aromatischen Rest verbunden sind und die Kohlenstoffatome im Zyklus mit H oder CH₃ substituiert sind, Sulfonamidsubstituenten der Formel-S(O)₂NR¹⁹,R²⁰, wobei R¹⁹ und R²⁰ unabhängig voneinander H oder C₁- bis C₂₀-Alkyl sind oder einen Zyklus mit 4 bis 5 Kohlenstoffatomen im Zyklus bilden und über das Schwefelatom mit dem aromatischen Substituenten verbunden sind und die Kohlenstoffatome im Zyklus mit H oder CH₃ substituiert sind, Nitril, Carboxyl, Carboxylester mit C₁-bis C₂₀-Alkyl, -CH₂-COOR²¹, wobei R²¹ H oder C₁- bis C₂₀-Alkyl ist, Pyridyl, Chlor und Brom.

Ein aromatischer Substituent weist einen oder mehrere aromatische Kerne auf. Weist der aromatische Substituent mehrere aromatische Kerne auf, können die Kerne anelliert, verbrückt, verknüpft oder verbrückt und verknüpft vorliegen. So können die aromatischen Kerne beispielsweise über eine oder mehrere CH₂-Gruppen oder über ein Heteroatome, wie Sauerstoffatom miteinander verbrückt sein. Verknüpfte aromatische Kerne sind über eine Einfachbindung direkt miteinander verbunden. Verbrückte und verknüpfte aromatische Kerne sind beispielsweise über eine CH₂-Gruppe oder ein Heteroatom, wie Sauerstoffatom miteinander verbrückt und weisen gleichzeitig eine direkte Verknüpfung über eine Einfachbindung auf. Es ist bevorzugt, dass der aromatische Substituent ein oder zwei aromatische Kerne aufweist. Ein aromatischer Substituent kann einen oder mehrere von H unterschiedliche Substituenten aufweisen. Die Substituenten sind ausgewählt aus der Gruppe bestehend aus C₁-bis C₂₀-Alkyl, Alkoxysubstituenten mit C₁- bis C₂₀-Alkyl, bevorzugt C₁- bis C₈-Alkyl, die über das Sauerstoffatom mit dem aromatischen Substituenten verbunden sind, Aminosubstituenten der Formel NR²²R²³, wobei R²² und R²³ unabhängig voneinander H oder C₁- bis C₂₀-Alkyl sind oder einen Zyklus mit 4 bis 5 Kohlenstoffatomen im Zyklus bilden und die über das Stickstoffatom mit dem aromatischen Substituenten verbunden sind und die Kohlenstoffatome im Zyklus mit H oder CH₃ substituiert sind, Sulfonamidsubstituenten der Formel -S(O)₂NR²⁴R²⁵, wobei R²⁴ und R²⁵ unabhängig voneinander H oder C₁- bis C₂₀-Alkyl sind oder einen Zyklus mit 4 bis 5 Kohlenstoffatomen im Zyklus bilden und über das Schwefelatom mit dem aromatischen Substituenten verbunden sind und die Kohlenstoffatome im Zyklus mit H oder CH₃ substituiert sind, Nitril, Carboxyl, Carboxylester mit C₁-bis C₂₀-Alkyl, -CH₂-COOR²⁶, wobei R²⁶ H oder C₁- bis C₂₀-Alkyl ist, Pyridyl, Chlor und Brom.

Ein anellierter heteroaromatischer Rest weist einen oder mehrere anellierte heteroaromatische Kerne auf. Es ist bevorzugt, dass der anellierte heteroaromatische Rest einen heteroaromatischen Kern aufweist. Der heteroaromatische Kern kann ein oder mehrere Stickstoffatome, ein oder mehrere Sauerstoffatome und/oder ein oder mehrere Schwefelatome enthalten. Der anellierte heteroaromatische Rest kann einen oder mehrere von H unterschiedliche Substituenten aufweisen. Die Substituenten sind ausgewählt aus der Gruppe bestehend aus C₁-bis C₂₀-Alkyl, Alkoxysubstituenten mit C₁- bis C₂₀-Alkyl, bevorzugt C₁- bis C₈-Alkyl, die über das Sauerstoffatom mit dem anellierten heteroaromatischen Rest verbunden sind, Aminosubstituenten der Formel NR²⁷R²⁸, wobei R²⁷ und R²⁸ unabhängig voneinander H oder C₁-bis C₂₀-Alkyl sind oder einen Zyklus mit 4 bis 5 Kohlenstoffatomen im Zyklus bilden und die über das Stickstoffatom mit dem anellierten heteroaromatischen Rest verbunden sind und die Kohlenstoffatome im Zyklus mit H oder CH₃ substituiert sind, Sulfonamidsubstituenten der Formel -S(O)₂NR²⁹R³⁰, wobei R²⁹ und R³⁰ unabhängig voneinander H oder C₁- bis C₂₀-Alkyl sind oder einen Zyklus mit 4 bis 5 Kohlenstoffatomen im Zyklus bilden und über das Schwefelatom mit dem aromatischen Substituenten verbunden sind und die Kohlenstoffatome im Zyklus mit H oder CH₃ substituiert sind, Nitril, Carboxyl, Carboxylester mit C₁-bis C₂₀-Alkyl, -CH₂-COOR³¹, wobei R³¹ H oder C₁- bis C₂₀-Alkyl ist, Pyridyl, Chlor und Brom.

Ein heteroaromatischer Substituent weist einen oder mehrere heteroaromatische Kerne auf. Weist der heteroaromatische Substituent mehrere heteroaromatische Kerne auf können die Kerne anelliert, verbrückt, verknüpft oder verbrückt und verknüpft vorliegen. So können die heteroaromatischen Kerne beispielsweise über eine oder mehrere CH₂-Gruppen oder über ein Heteroatom, wie Sauerstoffatom miteinander verbrückt sein. Verknüpfte heteroaromatische Kerne sind über eine Einfachbindung direkt miteinander verbunden. Verbrückte und verknüpfte heteroaromatische Kerne sind beispielsweise über eine CH₂-Gruppe oder ein Heteroatom, wie Sauerstoffatom miteinander verbrückt und weisen gleichzeitig eine direkte Verknüpfung über eine Einfachbindung auf. Es ist bevorzugt, dass der heteroaromatische Substitut ein oder zwei heteroaromatische Kerne aufweist. Ein heteroaromatischer Substituent kann einen oder mehrere von H unterschiedliche Substituenten aufweisen. Die Substituenten sind ausgewählt aus der Gruppe bestehend aus C₁-bis C₂₀-Alkyl, Alkoxysubstituenten mit C₁- bis C₂₀-Alkyl, bevorzugt mit C₁-bis C₈-Alkyl die über das Sauerstoffatom mit dem heteroaromatischen Substituenten verbunden sind, Aminosubstituenten der Formel NR³²R³³, wobei R³² und R³³ unabhängig voneinander H oder C₁- bis C₂₀-Alkyl sind oder einen Zyklus mit 4 bis 5 Kohlenstoffatomen im Zyklus bilden und die über das Stickstoffatom mit dem heteroaromatischen Substituenten verbunden sind und die Kohlenstoffatome im Zyklus mit H oder CH₃ substituiert sind, Sulfonamidsubstituenten der Formel -S(O)₂NR³⁴R³⁵, wobei R³⁴ und R³⁵ unabhängig voneinander H oder C₁- bis C₂₀-Alkyl sind oder einen Zyklus mit 4 bis 5 Kohlenstoffatomen im Zyklus bilden und über das Schwefelatom mit dem aromatischen Substituenten verbunden sind und die Kohlenstoffatome im Zyklus mit H oder CH₃ substituiert sind, Nitril, Carboxyl, Carboxylester mit C₁-bis C₂₀-Alkyl, -CH₂-COOR³⁶, wobei R³⁶H oder C₁-bis C₂₀-Alkyl ist, Pyridyl, Chlor und Brom.

Die Herstellung von Verbindungen der allgemeinen Formel (I), (II), (III) oder (IV) ist dem Fachmann bekannt oder erschließt sich ihm aus seinem allgemeinen Fachwissen. Verbindungen der allgemeinen Formel (I) liegen im Gleichgewicht mit ihrer Keto-Form vor.

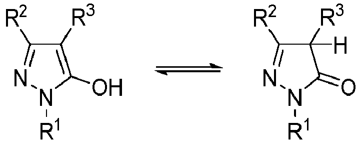

In ihrer Enol-Form können Verbindungen der allgemeinen Formel (I) Additionsreaktionen mit den polymerisationsfähigen Verbindungen eingehen. Dementsprechend können Additionsprodukte von Verbindungen der allgemeinen Formel (I) und polymerisationsfähigen Verbindungen in der erfindungsgemäßen Mischung vorliegen. Für den Fall, dass R³ gleich H ist, kann es neben der Einfachaddition auch zur Doppeladdition kommen. Handelt es sich bei der polymerisationsfähigen Verbindung beispielsweise um (Meth)acrylsäure oder um (Meth)acrylsäureester ist es denkbar, dass (Meth)acrylsäure oder (Meth)acrylsäureester an Verbindungen der allgemeinen Formel (I) addiert werden und die entsprechenden Addukte in der erfindungsgemäßen Mischung vorliegen.

Die Addition von (Meth)acrylsäure an 5-Methyl-2-phenyl-4H-pyrazol-3-on ist beispielhaft für die Addition von (Meth)acrylsäure an Verbindungen der allgemeinen Formel (I) dargestellt.

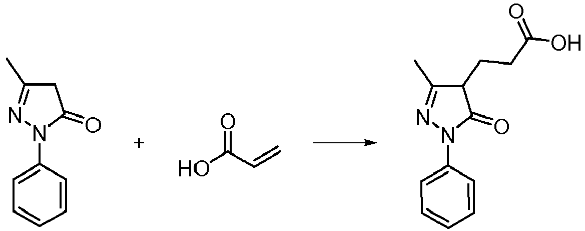

Gemäß der Addition von (Meth)acrylsäure an 5-Methyl-2-phenyl-4H-pyrazol-3-on kann auch die Addition von (Meth)acrylsäureester an Verbindungen der allgemeinen Formel (I) erfolgen. Neben Einfachaddition ist auch Doppeladdition möglich.

In bevorzugten Verbindungen der allgemeinen Formel (I) sind R¹ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Alkanoyl mit C₁- bis C₆-Alkyl oder 4-iso-Propoxyphenyl.

R² H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl oder 4-iso-Propoxyphenyl.

R³ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl, oder 4-iso-Propoxyphenyl.
wobei R² und R³ nicht gleichzeitig Methyl sind, wenn R¹ Phenyl ist.

Besonders bevorzugte Verbindungen der allgemeinen Formel (I) sind
2-Methyl-pyrazol-3-ol, 2-Ethyl-pyrazol-3-ol, 2-Propyl-pyrazol-3-ol, 2-lsopropyl-pyrazol-3-ol, 2-Butyl-pyrazol-3-ol, 2-Isobutyl- 2H-pyrazol-3-ol ,2-tert-Butyl-pyrazol-3-ol, 2-Phenyl-pyrazol-3-ol, 2-(4-tolyl)-pyrazol-3-ol, 2-(2-tolyl)-pyrazol-3-ol, 2-(3-tolyl)-pyrazol-3-ol, 2-(3,4-Dimethylphenyl)-pyrazol-3-ol, 2-Methyl-4-phenyl-pyrazol-3-ol, 2-Ethyl-4-phenyl-pyrazol-3-ol, 2-Propyl-4-phenyl-pyrazol-3-ol, 2-lsopropyl-4-phenyl-pyrazol-3-ol, 2-Butyl-4-phenyl-pyrazol-3-ol, 2-Isobutyl-4-phenyl-pyrazol-3-ol, 2-tert-Butyl-4-phenyl-pyrazol-3-ol, 2-Phenyl-4-phenyl-pyrazol-3-ol, 2-(4-tolyl)-4-phenyl-pyrazol-3-ol, 2-(2-tolyl)-4-phenyl-pyrazol-3-ol, 2-(3-tolyl)-4-phenyl-pyrazol-3-ol, 2-(3,4-Dimethylphenyl)-4-phenyl-pyrazol-3-ol, 2-Methyl-4-methyl-pyrazol-3-ol, 2-Ethyl-4-methyl-pyrazol-3-ol, 2-Propyl-4-methyl-pyrazol-3-ol, 2-Isopropyl-4-methyl -pyrazol-3-ol, 2-Butyl-4-methyl-pyrazol-3-ol, 2-Isobutyl-4-methyl-pyrazol-3-ol, 2-tert-Butyl-4-methyl-pyrazol-3-ol, 2-Phenyl-4-methyl-pyrazol-3-ol, 2-(4-tolyl)-4-methyl-pyrazol-3-ol, 2-(2-tolyl)-4-methyl -pyrazol-3-ol, 2-(3-tolyl)-4-methyl-pyrazol-3-ol, 2-(3,4-Dimethylphenyl)-4-methyl-pyrazol-3-ol, 2-methyl-4-ethyl-pyrazol-3-ol, 2-ethyl-4-ethyl-pyrazol-3-ol, 2-propyl-4-ethyl-pyrazol-3-ol, 2-isopropyl-4-ethyl-pyrazol-3-ol, 2-butyl-4-ethyl-pyrazol-3-ol, 2-Isobutyl-4-ethyl -pyrazol-3-ol, 2-tert-butyl-4-ethyl-pyrazol-3-ol, 2-Phenyl-4-ethyl-pyrazol-3-ol, 2-(4-tolyl)-4-ethyl-pyrazol-3-ol, 2-(2-tolyl)-4-ethyl-pyrazol-3-ol, 2-(3-tolyl)-4-ethyl-pyrazol-3-ol, 2-(3,4-Dimethylphenyl)-4-ethyl-pyrazol-3-ol, 2-Methyl-4-propyl-pyrazol-3-ol, 2-Ethyl-4-propyl-pyrazol-3-ol, 2-Propyl-4-propyl-pyrazol-3-ol, 2-Isopropyl-4-methyl-pyrazol-3-ol, 2-Butyl-4-propyl-pyrazol-3-ol, 2-Isobutyl-4-propyl-pyrazol-3-ol, 2-tert-Butyl-4-propyl-pyrazol-3-ol, 2-Phenyl-4-propyl-pyrazol-3-ol, 2-(4-tolyl)-4-propyl-pyrazol-3-ol, 2-(2-tolyl)-4-propyl-pyrazol-3-ol, 2-(3-tolyl)-4-propyl-pyrazol-3-ol, 2-(3,4-Dimethylphenyl)-4-propyl-pyrazol-3-ol, 2-Methyl-4-isopropyl-pyrazol-3-ol, 2-Ethyl-4-isopropyl-pyrazol-3-ol, 2-Propyl-4-isopropyl-pyrazol-3-ol, 2-Isopropyl-4-isopropyl-pyrazol-3-ol, 2-Butyl-4-isopropyl-pyrazol-3-ol, 2-Isobutyl-4-isopropyl-pyrazol-3-ol, 2-tert-Butyl-4-isopropyl-pyrazol-3-ol, 2-Phenyl-4-isopropyl-pyrazol-3-ol, 2-(4-tolyl)-4-isopropyl-pyrazol-3-ol, 2-(2-tolyl)-4-isopropyl-pyrazol-3-ol, 2-(3-tolyl)-4-isopropyl-pyrazol-3-ol, 2-(3,4-Dimethylphenyl)-4-isopropyl-pyrazol-3-ol, 2-Methyl-4-butyl-pyrazol-3-ol, 2-Ethyl-4-butyl-pyrazol-3-ol, 2-Propyl-4-butyl-pyrazol-3-ol, 2-Isopropyl-4-butyl-pyrazol-3-ol, 2-Butyl-4-butyl-pyrazol-3-ol, 2-Isobutyl-4-butyl-pyrazol-3-ol, 2-tert-Butyl-4-butyl-pyrazol-3-ol, 2-Phenyl-4-butyl-pyrazol-3-ol, 2-(4-tolyl)-4-butyl-pyrazol-3-ol, 2-(2-tolyl)-4-butyl-pyrazol-3-ol, 2-(3-tolyl)-4-butyl-pyrazol-3-ol, 2-(3,4-Dimethylphenyl)-4-butyl-pyrazol-3-ol, 2-Methyl-4-isobutyl-pyrazol-3-ol, 2-Ethyl-4-isobutyl-pyrazol-3-ol, 2-Propyl-4-isobutyl-pyrazol-3-ol, 2-Isopropyl-4-isobutyl-pyrazol-3-ol, 2-Butyl-4-isobutyl-pyrazol-3-ol, 2-Isobutyl-4-isobutyl-pyrazol-3-ol, 2-tert-Butyl-4-isobutyl-pyrazol-3-ol, 2-Phenyl-4-isobutyl-pyrazol-3-ol, 2-(4-tolyl)-4-isobutyl-pyrazol-3-ol, 2-(2-tolyl)-4-isobutyl-pyrazol-3-ol, 2-(3-tolyl)-4-isobutyl-pyrazol-3-ol, 2-(3,4-Dimethylphenyl)-4-isobutyl-pyrazol-3-ol, 2-Methyl-4-tert-butyl-pyrazol-3-ol, 2-Ethyl-4-tert-butyl-pyrazol-3-ol, 2-Propyl-4-tert-butyl-pyrazol-3-ol, 2-Isopropyl-4- tert-butyl-pyrazol-3-ol, 2-Butyl-4-tert-butyl-pyrazol-3-ol, 2-Isobutyl-4-tert-butyl-pyrazol-3-ol, 2-tert-Butyl-4-tert-butyl-pyrazol-3-ol, 2-Phenyl-4-tert-butyl-pyrazol-3-ol, 2-(4-tolyl)-4-tert-butyl -pyrazol-3-ol, 2-(2-tolyl)-4-tert-butyl-pyrazol-3-ol, 2-(3-tolyl)-4-tert-butyl-pyrazol-3-ol, 2-(3,4-Dimethylphenyl)-4-phenyl-pyrazol-3-ol, 2-Methyl-4-phenyl-pyrazol-3-ol, 2-Ethyl-4-phenyl-pyrazol-3-ol, 2-Propyl-4-phenyl-pyrazol-3-ol, 2-Isopropyl-4- phenyl-pyrazol-3-ol, 2-Butyl-4-phenyl-pyrazol-3-ol, 2-Isobutyl-4-phenyl-pyrazol-3-ol, 2-tert-Butyl-4-phenyl-pyrazol-3-ol, 2-Phenyl-4-phenyl-pyrazol-3-ol, 2-(4-tolyl)-4-phenyl-pyrazol-3-ol, 2-(2-tolyl)-4-phenyl-pyrazol-3-ol, 2-(3-tolyl)-4- phenyl -pyrazol-3-ol, 2-(3,4-Dimethylphenyl)-4-(2-tolyl)-pyrazol-3-ol, 2-Methyl-4-(2-tolyl)-pyrazol-3-ol, 2-Ethyl-4-(2-tolyl)-pyrazol-3-ol, 2-Propyl-4-(2-tolyl)-pyrazol-3-ol, 2-Isopropyl-4-(2-tolyl)-pyrazol-3-ol, 2-Butyl-4-(2-tolyl)-pyrazol-3-ol, 2-Isobutyl-4-(2-tolyl)-pyrazol-3-ol, 2-tert-Butyl-4-(2-tolyl)-pyrazol-3-ol, 2-Phenyl-4-(2-tolyl)-pyrazol-3-ol, 2-(4-tolyl)-4-(2-tolyl)-pyrazol-3-ol, 2-(2-tolyl)-4-(2-tolyl)-pyrazol-3-ol, 2-(3-tolyl)-4-(2-tolyl)-pyrazol-3-ol, 2-(3,4-Dimethylphenyl)-4-(3-tolyl)-pyrazol-3-ol, 2-Methyl-4-(3-tolyl)-pyrazol-3-ol, 2-Ethyl-4-(3-tolyl)-pyrazol-3-ol, 2-Propyl-4-(3-tolyl)-pyrazol-3-ol, 2-Isopropyl-4-(3-tolyl)-pyrazol-3-ol , 2-Butyl-4-(3-tolyl)-pyrazol-3-ol, 2-Isobutyl-4-(3-tolyl)-pyrazol-3-ol, 2-tert-Butyl-4-(3-tolyl)-pyrazol-3-ol, 2-Phenyl-4-(3-tolyl)-pyrazol-3-ol, 2-(4-tolyl)-4-(3-tolyl)-pyrazol-3-ol, 2-(2-tolyl)-4-(3-tolyl)-pyrazol-3-ol, 2-(3-tolyl)-4-(3-tolyl)-pyrazol-3-ol, 2-(3,4-Dimethylphenyl)-4-(3-tolyl)-pyrazol-3-ol, 2-Methyl-4-(3-tolyl)-pyrazol-3-ol, 2-Ethyl-4-(3-tolyl)-pyrazol-3-ol, 2-Propyl-4-(3-tolyl)-pyrazol-3-ol, 2-Isopropyl-4-(3-tolyl)-pyrazol-3-ol, 2-Butyl-4-(3-tolyl)-pyrazol-3-ol, 2-Isobutyl-4-(3-tolyl)-pyrazol-3-ol, 2-tert-Butyl-4-butyl-pyrazol-3-ol, 2-Phenyl-4-(3-tolyl)-pyrazol-3-ol, 2-(4-tolyl)-4-(3-tolyl)-pyrazol-3-ol, 2-(2-tolyl)-4-(3-tolyl)-pyrazol-3-ol, 2-(3-tolyl)-4-(3-tolyl)-pyrazol-3-ol, 2-(3,4-Dimethylphenyl)-4-butyl-pyrazol-3-ol, 2-Methyl-4-(4-tolyl)-pyrazol-3-ol, 2-Ethyl-4-(4-tolyl)-pyrazol-3-ol, 2-Propyl-4-(4-tolyl)-pyrazol-3-ol, 2-Isopropyl-4-(4-tolyl)-pyrazol-3-ol, 2-Butyl-4-(4-tolyl)-pyrazol-3-ol, 2-Isobutyl-4-(4-tolyl)-pyrazol-3-ol, 2-tert-Butyl-4-(4-tolyl)-pyrazol-3-ol, 2-Phenyl-4-(4-tolyl)-pyrazol-3-ol, 2-(4-tolyl)-4-(4-tolyl)-pyrazol-3-ol, 2-(2-tolyl)-4-(4-tolyl)-pyrazol-3-ol, 2-(3-tolyl)-4- (4-tolyl)-pyrazol-3-ol, 2-(3,4-Dimethylphenyl)-4-(4-tolyl)-pyrazol-3-ol, 2-Methyl-5-phenyl-pyrazol-3-ol, 2-Ethyl-5-phenyl-pyrazol-3-ol, 2-Propyl-5-phenyl-pyrazol-3-ol, 2-Isopropyl-5-phenyl-pyrazol-3-ol, 2-Butyl-5-phenyl-pyrazol-3-ol, 2-Isobutyl-5-phenyl-pyrazol-3-ol, 2-tert-Butyl-5-phenyl-pyrazol-3-ol, 2-Phenyl-5-phenyl-pyrazol-3-ol, 2-(4-tolyl)-5-phenyl-pyrazol-3-ol, 2-(2-tolyl)-5-phenyl-pyrazol-3-ol, 2-(3-tolyl)-5-phenyl-pyrazol-3-ol, 2-(3,4-Dimethylphenyl)-5-phenyl-pyrazol-3-ol, 2-Methyl-5-methyl-pyrazol-3-ol, 2-Ethyl-5-methyl-pyrazol-3-ol, 2-Propyl-5-methyl-pyrazol-3-ol, 2-Isopropyl-5-methyl-pyrazol-3-ol, 2-Butyl-5-methyl-pyrazol-3-ol, 2-Isobutyl-5-methyl-pyrazol-3-ol, 2-tert-Butyl-5-methyl-pyrazol-3-ol, 2-Phenyl-5-methyl-pyrazol-3-ol, 2-(4-tolyl)-5-methyl-pyrazol-3-ol, 2-(2-tolyl)-5-methyl-pyrazol-3-ol, 2-(3-tolyl)-5-methyl-pyrazol-3-ol, 2-(3,4-Dimethylphenyl)-5-methyl-pyrazol-3-ol, 2-methyl-5-ethyl-pyrazol-3-ol, 2-ethyl-5-ethyl-pyrazol-3-ol, 2-propyl-5-ethyl-pyrazol-3-ol, 2-isopropyl-5-ethyl-pyrazol-3-ol, 2-butyl-5-ethyl-pyrazol-3-ol, 2-Isobutyl-5-ethyl-pyrazol-3-ol, 2-tert-butyl-5-ethyl-pyrazol-3-ol, 2-Phenyl-5-ethyl-pyrazol-3-ol, 2-(4-tolyl)-5-ethyl-pyrazol-3-ol, 2-(2-tolyl)-5-ethyl-pyrazol-3-ol, 2-(3-tolyl)-5-ethyl-pyrazol-3-ol, 2-(3,4-Dimethylphenyl)-5-ethyl-pyrazol-3-ol, 2-Methyl-5-propyl-pyrazol-3-ol, 2-Ethyl-5-propyl-pyrazol-3-ol, 2-Propyl-5-propyl-pyrazol-3-ol, 2-Isopropyl-5-methyl-pyrazol-3-ol, 2-Butyl-5-propyl-pyrazol-3-ol, 2-Isobutyl-5-propyl-pyrazol-3-ol, 2-tert-Butyl-5-propyl-pyrazol-3-ol, 2-Phenyl-5-propyl-pyrazol-3-ol, 2-(4-tolyl)-5-propyl-pyrazol-3-ol, 2-(2-tolyl)-5-propyl-pyrazol-3-ol, 2-(3-tolyl)-5-propyl-pyrazol-3-ol, 2-(3,4-Dimethylphenyl)-5-propyl-pyrazol-3-ol, 2-Methyl-5-isopropyl-pyrazol-3-ol, 2-Ethyl-5-isopropyl-pyrazol-3-ol, 2-Propyl-5-isopropyl-pyrazol-3-ol, 2-Isopropyl-5-isopropyl-pyrazol-3-ol, 2-Butyl-5-isopropyl-pyrazol-3-ol, 2-Isobutyl-5-isopropyl-pyrazol-3-ol, 2-tert-Butyl-5-isopropyl-pyrazol-3-ol, 2-Phenyl-5-isopropyl-pyrazol-3-ol, 2-(4-tolyl)-5-isopropyl-pyrazol-3-ol, 2-(2-tolyl)-5-isopropyl-pyrazol-3-ol, 2-(3-tolyl)-5-isopropyl-pyrazol-3-ol, 2-(3,4-Dimethylphenyl)-5-isopropyl-pyrazol-3-ol, 2-Methyl-5-butyl-pyrazol-3-ol, 2-Ethyl-5-butyl-pyrazol-3-ol, 2-Propyl-5-butyl-pyrazol-3-ol, 2-Isopropyl-5-butyl-pyrazol-3-ol, 2-Butyl-5-butyl-pyrazol-3-ol, 2-Isobutyl-5-butyl-pyrazol-3-ol, 2-tert-Butyl-5-butyl-pyrazol-3-ol, 2-Phenyl-5-butyl-pyrazol-3-ol, 2-(4-tolyl)-5-butyl-pyrazol-3-ol, 2-(2-tolyl)-5-butyl-pyrazol-3-ol, 2-(3-tolyl)-5-butyl-pyrazol-3-ol, 2-(3,4-Dimethylphenyl)-5-butyl-pyrazol-3-ol, 2-Methyl-5-isobutyl-pyrazol-3-ol, 2-Ethyl-5-isobutyl-pyrazol-3-ol, 2-Propyl-5-isobutyl-pyrazol-3-ol, 2-Isopropyl-5-isobutyl -pyrazol-3-ol, 2-Butyl-5-isobutyl-pyrazol-3-ol, 2-Isobutyl-5-isobutyl-pyrazol-3-ol, 2-tert-Butyl-5-isobutyl-pyrazol-3-ol, 2-Phenyl-5-isobutyl-pyrazol-3-ol, 2-(4-tolyl)-5-isobutyl-pyrazol-3-ol, 2-(2-tolyl)-5-isobutyl-pyrazol-3-ol, 2-(3-tolyl)-5-isobutyl-pyrazol-3-ol, 2-(3,4-Dimethylphenyl)-5-isobutyl-pyrazol-3-ol, 2-Methyl-5-tert-butyl-pyrazol-3-ol, 2-Ethyl-5-tert-butyl-pyrazol-3-ol, 2-Propyl-5-tert-butyl-pyrazol-3-ol, 2-Isopropyl-5-tert-butyl-pyrazol-3-ol, 2-Butyl-5-tert-butyl -pyrazol-3-ol, 2-Isobutyl-5- tert-butyl -pyrazol-3-ol, 2-tert-Butyl-5- tert-butyl -pyrazol-3-ol, 2-Phenyl-5-tert-butyl-pyrazol-3-ol, 2-(4-tolyl)-5-tert-butyl-pyrazol-3-ol, 2-(2-tolyl)-5-tert-butyl-pyrazol-3-ol, 2-(3-tolyl)-5- tert-butyl -pyrazol-3-ol, 2-(3,4-Dimethylphenyl)-5-phenyl-pyrazol-3-ol, 2-Methyl-5-phenyl-pyrazol-3-ol, 2-Ethyl-5-phenyl-pyrazol-3-ol, 2-Propyl-5-phenyl-pyrazol-3-ol, 2-Isopropyl-5-phenyl-pyrazol-3-ol, 2-Butyl-5-phenyl-pyrazol-3-ol, 2-Isobutyl-5-phenyl-pyrazol-3-ol, 2-tert-Butyl-5-phenyl-pyrazol-3-ol, 2-Phenyl-5-phenyl-pyrazol-3-ol, 2-(4-tolyl)-5-phenyl-pyrazol-3-ol, 2-(2-tolyl)-5-phenyl-pyrazol-3-ol, 2-(3-tolyl)-5-phenyl-pyrazol-3-ol, 2-(3,4-Dimethylphenyl)-5-(2-tolyl)-pyrazol-3-ol, 2-Methyl-5-(2-tolyl)-pyrazol-3-ol, 2-Ethyl-5-(2-tolyl)-pyrazol-3-ol, 2-Propyl-5-(2-tolyl)-pyrazol-3-ol, 2-Isopropyl-5-(2-tolyl)-pyrazol-3-ol, 2-Butyl-5-(2-tolyl)-pyrazol-3-ol, 2-Isobutyl-5-(2-tolyl)-pyrazol-3-ol, 2-tert-Butyl-5-(2-tolyl)-pyrazol-3-ol, 2-Phenyl-5-(2-tolyl)-pyrazol-3-ol, 2-(4-tolyl)-5-(2-tolyl)-pyrazol-3-ol, 2-(2-tolyl)-5-(2-tolyl)-pyrazol-3-ol, 2-(3-tolyl)-5-(2-tolyl)-pyrazol-3-ol, 2-(3,4-Dimethylphenyl)-5-(3-tolyl)-pyrazol-3-ol, 2-Methyl-5-(3-tolyl)-pyrazol-3-ol, 2-Ethyl-5-(3-tolyl)-pyrazol-3-ol, 2-Propyl-5-(3-tolyl)-pyrazol-3-ol, 2-Isopropyl-5-(3-tolyl)-pyrazol-3-ol, 2-Butyl-5-(3-tolyl)-pyrazol-3-ol, 2-Isobutyl-5-(3-tolyl)-pyrazol-3-ol, 2-tert-Butyl-5-(3-tolyl)-pyrazol-3-ol, 2-Phenyl-5-(3-tolyl)-pyrazol-3-ol, 2-(4-tolyl)-5-(3-tolyl)-pyrazol-3-ol, 2-(2-tolyl)-5-(3-tolyl)-pyrazol-3-ol, 2-(3-tolyl)-5-(3-tolyl)-pyrazol-3-ol, 2-(3,4-Dimethylphenyl)-5-(3-tolyl)-pyrazol-3-ol, 2-Methyl-5-(3-tolyl)-pyrazol-3-ol, 2-Ethyl-5-(3-tolyl)-pyrazol-3-ol, 2-Propyl-5-(3-tolyl)-pyrazol-3-ol, 2-Isopropyl-5-(3-tolyl)-pyrazol-3-ol, 2-Butyl-5-(3-tolyl)-pyrazol-3-ol, 2-Isobutyl-5-(3-tolyl)-pyrazol-3-ol, 2-tert-Butyl-5-butyl-pyrazol-3-ol, 2-Phenyl-5-(3-tolyl)-pyrazol-3-ol, 2-(4-tolyl)-5-(3-tolyl)-pyrazol-3-ol, 2-(2-tolyl)-5-(3-tolyl)-pyrazol-3-ol, 2-(3-tolyl)-5-(3-tolyl)-pyrazol-3-ol, 2-(3,4-Dimethylphenyl)-5-butyl-pyrazol-3-ol, 2-Methyl-5-(4-tolyl)-pyrazol-3-ol, 2-Ethyl-5-(4-tolyl)-pyrazol-3-ol, 2-Propyl-5-(4-tolyl)-pyrazol-3-ol, 2-Isopropyl-5-(4-tolyl)-pyrazol-3-ol, 2-Butyl-5-(4-tolyl)-pyrazol-3-ol, 2-Isobutyl-5-(4-tolyl)-pyrazol-3-ol, 2-tert-Butyl-5-(4-tolyl)-pyrazol-3-ol, 2-Phenyl-5-(4-tolyl)-pyrazol-3-ol, 2-(4-tolyl)-5-(4-tolyl)-pyrazol-3-ol, 2-(2-tolyl)-5-(4-tolyl)-pyrazol-3-ol,2-(3-tolyl)-5-(4-tolyl)-pyrazol-3-ol, 2-(3,4-Dimethylphenyl)-5-(4-tolyl)-pyrazol-3-ol, 2-Methyl-4-methyl-5-phenyl-pyrazol-3-ol, 2-Ethyl-4-methyl5-phenyl-pyrazol-3-ol, 2-Propyl-4-methyl-5-phenyl-pyrazol-3-ol, 2-Isopropyl-4-methyl-5-phenyl-pyrazol-3-ol, 2-Butyl-4-methyl-5-phenyl-pyrazol-3-ol, 2-Isobutyl-4-methyl-5-phenyl-pyrazol-3-ol, 2-tert-Butyl-4-methyl-5-phenyl-pyrazol-3-ol, 2-Phenyl-4-methyl-5-phenyl-pyrazol-3-ol, 2-(4-tolyl)-4-methyl-5-phenyl-pyrazol-3-ol, 2-(2-tolyl)-4-methyl-5-phenyl-pyrazol-3-ol, 2-(3-tolyl)-4-methyl-5-phenyl-pyrazol-3-ol, 2-(3,4-Dimethylphenyl)-4-methyl-5-phenyl-pyrazol-3-ol, 2-Methyl-4-methyl-5-methyl-pyrazol-3-ol, 2-Ethyl-4-methyl-5-methyl-pyrazol-3-ol, 2-Propyl-4-methyl-5-methyl-pyrazol-3-ol, 2-Isopropyl-4-methyl-5-methyl -pyrazol-3-ol, 2-Butyl-4-methyl-5-methyl-pyrazol-3-ol, 2-Isobutyl- 4-methyl-5-methyl-pyrazol-3-ol, 2-tert-Butyl-4-methyl-5-methyl-pyrazol-3-ol, 2-(4-tolyl)-4-methyl-5-methyl-pyrazol-3-ol, 2-(2-tolyl)-4-methyl-5-methyl -pyrazol-3-ol, 2-(3-tolyl)-4-methyl-5-methyl-pyrazol-3-ol, 2-(3,4-Dimethylphenyl)-4-methyl-5-methyl-pyrazol-3-ol, 2-methyl-4-methyl-5-ethyl-pyrazol-3-ol, 2-ethyl-4-methyl-5-ethyl-pyrazol-3-ol, 2-propyl-4-methyl-5-ethyl-pyrazol-3-ol, 2-isopropyl-4-methyl-5-ethyl-pyrazol-3-ol, 2-butyl-4-methyl-5- ethyl -pyrazol-3-ol, 2-Isobutyl- 4-methyl-5-ethyl-pyrazol-3-ol, 2-tert-butyl-4-methyl-5-ethyl-pyrazol-3-ol, 2-Phenyl-4-methyl-5-ethyl-pyrazol-3-ol, 2-(4-tolyl)-4-methyl-5-ethyl -pyrazol-3-ol, 2-(2-tolyl)-4-methyl-5-ethyl-pyrazol-3-ol, 2-(3-tolyl)-4-methyl-5-ethyl-pyrazol-3-ol, 2-(3,4-Dimethylphenyl)-4-methyl-5-ethyl-pyrazol-3-ol,2-Methyl-4-methyl-5-propyl-pyrazol-3-ol, 2-Ethyl-4-methyl-5-propyl-pyrazol-3-ol, 2-Propyl-4-methyl-5-propyl-pyrazol-3-ol, 2-Isopropyl-4-methyl-5-methyl-pyrazol-3-ol, 2-Butyl-4-methyl-5-propyl-pyrazol-3-ol, 2-Isobutyl-4-methyl-5-propyl -pyrazol-3-ol, 2-tert-Butyl-4-methyl-5-propyl-pyrazol-3-ol, 2-Phenyl-4-methyl-5-propyl-pyrazol-3-ol, 2-(4-tolyl)-4-methyl-5-propyl-pyrazol-3-ol, 2-(2-tolyl)-4-methyl-5-propyl-pyrazol-3-ol, 2-(3-tolyl)-4-methyl-5-propyl-pyrazol-3-ol, 2-(3,4-Dimethylphenyl)-4-methyl-5-propyl-pyrazol-3-ol, 2-Methyl-4-methyl-5-isopropyl-pyrazol-3-ol, 2-Ethyl-4-methyl-5-isopropyl-pyrazol-3-ol, 2-Propyl-4-methyl-5- isopropyl -pyrazol-3-ol, 2-Isopropyl-4-methyl-5-isopropyl-pyrazol-3-ol, 2-Butyl-4-methyl-5- isopropyl -pyrazol-3-ol, 2-Isobutyl- 4-methyl-5-isopropyl-pyrazol-3-ol, 2-tert-Butyl-4-methyl-5-isopropyl-pyrazol-3-ol, 2-Phenyl-4-methyl-5-isopropyl-pyrazol-3-ol, 2-(4-tolyl)-4-methyl-5-isopropyl-pyrazol-3-ol, 2-(2-tolyl)-4-methyl-5-isopropyl-pyrazol-3-ol, 2-(3-tolyl)-4-methyl-5-isopropyl-pyrazol-3-ol, 2-(3,4-Dimethylphenyl)-4-methyl-5-isopropyl-pyrazol-3-ol, 2-Methyl-4-methyl-5-butyl-pyrazol-3-ol, 2-Ethyl-4-methyl-5-butyl-pyrazol-3-ol, 2-Propyl-4-methyl-5-butyl-pyrazol-3-ol, 2-Isopropyl-4-methyl-5-butyl-pyrazol-3-ol, 2-Butyl-4-methyl-5-butyl-pyrazol-3-ol, 2-Isobutyl-4-methyl-5-butyl-pyrazol-3-ol, 2-tert-Butyl-4-methyl-5-butyl-pyrazol-3-ol, 2-Phenyl-4-methyl-5- butyl -pyrazol-3-ol, 2-(4-tolyl)-4-methyl-5- butyl -pyrazol-3-ol, 2-(2-tolyl)-4-methyl-5-butyl-pyrazol-3-ol, 2-(3-tolyl)-4-methyl-5-butyl-pyrazol-3-ol, 2-(3,4-Dimethylphenyl)-4-methyl-5-butyl-pyrazol-3-ol, 2-Methyl-4-methyl-5-isobutyl-pyrazol-3-ol, 2-Ethyl-4-methyl-5-isobutyl-pyrazol-3-ol, 2-Propyl-4-methyl-5-isobutyl-pyrazol-3-ol, 2-Isopropyl-4-methyl-5-isobutyl-pyrazol-3-ol, 2-Butyl-4-methyl-5-isobutyl-pyrazol-3-ol, 2-Isobutyl-4-methyl-5-isobutyl-pyrazol-3-ol, 2-tert-Butyl-4-methyl-5-isobutyl-pyrazol-3-ol, 2-Phenyl-4-methyl-5-isobutyl-pyrazol-3-ol, 2-(4-tolyl)-4-methyl-5-isobutyl-pyrazol-3-ol, 2-(2-tolyl)-4-methyl-5-isobutyl-pyrazol-3-ol, 2-(3-tolyl)-4-methyl-5-isobutyl-pyrazol-3-ol, 2-(3,4-Dimethylphenyl)-4-methyl-5-isobutyl-pyrazol-3-ol, 2-Methyl-4-methyl-5-tert-butyl-pyrazol-3-ol, 2-Ethyl-4-methyl-5-tert-butyl-pyrazol-3-ol, 2-Propyl-4-methyl-5-tert-butyl-pyrazol-3-ol, 2-Isopropyl-4-methyl-5-tert-butyl-pyrazol-3-ol, 2-Butyl-4-methyl-5-tert-butyl-pyrazol-3-ol, 2-Isobutyl- 4-methyl-5-tert-butyl-pyrazol-3-ol, 2-tert-Butyl-4-methyl-5-tert-butyl-pyrazol-3-ol, 2-Phenyl-4-methyl-5-tert-butyl-pyrazol-3-ol, 2-(4-tolyl)-4-methyl-5-tert-butyl-pyrazol-3-ol, 2-(2-tolyl)-4-methyl-5-tert-butyl-pyrazol-3-ol, 2-(3-tolyl)-4-methyl-5-tert-butyl-pyrazol-3-ol, 2-(3,4-Dimethylphenyl)-4-methyl-5-phenyl-pyrazol-3-ol, 2-Methyl-4-methyl-5-phenyl-pyrazol-3-ol, 2-Ethyl-4-methyl-5-phenyl-pyrazol-3-ol, 2-Propyl-4-methyl-5-phenyl-pyrazol-3-ol, 2-Isopropyl-4-methyl-5-phenyl-pyrazol-3-ol, 2-Butyl-4-methyl-5-phenyl-pyrazol-3-ol, 2-Isobutyl-4-methyl-5-phenyl-pyrazol-3-ol, 2-tert-Butyl-4-methyl-5-phenyl-pyrazol-3-ol, 2-Phenyl-4-methyl-5-phenyl-pyrazol-3-ol, 2-(4-tolyl)-4-methyl-5-phenyl-pyrazol-3-ol, 2-(2-tolyl)-4-methyl-5-phenyl-pyrazol-3-ol, 2-(3-tolyl)-4-methyl-5-phenyl-pyrazol-3-ol, 2-(3,4-Dimethylphenyl)-4-methyl-5-(2-tolyl)-pyrazol-3-ol, 2-Methyl-4-methyl-5-(2-tolyl)-pyrazol-3-ol, 2-Ethyl-4-methyl-5-(2-tolyl)-pyrazol-3-ol, 2-Propyl-4-methyl-5-(2-tolyl)-pyrazol-3-ol, 2-Isopropyl-4-methyl-5-(2-tolyl)-pyrazol-3-ol, 2-Butyl-4-methyl-5-(2-tolyl)-pyrazol-3-ol, 2-Isobutyl-4-methyl-5- (2-tolyl)-pyrazol-3-ol, 2-tert-Butyl-4-methyl-5-(2-tolyl)-pyrazol-3-ol, 2-Phenyl-4-methyl-5- (2-tolyl)-pyrazol-3-ol, 2-(4-tolyl)-4-methyl-5-(2-tolyl)-pyrazol-3-ol, 2-(2-tolyl)-4-methyl-5- (2-tolyl)-pyrazol-3-ol, 2-(3-tolyl)-4-methyl-5-(2-tolyl)-pyrazol-3-ol, 2-(3,4-Dimethylphenyl)-4-methyl-5-(3-tolyl)-pyrazol-3-ol, 2-Methyl-4-methyl-5-(3-tolyl)-pyrazol-3-ol, 2-Ethyl-4-methyl-5- (3-tolyl)-pyrazol-3-ol, 2-Propyl-4-methyl-5-(3-tolyl)-pyrazol-3-ol, 2-Isopropyl-4-methyl-5-(3-tolyl)-pyrazol-3-ol, 2-Butyl-4-methyl-5-(3-tolyl)-pyrazol-3-ol, 2-Isobutyl-4-methyl-5- (3-tolyl)-pyrazol-3-ol, 2-tert-Butyl-4-methyl-5-(3-tolyl)-pyrazol-3-ol, 2-Phenyl-4-methyl-5- (3-tolyl)-pyrazol-3-ol, 2-(4-tolyl)-4-methyl-5-(3-tolyl)-pyrazol-3-ol, 2-(2-tolyl)-4-methyl-5- (3-tolyl)-pyrazol-3-ol, 2-(3-tolyl)-4-methyl-5-(3-tolyl)-pyrazol-3-ol, 2-(3,4-Dimethylphenyl)-4-methyl-5-(3-tolyl)-pyrazol-3-ol, 2-Methyl-4-methyl-5-(3-tolyl)-pyrazol-3-ol, 2-Ethyl-4-methyl-5-(3-tolyl)-pyrazol-3-ol, 2-Propyl-4-methyl-5-(3-tolyl)-pyrazol-3-ol, 2-Isopropyl-4-methyl-5-(3-tolyl)-pyrazol-3-ol, 2-Butyl-4-methyl-5-(3-tolyl)-pyrazol-3-ol, 2-Isobutyl-4-methyl-5- (3-tolyl)-pyrazol-3-ol, 2-tert-Butyl-4-methyl-5-butyl-pyrazol-3-ol, 2-Phenyl-4-methyl-5-(3-tolyl)-pyrazol-3-ol, 2-(4-tolyl)-4-methyl-5-(3-tolyl)-pyrazol-3-ol, 2-(2-tolyl)-4-methyl-5-(3-tolyl)-pyrazol-3-ol, 2-(3-tolyl)-4-methyl-5-(3-tolyl)-pyrazol-3-ol, 2-(3,4-Dimethylphenyl)-4-methyl-5-butyl -pyrazol-3-ol, 2-Methyl-4-methyl-5-(4-tolyl)-pyrazol-3-ol, 2-Ethyl-4-methyl-5-(4-tolyl)-pyrazol-3-ol, 2-Propyl-4-methyl-5-(4-tolyl)-pyrazol-3-ol, 2-Isopropyl-4-methyl-5-(4-tolyl)-pyrazol-3-ol, 2-Butyl-4-methyl-5-(4-tolyl)-pyrazol-3-ol, 2-Isobutyl-4-methyl-5-(4-tolyl)-pyrazol-3-ol, 2-tert-Butyl-4-methyl-5-(4-tolyl)-pyrazol-3-ol, 2-Phenyl-4-methyl-5-(4-tolyl)-pyrazol-3-ol, 2-(4-tolyl)-4-methyl-5-(4-tolyl)-pyrazol-3-ol, 2-(2-tolyl)-4-methyl-5-(4-tolyl)-pyrazol-3-ol, 2-(3-tolyl)-4-methyl-5-(4-tolyl)-pyrazol-3-ol, 2-(3,4-Dimethylphenyl)-4-methyl-5-(4-tolyl)-pyrazol-3-ol, 2-Methyl-4-ethyl-5-phenyl-pyrazol-3-ol, 2-Ethyl-4-ethyl-5-phenyl-pyrazol-3-ol, 2-Propyl-4-ethyl-5-phenyl-pyrazol-3-ol, 2-Isopropyl-4-ethyl-5-phenyl-pyrazol-3-ol, 2-Butyl-4-ethyl-5-phenyl-pyrazol-3-ol, 2-Isobutyl- 4-ethyl-5-phenyl-pyrazol-3-ol, 2-tert-Butyl-4-ethyl-5-phenyl-pyrazol-3-ol, 2-Phenyl-4-ethyl-5-phenyl-pyrazol-3-ol, 2-(4-tolyl)-4-ethyl-5-phenyl-pyrazol-3-ol, 2-(2-tolyl)-4-ethyl-5-phenyl-pyrazol-3-ol, 2-(3-tolyl)-4-ethyl-5-phenyl-pyrazol-3-ol, 2-(3,4-Dimethylphenyl)-4-ethyl-5-phenyl-pyrazol-3-ol,2-Methyl-4-ethyl-5-methyl-pyrazol-3-ol, 2-Ethyl-4-ethyl-5-methyl-pyrazol-3-ol, 2-Propyl-4-ethyl-5-methyl-pyrazol-3-ol, 2-Isopropyl-4-ethyl-5-methyl-pyrazol-3-ol, 2-Butyl-4-ethyl-5-methyl-pyrazol-3-ol, 2-Isobutyl-4-ethyl-5-methyl -pyrazol-3-ol, 2-tert-Butyl-4-ethyl-5-methyl-pyrazol-3-ol, 2-Phenyl-4-ethyl-5-methyl-pyrazol-3-ol, 2-(4-tolyl)-4-ethyl-5-methyl-pyrazol-3-ol, 2-(2-tolyl)-4-ethyl-5-methyl-pyrazol-3-ol, 2-(3-tolyl)-4-ethyl-5-methyl-pyrazol-3-ol, 2-(3,4-Dimethylphenyl)-4-ethyl-5-methyl-pyrazol-3-ol,2-methyl-4-ethyl-5-ethyl-pyrazol-3-ol, 2-ethyl-4-ethyl-5-ethyl-pyrazol-3-ol, 2-propyl-4-ethyl-5-ethyl-pyrazol-3-ol, 2-isopropyl-4-ethyl-5-ethyl-pyrazol-3-ol, 2-butyl-4-ethyl-5-ethyl-pyrazol-3-ol, 2-Isobutyl- 4-ethyl-5-ethyl-pyrazol-3-ol, 2-tert-butyl-4-ethyl-5-ethyl -pyrazol-3-ol, 2-Phenyl-4-ethyl-5- ethyl-pyrazol-3-ol, 2-(4-tolyl)-4-ethyl-5-ethyl -pyrazol-3-ol, 2-(2-tolyl)-4-ethyl-5-ethyl-pyrazol-3-ol, 2-(3-tolyl)-4-ethyl-5-ethyl-pyrazol-3-ol, 2-(3,4-Dimethylphenyl)-4-ethyl-5-ethyl-pyrazol-3-ol, 2-Methyl-4-ethyl-5-propyl-pyrazol-3-ol, 2-Ethyl-4-ethyl-5- propyl-pyrazol-3-ol, 2-Propyl-4-ethyl-5-propyl-pyrazol-3-ol, 2-Isopropyl-4-ethyl-5-methyl-pyrazol-3-ol, 2-Butyl-4-ethyl-5-propyl-pyrazol-3-ol, 2-Isobutyl-4-ethyl-5-propyl-pyrazol-3-ol, 2-tert-Butyl-4-ethyl-5-propyl-pyrazol-3-ol, 2-Phenyl-4-ethyl-5-propyl-pyrazol-3-ol, 2-(4-tolyl)-4-ethyl-5-propyl-pyrazol-3-ol, 2-(2-tolyl)-4-ethyl-5-propyl-pyrazol-3-ol, 2-(3-tolyl)-4-ethyl-5-propyl-pyrazol-3-ol, 2-(3,4-Dimethylphenyl)-4-ethyl-5-propyl-pyrazol-3-ol, 2-Methyl-4-ethyl-5-isopropyl-pyrazol-3-ol, 2-Ethyl-4-ethyl-5-isopropyl-pyrazol-3-ol, 2-Propyl-4-ethyl-5-isopropyl-pyrazol-3-ol, 2-Isopropyl-4-ethyl-5-isopropyl-pyrazol-3-ol, 2-Butyl-4-ethyl-5-isopropyl-pyrazol-3-ol, 2-Isobutyl-4-ethyl-5-isopropyl-pyrazol-3-ol, 2-tert-Butyl-4-ethyl-5-isopropyl-pyrazol-3-ol, 2-Phenyl-4-ethyl-5-isopropyl -pyrazol-3-ol, 2-(4-tolyl)-4-ethyl-5-isopropyl-pyrazol-3-ol, 2-(2-tolyl)-4-ethyl-5-isopropyl-pyrazol-3-ol, 2-(3-tolyl)-4-ethyl-5-isopropyl -pyrazol-3-ol, 2-(3,4-Dimethylphenyl)-4-ethyl-5-isopropyl-pyrazol-3-ol, 2-Methyl-4-ethyl-5-butyl-pyrazol-3-ol, 2-Ethyl-4-ethyl-5-butyl-pyrazol-3-ol, 2-Propyl-4-ethyl-5-butyl-pyrazol-3-ol, 2-Isopropyl-4-ethyl-5-butyl-pyrazol-3-ol, 2-Butyl-4-ethyl-5-butyl-pyrazol-3-ol, 2-Isobutyl-4-ethyl-5-butyl-pyrazol-3-ol, 2-tert-Butyl-4-ethyl-5-butyl-pyrazol-3-ol, 2-Phenyl-4-ethyl-5-butyl-pyrazol-3-ol, 2-(4-tolyl)-4-ethyl-5-butyl-pyrazol-3-ol, 2-(2-tolyl)-4-ethyl-5-butyl-pyrazol-3-ol, 2-(3-tolyl)-4-ethyl-5-butyl-pyrazol-3-ol, 2-(3,4-Dimethylphenyl)-4-ethyl-5-butyl-pyrazol-3-ol, 2-Methyl-4-ethyl-5-isobutyl-pyrazol-3-ol, 2-Ethyl-4-ethyl-5-isobutyl-pyrazol-3-ol, 2-Propyl-4-ethyl-5-isobutyl-pyrazol-3-ol, 2-Isopropyl-4-ethyl-5-isobutyl-pyrazol-3-ol, 2-Butyl-4-ethyl-5-isobutyl-pyrazol-3-ol, 2-Isobutyl-4-ethyl-5-isobutyl-pyrazol-3-ol, 2-tert-Butyl-4-ethyl-5-isobutyl-pyrazol-3-ol, 2-Phenyl-4-ethyl-5-isobutyl-pyrazol-3-ol, 2-(4-tolyl)-4-ethyl-5-isobutyl-pyrazol-3-ol, 2-(2-tolyl)-4-ethyl-5-isobutyl-pyrazol-3-ol, 2-(3-tolyl)-4-ethyl-5-isobutyl-pyrazol-3-ol, 2-(3,4-Dimethylphenyl)-4-ethyl-5-isobutyl-pyrazol-3-ol, 2-Methyl-4-ethyl-5-tert-butyl-pyrazol-3-ol, 2-Ethyl-4-ethyl-5-tert-butyl-pyrazol-3-ol, 2-Propyl-4-ethyl-5-tert-butyl-pyrazol-3-ol, 2-Isopropyl-4-ethyl-5-tert-butyl-pyrazol-3-ol, 2-Butyl-4-ethyl-5-tert-butyl-pyrazol-3-ol, 2-Isobutyl-4-ethyl-5-tert-butyl-pyrazol-3-ol, 2-tert-Butyl-4-ethyl-5-tert-butyl-pyrazol-3-ol, 2-Phenyl-4-ethyl-5-tert-butyl-pyrazol-3-ol, 2-(4-tolyl)-4-ethyl-5-tert-butyl-pyrazol-3-ol, 2-(2-tolyl)-4-ethyl-5-tert-butyl-pyrazol-3-ol, 2-(3-tolyl)-4-ethyl-5-tert-butyl-pyrazol-3-ol, 2-(3,4-Dimethylphenyl)-4-ethyl-5-phenyl-pyrazol-3-ol, 2-Methyl-4-ethyl-5-phe-nyl-pyrazol-3-ol, 2-Ethyl-4-ethyl-5-phenyl-pyrazol-3-ol, 2-Propyl-4-ethyl-5-phenyl-pyrazol-3-ol, 2-Isopropyl-4-ethyl-5-phenyl-pyrazol-3-ol, 2-Butyl-4-ethyl-5-phenyl-pyrazol-3-ol,2-Isobutyl-4-ethyl-5-phenyl-pyrazol-3-ol, 2-tert-Butyl-4-ethyl-5-phenyl-pyrazol-3-ol,2-Phenyl-4-ethyl-5-phenyl-pyrazol-3-ol, 2-(4-tolyl)-4-ethyl-5-phenyl-pyrazol-3-ol, 2-(2-tolyl)-4-ethyl-5-phenyl-pyrazol-3-ol, 2-(3-tolyl)-4-ethyl-5-phenyl-pyrazol-3-ol, 2-(3,4-Dimethyl-phenyl)-4-ethyl-5-(2-tolyl)-pyrazol-3-ol, 2-Methyl-4-ethyl-5-(2-tolyl)-pyrazol-3-ol, 2-Ethyl-4-ethyl-5-(2-tolyl)-pyrazol-3-ol, 2-Propyl-4-ethyl-5-(2-tolyl)-pyrazol-3-ol, 2-Isopropyl-4-ethyl-5-(2-tolyl)-pyrazol-3-ol, 2-Butyl-4-ethyl-5-(2-tolyl)-pyrazol-3-ol, 2-Isobutyl-4-ethyl-5-(2-tolyl)-pyrazol-3-ol, 2-tert-Butyl-4-ethyl-5-(2-tolyl)-pyrazol-3-ol, 2-Phenyl-4-ethyl-5-(2-tolyl)-pyrazol-3-ol, 2-(4-tolyl)-4-ethyl-5-(2-tolyl)-pyrazol-3-ol, 2-(2-tolyl)-4-ethyl-5-(2-tolyl)-pyrazol-3-ol, 2-(3-tolyl)-4-ethyl-5-(2-tolyl)-pyrazol-3-ol, 2-(3,4-Dimethylphenyl)-4-ethyl-5-(3-tolyl)-pyrazol-3-ol, 2-Methyl-4-ethyl-5-(3-tolyl)-pyrazol-3-ol, 2-Ethyl-4-ethyl-5-(3-tolyl)-pyrazol-3-ol, 2-Propyl-4-ethyl-5-(3-tolyl)-pyrazol-3-ol, 2-Isopropyl-4-ethyl-5-(3-tolyl)-pyrazol-3-ol, 2-Butyl-4-ethyl-5-(3-tolyl)-pyrazol-3-ol, 2-Isobutyl-4-ethyl-5-(3-tolyl)-pyrazol-3-ol, 2-tert-Butyl-4-ethyl-5-(3-tolyl)-pyrazol-3-ol, 2-Phenyl-4-ethyl-5-(3-tolyl)-pyrazol-3-ol, 2-(4-tolyl)-4-ethyl-5-(3-tolyl)-pyrazol-3-ol, 2-(2-tolyl)-4-ethyl-5-(3-tolyl)-pyrazol-3-ol, 2-(3-tolyl)-4-ethyl-5-(3-tolyl)-pyrazol-3-ol, 2-(3,4-Dimethylphenyl)-4-ethyl-5-(3-tolyl)-pyrazol-3-ol, 2-Methyl-4-ethyl-5-(3-tolyl)-pyrazol-3-ol, 2-Ethyl-4-ethyl-5-(3-tolyl)-pyrazol-3-ol, 2-Propyl-4-ethyl-5-(3-tolyl)-pyrazol-3-ol, 2-Isopropyl-4-ethyl-5-(3-tolyl)-pyrazol-3-ol, 2-Butyl-4-ethyl-5-(3-tolyl)-pyrazol-3-ol, 2-Isobutyl-4-ethyl-5-(3-tolyl)-pyrazol-3-ol, 2-tert-Butyl-4-ethyl-5-butyl-pyrazol-3-ol,2-Phenyl-4-ethyl-5-(3-tolyl)-pyrazol-3-ol, 2-(4-tolyl)-4-ethyl-5-(3-tolyl)-pyrazol-3-ol, 2-(2-tolyl)-4-ethyl-5-(3-tolyl)-pyrazol-3-ol, 2-(3-tolyl)-4-ethyl-5-(3-tolyl)-pyrazol-3-ol, 2-(3,4-Dimethylphenyl)-4-ethyl-5-butyl-pyrazol-3-ol, 2-Methyl-4-ethyl-5-(4-tolyl)-pyrazol-3-ol, 2-Ethyl-4-ethyl-5-(4-tolyl)-pyrazol-3-ol, 2-Propyl-4-ethyl-5-(4-tolyl)-pyrazol-3-ol, 2-Isopropyl-4-ethyl-5-(4-tolyl)-pyrazol-3-ol, 2-Butyl-4-ethyl-5-(4-tolyl)-pyrazol-3-ol, 2-Isobutyl-4-ethyl-5-(4-tolyl)-pyrazol-3-ol, 2-tert-Butyl-4-ethyl-5-(4-tolyl)-pyrazol-3-ol, 2-Phenyl-4-ethyl-5-(4-tolyl)-pyrazol-3-ol, 2-(4-tolyl)-4-ethyl-5-(4-tolyl)-pyrazol-3-ol, 2-(2-tolyl)-4-ethyl-5-(4-tolyl)-pyrazol-3-ol, 2-(3-tolyl)-4-ethyl-5-(4-tolyl)-pyrazol-3-ol, 2-(3,4-Dimethylphenyl)-4-ethyl-5-(4-tolyl)-pyrazol-3-ol, 2-Methyl-4-propyl-5-phenyl-pyrazol-3-ol, 2-Ethyl-4-propyl-5-phenyl-pyrazol-3-ol, 2-Propyl-4-propyl-5-phenyl-pyrazol-3-ol, 2-Isopropyl-4-propyl-5-phenyl-pyrazol-3-ol, 2-Butyl-4-propyl-5-phenyl-pyrazol-3-ol, 2-Isobutyl-4-propyl-5-phenyl-pyrazol-3-ol, 2-tert-Butyl-4-propyl-5-phenyl-pyrazol-3-ol, 2-Phenyl-4-propyl-5-phenyl-pyrazol-3-ol, 2-(4-tolyl)-4-propyl-5-phenyl-pyrazol-3-ol, 2-(2-tolyl)-4-propyl-5-phenyl-pyrazol-3-ol, 2-(3-tolyl)-4-propyl-5-phenyl-pyrazol-3-ol, 2-(3,4-Dimethylphenyl)-4-propyl-5-phenyl-pyrazol-3-ol, 2-Methyl-4-propyl-5-methyl-pyrazol-3-ol, 2-Ethyl-4-propyl-5-methyl-pyrazol-3-ol, 2-Propyl-4-propyl-5-methyl-pyrazol-3-ol, 2-Isopropyl-4-propyl-5-methyl-pyrazol-3-ol, 2-Butyl-4-propyl-5-methyl-pyrazol-3-ol, 2-Isobutyl-4-propyl-5-methyl-pyrazol-3-ol, 2-tert-Butyl-4-propyl-5-methyl-pyrazol-3-ol, 2-Phenyl-4-propyl-5-methyl-pyrazol-3-ol, 2-Phenyl-4-propyl-5-methyl-pyrazol-3-ol, 2-(4-tolyl)-4-propyl-5-methyl-pyrazol-3-ol, 2-(2-tolyl)-4-propyl-5-methyl-pyrazol-3-ol, 2-(3-tolyl)-4-propyl-5-methyl-pyrazol-3-ol, 2-(3,4-Dimethylphenyl)-4-propyl-5-methyl-pyrazol-3-ol, 2-methyl-4-propyl-5-ethyl-pyrazol-3-ol, 2-ethyl-4-propyl-5-ethyl-pyrazol-3-ol, 2-propyl-4-propyl-5-ethyl-pyrazol-3-ol, 2-isopropyl-4-propyl-5-ethyl-pyrazol-3-ol, 2-butyl-4-propyl-5-ethyl-pyrazol-3-ol, 2-Isobutyl-4-propyl-5-ethyl-pyrazol-3-ol, 2-tert-butyl-4-propyl-5-ethyl-pyrazol-3-ol, 2-Phenyl-4-propyl-5-ethyl-pyrazol-3-ol, 2-(4-tolyl)-4-propyl-5-ethyl-pyrazol-3-ol, 2-(2-tolyl)-4-propyl-5-ethyl-pyrazol-3-ol, 2-(3-tolyl)-4-propyl-5-ethyl-pyrazol-3-ol, 2-(3,4-Dimethylphenyl)-4-propyl-5-ethyl-pyrazol-3-ol, 2-Methyl-4-propyl-5-propyl-pyrazol-3-ol, 2-Ethyl-4-propyl-5-propyl-pyrazol-3-ol, 2-Propyl-4-propyl-5-propyl-pyrazol-3-ol, 2-Isopropyl-4-propyl-5-methyl-pyrazol-3-ol, 2-Butyl-4-propyl-5-propyl-pyrazol-3-ol, 2-Isobutyl-4-propyl-5-propyl-pyrazol-3-ol, 2-tert-Butyl-4-propyl-5-propyl-pyrazol-3-ol, 2-Phenyl-4-propyl-5-propyl -pyrazol-3-ol, 2-(4-tolyl)-4-propyl-5-propyl-pyrazol-3-ol, 2-(2-tolyl)-4-propyl-5-propyl-pyrazol-3-ol, 2-(3-tolyl)-4-propyl-5-propyl-pyrazol-3-ol, 2-(3,4-Dimethylphenyl)-4-propyl-5-propyl-pyrazol-3-ol, 2-Methyl-4-propyl-5-isopropyl-pyrazol-3-ol, 2-Ethyl-4-propyl-5-isopropyl -pyrazol-3-ol, 2-Propyl-4-propyl-5-isopropyl-pyrazol-3-ol, 2-Isopropyl-4-propyl-5-isopropyl-pyrazol-3-ol, 2-Butyl-4-propyl-5-isopropyl-pyrazol-3-ol, 2-Isobutyl-4-propyl-5-isopropyl-pyrazol-3-ol, 2-tert-Butyl-4-propyl-5-isopropyl-pyrazol-3-ol, 2-Phenyl-4-propyl-5-isopropyl-pyrazol-3-ol, 2-(4-tolyl)-4-propyl-5-isopropyl-pyrazol-3-ol, 2-(2-tolyl)-4-propyl-5-isopropyl-pyrazol-3-ol, 2-(3-tolyl)-4-propyl-5-isopropyl-pyrazol-3-ol, 2-(3,4-Dimethylphenyl)-4-propyl-5-isopropyl-pyrazol-3-ol, 2-Methyl-4-propyl-5-butyl-pyrazol-3-ol, 2-Ethyl-4-propyl-5-butyl-pyrazol-3-ol, 2-Propyl-4-propyl-5-butyl-pyrazol-3-ol, 2-Isopropyl-4-propyl-5-butyl-pyrazol-3-ol, 2-Butyl-4-propyl-5-butyl-pyrazol-3-ol, 2-Isobutyl-4-propyl-5-butyl-pyrazol-3-ol, 2-tert-Butyl-4-propyl-5-butyl-pyrazol-3-ol, 2-Phenyl-4-propyl-5-butyl-pyrazol-3-ol, 2-(4-tolyl)-4-propyl-5-butyl-pyrazol-3-ol, 2-(2-tolyl)-4-propyl-5-butyl-pyrazol-3-ol, 2-(3-tolyl)-4-propyl-5-butyl-pyrazol-3-ol, 2-(3,4-Dimethylphenyl)-4-propyl-5-butyl-pyrazol-3-ol, 2-Methyl-4-propyl-5-isobutyl-pyrazol-3-ol, 2-Ethyl-4-propyl-5-isobutyl-pyrazol-3-ol, 2-Propyl-4-propyl-5-isobutyl-pyrazol-3-ol, 2-Isopropyl-4-propyl-5-isobutyl-pyrazol-3-ol, 2-Butyl-4-propyl-5-isobutyl-pyrazol-3-ol, 2-Isobutyl-4-propyl-5-isobutyl-pyrazol-3-ol, 2-tert-Butyl-4-propyl-5-isobutyl-pyrazol-3-ol, 2-Phenyl-4-propyl-5-isobutyl-pyrazol-3-ol, 2-(4-tolyl)-4-propyl-5-isobutyl-pyrazol-3-ol, 2-(2-tolyl)-4-propyl-5-isobutyl-pyrazol-3-ol, 2-(3-tolyl)-4-propyl-5-isobutyl-pyrazol-3-ol, 2-(3,4-Dimethylphenyl)-4-propyl-5-isobutyl-pyrazol-3-ol, 2-Methyl-4-propyl-5-tert-butyl-pyrazol-3-ol, 2-Ethyl-4-propyl-5-tert-butyl-pyrazol-3-ol, 2-Propyl-4-propyl-5-tert-butyl-pyrazol-3-ol, 2-Isopropyl-4-propyl-5-tert-butyl-pyrazol-3-ol, 2-Butyl-4-propyl-5-tert-butyl-pyrazol-3-ol, 2-Isobutyl-4-propyl-5-tert-butyl-pyrazol-3-ol, 2-tert-Butyl-4-propyl-5-tert-butyl-pyrazol-3-ol, 2-Phenyl-4-propyl-5-tert-butyl-pyrazol-3-ol, 2-(4-tolyl)-4-propyl-5-tert-butyl-pyrazol-3-ol, 2-(2-tolyl)-4-propyl-5-tert-butyl-pyrazol-3-ol, 2-(3-tolyl)-4-propyl-5-tert-butyl-pyrazol-3-ol, 2-(3,4-Dimethylphenyl)-4-propyl-5-phenyl-pyrazol-3-ol, 2-Methyl-4-propyl-5-phenyl-pyrazol-3-ol, 2-Ethyl-4-propyl-5-phenyl-pyrazol-3-ol, 2-Propyl-4-propyl-5-phenyl-pyrazol-3-ol, 2-Isopropyl-4-propyl-5-phenyl-pyrazol-3-ol, 2-Butyl-4-propyl-5-phenyl-pyrazol-3-ol, 2-Isobutyl-4-propyl-5-phenyl-pyrazol-3-ol, 2-tert-Butyl-4-propyl-5-phenyl-pyrazol-3-ol, 2-Phenyl-4-propyl-5-phenyl-pyrazol-3-ol, 2-(4-tolyl)-4-propyl-5-phenyl -pyrazol-3-ol, 2-(2-tolyl)-4-propyl-5-phenyl-pyrazol-3-ol, 2-(3-tolyl)-4-propyl-5-phenyl-pyrazol-3-ol, 2-(3,4-Dimethylphenyl)-4-propyl-5-(2-tolyl)-pyrazol-3-ol, 2-Methyl-4-propyl-5-(2-tolyl)-pyrazol-3-ol,2-Ethyl-4-propyl-5-(2-tolyl)-pyrazol-3-ol, 2-Propyl-4-propyl-5-(2-tolyl)-pyrazol-3-ol, 2-Isopropyl-4-propyl-5-(2-tolyl)-pyrazol-3-ol, 2-Butyl-4-propyl-5-(2-tolyl)-pyrazol-3-ol, 2-Isobutyl-4-propyl-5-(2-tolyl)-pyrazol-3-ol, 2-tert-Butyl-4-propyl-5-(2-tolyl)-pyrazol-3-ol, 2-Phenyl-4-propyl-5-(2-tolyl)-pyrazol-3-ol, 2-(4-tolyl)-4-propyl-5-(2-tolyl)-pyrazol-3-ol, 2-(2-tolyl)-4-propyl-5-(2-tolyl)-pyrazol-3-ol, 2-(3-tolyl)-4-propyl-5-(2-tolyl)-pyrazol-3-ol, 2-(3,4-Dimethylphenyl)-4-propyl-5-(3-tolyl)-pyrazol-3-ol, 2-Methyl-4-propyl-5-(3-tolyl)-pyrazol-3-ol, 2-Ethyl-4-propyl-5-(3-tolyl)-pyrazol-3-ol, 2-Propyl-4-propyl-5-(3-tolyl)-pyrazol-3-ol, 2-Isopropyl-4-propyl-5-(3-tolyl)-pyrazol-3-ol, 2-Butyl-4-propyl-5-(3-tolyl)-pyrazol-3-ol, 2-Isobutyl-4-propyl-5-(3-tolyl)-pyrazol-3-ol, 2-tert-Butyl-4-propyl-5-(3-tolyl)-pyrazol-3-ol, 2-Phenyl-4-propyl-5-(3-tolyl)-pyrazol-3-ol, 2-(4-tolyl)-4-propyl-5-(3-tolyl)-pyrazol-3-ol, 2-(2-tolyl)-4-propyl-5-(3-tolyl)-pyrazol-3-ol, 2-(3-tolyl)-4-propyl-5-(3-tolyl)-pyrazol-3-ol, 2-(3,4-Dimethylphenyl)-4-propyl-5-(3-tolyl)-pyrazol-3-ol, 2-Methyl-4-propyl-5-(3-tolyl)-pyrazol-3-ol, 2-Ethyl-4-propyl-5-(3-tolyl)-pyrazol-3-ol, 2-Propyl-4-propyl-5-(3-tolyl)-pyrazol-3-ol, 2-Isopropyl-4-propyl-5-(3-tolyl)-pyrazol-3-ol, 2-Butyl-4-propyl-5-(3-tolyl)-pyrazol-3-ol, 2-Isobutyl-4-propyl-5-(3-tolyl)-pyrazol-3-ol, 2-tert-Butyl-4-propyl-5-butyl-pyrazol-3-ol, 2-Phenyl-4-propyl-5-(3-tolyl)-pyrazol-3-ol, 2-(4-tolyl)-4-propyl-5-(3-tolyl)-pyrazol-3-ol, 2-(2-tolyl)-4-propyl-5-(3-tolyl)-pyrazol-3-ol, 2-(3-tolyl)-4-propyl-5-(3-tolyl)-pyrazol-3-ol, 2-(3,4-Dimethylphenyl)-4-propyl-5-butyl-pyrazol-3-ol, 2-Methyl-4-propyl-5-(4-tolyl)-pyrazol-3-ol, 2-Ethyl-4-propyl-5-(4-tolyl)-pyrazol-3-ol, 2-Propyl-4-propyl-5-(4-tolyl)-pyrazol-3-ol, 2-Isopropyl-4-propyl-5-(4-tolyl)-pyrazol-3-ol, 2-Butyl-4-propyl-5-(4-tolyl)-pyrazol-3-ol, 2-Isobutyl-4-propyl-5-(4-tolyl)-pyrazol-3-ol, 2-tert-Butyl-4-propyl-5-(4-tolyl)-pyrazol-3-ol, 2-Phenyl-4-propyl-5-(4-tolyl)-pyrazol-3-ol, 2-(4-tolyl)-4-propyl-5-(4-tolyl)-pyrazol-3-ol, 2-(2-tolyl)-4-propyl-5-(4-tolyl)-pyrazol-3-ol, 2-(3-tolyl)-4-propyl-5-(4-tolyl)-pyrazol-3-ol, 2-(3,4-Dimethylphenyl)-4-propyl-5-(4-tolyl)-pyrazol-3-ol,
2-Phenyl-4-isopropyl-5-phenyl-pyrazol-3-ol, 2-(4-tolyl)-4-isopropyl-5-phenyl-pyrazol-3-ol, 2-(2-tolyl)-4-isopropyl-5-phenyl-pyrazol-3-ol, 2-(3-tolyl)-4-isopropyl-5-phenyl-pyrazol-3-ol, 2-(3,4-Dimethylphenyl)-4-isopropyl-5-phenyl-pyrazol-3-ol, 2-Methyl-4-isopropyl-5-methyl-pyrazol-3-ol, 2-Ethyl-4-isopropyl-5-methyl-pyrazol-3-ol, 2-Propyl-4-isopropyl-5-methyl-pyrazol-3-ol, 2-Isopropyl-4-isopropyl-5-methyl-pyrazol-3-ol, 2-Butyl-4-isopropyl-5-methyl-pyrazol-3-ol, 2-Isobutyl-4-isopropyl-5-methyl-pyrazol-3-ol, 2-tert-Butyl-4-isopropyl-5-methyl-pyrazol-3-ol, 2-Phenyl-4-isopropyl-5-methyl-pyrazol-3-ol, 2-Phenyl-4-isopropyl-5-methyl-pyrazol-3-ol, 2-(4-tolyl)-4-isopropyl-5-methyl-pyrazol-3-ol, 2-(2-tolyl)-4-isopropyl-5-methyl-pyrazol-3-ol, 2-(3-tolyl)-4-isopropyl-5-methyl-pyrazol-3-ol, 2-(3,4-Dimethylphenyl)-4-isopropyl-5-methyl-pyrazol-3-ol, 2-methyl-4-isopropyl-5-ethyl-pyrazol-3-ol, 2-ethyl-4-isopropyl-5-ethyl-pyrazol-3-ol, 2-propyl-4-isopropyl-5-ethyl-pyrazol-3-ol, 2-isopropyl-4-isopropyl-5-ethyl-pyrazol-3-ol, 2-butyl-4-isopropyl-5-ethyl-pyrazol-3-ol, 2-Isobutyl-4-isopropyl-5-ethyl-pyrazol-3-ol, 2-tert-butyl-4-isopropyl-5-ethyl-pyrazol-3-ol, 2-Phenyl-4-isopropyl-5-ethyl-pyrazol-3-ol, 2-(4-tolyl)-4-isopropyl-5-ethyl-pyrazol-3-ol, 2-(2-tolyl)-4-isopropyl-5-ethyl-pyrazol-3-ol, 2-(3-tolyl)-4-isopropyl-5-ethyl-pyrazol-3-ol, 2-(3,4-Dimethylphenyl)-4-isopropyl-5-ethyl-pyrazol-3-ol, 2-Methyl-4-isopropyl-5-propyl-pyrazol-3-ol, 2-Ethyl-4-isopropyl-5-propyl-pyrazol-3-ol, 2-Propyl-4-isopropyl-5-propyl-pyrazol-3-ol,2-Isopropyl-4-isopropyl-5-methyl-pyrazol-3-ol, 2-Butyl-4-isopropyl-5-propyl-pyrazol-3-ol,2-Isobutyl-4-isopropyl-5-propyl-pyrazol-3-ol, 2-tert-Butyl-4-isopropyl-5-propyl-pyrazol-3-ol,2-Phenyl-4-isopropyl-5-propyl-pyrazol-3-ol, 2-(4-tolyl)-4-isopropyl-5-propyl-pyrazol-3-ol, 2-(2-tolyl)-4-isopropyl-5-propyl-pyrazol-3-ol, 2-(3-tolyl)-4-isopropyl-5-propyl-pyrazol-3-ol, 2-(3,4-Dimethylphenyl)-4-isopropyl-5-propyl-pyrazol-3-ol, 2-Methyl-4-isopropyl-5-isopropyl-pyrazol-3-ol, 2-Ethyl-4-isopropyl-5-isopropyl-pyrazol-3-ol, 2-Propyl-4-isopropyl-5-isopropyl-pyrazol-3-ol, 2-Isopropyl-4-isopropyl-5-isopropyl-pyrazol-3-ol, 2-Butyl-4-isopropyl-5-isopropyl-pyrazol-3-ol, 2-Isobutyl-4-isopropyl-5-isopropyl-pyrazol-3-ol, 2-tert-Butyl-4-isopropyl-5-isopropyl-pyrazol-3-ol, 2-Phenyl-4-isopropyl-5-isopropyl-pyrazol-3-ol, 2-(4-tolyl)-4-isopropyl-5-isopropyl-pyrazol-3-ol, 2-(2-tolyl)-4-isopropyl-5-isopropyl-pyrazol-3-ol, 2-(3-tolyl)-4-isopropyl-5-isopropyl-pyrazol-3-ol, 2-(3,4-Dimethylphenyl)-4-isopropyl-5-isopropyl-pyrazol-3-ol, 2-Methyl-4-isopropyl-5-butyl-pyrazol-3-ol, 2-Ethyl-4-isopropyl-5-butyl-pyrazol-3-ol, 2-Propyl-4-isopropyl-5-butyl-pyrazol-3-ol, 2-Isopropyl-4-isopropyl-5-butyl-pyrazol-3-ol, 2-Butyl-4-isopropyl-5-butyl-pyrazol-3-ol, 2-Isobutyl-4-isopropyl-5-butyl-pyrazol-3-ol, 2-tert-Butyl-4-isopropyl-5-butyl-pyrazol-3-ol, 2-Phenyl-4-isopropyl-5-butyl-pyrazol-3-ol, 2-(4-tolyl)-4-isopropyl-5-butyl-pyrazol-3-ol, 2-(2-tolyl)-4-isopropyl-5-butyl-pyrazol-3-ol, 2-(3-tolyl)-4-isopropyl-5-butyl-pyrazol-3-ol, 2-(3,4-Dimethylphenyl)-4-isopropyl-5-butyl-pyrazol-3-ol, 2-Methyl-4-isopropyl-5-isobutyl-pyrazol-3-ol, 2-Ethyl-4-isopropyl-5-isobutyl-pyrazol-3-ol, 2-Propyl-4-isopropyl-5-isobutyl-pyrazol-3-ol, 2-Isopropyl-4-isopropyl-5-isobutyl-pyrazol-3-ol, 2-Butyl-4-isopropyl-5-isobutyl-pyrazol-3-ol, 2-Isobutyl-4-isopropyl-5-isobutyl-pyrazol-3-ol, 2-tert-Butyl-4-isopropyl-5-isobutyl-pyrazol-3-ol, 2-Phenyl-4-isopropyl-5-isobutyl-pyrazol-3-ol, 2-(4-tolyl)-4-isopropyl-5-isobutyl-pyrazol-3-ol, 2-(2-tolyl)-4-isopropyl-5-isobutyl-pyrazol-3-ol, 2-(3-tolyl)-4-isopropyl-5-isobutyl-pyrazol-3-ol, 2-(3,4-Dimethylphenyl)-4-isopropyl-5-isobutyl-pyrazol-3-ol, 2-Methyl-4-isopropyl-5-tert-butyl-pyrazol-3-ol, 2-Ethyl-4-isopropyl-5-tert-butyl-pyrazol-3-ol, 2-Propyl-4-isopropyl-5-tert-butyl-pyrazol-3-ol, 2-Isopropyl-4-isopropyl-5-tert-butyl-pyrazol-3-ol, 2-Butyl-4-isopropyl-5-tert-butyl-pyrazol-3-ol, 2-Isobutyl-4-isopropyl-5-tert-butyl-pyrazol-3-ol, 2-tert-Butyl-4-isopropyl-5-tert-butyl-pyrazol-3-ol, 2-Phenyl-4-isopropyl-5-tert-butyl-pyrazol-3-ol, 2-(4-tolyl)-4-isopropyl-5-tert-butyl-pyrazol-3-ol,2-(2-tolyl)-4-isopropyl-5-tert-butyl-pyrazol-3-ol, 2-(3-tolyl)-4-isopropyl-5-tert-butyl-pyrazol-3-ol,2-(3,4-Dimethylphenyl)-4-isopropyl-5-phenyl-pyrazol-3-ol, 2-Methyl-4-isopropyl-5-phenyl-pyrazol-3-ol, 2-Ethyl-4-isopropyl-5-phenyl-pyrazol-3-ol, 2-Propyl-4-isopropyl-5-phenyl-pyrazol-3-ol, 2-Isopropyl-4-isopropyl-5-phenyl-pyrazol-3-ol, 2-Butyl-4-isopropyl-5-phenyl-pyrazol-3-ol, 2-Isobutyl-4-isopropyl-5-phenyl-pyrazol-3-ol, 2-tert-Butyl-4-isopropyl-5-phenyl -pyrazol-3-ol, 2-Phenyl-4-isopropyl-5-phenyl-pyrazol-3-ol, 2-(4-tolyl)-4-isopropyl-5-phenyl-pyrazol-3-ol, 2-(2-tolyl)-4-isopropyl-5-phenyl-pyrazol-3-ol, 2-(3-tolyl)-4-isopropyl-5-phenyl-pyrazol-3-ol, 2-(3,4-Dimethylphenyl)-4-isopropyl-5-(2-tolyl)-pyrazol-3-ol, 2-Methyl-4-isopropyl-5-(2-tolyl)-pyrazol-3-ol, 2-Ethyl-4-isopropyl-5-(2-tolyl)-pyrazol-3-ol, 2-Propyl-4-isopropyl-5-(2-tolyl)-pyrazol-3-ol, 2-Isopropyl-4-isopropyl-5-(2-tolyl)-pyrazol-3-ol, 2-Butyl-4-isopropyl-5-(2-tolyl)-pyrazol-3-ol, 2-Isobutyl-4-isopropyl-5-(2-tolyl)-pyrazol-3-ol, 2-tert-Butyl-4-isopropyl-5-(2-tolyl)-pyrazol-3-ol, 2-Phenyl-4-isopropyl-5-(2-tolyl)-pyrazol-3-ol, 2-(4-tolyl)-4-isopropyl-5-(2-tolyl)-pyrazol-3-ol, 2-(2-tolyl)-4-isopropyl-5-(2-tolyl)-pyrazol-3-ol, 2-(3-tolyl)-4-isopropyl-5-(2-tolyl)-pyrazol-3-ol, 2-(3,4-Dimethylphenyl)-4-isopropyl-5-(3-tolyl)-pyrazol-3-ol, 2-Methyl-4-isopropyl-5-(3-tolyl)-pyrazol-3-ol, 2-Ethyl-4-isopropyl-5-(3-tolyl)-pyrazol-3-ol, 2-Propyl-4-isopropyl-5-(3-tolyl)-pyrazol-3-ol, 2-Isopropyl-4-isopropyl-5-(3-tolyl) -pyrazol-3-ol, 2-Butyl-4-isopropyl-5-(3-tolyl)-pyrazol-3-ol, 2-Isobutyl-4-isopropyl-5-(3-tolyl)-pyrazol-3-ol, 2-tert-Butyl-4-isopropyl-5-(3-tolyl)-pyrazol-3-ol, 2-Phenyl-4-isopropyl-5-(3-tolyl) -pyrazol-3-ol, 2-(4-tolyl)-4-isopropyl-5-(3-tolyl)-pyrazol-3-ol, 2-(2-tolyl)-4-isopropyl-5-(3-tolyl) -pyrazol-3-ol, 2-(3-tolyl)-4-isopropyl-5-(3-tolyl)-pyrazol-3-ol, 2-(3,4-Dimethylphenyl)-4-isopropyl-5-(3-tolyl)-pyrazol-3-ol, 2-Methyl-4-isopropyl-5-(3-tolyl)-pyrazol-3-ol, 2-Ethyl-4-isopropyl-5-(3-tolyl)-pyrazol-3-ol, 2-Propyl-4-isopropyl-5-(3-tolyl)-pyrazol-3-ol, 2-Isopropyl-4-isopropyl-5-(3-tolyl)-pyrazol-3-ol, 2-Butyl-4-isopropyl-5-(3-tolyl)-pyrazol-3-ol, 2-Isobutyl-4-isopropyl-5-(3-tolyl)-pyrazol-3-ol, 2-tert-Butyl-4-isopropyl-5-butyl-pyrazol-3-ol, 2-Phenyl-4-isopropyl-5-(3-tolyl)-pyrazol-3-ol, 2-(4-tolyl)-4-isopropyl-5-(3-tolyl)-pyrazol-3-ol, 2-(2-tolyl)-4-isopropyl-5-(3-tolyl)-pyrazol-3-ol, 2-(3-tolyl)-4-isopropyl-5-(3-tolyl)-pyrazol-3-ol, 2-(3,4-Dimethylphenyl)-4-isopropyl-5-butyl-pyrazol-3-ol, 2-Methyl-4-isopropyl-5-(4-tolyl)-pyrazol-3-ol, 2-Ethyl-4-isopropyl-5-(4-tolyl)-pyrazol-3-ol, 2-Propyl-4-isopropyl-5-(4-tolyl)-pyrazol-3-ol, 2-Isopropyl-4-isopropyl-5-(4-tolyl)-pyrazol-3-ol, 2-Butyl-4-isopropyl-5-(4-tolyl)-pyrazol-3-ol, 2-Isobutyl-4-isopropyl-5-(4-tolyl)-pyrazol-3-ol, 2-tert-Butyl-4-isopropyl-5-(4-tolyl)-pyrazol-3-ol, 2-Phenyl-4-isopropyl-5-(4-tolyl)-pyrazol-3-ol, 2-(4-tolyl)-4-isopropyl-5-(4-tolyl)-pyrazol-3-ol, 2-(2-tolyl)-4-isopropyl-5-(4-tolyl)-pyrazol-3-ol, 2-(3-tolyl)-4-isopropyl-5-(4-tolyl) -pyrazol-3-ol, 2-(3,4-Dimethylphenyl)-4-isopropyl-5-(4-tolyl)-pyrazol-3-ol,
2-Methyl-4-butyl-5-phenyl-pyrazol-3-ol, 2-Ethyl-4-butyl5-phenyl-pyrazol-3-ol, 2-Propyl-4-butyl-5-phenyl-pyrazol-3-ol, 2-Isopropyl-4-butyl-5-phenyl-pyrazol-3-ol, 2-Butyl-4-butyl-5-phenyl-pyrazol-3-ol, 2-Isobutyl-4-butyl-5-phenyl-pyrazol-3-ol, 2-tert-Butyl-4-butyl-5-phenyl-pyrazol-3-ol, 2-Phenyl-4-butyl-5-phenyl-pyrazol-3-ol,2-(4-tolyl)-4-butyl-5-phenyl-pyrazol-3-ol, 2-(2-tolyl)-4-butyl-5-phenyl-pyrazol-3-ol, 2-(3-tolyl)-4-butyl-5-phenyl-pyrazol-3-ol,2-(3,4-Dimethylphenyl)-4-butyl-5-phenyl-pyrazol-3-ol, 2-Methyl-4-butyl-5-methyl-pyrazol-3-ol,2-Ethyl-4-butyl-5-methyl-pyrazol-3-ol, 2-Propyl-4-butyl-5-methyl-pyrazol-3-ol, 2-Isopropyl-4-butyl-5-methyl-pyrazol-3-ol, 2-Butyl-4-butyl-5-methyl-pyrazol-3-ol, 2-Isobutyl-4-butyl-5-methyl-pyrazol-3-ol, 2-tert-Butyl-4-butyl-5-methyl-pyrazol-3-ol, 2-Phenyl-4-butyl-5-methyl-pyrazol-3-ol, 2-(4-tolyl)-4-butyl-5-methyl-pyrazol-3-ol,2-(2-tolyl)-4-butyl-5-methyl-pyrazol-3-ol, 2-(3-tolyl)-4-butyl-5-methyl-pyrazol-3-ol, 2-(3,4-Dimethylphenyl)-4-butyl-5-methyl-pyrazol-3-ol, 2-methyl-4-butyl-5-ethyl-pyrazol-3-ol, 2-ethyl-4-butyl-5-ethyl-pyrazol-3-ol,2-propyl-4-butyl-5-ethyl-pyrazol-3-ol, 2-isopropyl-4-butyl-5-ethyl-pyrazol-3-ol, 2-butyl-4-butyl-5-ethyl-pyrazol-3-ol, 2-Isobutyl-4-butyl-5-ethyl-pyrazol-3-ol, 2-tert-butyl-4-butyl-5-ethyl-pyrazol-3-ol, 2-Phenyl-4-butyl-5-ethyl-pyrazol-3-ol, 2-(4-tolyl)-4-butyl-5-ethyl-pyrazol-3-ol, 2-(2-tolyl)-4-butyl-5-ethyl-pyrazol-3-ol, 2-(3-tolyl)-4-butyl-5-ethyl-pyrazol-3-ol, 2-(3,4-Dimethylphenyl)-4-butyl-5-ethyl-pyrazol-3-ol, 2-Methyl-4-butyl-5-propyl-pyrazol-3-ol, 2-Ethyl-4-butyl-5-propyl -pyrazol-3-ol, 2-Propyl-4-butyl-5-propyl-pyrazol-3-ol, 2-Isopropyl-4-butyl-5-methyl-pyrazol-3-ol, 2-Butyl-4-butyl-5-propyl-pyrazol-3-ol, 2-Isobutyl-4-butyl-5-propyl-pyrazol-3-ol, 2-tert-Butyl-4-butyl-5-propyl-pyrazol-3-ol, 2-Phenyl-4-butyl-5-propyl-pyrazol-3-ol, 2-(4-tolyl)-4-butyl-5-propyl-pyrazol-3-ol, 2-(2-tolyl)-4-butyl-5-propyl-pyrazol-3-ol, 2-(3-tolyl)-4-butyl-5-propyl-pyrazol-3-ol, 2-(3,4-Dimethylphenyl)-4-butyl-5-propyl-pyrazol-3-ol, 2-Methyl-4-butyl-5-isopropyl-pyrazol-3-ol, 2-Ethyl-4-butyl-5-isopropyl-pyrazol-3-ol, 2-Propyl-4-butyl-5-isopro-pyl-pyrazol-3-ol, 2-Isopropyl-4-butyl-5-isopropyl-pyrazol-3-ol, 2-Butyl-4-butyl-5-isopropyl-pyrazol-3-ol, 2-Isobutyl-4-butyl-5-isopropyl-pyrazol-3-ol, 2-tert-Butyl-4-butyl-5-isopropyl-pyrazol-3-ol, 2-Phenyl-4-butyl-5-isopropyl-pyrazol-3-ol, 2-(4-tolyl)-4-butyl-5-isopropyl-pyrazol-3-ol, 2-(2-tolyl)-4-butyl-5-isopropyl-pyrazol-3-ol, 2-(3-tolyl)-4-butyl-5-isopropyl-pyrazol-3-ol, 2-(3,4-Dimethylphenyl)-4-butyl-5-isopropyl-pyrazol-3-ol, 2-Methyl-4-butyl-5-butyl-pyrazol-3-ol, 2-Ethyl-4-butyl-5-butyl-pyrazol-3-ol, 2-Propyl-4-butyl-5-butyl-pyrazol-3-ol, 2-Isopropyl-4-butyl-5-butyl-pyrazol-3-ol, 2-Butyl-4-butyl-5-butyl-pyrazol-3-ol, 2-Isobutyl-4-butyl-5-butyl-pyrazol-3-ol, 2-tert-Butyl-4-butyl-5-butyl-pyrazol-3-ol, 2-Phenyl-4-butyl-5-butyl -pyrazol-3-ol, 2-(4-tolyl)-4-butyl-5-butyl-pyrazol-3-ol, 2-(2-tolyl)-4-butyl-5-butyl-pyrazol-3-ol, 2-(3-tolyl)-4-butyl-5-butyl-pyrazol-3-ol, 2-(3,4-Dimethylphenyl)-4-butyl-5-butyl-pyrazol-3-ol, 2-Methyl-4-butyl-5-isobutyl-pyrazol-3-ol, 2-Ethyl-4-butyl-5-isobutyl-pyrazol-3-ol, 2-Propyl-4-butyl-5-isobutyl-pyrazol-3-ol, 2-Isopropyl-4-butyl-5-isobutyl-pyrazol-3-ol, 2-Butyl-4-butyl-5-isobutyl-pyrazol-3-ol, 2-Isobutyl-4-butyl-5-isobutyl-pyrazol-3-ol, 2-tert-Butyl-4-butyl-5-isobutyl-pyrazol-3-ol, 2-Phenyl-4-butyl-5-isobutyl-pyrazol-3-ol, 2-(4-tolyl)-4-butyl-5-isobutyl -pyrazol-3-ol, 2-(2-tolyl)-4-butyl-5-isobutyl-pyrazol-3-ol, 2-(3-tolyl)-4-butyl-5-isobutyl-pyrazol-3-ol, 2-(3,4-Dimethylphenyl)-4-butyl-5-isobutyl-pyrazol-3-ol, 2-Methyl-4-butyl-5-tert-butyl-pyrazol-3-ol,, 2-Ethyl-4-butyl-5-tert-butyl-pyrazol-3-ol, 2-Propyl-4-butyl-5-tert-butyl-pyrazol-3-ol, 2-Isopropyl-4-butyl-5-tert-butyl-pyrazol-3-ol, 2-Butyl-4-butyl-5-tert-butyl-pyrazol-3-ol, 2-Isobutyl-4-butyl-5-tert-butyl-pyrazol-3-ol, 2-tert-Butyl-4-butyl-5-tert-butyl-pyrazol-3-ol, 2-Phenyl-4-butyl-5-tert-butyl-pyrazol-3-ol, 2-(4-tolyl)-4-butyl-5-tert-butyl-pyrazol-3-ol, 2-(2-tolyl)-4-butyl-5-tert-butyl-pyrazol-3-ol, 2-(3-tolyl)-4-butyl-5-tert-butyl-pyrazol-3-ol, 2-(3,4-Dimethylphenyl)-4-butyl-5-phenyl-pyrazol-3-ol, 2-Methyl-4-butyl-5-phenyl -pyrazol-3-ol,, 2-Ethyl-4-butyl-5-phenyl-pyrazol-3-ol, 2-Propyl-4-butyl-5-phenyl-pyrazol-3-ol, 2-Isopropyl-4-butyl-5-phenyl-pyrazol-3-ol, 2-Butyl-4-butyl-5-phenyl-pyrazol-3-ol, 2-Isobutyl-4-butyl-5-phenyl-pyrazol-3-ol, 2-tert-Butyl-4-butyl-5-phenyl-pyrazol-3-ol, 2-Phenyl-4-butyl-5-phenyl-pyrazol-3-ol, 2-(4-tolyl)-4-butyl-5-phenyl-pyrazol-3-ol, 2-(2-tolyl)-4-butyl-5-phenyl-pyrazol-3-ol, 2-(3-tolyl)-4-butyl-5-phenyl-pyrazol-3-ol, 2-(3,4-Dimethylphenyl)-4-butyl-5-(2-tolyl)-pyrazol-3-ol, 2-Methyl-4-butyl-5-(2-tolyl)-pyrazol-3-ol,, 2-Ethyl-4-butyl-5-(2-tolyl)-pyrazol-3-ol, 2-Propyl-4-butyl-5-(2-tolyl)-pyrazol-3-ol, 2-Isopropyl-4-butyl-5-(2-tolyl)-pyrazol-3-ol, 2-Butyl-4-butyl-5-(2-tolyl)-pyrazol-3-ol, 2-Isobutyl-4-butyl-5-(2-tolyl)-pyrazol-3-ol, 2-tert-Butyl-4-butyl-5-(2-tolyl)-pyrazol-3-ol, 2-Phenyl-4-butyl-5-(2-tolyl)-pyrazol-3-ol, 2-(4-tolyl)-4-butyl-5-(2-tolyl)-pyrazol-3-ol, 2-(2-tolyl)-4-butyl-5-(2-tolyl)-pyrazol-3-ol, 2-(3-tolyl)-4-butyl-5-(2-tolyl)-pyrazol-3-ol, 2-(3,4-Dimethylphenyl)-4-butyl-5-(3-tolyl)-pyrazol-3-ol, 2-Methyl-4-butyl-5-(3-tolyl)-pyrazol-3-ol,, 2-Ethyl-4-butyl-5-(3-tolyl)-pyrazol-3-ol, 2-Propyl-4-butyl-5-(3-tolyl)-pyrazol-3-ol, 2-Isopropyl-4-butyl-5-(3-tolyl)-pyrazol-3-ol, 2-Butyl-4-butyl-5-(3-tolyl)-pyrazol-3-ol, 2-Isobutyl-4-butyl-5-(3-tolyl)-pyrazol-3-ol, 2-tert-Butyl-4-butyl-5-(3-tolyl)-pyrazol-3-ol, 2-Phenyl-4-butyl-5-(3-tolyl)-pyrazol-3-ol, 2-(4-tolyl)-4-butyl-5-(3-tolyl)-pyrazol-3-ol, 2-(2-tolyl)-4-butyl-5-(3-tolyl)-pyrazol-3-ol, 2-(3-tolyl)-4-butyl-5-(3-tolyl)-pyrazol-3-ol, 2-(3,4-Dimethylphenyl)-4-butyl-5-(3-tolyl)-pyrazol-3-ol, 2-Methyl-4-butyl-5-(3-tolyl)-pyrazol-3-ol,, 2-Ethyl-4-butyl-5-(3-tolyl)-pyrazol-3-ol, 2-Propyl-4-butyl-5-(3-tolyl)-pyrazol-3-ol, 2-Isopropyl-4-butyl-5-(3-tolyl)-pyrazol-3-ol, 2-Butyl-4-butyl-5-(3-tolyl)-pyrazol-3-ol, 2-Isobutyl-4-butyl-5-(3-tolyl)-pyrazol-3-ol, 2-tert-Butyl-4-butyl-5-butyl-pyrazol-3-ol, 2-Phenyl-4-butyl-5-(3-tolyl)-pyrazol-3-ol, 2-(4-tolyl)-4-butyl-5-(3-tolyl)-pyrazol-3-ol, 2-(2-tolyl)-4-butyl-5-(3-tolyl)-pyrazol-3-ol, 2-(3-tolyl)-4-butyl-5-(3-tolyl)-pyrazol-3-ol, 2-(3,4-Dimethylphenyl)-4-butyl-5-butyl-pyrazol-3-ol, 2-Methyl-4-butyl-5-(4-tolyl)-pyrazol-3-ol, 2-Ethyl-4-butyl-5-(4-tolyl)-pyrazol-3-ol, 2-Propyl-4-butyl-5-(4-tolyl)-pyrazol-3-ol, 2-Isopropyl-4-butyl-5-(4-tolyl)-pyrazol-3-ol, 2-Butyl-4-butyl-5-(4-tolyl)-pyrazol-3-ol, 2-Isobutyl-4-butyl-5-(4-tolyl)-pyrazol-3-ol, 2-tert-Butyl-4-butyl-5-(4-tolyl)-pyrazol-3-ol, 2-Phenyl-4-butyl-5-(4-tolyl)-pyrazol-3-ol, 2-(4-tolyl)-4-butyl-5-(4-tolyl)-pyrazol-3-ol, 2-(2-tolyl)-4-butyl-5-(4-tolyl)-pyrazol-3-ol, 2-(3-tolyl)-4-butyl-5-(4-tolyl)-pyrazol-3-ol, 2-(3,4-Dimethylphenyl)-4-butyl-5-(4-tolyl)-pyrazol-3-ol, 2-Methyl-4-isobutyl-5-phenyl-pyrazol-3-ol, , 2-Ethyl-4-isobutyl5-phenyl-pyrazol-3-ol, 2-Propyl-4-isobutyl-5-phenyl-pyrazol-3-ol, 2-Isopropyl-4-isobutyl-5-phenyl-pyrazol-3-ol, 2-Butyl-4-isobutyl-5-phenyl-pyrazol-3-ol, 2-Isobutyl- 4-isobutyl-5-phenyl-pyrazol-3-ol, 2-tert-Butyl-4-isobutyl-5-phenyl-pyrazol-3-ol, 2-Phenyl-4-isobutyl-5-phenyl-pyrazol-3-ol, 2-(4-tolyl)-4-isobutyl-5-phenyl-pyrazol-3-ol, 2-(2-tolyl)-4-isobutyl-5-phenyl-pyrazol-3-ol, 2-(3-tolyl)-4-isobutyl-5-phenyl-pyrazol-3-ol, 2-(3,4-Dimethylphenyl)-4-isobutyl-5-phenyl-pyrazol-3-ol, 2-Methyl-4-isobutyl-5-methyl-pyrazol-3-ol, , 2-Ethyl-4-isobutyl-5-methyl-pyrazol-3-ol, 2-Propyl-4-isobutyl-5-methyl-pyrazol-3-ol, 2-Isopropyl-4-isobutyl-5-methyl-pyrazol-3-ol, 2-Butyl-4-isobutyl-5-methyl-pyrazol-3-ol, 2-Isobutyl-4-isobutyl-5-methyl-pyrazol-3-ol, 2-tert-Butyl-4-isobutyl-5-methyl-pyrazol-3-ol, 2-Phenyl-4-isobutyl-5-methyl-pyrazol-3-ol, 2-(4-tolyl)-4-isobutyl-5-methyl-pyrazol-3-ol, 2-(2-tolyl)-4-isobutyl-5-methyl-pyrazol-3-ol, 2-(3-tolyl)-4-isobutyl-5-methyl-pyrazol-3-ol, 2-(3,4-Dimethylphenyl)-4-isobutyl-5-methyl-pyrazol-3-ol,2-methyl-4-isobutyl-5-ethyl-pyrazol-3-ol,, 2-ethyl-4-isobutyl-5-ethyl-pyrazol-3-ol, 2-propyl-4-isobutyl-5-ethyl-pyrazol-3-ol, 2-isopropyl-4-isobutyl-5-ethyl-pyrazol-3-ol, 2-butyl-4-isobutyl-5-ethyl-pyrazol-3-ol, 2-Isobutyl-4-isobutyl-5-ethyl-pyrazol-3-ol, 2-tert-butyl-4-isobutyl-5-ethyl-pyrazol-3-ol, 2-Phenyl-4-isobutyl-5-ethyl-pyrazol-3-ol, 2-(4-tolyl)-4-isobutyl-5-ethyl-pyrazol-3-ol, 2-(2-tolyl)-4-isobutyl-5-ethyl-pyrazol-3-ol, 2-(3-tolyl)-4-isobutyl-5-ethyl-pyrazol-3-ol,2-(3,4-Dimethylphenyl)-4-isobutyl-5-ethyl-pyrazol-3-ol, 2-Methyl-4-isobutyl-5-propyl-pyrazol-3-ol,, 2-Ethyl-4-isobutyl-5-propyl-pyrazol-3-ol, 2-Propyl-4-isobutyl-5-propyl-pyrazol-3-ol, 2-Isopropyl-4-isobutyl-5-methyl-pyrazol-3-ol, 2-Butyl-4-isobutyl-5-propyl-pyrazol-3-ol, 2-Isobutyl-4-isobutyl-5-propyl-pyrazol-3-ol, 2-tert-Butyl-4-isobutyl-5-propyl-pyrazol-3-ol, 2-Phenyl-4-isobutyl-5-propyl-pyrazol-3-ol, 2-(4-tolyl)-4-isobutyl-5-propyl-pyrazol-3-ol, 2-(2-tolyl)-4-isobutyl-5-propyl-pyrazol-3-ol, 2-(3-tolyl)-4-isobutyl-5-propyl-pyrazol-3-ol, 2-(3,4-Dimethylphenyl)-4-isobutyl-5-propyl-pyrazol-3-ol, 2-Methyl-4-isobutyl-5-isopropyl-pyrazol-3-ol,, 2-Ethyl-4-isobutyl-5-isopropyl-pyrazol-3-ol, 2-Propyl-4-isobutyl-5-isopropyl-pyrazol-3-ol, 2-Isopropyl-4-isobutyl-5-isopropyl-pyrazol-3-ol, 2-Butyl-4-isobutyl-5-isopropyl-pyrazol-3-ol, 2-Isobutyl-4-isobutyl-5-isopropyl-pyrazol-3-ol, 2-tert-Butyl-4-isobutyl-5-isopropyl-pyrazol-3-ol, 2-Phenyl-4-isobutyl-5-isopropyl-pyrazol-3-ol, 2-(4-tolyl)-4-isobutyl-5-isopropyl-pyrazol-3-ol, 2-(2-tolyl)-4-isobutyl-5-isopropyl-pyrazol-3-ol, 2-(3-tolyl)-4-isobutyl-5-isopropyl-pyrazol-3-ol, 2-(3,4-Dimethylphenyl)-4-isobutyl-5-isopropyl-pyrazol-3-ol, 2-Methyl-4-isobutyl-5-butyl-pyrazol-3-ol,, 2-Ethyl-4-isobutyl-5-butyl-pyrazol-3-ol, 2-Propyl-4-isobutyl-5-butyl-pyrazol-3-ol, 2-Isopropyl-4-isobutyl-5-butyl-pyrazol-3-ol, 2-Butyl-4-isobutyl-5-butyl-pyrazol-3-ol, 2-Isobutyl-4-isobutyl-5-butyl-pyrazol-3-ol, 2-tert-Butyl-4-isobutyl-5-butyl-pyrazol-3-ol, 2-Phenyl-4-isobutyl-5-butyl-pyrazol-3-ol, 2-(4-tolyl)-4-isobutyl-5-butyl-pyrazol-3-ol, 2-(2-tolyl)-4-isobutyl-5-butyl-pyrazol-3-ol, 2-(3-tolyl)-4-isobutyl-5-butyl -pyrazol-3-ol, 2-(3,4-Dimethylphenyl)-4-isobutyl-5-butyl-pyrazol-3-ol, 2-Methyl-4-isobutyl-5-isobutyl-pyrazol-3-ol,, 2-Ethyl-4-isobutyl-5-isobutyl-pyrazol-3-ol, 2-Propyl-4-isobutyl-5-isobutyl-pyrazol-3-ol, 2-Isopropyl-4-isobutyl-5-isobutyl-pyrazol-3-ol, 2-Butyl-4-isobutyl-5-isobutyl-pyrazol-3-ol, 2-Isobutyl-4-isobutyl-5-isobutyl-pyrazol-3-ol, 2-tert-Butyl-4-isobutyl-5-isobutyl-pyrazol-3-ol, 2-Phenyl-4-isobutyl-5-isobutyl-pyrazol-3-ol, 2-(4-tolyl)-4-isobutyl-5-isobutyl-pyrazol-3-ol, 2-(2-tolyl)-4-isobutyl-5-isobutyl-pyrazol-3-ol, 2-(3-tolyl)-4-isobutyl-5-isobutyl-pyrazol-3-ol, 2-(3,4-Dimethylphenyl)-4-isobutyl-5-isobutyl-pyrazol-3-ol, 2-Methyl-4-isobutyl-5-tert-butyl-pyrazol-3-ol,, 2-Ethyl-4-isobutyl-5-tert-butyl-pyrazol-3-ol, 2-Propyl-4-isobutyl-5-tert-butyl-pyrazol-3-ol, 2-Isopropyl-4-isobutyl-5-tert-butyl-pyrazol-3-ol, 2-Butyl-4-isobutyl-5-tert-butyl-pyrazol-3-ol, 2-Isobutyl-4-isobutyl-5-tert-butyl-pyrazol-3-ol,2-tert-Butyl-4-isobutyl-5-tert-butyl-pyrazol-3-ol, 2-Phenyl-4-isobutyl-5-tert-butyl-pyrazol-3-ol, 2-(4-tolyl)-4-isobutyl-5-tert-butyl-pyrazol-3-ol, 2-(2-tolyl)-4-isobutyl-5-tert-butyl-pyrazol-3-ol, 2-(3-tolyl)-4-isobutyl-5-tert-butyl-pyrazol-3-ol, 2-(3,4-Dimethylphenyl)-4-isobutyl-5-phenyl-pyrazol-3-ol, 2-Methyl-4-isobutyl-5-phenyl-pyrazol-3-ol,, 2-Ethyl-4-isobutyl-5-phenyl-pyrazol-3-ol, 2-Propyl-4-isobutyl-5-phenyl-pyrazol-3-ol, 2-Isopropyl-4-isobutyl-5-phenyl-pyrazol-3-ol, 2-Butyl-4-isobutyl-5-phenyl-pyrazol-3-ol, 2-Isobutyl-4-isobutyl-5-phenyl-pyrazol-3-ol, 2-tert-Butyl-4-isobutyl-5-phenyl-pyrazol-3-ol, 2-Phenyl-4-isobutyl-5-phenyl-pyrazol-3-ol, 2-(4-tolyl)-4-isobutyl-5-phenyl-pyrazol-3-ol, 2-(2-tolyl)-4-isobutyl-5-phenyl-pyrazol-3-ol, 2-(3-tolyl)-4-isobutyl-5-phenyl-pyrazol-3-ol, 2-(3,4-Dimethylphenyl)-4-isobutyl-5-(2-tolyl)-pyrazol-3-ol, 2-Methyl-4-isobutyl-5-(2-tolyl)-pyrazol-3-ol,, 2-Ethyl-4-isobutyl-5-(2-tolyl)-pyrazol-3-ol, 2-Propyl-4-isobutyl-5-(2-tolyl)-pyrazol-3-ol, 2-Isopropyl-4-isobutyl-5-(2-tolyl)-pyrazol-3-ol, 2-Butyl-4-isobutyl-5-(2-tolyl)-pyrazol-3-ol, 2-Isobutyl-4-isobutyl-5-(2-tolyl)-pyrazol-3-ol, 2-tert-Butyl-4-isobutyl-5-(2-tolyl)-pyrazol-3-ol, 2-Phenyl-4-isobutyl-5-(2-tolyl)-pyrazol-3-ol, 2-(4-tolyl)-4-isobutyl-5-(2-tolyl)-pyrazol-3-ol, 2-(2-tolyl)-4-isobutyl-5-(2-tolyl)-pyrazol-3-ol, 2-(3-tolyl)-4-isobutyl-5-(2-tolyl)-pyrazol-3-ol, 2-(3,4-Dimethylphenyl)-4-isobutyl-5-(3-tolyl)-pyrazol-3-ol, 2-Methyl-4-isobutyl-5-(3-tolyl)-pyrazol-3-ol,, 2-Ethyl-4-isobutyl-5-(3-tolyl)-pyrazol-3-ol, 2-Propyl-4-isobutyl-5-(3-tolyl)-pyrazol-3-ol, 2-Isopropyl-4-isobutyl-5-(3-tolyl)-pyrazol-3-ol, 2-Butyl-4-isobutyl-5-(3-tolyl)-pyrazol-3-ol, 2-Isobutyl-4-isobutyl-5-(3-tolyl)-pyrazol-3-ol, 2-tert-Butyl-4-isobutyl-5-(3-tolyl)-pyrazol-3-ol, 2-Phenyl-4-isobutyl-5-(3-tolyl)-pyrazol-3-ol, 2-(4-tolyl)-4-isobutyl-5-(3-tolyl)-pyrazol-3-ol, 2-(2-tolyl)-4-isobutyl-5-(3-tolyl)-pyrazol-3-ol, 2-(3-tolyl)-4-isobutyl-5-(3-tolyl)-pyrazol-3-ol, 2-(3,4-Dimethylphenyl)-4-isobutyl-5-(3-tolyl)-pyrazol-3-ol, 2-Methyl-4-isobutyl-5-(3-tolyl)-pyrazol-3-ol,, 2-Ethyl-4-isobutyl-5-(3-tolyl)-pyrazol-3-ol, 2-Propyl-4-isobutyl-5-(3-tolyl)-pyrazol-3-ol, 2-Isopropyl-4-isobutyl-5-(3-tolyl)-pyrazol-3-ol, 2-Butyl-4-isobutyl-5-(3-tolyl)-pyrazol-3-ol, 2-Isobutyl-4-isobutyl-5-(3-tolyl)-pyrazol-3-ol, 2-tert-Butyl-4-isobutyl-5-butyl-pyrazol-3-ol, 2-Phenyl-4-isobutyl-5-(3-tolyl)-pyrazol-3-ol,2-(4-tolyl)-4-isobutyl-5-(3-tolyl)-pyrazol-3-ol, 2-(2-tolyl)-4-isobutyl-5-(3-tolyl)-pyrazol-3-ol, 2-(3-tolyl)-4-isobutyl-5-(3-tolyl)-pyrazol-3-ol, 2-(3,4-Dimethylphenyl)-4-isobutyl-5-butyl-pyrazol-3-ol, 2-Methyl-4-isobutyl-5-(4-tolyl)-pyrazol-3-ol, ,2-Ethyl-4-isobutyl-5-(4-tolyl)-pyrazol-3-ol, 2-Propyl-4-isobutyl-5-(4-tolyl)-pyrazol-3-ol, 2-Isopropyl-4-isobutyl-5-(4-tolyl)-pyrazol-3-ol, 2-Butyl-4-isobutyl-5-(4-tolyl)-pyrazol-3-ol, 2-Isobutyl-4-isobutyl-5-(4-tolyl)-pyrazol-3-ol, 2-tert-Butyl-4-isobutyl-5-(4-tolyl)-pyrazol-3-ol, 2-Phenyl-4-isobutyl-5-(4-tolyl)-pyrazol-3-ol, 2-(4-tolyl)-4-isobutyl-5-(4-tolyl)-pyrazol-3-ol, 2-(2-tolyl)-4-isobutyl-5-(4-tolyl)-pyrazol-3-ol, 2-(3-tolyl)-4-isobutyl-5-(4-tolyl)-pyrazol-3-ol, 2-(3,4-Dimethylphenyl)-4-isobutyl-5-(4-tolyl)-pyrazol-3-ol, 2-Methyl-4-tert-butyl-5-phenyl-pyrazol-3-ol,,2-Ethyl-4-tert-butyl5-phenyl-pyrazol-3-ol, 2-Propyl-4-tert-butyl-5-phenyl-pyrazol-3-ol, 2-Isopropyl-4-tert-butyl-5-phenyl-pyrazol-3-ol, 2-Butyl-4-tert-butyl-5-phenyl-pyrazol-3-ol, 2-Isobutyl-4-tert-butyl-5-phenyl-pyrazol-3-ol, 2-tert-Butyl-4-tert-butyl-5-phenyl-pyrazol-3-ol, 2-Phenyl-4-tert-butyl-5-phenyl-pyrazol-3-ol, 2-(4-tolyl)-4-tert-butyl-5-phenyl-pyrazol-3-ol, 2-(2-tolyl)-4-tert-butyl-5-phenyl-pyrazol-3-ol, 2-(3-tolyl)-4-tert-butyl-5-phenyl-pyrazol-3-ol, 2-(3,4-Dimethylphenyl)-4-tert-butyl-5-phenyl-pyrazol-3-ol, 2-Methyl-4-tert-butyl-5-methyl-pyrazol-3-ol,, 2-Ethyl-4-tert-butyl-5-methyl-pyrazol-3-ol, 2-Propyl-4-tert-butyl-5-methyl-pyrazol-3-ol, 2-Isopropyl-4-tert-butyl-5-methyl-pyrazol-3-ol, 2-Butyl-4-tert-butyl-5-methyl-pyrazol-3-ol, 2-Isobutyl-4-tert-butyl-5-methyl-pyrazol-3-ol, 2-tert-Butyl-4-tert-butyl-5-methyl-pyrazol-3-ol, 2-Phenyl-4-tert-butyl-5-methyl-pyrazol-3-ol, 2-(4-tolyl)-4-tert-butyl-5-methyl-pyrazol-3-ol, 2-(2-tolyl)-4-tert-butyl-5-methyl-pyrazol-3-ol, 2-(3-tolyl)-4-tert-butyl-5-methyl-pyrazol-3-ol, 2-(3,4-Dimethylphenyl)-4-tert-butyl-5-methyl-pyrazol-3-ol, 2-methyl-4-tert-butyl-5-ethyl-pyrazol-3-ol, 2-ethyl-4-tert-butyl-5-ethyl-pyrazol-3-ol, 2-propyl-4-tert-butyl-5-ethyl-pyrazol-3-ol, 2-isopropyl-4-tert-butyl-5-ethyl-pyrazol-3-ol, 2-butyl-4-tert-butyl-5-ethyl-pyrazol-3-ol, 2-Isobutyl-4-tert-butyl-5-ethyl-pyrazol-3-ol, 2-tert-butyl-4-tert-butyl-5-ethyl-pyrazol-3-ol, 2-Phenyl-4-tert-butyl-5-ethyl-pyrazol-3-ol, 2-(4-tolyl)-4-tert-butyl-5-ethyl-pyrazol-3-ol, 2-(2-tolyl)-4-tert-butyl-5-ethyl-pyrazol-3-ol, 2-(3-tolyl)-4-tert-butyl-5-ethyl-pyrazol-3-ol, 2-(3,4-Dimethylphenyl)-4-tert-butyl-5-ethyl-pyrazol-3-ol, 2-Methyl-4-tert-butyl-5-propyl-pyrazol-3-ol,, 2-Ethyl-4-tert-butyl-5-propyl-pyrazol-3-ol, 2-Propyl-4-tert-butyl-5-propyl-pyrazol-3-ol, 2-Isopropyl-4-tert-butyl-5-methyl-pyrazol-3-ol, 2-Butyl-4-tert-butyl-5-propyl-pyrazol-3-ol, 2-Isobutyl-4-tert-butyl-5-pro-pyl-pyrazol-3-ol, 2-tert-Butyl-4-tert-butyl-5-propyl-pyrazol-3-ol, 2-Phenyl-4-tert-butyl-5-propyl -pyrazol-3-ol, 2-(4-tolyl)-4-tert-butyl-5-propy-pyrazol-3-ol, 2-(2-tolyl)-4-tert-butyl-5-propyl-pyrazol-3-ol, 2-(3-tolyl)-4-tert-butyl-5-propyl-pyrazol-3-ol, 2-(3,4-Dimethylphenyl)-4-tert-butyl-5-propyl-pyrazol-3-ol, 2-Methyl-4-tert-butyl-5-isopropyl-pyrazol-3-ol, 2-Ethyl-4-tert-butyl-5-isopropyl-pyrazol-3-ol, 2-Propyl-4-tert-butyl-5-isopropyl-pyrazol-3-ol, 2-Isopropyl-4-tert-butyl-5-isopropyl-pyrazol-3-ol, 2-Butyl-4-tert-butyl-5-isopropyl-pyrazol-3-ol,2-Isobutyl-4-tert-butyl-5-isopropyl-pyrazol-3-ol, 2-tert-Butyl-4-tert-butyl-5-isopropyl-pyrazol-3-ol, 2-Phenyl-4-tert-butyl-5-isopropyl-pyrazol-3-ol, 2-(4-tolyl)-4-tert-butyl-5-isopropyl-pyrazol-3-ol,2-(2-tolyl)-4-tert-butyl-5-isopropyl-pyrazol-3-ol, 2-(3-tolyl)-4-tert-butyl-5-isopropyl-pyrazol-3-ol, 2-(3,4-Dimethylphenyl)-4-tert-butyl-5-isopropyl-pyrazol-3-ol, 2-Methyl-4-tert-butyl-5-butyl-pyrazol-3-ol,, 2-Ethyl-4-tert-butyl-5-butyl-pyrazol-3-ol, 2-Propyl-4-tert-butyl-5-butyl-pyrazol-3-ol,2-Isopropyl-4-tert-butyl-5-butyl-pyrazol-3-ol, 2-Butyl-4-tert-butyl-5-butyl-pyrazol-3-ol, 2-Isobutyl-4-tert-butyl-5-butyl-pyrazol-3-ol, 2-tert-Butyl-4-tert-butyl-5-butyl-pyrazol-3-ol, 2-Phenyl-4-tert-butyl-5-butyl-pyrazol-3-ol, 2-(4-tolyl)-4-tert-butyl-5-butyl-pyrazol-3-ol, 2-(2-tolyl)-4-tert-butyl-5-butyl-pyrazol-3-ol, 2-(3-tolyl)-4-tert-butyl-5-butyl-pyrazol-3-ol, 2-(3,4-Dimethylphenyl)-4-tert-butyl-5-butyl-pyrazol-3-ol, 2-Methyl-4-tert-butyl-5-isobutyl-pyrazol-3-ol,, 2-Ethyl-4-tert-butyl-5-isobutyl-pyrazol-3-ol, 2-Propyl-4-tert-butyl-5-isobutyl-pyrazol-3-ol, 2-Isopropyl-4-tert-butyl-5-isobutyl-pyrazol-3-ol, 2-Butyl-4-tert-butyl-5-isobutyl-pyrazol-3-ol, 2-Isobutyl-4-tert-butyl-5-isobutyl-pyrazol-3-ol, 2-tert-Butyl-4-tert-butyl-5-isobutyl-pyrazol-3-ol, 2-Phenyl-4-tert-butyl-5-isobutyl-pyrazol-3-ol, 2-(4-tolyl)-4-tert-butyl-5-isobutyl-pyrazol-3-ol, 2-(2-tolyl)-4-tert-butyl-5-isobutyl-pyrazol-3-ol, 2-(3-tolyl)-4-tert-butyl-5-isobutyl-pyrazol-3-ol, 2-(3,4-Dimethylphenyl)-4-tert-butyl-5-isobutyl-pyrazol-3-ol, 2-Methyl-4-tert-butyl-5-tert-butyl-pyrazol-3-ol,, 2-Ethyl-4-tert-butyl-5-tert-butyl-pyrazol-3-ol, 2-Propyl-4-tert-butyl-5-tert-butyl-pyrazol-3-ol, 2-Isopropyl-4-tert-butyl-5-tert-butyl-pyrazol-3-ol, 2-Butyl-4-tert-butyl-5-tert-butyl-pyrazol-3-ol, 2-Isobutyl-4-tert-butyl-5-tert-butyl-pyrazol-3-ol, 2-tert-Butyl-4-tert-butyl-5-tert-butyl-pyrazol-3-ol, 2-Phenyl-4-tert-butyl-5-tert-butyl-pyrazol-3-ol, 2-(4-tolyl)-4-tert-butyl-5-tert-butyl-pyrazol-3-ol, 2-(2-tolyl)-4-tert-butyl-5-tert-butyl-pyrazol-3-ol, 2-(3-tolyl)-4-tert-butyl-5-tert-butyl-pyrazol-3-ol,
2-(3,4-Dimethylphenyl)-4-tert-butyl-5-phenyl-pyrazol-3-ol, 2-Methyl-4-tert-butyl-5-phenyl-pyrazol-3-ol,, 2-Ethyl-4-tert-butyl-5-phenyl-pyrazol-3-ol, 2-Propyl-4-tert-butyl-5-phenyl-pyrazol-3-ol, 2-Isopropyl-4-tert-butyl-5-phenyl-pyrazol-3-ol, 2-Butyl-4-tert-butyl-5-phenyl-pyrazol-3-ol, 2-Isobutyl-4-tert-butyl-5-phenyl-pyrazol-3-ol, 2-tert-Butyl-4-tert-butyl-5-phenyl-pyrazol-3-ol, 2-Phenyl-4-tert-butyl-5-phenyl-pyrazol-3-ol, 2-(4-tolyl)-4-tert-butyl-5-phenyl-pyrazol-3-ol, 2-(2-tolyl)-4-tert-butyl-5-phenyl-pyrazol-3-ol, 2-(3-tolyl)-4-tert-butyl-5-phenyl-pyrazol-3-ol, 2-(3,4-Dimethylphenyl)-4-tert-butyl-5-(2-tolyl)-pyrazol-3-ol, 2-Methyl-4-tert-butyl-5-(2-tolyl)-pyrazol-3-ol, 2-Ethyl-4-tert-butyl-5-(2-tolyl)-pyrazol-3-ol, 2-Propyl-4-tert-butyl-5-(2-tolyl)-pyrazol-3-ol, 2-Isopropyl-4-tert-butyl-5-(2-tolyl)-pyrazol-3-ol, 2-Butyl-4-tert-butyl-5-(2-tolyl)-pyrazol-3-ol, 2-Isobutyl-4-tert-butyl-5-(2-tolyl)-pyrazol-3-ol, 2-tert-Butyl-4-tert-butyl-5-(2-tolyl)-pyrazol-3-ol, 2-Phenyl-4-tert-butyl-5-(2-tolyl)-pyrazol-3-ol, 2-(4-tolyl)-4-tert-butyl-5-(2-tolyl)-pyrazol-3-ol, 2-(2-tolyl)-4-tert-butyl-5-(2-tolyl)-pyrazol-3-ol, 2-(3-tolyl)-4-tert-butyl-5-(2-tolyl)-pyrazol-3-ol, 2-(3,4-Dimethylphenyl)-4-tert-butyl-5-(3-tolyl)-pyrazol-3-ol, 2-Methyl-4-tert-butyl-5-(3-tolyl)-pyrazol-3-ol,, 2-Ethyl-4-tert-butyl-5-(3-tolyl)-pyrazol-3-ol,2-Propyl-4-tert-butyl-5-(3-tolyl)-pyrazol-3-ol, 2-Isopropyl-4-tert-butyl-5-(3-tolyl)-pyrazol-3-ol, 2-Butyl-4-tert-butyl-5-(3-tolyl)-pyrazol-3-ol, 2-Isobutyl-4-tert-butyl-5-(3-tolyl)-pyrazol-3-ol, 2-tert-Butyl-4-tert-butyl-5-(3-tolyl)-pyrazol-3-ol, 2-Phenyl-4-tert-butyl-5-(3-tolyl)-pyrazol-3-ol, 2-(4-tolyl)-4-tert-butyl-5-(3-tolyl)-pyrazol-3-ol, 2-(2-tolyl)-4-tert-butyl-5-(3-tolyl)-pyrazol-3-ol, 2-(3-tolyl)-4-tert-butyl-5-(3-tolyl)-pyrazol-3-ol, 2-(3,4-Dimethylphenyl)-4-tert-butyl-5-(3-tolyl)-pyrazol-3-ol, 2-Methyl-4-tert-butyl-5-(3-tolyl)-pyrazol-3-ol,, 2-Ethyl-4-tert-butyl-5-(3-tolyl)-pyrazol-3-ol, 2-Propyl-4-tert-butyl-5-(3-tolyl)-pyrazol-3-ol, 2-Isopropyl-4-tert-butyl-5-(3-tolyl) -pyrazol-3-ol, 2-Butyl-4-tert-butyl-5-(3-tolyl)-pyrazol-3-ol, 2-Isobutyl-4-tert-butyl-5-(3-tolyl)-pyrazol-3-ol, 2-tert-Butyl-4-tert-butyl-5-butyl-pyrazol-3-ol, 2-Phenyl-4-tert-butyl-5-(3-tolyl)-pyrazol-3-ol, 2-(4-tolyl)-4-tert-butyl-5-(3-tolyl)-pyrazol-3-ol, 2-(2-tolyl)-4-tert-butyl-5-(3-tolyl)-pyrazol-3-ol, 2-(3-tolyl)-4-tert-butyl-5-(3-tolyl)-pyrazol-3-ol, 2-(3,4-Dimethylphenyl)-4-tert-butyl-5-butyl-pyrazol-3-ol, 2-Methyl-4-tert-butyl-5-(4-tolyl)-pyrazol-3-ol,, 2-Ethyl-4-tert-butyl-5-(4-tolyl)-pyrazol-3-ol, 2-Propyl-4-tert-butyl-5-(4-tolyl)-pyrazol-3-ol, 2-Isopropyl-4-tert-butyl-5-(4-tolyl)-pyrazol-3-ol, 2-Butyl-4-tert-butyl-5-(4-tolyl)-pyrazol-3-ol, 2-Isobutyl-4-tert-butyl-5-(4-tolyl)-pyrazol-3-ol, 2-tert-Butyl-4-tert-butyl-5-(4-tolyl)-pyrazol-3-ol, 2-Phenyl-4-tert-butyl-5-(4-tolyl)-pyrazol-3-ol, 2-(4-tolyl)-4-tert-butyl-5-(4-tolyl)-pyrazol-3-ol, 2-(2-tolyl)-4-tert-butyl-5-(4-tolyl)-pyrazol-3-ol, 2-(3-tolyl)-4-tert-butyl-5-(4-tolyl)-pyrazol-3-ol, 2-(3,4-Dimethylphenyl)-4-tert-butyl-5-(4-tolyl)-pyrazol-3-ol, 2-Methyl-4-phenyl-5-phenyl-pyrazol-3-ol, 2-Ethyl-4-phenyl-5-phenyl-pyrazol-3-ol, 2-Propyl-4-phenyl-5-phenyl-pyrazol-3-ol, 2-Isopropyl-4-phenyl-5-phenyl-pyrazol-3-ol, 2-Butyl-4-phenyl-5-phenyl-pyrazol-3-ol, 2-Isobutyl-4-phenyl-5-phenyl-pyrazol-3-ol, 2-tert-Butyl-4-phenyl-5-phenyl-pyrazol-3-ol, 2-Phenyl-4-phenyl-5-phenyl-pyrazol-3-ol, 2-(4-tolyl)-4-phenyl-5-phenyl-pyrazol-3-ol, 2-(2-tolyl)-4-phenyl-5-phenyl-pyrazol-3-ol, 2-(3-tolyl)-4-phenyl-5-phenyl-pyrazol-3-ol, 2-(3,4-Dimethylphenyl)-4-phenyl-5-phenyl-pyrazol-3-ol, 2-Methyl-4-phenyl-5-methyl-pyrazol-3-ol,, 2-Ethyl-4-phenyl-5-methyl-pyrazol-3-ol, 2-Propyl-4-phenyl-5-methyl-pyrazol-3-ol, 2-Isopropyl-4-phenyl-5-methyl-pyrazol-3-ol, 2-Butyl-4-phenyl-5-methyl-pyrazol-3-ol, 2-Isobutyl-4-phenyl-5-methyl-pyrazol-3-ol, 2-tert-Butyl-4-phenyl-5-methyl-pyrazol-3-ol, 2-Phenyl-4-phenyl-5-methyl-pyrazol-3-ol, 2-(4-tolyl)-4-phenyl-5-methyl-pyrazol-3-ol, 2-(2-tolyl)-4-phenyl-5-methyl-pyrazol-3-ol, 2-(3-tolyl)-4-phenyl-5-methyl-pyrazol-3-ol, 2-(3,4-Dimethylphenyl)-4-phenyl-5-methyl-pyrazol-3-ol, 2-methyl-4-phenyl-5-ethyl-pyrazol-3-ol,, 2-ethyl-4-phenyl-5-ethyl-pyrazol-3-ol, 2-propyl-4-phenyl-5-ethyl-pyrazol-3-ol, 2-isopropyl-4-phenyl-5-ethyl-pyrazol-3-ol,2-butyl-4-phenyl-5-ethyl-pyrazol-3-ol, 2-Isobutyl-4-phenyl-5-ethyl-pyrazol-3-ol, 2-tert-butyl-4-phenyl-5-ethyl-pyrazol-3-ol, 2-Phenyl-4-phenyl-5-ethyl-pyrazol-3-ol, 2-(4-tolyl)-4-phenyl-5-ethyl-pyrazol-3-ol, 2-(2-tolyl)-4-phenyl-5-ethyl-pyrazol-3-ol, 2-(3-tolyl)-4-phenyl-5-ethyl-pyrazol-3-ol, 2-(3,4-Dimethylphenyl)-4-phenyl-5-ethyl-pyrazol-3-ol, 2-Methyl-4-phenyl-5-propyl-pyrazol-3-ol,, 2-Ethyl-4-phenyl-5-propyl-pyrazol-3-ol, 2-Propyl-4-phenyl-5-propyl-pyrazol-3-ol, 2-Isopropyl-4-phenyl-5-methyl-pyrazol-3-ol, 2-Butyl-4-phenyl-5-propyl-pyrazol-3-ol, 2-Isobutyl-4-phenyl-5-propyl-pyrazol-3-ol, 2-tert-Butyl-4-phenyl-5-propyl-pyrazol-3-ol, 2-Phenyl-4-phenyl-5-propyl-pyrazol-3-ol, 2-(4-tolyl)-4-phenyl-5-propyl-pyrazol-3-ol, 2-(2-tolyl)-4-phenyl-5-propyl-pyrazol-3-ol, 2-(3-tolyl)-4-phenyl-5-propyl-pyrazol-3-ol, 2-(3,4-Dimethylphenyl)-4-phenyl-5-propyl-pyrazol-3-ol, 2-Methyl-4-phenyl-5-isopropyl-pyrazol-3-ol,, 2-Ethyl-4-phenyl-5-isopropyl-pyrazol-3-ol, 2-Propyl-4-phenyl-5-isopropyl-pyrazol-3-ol, 2-Isopropyl-4-phenyl-5-isopropyl-pyrazol-3-ol, 2-Butyl-4-phenyl-5-isopropyl-pyrazol-3-ol, 2-Isobutyl-4-phenyl-5-isopropyl-pyrazol-3-ol, 2-tert-Butyl-4-phenyl-5-isopropyl-pyrazol-3-ol, 2-Phenyl-4-phenyl-5-isopropyl-pyrazol-3-ol, 2-(4-tolyl)-4-phenyl-5-isopropyl-pyrazol-3-ol, 2-(2-tolyl)-4-phenyl-5-isopropyl-pyrazol-3-ol, 2-(3-tolyl)-4-phenyl-5-isopropyl-pyrazol-3-ol, 2-(3,4-Dimethylphenyl)-4-phenyl-5-isopropyl-pyrazol-3-ol, 2-Methyl-4-phenyl-5-butyl-pyrazol-3-ol,, 2-Ethyl-4-phenyl-5-butyl-pyrazol-3-ol, 2-Propyl-4-phenyl-5-butyl-pyrazol-3-ol, 2-Isopropyl-4-phenyl-5-butyl-pyrazol-3-ol, 2-Butyl-4-phenyl-5-butyl-pyrazol-3-ol, 2-Isobutyl-4-phenyl-5-butyl-pyrazol-3-ol, 2-tert-Butyl-4-phenyl-5-butyl-pyrazol-3-ol, 2-Phenyl-4-phenyl-5-butyl-pyrazol-3-ol, 2-(4-tolyl)-4-phenyl-5-butyl-pyrazol-3-ol,2-(2-tolyl)-4-phenyl-5-butyl-pyrazol-3-ol, 2-(3-tolyl)-4-phenyl-5-butyl-pyrazol-3-ol, 2-(3,4-Dimethylphenyl)-4-phenyl-5-butyl-pyrazol-3-ol,2-Methyl-4-phenyl-5-isobutyl-pyrazol-3-ol, 2-Ethyl-4-phenyl-5-isobutyl-pyrazol-3-ol, 2-Propyl-4-phenyl-5-isobutyl-pyrazol-3-ol, 2-Isopropyl-4-phenyl-5-isobutyl-pyrazol-3-ol, 2-Butyl-4-phenyl-5-isobutyl-pyrazol-3-ol, 2-Isobutyl-4-phenyl-5-isobutyl-pyrazol-3-ol, 2-tert-Butyl-4-phenyl-5-isobutyl-pyrazol-3-ol,2-Phenyl-4-phenyl-5-isobutyl-pyrazol-3-ol, 2-(4-tolyl)-4-phenyl-5-isobutyl-pyrazol-3-ol, 2-(2-tolyl)-4-phenyl-5-isobutyl-pyrazol-3-ol, 2-(3-tolyl)-4-phenyl-5-isobutyl-pyrazol-3-ol, 2-(3,4-Dimethylphenyl)-4-phenyl-5-isobutyl-pyrazol-3-ol, 2-Methyl-4-phenyl-5-tert-butyl-pyrazol-3-ol,, 2-Ethyl-4-phenyl-5-tert-butyl-pyrazol-3-ol, 2-Propyl-4-phenyl-5-tert-butyl-pyrazol-3-ol, 2-Isopropyl-4-phenyl-5-tert-butyl-pyrazol-3-ol, 2-Butyl-4-phenyl-5-tert-butyl-pyrazol-3-ol, 2-Isobutyl-4-phenyl-5-tert-butyl-pyrazol-3-ol, 2-tert-Butyl-4-phenyl-5-tert-butyl-pyrazol-3-ol, 2-Phenyl-4-phenyl-5-tert-butyl-pyrazol-3-ol, 2-(4-tolyl)-4-phenyl-5-tert-butyl-pyrazol-3-ol, 2-(2-tolyl)-4-phenyl-5-tert-butyl-pyrazol-3-ol, 2-(3-tolyl)-4-phenyl-5-tert-butyl-pyrazol-3-ol, 2-(3,4-Dimethylphenyl)-4-phenyl-5-phenyl-pyrazol-3-ol, 2-Methyl-4-phenyl-5-phenyl-pyrazol-3-ol,, 2-Ethyl-4-phenyl-5-phenyl-pyrazol-3-ol, 2-Propyl-4-phenyl-5-phenyl-pyrazol-3-ol, 2-Isopropyl-4-phenyl-5-phenyl-pyrazol-3-ol, 2-Butyl-4-phenyl-5-phenyl-pyrazol-3-ol, 2-Isobutyl-4-phenyl-5-phenyl-pyrazol-3-ol, 2-tert-Butyl-4-phenyl-5-phenyl-pyrazol-3-ol, 2-Phenyl-4-phenyl-5-phenyl-pyrazol-3-ol, 2-(4-tolyl)-4-phenyl-5-phenyl-pyrazol-3-ol, 2-(2-tolyl)-4-phenyl-5-phenyl-pyrazol-3-ol, 2-(3-tolyl)-4-phenyl-5-phenyl-pyrazol-3-ol, 2-(3,4-Dimethylphenyl)-4-phenyl-5-(2-tolyl)-pyrazol-3-ol, 2-Methyl-4-phenyl-5-(2-tolyl)-pyrazol-3-ol, , 2-Ethyl-4-phenyl-5-(2-tolyl)-pyrazol-3-ol, 2-Propyl-4-phenyl-5-(2-tolyl)-pyrazol-3-ol, 2-Isopropyl-4-phenyl-5-(2-tolyl)-pyrazol-3-ol, 2-Butyl-4-phenyl-5-(2-tolyl)-pyrazol-3-ol, 2-Isobutyl- 4-phenyl-5-(2-tolyl)-pyrazol-3-ol, 2-tert-Butyl-4-phenyl-5-(2-tolyl)-pyrazol-3-ol, 2-Phenyl-4-phenyl-5-(2-tolyl)-pyrazol-3-ol, 2-(4-tolyl)-4-phenyl-5-(2-tolyl)-pyrazol-3-ol, 2-(2-tolyl)-4-phenyl-5-(2-tolyl)-pyrazol-3-ol, 2-(3-tolyl)-4-phenyl-5-(2-tolyl)-pyrazol-3-ol, 2-(3,4-Dimethylphenyl)-4-phenyl-5-(3-tolyl)-pyrazol-3-ol, 2-Methyl-4-phenyl-5-(3-tolyl)-pyrazol-3-ol, , 2-Ethyl-4-phenyl-5-(3-tolyl)-pyrazol-3-ol, 2-Propyl-4-phenyl-5-(3-tolyl)-pyrazol-3-ol, 2-Isopropyl-4-phenyl-5-(3-tolyl)-pyrazol-3-ol, 2-Butyl-4-phenyl-5-(3-tolyl)-pyrazol-3-ol, 2-Isobutyl- 4-phenyl-5-(3-tolyl)-pyrazol-3-ol, 2-tert-Butyl-4-phenyl-5-(3-tolyl)-pyrazol-3-ol, 2-Phenyl-4-phenyl-5-(3-tolyl)-pyrazol-3-ol, 2-(4-tolyl)-4-phenyl-5-(3-tolyl)-pyrazol-3-ol, 2-(2-tolyl)-4-phenyl-5-(3-tolyl)-pyrazol-3-ol, 2-(3-tolyl)-4-phenyl-5-(3-tolyl)-pyrazol-3-ol, 2-(3,4-Dimethylphenyl)-4-phenyl-5-(3-tolyl)-pyrazol-3-ol, 2-Methyl-4-phenyl-5-(3-tolyl)-pyrazol-3-ol, , 2-Ethyl-4-phenyl-5-(3-tolyl)-pyrazol-3-ol, 2-Propyl-4-phenyl-5-(3-tolyl)-pyrazol-3-ol, 2-Isopropyl-4-phenyl-5-(3-tolyl)-pyrazol-3-ol, 2-Butyl-4-phenyl-5-(3-tolyl)-pyrazol-3-ol, 2-Isobutyl-4-phenyl-5-(3-tolyl)-pyrazol-3-ol, 2-tert-Butyl-4-phenyl-5-butyl-pyrazol-3-ol, 2-Phenyl-4-phenyl-5-(3-tolyl)-pyrazol-3-ol, 2-(4-tolyl)-4-phenyl-5-(3-tolyl)-pyrazol-3-ol, 2-(2-tolyl)-4-phenyl-5-(3-tolyl)-pyrazol-3-ol, 2-(3-tolyl)-4-phenyl-5-(3-tolyl)-pyrazol-3-ol, 2-(3,4-Dimethylphenyl)-4-phenyl-5-butyl-pyrazol-3-ol, 2-Methyl-4-phenyl-5-(4-tolyl)-pyrazol-3-ol, 2-Ethyl-4-phenyl-5-(4-tolyl)-pyrazol-3-ol, 2-Propyl-4-phenyl-5-(4-tolyl)-pyrazol-3-ol, 2-Isopropyl-4-phenyl-5-(4-tolyl)-pyrazol-3-ol, 2-Butyl-4-phenyl-5-(4-tolyl)-pyrazol-3-ol, 2-Isobutyl- 4-phenyl-5-(4-tolyl)-pyrazol-3-ol, 2-tert-Butyl-4-phenyl-5-(4-tolyl)-pyrazol-3-ol, 2-Phenyl-4-phenyl-5-(4-tolyl)-pyrazol-3-ol, 2-(4-tolyl)-4-phenyl-5-(4-tolyl)-pyrazol-3-ol, 2-(2-tolyl)-4-phenyl-5-(4-tolyl)-pyrazol-3-ol, 2-(3-tolyl)-4-phenyl-5-(4-tolyl)-pyrazol-3-ol, 2-(3,4-Dimethylphenyl)-4-phenyl-5-(4-tolyl)-pyrazol-3-ol, 2-Methyl-4-(4-tolyl)-5-phenyl-pyrazol-3-ol, , 2-Ethyl-4-(4-tolyl)5-phenyl-pyrazol-3-ol, 2-Propyl-4-(4-tolyl)-5-phenyl-pyrazol-3-ol, 2-Isopropyl-4-(4-tolyl)-5-phenyl-pyrazol-3-ol, 2-Butyl-4-(4-tolyl)-5-phenyl-pyrazol-3-ol, 2-Isobutyl-4-(4-tolyl)-5-phenyl-pyrazol-3-ol, 2-tert-Butyl-4-(4-tolyl)-5-phenyl-pyrazol-3-ol, 2-Phenyl-4-(4-tolyl)-5-phenyl-pyrazol-3-ol, 2-(4-tolyl)-4-(4-tolyl)-5-phenyl-pyrazol-3-ol, 2-(2-tolyl)-4-(4-tolyl)-5-phenyl-pyrazol-3-ol, 2-(3-tolyl)-4-(4-tolyl)-5-phenyl-pyrazol-3-ol, 2-(3,4-Dimethylphenyl)-4-(4-tolyl)-5-phenyl-pyrazol-3-ol, 2-Methyl-4-(4-tolyl)-5-methyl-pyrazol-3-ol, 2-Ethyl-4-(4-tolyl)-5-methyl-pyrazol-3-ol, 2-propyl-4-(4-tolyl)-5-methyl-pyrazol-3-ol, 2-Isopropyl-4-(4-tolyl)-5-methyl-pyrazol-3-ol, 2-Butyl-4-(4-tolyl)-5-methyl-pyrazol-3-ol, 2-Isobutyl-4-(4-tolyl)-5-methyl-pyrazol-3-ol, 2-tert-Butyl-4-(4-tolyl)-5-methyl-pyrazol-3-ol, 2-Phenyl-4-(4-tolyl)-5-methyl-pyrazol-3-ol, 2-(4-tolyl)-4-(4-tolyl)-5-methyl-pyrazol-3-ol, 2-(2-tolyl)-4-(4-tolyl)-5-methyl-pyrazol-3-ol, 2-(3-tolyl)-4-(4-tolyl)-5-methyl-pyrazol-3-ol, 2-(3,4-Dimethylphenyl)-4-(4-tolyl)-5-methyl-pyrazol-3-ol, 2-methyl-4-(4-tolyl)-5-ethyl-pyrazol-3-ol, , 2-ethyl-4-(4-tolyl)-5-ethyl-pyrazol-3-ol, 2-propyl-4-(4-tolyl)-5-ethyl-pyrazol-3-ol, 2-isopropyl-4-(4-tolyl)-5-ethyl-pyrazol-3-ol, 2-butyl-4-(4-tolyl)-5-ethyl-pyrazol-3-ol, 2-Isobutyl-4-(4-tolyl)-5-ethyl-pyrazol-3-ol, 2-tert-butyl-4-(4-tolyl)-5-ethyl -pyrazol-3-ol, 2-Phenyl-4-(4-tolyl)-5-ethyl-pyrazol-3-ol, 2-(4-tolyl)-4-(4-tolyl)-5-ethyl-pyrazol-3-ol, 2-(2-tolyl)-4-(4-tolyl)-5-ethyl-pyrazol-3-ol, 2-(3-tolyl)-4-(4-tolyl)-5-ethyl-pyrazol-3-ol, 2-(3,4-Dimethylphenyl)-4-(4-tolyl)-5-ethyl-pyrazol-3-ol, 2-Methyl-4-(4-tolyl)-5-propyl-pyrazol-3-ol, 2-Ethyl-4-(4-tolyl)-5-propyl-pyrazol-3-ol, 2-Propyl-4-(4-tolyl)-5-propyl-pyrazol-3-ol, 2-Isopropyl-4-(4-tolyl)-5-methyl-pyrazol-3-ol, 2-Butyl-4-(4-tolyl)-5-propyl-pyrazol-3-ol, 2-Isobutyl- 4-(4-tolyl)-5-propyl-pyrazol-3-ol, 2-tert-Butyl-4-(4-tolyl)-5-propyl-pyrazol-3-ol, 2-Phenyl-4-(4-tolyl)-5-propyl-pyrazol-3-ol, 2-(4-tolyl)-4-(4-tolyl)-5-propyl-pyrazol-3-ol, 2-(2-tolyl)-4-(4-tolyl)-5-propyl-pyrazol-3-ol, 2-(3-tolyl)-4-(4-tolyl)-5-propyl-pyrazol-3-ol, 2-(3,4-Dimethylphenyl)-4-(4-tolyl)-5-propyl-pyrazol-3-ol, 2-Methyl-4-(4-tolyl)-5-isopropyl-pyrazol-3-ol, 2-Ethyl-4-(4-tolyl)-5-isopropyl-pyrazol-3-ol, 2-Propyl-4-(4-tolyl)-5-isopropyl-pyrazol-3-ol, 2-Isopropyl-4-(4-tolyl)-5-isopropyl-pyrazol-3-ol, 2-Butyl-4-(4-tolyl)-5-isopropyl-pyrazol-3-ol, 2-Isobutyl-4-(4-tolyl)-5-isopropyl-pyrazol-3-ol, 2-tert-Butyl-4-(4-tolyl)-5-isopropyl -pyrazol-3-ol, 2-Phenyl-4-(4-tolyl)-5-isopropyl-pyrazol-3-ol, 2-(4-tolyl)-4-(4-tolyl)-5-isopropyl -pyrazol-3-ol, 2-(2-tolyl)-4-(4-tolyl)-5-isopropyl-pyrazol-3-ol, 2-(3-tolyl)-4-(4-tolyl)-5-isopropyl -pyrazol-3-ol, 2-(3,4-Dimethylphenyl)-4-(4-tolyl)-5-isop-ropyl-pyrazol-3-ol, 2-Methyl-4-(4-tolyl)-5-butyl-pyrazol-3-ol, 2-Ethyl-4-(4-tolyl)-5-butyl-pyrazol-3-ol, 2-Propyl-4-(4-tolyl)-5-butyl-pyrazol-3-ol, 2-Isopropyl-4-(4-tolyl)-5-butyl-pyrazol-3-ol, 2-Butyl-4-(4-tolyl)-5-butyl-pyrazol-3-ol, 2-Isobutyl- 4-(4-tolyl)-5-butyl-pyrazol-3-ol, 2-tert-Butyl-4-(4-tolyl)-5-butyl-pyrazol-3-ol, 2-Phenyl-4-(4-tolyl)-5-butyl-pyrazol-3-ol, 2-(4-tolyl)-4-(4-tolyl)-5-butyl-pyrazol-3-ol, 2-(2-tolyl)-4-(4-tolyl)-5-butyl-pyrazol-3-ol, 2-(3-tolyl)-4-(4-tolyl)-5-butyl-pyrazol-3-ol, 2-(3,4-Dimethylphenyl)-4-(4-tolyl)-5-butyl-pyrazol-3-ol, 2-Methyl-4-(4-tolyl)-5-isobutyl-pyrazol-3-ol,2-Ethyl-4-(4-tolyl)-5-isobutyl-pyrazol-3-ol, 2-Propyl-4-(4-tolyl)-5-isobutyl-pyrazol-3-ol, 2-Isopropyl-4-(4-tolyl)-5-isobutyl-pyrazol-3-ol, 2-Butyl-4-(4-tolyl)-5-isobutyl-pyrazol-3-ol, 2-Isobutyl-4-(4-tolyl)-5-isobutyl-pyrazol-3-ol, 2-tert-Butyl-4-(4-tolyl)-5-isobutyl-pyrazol-3-ol, 2-Phenyl-4-(4-tolyl)-5-isobutyl-pyrazol-3-ol,
2-(4-tolyl)-4-(4-tolyl)-5-isobutyl-pyrazol-3-ol, 2-(2-tolyl)-4-(4-tolyl)-5-isobutyl-pyrazol-3-ol, 2-(3-tolyl)-4-(4-tolyl)-5-isobutyl-pyrazol-3-ol, 2-(3,4-Dimethylphenyl)-4-(4-tolyl)-5-isobutyl-pyrazol-3-ol, 2-Methyl-4-(4-tolyl)-5-tert-butyl-pyrazol-3-ol, 2-Ethyl-4-(4-tolyl)-5-tert-butyl-pyrazol-3-ol, 2-Propyl-4-(4-tolyl)-5-tert-butyl-pyrazol-3-ol, 2-Isopropyl-4-(4-tolyl)-5-tert-butyl -pyrazol-3-ol, 2-Butyl-4-(4-tolyl)-5-tert-butyl-pyrazol-3-ol, 2-Isobutyl-4-(4-tolyl)-5-tert-butyl-pyrazol-3-ol,
2-tert-Butyl-4-(4-tolyl)-5-tert-butyl -pyrazol-3-ol, 2-Phenyl-4-(4-tolyl)-5-tert-butyl-pyrazol-3-ol, 2-(4-tolyl)-4-(4-tolyl)-5-tert-butyl -pyrazol-3-ol, 2-(2-tolyl)-4-(4-tolyl)-5-tert-butyl-pyrazol-3-ol, 2-(3-tolyl)-4-(4-tolyl)-5-tert-butyl -pyrazol-3-ol, 2-(3,4-Dimethylphenyl)-4-(4-tolyl)-5-phenyl-pyrazol-3-ol, 2-Methyl-4-(4-tolyl)-5-phenyl-pyrazol-3-ol, 2-Ethyl-4-(4-tolyl)-5-phenyl-pyrazol-3-ol, 2-Propyl-4-(4-tolyl)-5-phenyl-pyrazol-3-ol, 2-Isopropyl-4-(4-tolyl)-5-phenyl-pyrazol-3-ol, 2-Butyl-4-(4-tolyl)-5-phenyl-pyrazol-3-ol, 2-Isobutyl-4-(4-tolyl)-5-phenyl-pyrazol-3-ol, 2-tert-Butyl-4-(4-tolyl)-5-phenyl-pyrazol-3-ol, 2-Phenyl-4-(4-tolyl)-5-phenyl-pyrazol-3-ol, 2-(4-tolyl)-4-(4-tolyl)-5-phenyl-pyrazol-3-ol, 2-(2-tolyl)-4-(4-tolyl)-5-phenyl -pyrazol-3-ol, 2-(3-tolyl)-4-(4-tolyl)-5-phenyl-pyrazol-3-ol, 2-(3,4-Dimethylphenyl)-4-(4-tolyl)-5-(2-tolyl)-pyrazol-3-ol, 2-Methyl-4-(4-tolyl)-5-(2-tolyl)-pyrazol-3-ol, 2-Ethyl-4-(4-tolyl)-5-(2-tolyl)-pyrazol-3-ol, 2-Propyl-4-(4-tolyl)-5-(2-tolyl)-pyrazol-3-ol, 2-Isopropyl-4-(4-tolyl)-5-(2-tolyl)-pyrazol-3-ol, 2-Butyl-4-(4-tolyl)-5-(2-tolyl)-pyrazol-3-ol,2-Isobutyl- 4-(4-tolyl)-5-(2-tolyl)-pyrazol-3-ol, 2-tert-Butyl-4-(4-tolyl)-5-(2-tolyl)-pyrazol-3-ol, 2-Phenyl-4-(4-tolyl)-5-(2-tolyl)-pyrazol-3-ol, 2-(4-tolyl)-4-(4-tolyl)-5-(2-tolyl)-pyrazol-3-ol, 2-(2-tolyl)-4-(4-tolyl)-5-(2-tolyl)-pyrazol-3-ol, 2-(3-tolyl)-4-(4-tolyl)-5-(2-tolyl)-pyrazol-3-ol, 2-(3,4-Dimethylphenyl)-4-(4-tolyl)-5-(3-tolyl)-pyrazol-3-ol, 2-Methyl-4-(4-tolyl)-5-(3-tolyl)-pyrazol-3-ol, 2-Ethyl-4-(4-tolyl)-5-(3-tolyl)-pyrazol-3-ol, 2-Propyl-4-(4-tolyl)-5-(3-tolyl)-pyrazol-3-ol, 2-Isopropyl-4-(4-tolyl)-5-(3-tolyl)-pyrazol-3-ol, 2-Butyl-4-(4-tolyl)-5-(3-tolyl)-pyrazol-3-ol, 2-Isobutyl-4-(4-tolyl)-5-(3-tolyl)-pyrazol-3-ol, 2-tert-Butyl-4-(4-tolyl)-5-(3-tolyl)-pyrazol-3-ol, 2-Phenyl-4-(4-tolyl)-5-(3-tolyl)-pyrazol-3-ol, 2-(4-tolyl)-4-(4-tolyl)-5-(3-tolyl)-pyrazol-3-ol, 2-(2-tolyl)-4-(4-tolyl)-5-(3-tolyl)-pyrazol-3-ol, 2-(3-tolyl)-4-(4-tolyl)-5-(3-tolyl)-pyrazol-3-ol, 2-(3,4-Dimethylphenyl)-4-(4-tolyl)-5-(3-tolyl)-pyrazol-3-ol, 2-Methyl-4-(4-tolyl)-5-(3-tolyl)-pyrazol-3-ol, 2-Ethyl-4-(4-tolyl)-5-(3-tolyl)-pyrazol-3-ol, 2-Propyl-4-(4-tolyl)-5-(3-tolyl)-pyrazol-3-ol, 2-Isopropyl-4-(4-tolyl)-5-(3-tolyl)-pyrazol-3-ol, 2-Butyl-4-(4-tolyl)-5-(3-tolyl)-pyrazol-3-ol, 2-Isobutyl-4-(4-tolyl)-5-(3-tolyl)-pyrazol-3-ol, 2-tert-Butyl-4-(4-tolyl)-5-butyl-pyrazol-3-ol, 2-Phenyl-4-(4-tolyl)-5-(3-tolyl)-pyrazol-3-ol, 2-(4-tolyl)-4-(4-tolyl)-5-(3-tolyl)-pyrazol-3-ol, 2-(2-tolyl)-4-(4-tolyl)-5-(3-tolyl)-pyrazol-3-ol, 2-(3-tolyl)-4-(4-tolyl)-5-(3-tolyl)-pyrazol-3-ol, 2-(3,4-Dimethylphenyl)-4-(4-tolyl)-5-butyl-pyrazol-3-ol, 2-Methyl-4-(4-tolyl)-5-(4-tolyl)-pyrazol-3-ol, 2-Ethyl-4-(4-tolyl)-5-(4-tolyl)-pyrazol-3-ol, 2-Propyl-4-(4-tolyl)-5-(4-tolyl)-pyrazol-3-ol, 2-Isopropyl-4-(4-tolyl)-5-(4-tolyl)-pyrazol-3-ol, 2-Butyl-4-(4-tolyl)-5-(4-tolyl)-pyrazol-3-ol, 2-Isobutyl- 4-(4-tolyl)-5-(4-tolyl)-pyrazol-3-ol, 2-tert-Butyl-4-(4-tolyl)-5-(4-tolyl)-pyrazol-3-ol, 2-Phenyl-4-(4-tolyl)-5-(4-tolyl)-pyrazol-3-ol, 2-(4-tolyl)-4-(4-tolyl)-5-(4-tolyl)-pyrazol-3-ol, 2-(2-tolyl)-4-(4-tolyl)-5-(4-tolyl)-pyrazol-3-ol, 2-(3-tolyl)-4-(4-tolyl)-5-(4-tolyl)-pyrazol-3-ol, 2-(3,4-Dimethylphenyl)-4-(4-tolyl)-5-(4-tolyl)-pyrazol-3-ol,
2-(4-Methoxyphenyl)-5-methyl-pyrazol-3-ol, 2-(4-Isopropylphenyl)-5-methyl-pyrazol-3-ol, 2-[4-(3-Methyl-5-hydroxy-pyrazol-1-yl)phenyl]essigsäure, 5-Methyl-2-(2-pyridyl)-pyrazol-3-ol, 2-Cyclopentylpyrazol-3-ol oder 2-Cyclohexylpyrazol-3-ol.

Sofern nicht näher spezifiziert steht Butyl für iso-Butyl oder n-Butyl. Insbesondere sind solche Verbindungen bevorzugt, die keinen Pyridyl-Substituenten aufweisen.

In bevorzugten Verbindungen der allgemeinen Formel (II) sind
R⁶ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Alkanoyl mit C₁- bis C₆-Alkyl, oder 4-iso-Propoxyphenyl,
R⁷ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl oder 4-iso-Propoxyphenyl,
R⁸ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁-bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl, oder 4-iso-Propoxyphenyl,
wobei R⁶ und R⁷ nicht gleichzeitig Phenyl sind, wenn R⁸ Phenyl oder Methyl ist,
R⁶ und R⁸ nicht gleichzeitig Phenyl sind, wenn R⁷ H oder Methyl ist,
R⁶ nicht Phenyl und R⁸ nicht Methyl ist, wenn R⁷ H oder Methyl ist.

Besonders bevorzugte Verbindungen der allgemeinen Formel (II) sind
1-Methyl-pyrazol-4-ol, 1-Ethyl-pyrazol-4-ol, 1-Propyl-pyrazol-4-ol, 1-lsopropyl-pyrazol-4-ol, 1-Butyl-pyrazol-4-ol, 1-Isobutyl-1H-pyrazol-4-ol ,1-tert-Butyl-pyrazol-4-ol, 1-Cyclopentyl-pyrazol-4-ol, 1-Cyclohexyl-pyrazol-4-ol,1-Phenyl-pyrazol-4-ol, 1-Benzyl-pyrazol-4-ol, 1-(4-Methylphenyl)-pyrazol-4-ol, 1-(4-Methoxyphenyl)-pyrazol-4-ol, 1-(2-methylphenyl)-pyrazol-4-ol, 1-(3-methylphenyl)-pyrazol-4-ol, 1-(4-methoxyphenyl)-pyrazol-4-ol, 1-Methyl-3-phenyl-pyrazol-4-ol, 1-Ethyl-3-phenyl-pyrazol-4-ol, 1-Propyl-3-phenyl-pyrazol-4-ol, 1-Isopropyl-3-phenyl-pyrazol-4-ol, 1-Butyl-3-phenyl-pyrazol-4-ol, 1-Isobutyl-3-phenyl-pyrazol-4-ol, 1-tert-Butyl-3-phenyl-pyrazol-4-ol, 1-Phenyl-3-phenyl-pyrazol-4-ol, 1-(4-methylphenyl)-3-phenyl-pyrazol-4-ol, 1-(4-methoxyphenyl)-3-phenyl-pyrazol-4-ol, 1-(2-methylphenyl)-3-phenyl-pyrazol-4-ol, 1-(3-methylphenyl)-3-phenyl-pyrazol-4-ol, 1-(4-methoxyphenyl)-3-phenyl-pyrazol-4-ol, 1-Methyl-3-methyl-pyrazol-4-ol, 1-Ethyl-3-methyl-pyrazol-4-ol, 1-Propyl-3-methyl-pyrazol-4-ol, 1-Isopropyl-3-methyl-pyrazol-4-ol, 1-Butyl-3-methyl-pyrazol-4-ol, 1-Isobutyl-3- methyl -pyrazol-4-ol, 1-tert-Butyl-3- methyl-pyrazol-4-ol, 1-Phenyl-3-methyl-pyrazol-4-ol, 1-(4-methylphenyl)-3-methyl-pyrazol-4-ol, 1-(2-methylphenyl)-3-methyl-pyrazol-4-ol, 1-(3-methylphenyl)-3-methyl-pyrazol-4-ol, 1-(4-methoxyphenyl)-3-methyl-pyrazol-4-ol, 1-methyl-3-ethyl-pyrazol-4-ol, 1-ethyl-3-ethyl-pyrazol-4-ol, 1-propyl-3-ethyl-pyrazol-4-ol, 1-isopropyl-3-ethyl-pyrazol-4-ol, 1-butyl-3-ethyl-pyrazol-4-ol, 1-Isobutyl-3-ethyl-pyrazol-4-ol, 1-tert-butyl-3-ethyl-pyrazol-4-ol, 1-Phenyl-3-ethyl-pyrazol-4-ol, 1-(4-methylphenyl)-3-ethyl-pyrazol-4-ol, 1-(2-methylphenyl)-3-ethyl-pyrazol-4-ol, 1-(3-methylphenyl)-3-ethyl-pyrazol-4-ol, 1-(4-methoxyphenyl)-3-ethyl-pyrazol-4-ol, 1-Methyl-3-propyl-pyrazol-4-ol, 1-Ethyl-3-propyl-pyrazol-4-ol, 1-Propyl-3-propyl-pyrazol-4-ol, 1-Isopropyl-3-methyl-pyrazol-4-ol, 1-Butyl-3-propyl-pyrazol-4-ol, 1-Isobutyl-3-propyl-pyrazol-4-ol, 1-tert-Butyl-3-propyl-pyrazol-4-ol, 1-Phenyl-3-propyl-pyrazol-4-ol, 1-(4-methylphenyl)-3-propyl-pyrazol-4-ol, 1-(2-methylphenyl)-3-propyl-pyrazol-4-ol, 1-(3-methylphenyl)-3-propyl-pyrazol-4-ol, 1-(4-methoxyphenyl)-3-propyl-pyrazol-4-ol, 1-Methyl-3-isopropyl-pyrazol-4-ol, 1-Ethyl-3-isopropyl-pyrazol-4-ol, 1-Propyl-3-isopropyl -pyrazol-4-ol, 1-Isopropyl-3-isopropyl-pyrazol-4-ol, 1-Butyl-3-isopropyl-pyrazol-4-ol, 1-Isobutyl-3-isopropyl-pyrazol-4-ol, 1-tert-Butyl-3-isopropyl-pyrazol-4-ol, 1-Phenyl-3-isopropyl-pyrazol-4-ol, 1-(4-methylphenyl)-3-isopropyl-pyrazol-4-ol, 1-(2-methylphenyl)-3-isopropyl-pyrazol-4-ol, 1-(3-methylphenyl)-3-isopropyl-pyrazol-4-ol, 1-(4-methoxyphenyl)-3-isopropyl-pyrazol-4-ol, 1-Methyl-3-butyl-pyrazol-4-ol, 1-Ethyl-3-butyl-pyrazol-4-ol, 1-Propyl-3-butyl-pyrazol-4-ol, 1-Isopropyl-3-butyl-pyrazol-4-ol, 1-Butyl-3-butyl-pyrazol-4-ol, 1-Isobutyl-3-butyl-pyrazol-4-ol, 1-tert-Butyl-3-butyl-pyrazol-4-ol, 1-Phenyl-3-butyl-pyrazol-4-ol, 1-(4-methylphenyl)-3-butyl-pyrazol-4-ol, 1-(2-methylphenyl)-3-butyl-pyrazol-4-ol, 1-(3-methylphenyl)-3-butyl-pyrazol-4-ol, 1-(4-methoxyphenyl)-3-butyl-pyrazol-4-ol, 1-Methyl-3-isobutyl-pyrazol-4-ol, 1-Ethyl-3-isobutyl -pyrazol-4-ol, 1-Propyl-3-isobutyl-pyrazol-4-ol, 1-Isopropyl-3-isobutyl-pyrazol-4-ol, 1-Butyl-3-isobutyl-pyrazol-4-ol, 1-Isobutyl-3-isobutyl-pyrazol-4-ol, 1-tert-Butyl-3-isobutyl-pyrazol-4-ol, 1-Phenyl-3-isobutyl-pyrazol-4-ol, 1-(4-methylphenyl)-3-isobutyl-pyrazol-4-ol, 1-(2-methylphenyl)-3-isobutyl-pyrazol-4-ol, 1-(3-methylphenyl)-3-isobutyl-pyrazol-4-ol, 1-(4-methoxyphenyl)-3-isobutyl-pyrazol-4-ol, 1-Methyl-3-tert-butyl-pyrazol-4-ol, 1-Ethyl-3-tert-butyl-pyrazol-4-ol,1-Propyl-3-tert-butyl-pyrazol-4-ol, 1-Isopropyl-3-tert-butyl-pyrazol-4-ol, 1-Butyl-3-tert-butyl-pyrazol-4-ol, 1-Isobutyl-3-tert-butyl-pyrazol-4-ol, 1-tert-Butyl-3-tert-butyl-pyrazol-4-ol, 1-Phenyl-3-tert-butyl-pyrazol-4-ol, 1-(4-methylphenyl)-3-tert-butyl-pyrazol-4-ol, 1-(2-methylphenyl)-3-tert-butyl-pyrazol-4-ol, 1-(3-methylphenyl)-3-tert-butyl-pyrazol-4-ol, 1-(4-methoxyphenyl)-3-phenyl-pyrazol-4-ol, 1-Methyl-3-phenyl-pyrazol-4-ol, 1-Ethyl-3-phenyl-pyrazol-4-ol, 1-Propyl-3-phenyl-pyrazol-4-ol, 1-Isopropyl-3-phenyl-pyrazol-4-ol, 1-Butyl-3-phenyl-pyrazol-4-ol, 1-Isobutyl-3-phenyl-pyrazol-4-ol, 1-tert-Butyl-3-phenyl-pyrazol-4-ol, 1-Phenyl-3-phenyl-pyrazol-4-ol, 1-(4-methylphenyl)-3-phenyl -pyrazol-4-ol, 1-(2-methylphenyl)-3-phenyl-pyrazol-4-ol, 1-(3-methylphenyl)-3-phenyl-pyrazol-4-ol, 1-(4-methoxyphenyl)-3-(2-methylphenyl)-pyrazol-4-ol, 1-Methyl-3-(2-methylphenyl)-pyrazol-4-ol, 1-Ethyl-3-(2-methylphenyl) -pyrazol-4-ol, 1-Propyl-3-(2-methylphenyl)-pyrazol-4-ol, 1-Isopropyl-3-(2-methylphenyl)-pyrazol-4-ol, 1-Butyl-3-(2-methylphenyl)-pyrazol-4-ol, 1-Isobutyl-3-(2-methylphenyl)-pyrazol-4-ol, 1-tert-Butyl-3-(2-methylphenyl)-pyrazol-4-ol, 1-Phenyl-3-(2-methylphenyl)-pyrazol-4-ol, 1-(4-methylphenyl)-3-(2-methylphenyl)-pyrazol-4-ol, 1-(2-methylphenyl)-3-(2-methylphenyl)-pyrazol-4-ol, 1-(3-methylphenyl)-3-(2-methylphenyl) -pyrazol-4-ol, 1-(4-methoxyphenyl)-3-(3-methylphenyl)-pyrazol-4-ol, 1-Methyl-3-(3-methylphenyl)-pyrazol-4-ol, 1-Ethyl-3-(3-methylphenyl)-pyrazol-4-ol, 1-Propyl-3-(3-methylphenyl)-pyrazol-4-ol, 1-Isopropyl-3-(3-methylphenyl)-pyrazol-4-ol , 1-Butyl-3-(3-methylphenyl)-pyrazol-4-ol, 1-Isobutyl-3-(3-methylphenyl)-pyrazol-4-ol, 1-tert-Butyl-3-(3-methylphenyl)-pyrazol-4-ol, 1-Phenyl-3-(3-methylphenyl)-pyrazol-4-ol, 1-(4-methylphenyl)-3-(3-methylphenyl)-pyrazol-4-ol, 1-(2-methylphenyl)-3-(3-methylphenyl)-pyrazol-4-ol, 1-(3-methylphenyl)-3-(3-methylphenyl)-pyrazol-4-ol, 1-(4-methoxyphenyl)-3-(3-methylphenyl)-pyrazol-4-ol, 1-Methyl-3-(3-methylphenyl)-pyrazol-4-ol, 1-Ethyl-3-(3-methylphenyl)-pyrazol-4-ol, 1-Propyl-3-(3-methylphenyl)-pyrazol-4-ol, 1-Isopropyl-3-(3-methylphenyl)-pyrazol-4-ol, 1-Butyl-3-(3-methylphenyl)-pyrazol-4-ol, 1-Isobutyl-3-(3-methylphenyl)-pyrazol-4-ol, 1-tert-Butyl-3-butyl-pyrazol-4-ol, 1-Phenyl-3-(3-methylphenyl)-pyrazol-4-ol, 1-(4-methylphenyl)-3-(3-methylphenyl)-pyrazol-4-ol, 1-(2-methylphenyl)-3-(3-methylphenyl)-pyrazol-4-ol, 1-(3-methylphenyl)-3-(3-methylphenyl)-pyrazol-4-ol, 1-(4-methoxyphenyl)-3-butyl-pyrazol-4-ol, 1-Methyl-3-(4-methylphenyl)-pyrazol-4-ol, 1-Ethyl-3-(4-methylphenyl)-pyrazol-4-ol, 1-Propyl-3-(4-methylphenyl)-pyrazol-4-ol, 1-Isopropyl-3-(4-methylphenyl)-pyrazol-4-ol, 1-Butyl-3-(4-methylphenyl) -pyrazol-4-ol, 1-Isobutyl-3-(4-methylphenyl)-pyrazol-4-ol, 1-tert-Butyl-3-(4-methylphenyl)-pyrazol-4-ol, 1-Phenyl-3-(4-methylphenyl)-pyrazol-4-ol, 1-(4-methylphenyl)-3-(4-methylphenyl)-pyrazol-4-ol, 1-(2-methylphenyl)-3-(4-methylphenyl)-pyrazol-4-ol, 1-(3-methylphenyl)-3-(4-methylphenyl)-pyrazol-4-ol, 1-(4-methoxyphenyl)-3-(4-methylphenyl)-pyrazol-4-ol, 1-Methyl-5-phenyl-pyrazol-4-ol, 1-Ethyl-5-phenyl-pyrazol-4-ol, 1-Propyl-5-phenyl-pyrazol-4-ol, 1-Isopropyl-5-phenyl-pyrazol-4-ol, 1-Butyl-5-phenyl-pyrazol-4-ol, 1-Isobutyl-5-phenyl-pyrazol-4-ol, 1-tert-Butyl-5-phenyl-pyrazol-4-ol, 1-Phenyl-5-phenyl-pyrazol-4-ol, 1-(4-methylphenyl)-5-phenyl-pyrazol-4-ol, 1-(2-methylphenyl)-5-phenyl-pyrazol-4-ol, 1-(3-methylphenyl)-5-phenyl-pyrazol-4-ol, 1-(4-methoxyphenyl)-5-phenyl-pyrazol-4-ol, 1-Methyl-5-methyl-pyrazol-4-ol, 1-Ethyl-5-methyl-pyrazol-4-ol, 1-Propyl-5-methyl-pyrazol-4-ol, 1-Isopropyl-5-methyl-pyrazol-4-ol, 1-Butyl-5-methyl-pyrazol-4-ol, 1-Isobutyl-5-methyl-pyrazol-4-ol, 1-tert-Butyl-5-methyl-pyrazol-4-ol, 1-Phenyl-5-methyl-pyrazol-4-ol, 1-(4-methylphenyl)-5-methyl-pyrazol-4-ol, 1-(2-methylphenyl)-5-methyl-pyrazol-4-ol, 1-(3-methylphenyl)-5-methyl-pyrazol-4-ol, 1-(4-methoxyphenyl)-5-methyl-pyrazol-4-ol, 1-methyl-5-ethyl-pyrazol-4-ol, 1-ethyl-5-ethyl-pyrazol-4-ol, 1-propyl-5-ethyl-pyrazol-4-ol, 1-isopropyl-5-ethyl -pyrazol-4-ol, 1-butyl-5-ethyl-pyrazol-4-ol, 1-Isobutyl-5-ethyl-pyrazol-4-ol, 1-tert-butyl-5- ethyl-pyrazol-4-ol, 1-Phenyl-5-ethyl-pyrazol-4-ol, 1-(4-methylphenyl)-5-ethyl-pyrazol-4-ol, 1-(2-methylphenyl)-5-ethyl-pyrazol-4-ol, 1-(3-methylphenyl)-5-ethyl-pyrazol-4-ol, 1-(4-methoxyphenyl)-5-ethyl-pyrazol-4-ol, 1-Methyl-5-propyl-pyrazol-4-ol, 1-Ethyl-5-propyl-pyrazol-4-ol, 1-Propyl-5-propyl-pyrazol-4-ol, 1-Isopropyl-5-methyl-pyrazol-4-ol, 1-Butyl-5-propyl-pyrazol-4-ol, 1-Isobutyl-5-propyl-pyrazol-4-ol, 1-tert-Butyl-5- propyl-pyrazol-4-ol, 1-Phenyl-5-propyl-pyrazol-4-ol, 1-(4-methylphenyl)-5-propyl-pyrazol-4-ol, 1-(2-methylphenyl)-5-propyl-pyrazol-4-ol, 1-(3-methylphenyl)-5-propyl-pyrazol-4-ol, 1-(4-methoxyphenyl)-5-propyl-pyrazol-4-ol, 1-Methyl-5-isopropyl-pyrazol-4-ol, 1-Ethyl-5-isopropyl-pyrazol-4-ol, 1-Propyl-5-isopropyl-pyrazol-4-ol, 1-Isopropyl-5-isopropyl-pyrazol-4-ol, 1-Butyl-5-isopropyl-pyrazol-4-ol, 1-Isobutyl-5-isopropyl-pyrazol-4-ol, 1-tert-Butyl-5-isopropyl-pyrazol-4-ol, 1-Phenyl-5-isopropyl-pyrazol-4-ol, 1-(4-methylphenyl)-5-isopropyl-pyrazol-4-ol, 1-(2-methylphenyl)-5-isopropyl-pyrazol-4-ol, 1-(3-methylphenyl)-5-isopropyl-pyrazol-4-ol, 1-(4-methoxyphenyl)-5-isopropyl-pyrazol-4-ol, 1-Methyl-5-butyl-pyrazol-4-ol, 1-Ethyl-5-butyl-pyrazol-4-ol, 1-Propyl-5-butyl-pyrazol-4-ol, 1-Isopropyl-5-butyl-pyrazol-4-ol, 1-Butyl-5-butyl-pyrazol-4-ol, 1-Isobutyl-5-butyl-pyrazol-4-ol, 1-tert-Butyl-5-butyl-pyrazol-4-ol, 1-Phenyl-5-butyl-pyrazol-4-ol, 1-(4-methylphenyl)-5-butyl -pyrazol-4-ol, 1-(2-methylphenyl)-5-butyl-pyrazol-4-ol, 1-(3-methylphenyl)-5-butyl-pyrazol-4-ol, 1-(4-methoxyphenyl)-5-butyl-pyrazol-4-ol, 1-Methyl-5-isobutyl-pyrazol-4-ol, 1-Ethyl-5-isobutyl-pyrazol-4-ol, 1-Propyl-5-isobutyl-pyrazol-4-ol, 1-Isopropyl-5-isobutyl-pyrazol-4-ol, 1-Butyl-5-isobutyl-pyrazol-4-ol, 1-Isobutyl-5-isobutyl-pyrazol-4-ol, 1-tert-Butyl-5-isobutyl-pyrazol-4-ol, 1-Phenyl-5-isobutyl-pyrazol-4-ol, 1-(4-methylphenyl)-5-isobutyl-pyrazol-4-ol, 1-(2-methylphenyl)-5-isobutyl-pyrazol-4-ol, 1-(3-methylphenyl)-5-isobutyl-pyrazol-4-ol, 1-(4-methoxyphenyl)-5-isobutyl-pyrazol-4-ol, 1-Methyl-5-tert-butyl-pyrazol-4-ol, 1-Ethyl-5-tert-butyl-pyrazol-4-ol, 1-Propyl-5-tert-butyl-pyrazol-4-ol, 1-Isopropyl-5-tert-butyl-pyrazol-4-ol, 1-Butyl-5-tert-butyl-pyrazol-4-ol, 1-Isobutyl-5-tert-butyl-pyrazol-4-ol, 1-tert-Butyl-5-tert-butyl-pyrazol-4-ol, 1-Phenyl-5-tert-butyl-pyrazol-4-ol, 1-(4-methylphenyl)-5-tert-butyl-pyrazol-4-ol, 1-(2-methylphenyl)-5-tert-butyl-pyrazol-4-ol, 1-(3-methylphenyl)-5-tert-butyl-pyrazol-4-ol, 1-(4-methoxyphenyl)-5- phenyl-pyrazol-4-ol, 1-Methyl-5-phenyl-pyrazol-4-ol, 1-Ethyl-5-phenyl-pyrazol-4-ol, 1-Propyl-5-phenyl-pyrazol-4-ol, 1-Isopropyl-5-phenyl-pyrazol-4-ol, 1-Butyl-5-phenyl-pyrazol-4-ol, 1-Isobutyl-5-phenyl-pyrazol-4-ol, 1-tert-Butyl-5-phenyl-pyrazol-4-ol, 1-Phenyl-5-phenyl-pyrazol-4-ol, 1-(4-methylphenyl)-5-phenyl-pyrazol-4-ol, 1-(2-methylphenyl)-5-phenyl-pyrazol-4-ol, 1-(3-methylphenyl)-5-phenyl-pyrazol-4-ol, 1-(4-methoxyphenyl)-5-(2-methylphenyl)-pyrazol-4-ol, 1-Methyl-5-(2-methylphenyl)-pyrazol-4-ol, 1-Ethyl-5-(2-methylphenyl)-pyrazol-4-ol, 1-Propyl-5-(2-methylphenyl)-pyrazol-4-ol, 1-Isopropyl-5-(2-methylphenyl)-pyrazol-4-ol, 1-Butyl-5-(2-methylphenyl)-pyrazol-4-ol, 1-Isobutyl-5-(2-methylphenyl)-pyrazol-4-ol, 1-tert-Butyl-5-(2-methylphenyl)-pyrazol-4-ol, 1-Phenyl-5-(2-methylphenyl)-pyrazol-4-ol, 1-(4-methylphenyl)-5-(2-methylphenyl)-pyrazol-4-ol, 1-(2-methylphenyl)-5-(2-methylphenyl)-pyrazol-4-ol, 1-(3-methylphenyl)-5-(2-methylphenyl)-pyrazol-4-ol, 1-(4-methoxyphenyl)-5-(3-methylphenyl)-pyrazol-4-ol, 1-Methyl-5-(3-methylphenyl)-pyrazol-4-ol, 1-Ethyl-5-(3-methylphenyl)-pyrazol-4-ol, 1-Propyl-5-(3-methylphenyl)-pyrazol-4-ol, 1-Isopropyl-5-(3-methylphenyl)-pyrazol-4-ol, 1-Butyl-5-(3-methylphenyl)-pyrazol-4-ol, 1-Isobutyl-5-(3-methylphenyl)-pyrazol-4-ol, 1-tert-Butyl-5-(3-methylphenyl)-pyrazol-4-ol, 1-Phenyl-5-(3-methylphenyl)-pyrazol-4-ol, 1-(4-methylphenyl)-5-(3-methylphenyl)-pyrazol-4-ol, 1-(2-methylphenyl)-5-(3-methylphenyl)-pyrazol-4-ol, 1-(3-methylphenyl)-5-(3-methylphenyl)-pyrazol-4-ol, 1-(4-methoxyphenyl)-5-(3-methylphenyl)-pyrazol-4-ol, 1-Methyl-5-(3-methylphenyl)-pyrazol-4-ol, 1-Ethyl-5-(3-methylphenyl)-pyrazol-4-ol, 1-Propyl-5-(3-methylphenyl)-pyrazol-4-ol, 1-Isopropyl-5-(3-methylphenyl)-pyrazol-4-ol, 1-Butyl-5-(3-methylphenyl)-pyrazol-4-ol, 1-Isobutyl-5-(3-methylphenyl)-pyrazol-4-ol, 1-tert-Butyl-5-butyl-pyrazol-4-ol, 1-Phenyl-5-(3-methylphenyl)-pyrazol-4-ol, 1-(4-methyl-phenyl)-5-(3-methylphenyl)-pyrazol-4-ol, 1-(2-methylphenyl)-5-(3-methylphenyl)-pyrazol-4-ol, 1-(3-methylphenyl)-5-(3-methylphenyl)-pyrazol-4-ol, 1-(4-methoxyphenyl)-5-butyl-pyrazol-4-ol, 1-Methyl-5-(4-methylphenyl)-pyrazol-4-ol, 1-Ethyl-5-(4-methylphenyl)-pyrazol-4-ol, 1-Propyl-5-(4-methylphenyl)-pyrazol-4-ol, 1-Isopropyl-5-(4-methylphenyl)-pyrazol-4-ol, 1-Butyl-5-(4-methylphenyl)-pyrazol-4-ol, 1-Isobutyl-5-(4-methylphenyl)-pyrazol-4-ol, 1-tert-Butyl-5-(4-methylphenyl)-pyrazol-4-ol, 1-Phenyl-5-(4-methylphenyl)-pyrazol-4-ol, 1-(4-methylphenyl)-5-(4-methylphenyl)-pyrazol-4-ol, 1-(2-methylphenyl)-5-(4-methylphenyl)-pyrazol-4-ol, 1-(3-methylphenyl)-5-(4-methylphenyl)-pyrazol-4-ol, 1-(4-methoxyphenyl)-5-(4-methylphenyl)-pyrazol-4-ol, 1-Methyl-3-methyl-5-phenyl-pyrazol-4-ol, 1-Ethyl-3-methyl5-phenyl-pyrazol-4-ol, 1-Propyl-3-methyl-5-phenyl-pyrazol-4-ol, 1-Isopropyl-3-methyl-5-phenyl-pyrazol-4-ol, 1-Butyl-3-methyl-5-phenyl-pyrazol-4-ol, 1-Isobutyl- 3-methyl-5-phenyl-pyrazol-4-ol, 1-tert-Butyl-3-methyl-5-phenyl-pyrazol-4-ol, 1-(4-methylphenyl)-3-methyl-5-phenyl-pyrazol-4-ol, 1-(2-methylphenyl)-3-methyl-5-phenyl-pyrazol-4-ol, 1-(3-methylphenyl)-3-methyl-5-phenyl-pyrazol-4-ol, 1-(4-methoxyphenyl)-3-methyl-5-phenyl-pyrazol-4-ol, 1-Methyl-3-methyl-5-methyl-pyrazol-4-ol, 1-Ethyl-3-methyl-5-methyl-pyrazol-4-ol, 1-Propyl-3-methyl-5-methyl-pyrazol-4-ol, 1-Isopropyl-3-methyl-5-methyl-pyrazol-4-ol, 1-Butyl-3-methyl-5-methyl-pyrazol-4-ol, 1-Isobutyl-3-methyl-5-methyl-pyrazol-4-ol, 1-tert-Butyl-3-methyl-5-methyl-pyrazol-4-ol, 1-(4-methylphenyl)-3-methyl-5-methyl-pyrazol-4-ol, 1-(2-methylphenyl)-3-methyl-5-methyl-pyrazol-4-ol, 1-(3-methylphenyl)-3-methyl-5-methyl-pyrazol-4-ol, 1-(4-methoxyphenyl)-3-methyl-5-methyl-pyrazol-4-ol, 1-methyl-3-methyl-5-ethyl-pyrazol-4-ol, 1-ethyl-3-methyl-5-ethyl-pyrazol-4-ol, 1-propyl-3-methyl-5-ethyl-pyrazol-4-ol, 1-isopropyl-3-methyl-5-ethyl-pyrazol-4-ol, 1-butyl-3-methyl-5-ethyl-pyrazol-4-ol, 1-Isobutyl- 3-methyl-5-ethyl-pyrazol-4-ol, 1-tert-butyl-3-methyl-5-ethyl-pyrazol-4-ol, 1-Phenyl-3-methyl-5-ethyl-pyrazol-4-ol, 1-(4-methylphenyl)-3-methyl-5-ethyl-pyrazol-4-ol, 1-(2-methylphenyl)-3-methyl-5- ethyl-pyrazol-4-ol, 1-(3-methylphenyl)-3-methyl-5-ethyl-pyrazol-4-ol, 1-(4-methoxyphenyl)-3-methyl-5-ethyl -pyrazol-4-ol,1-Methyl-3-methyl-5-propyl-pyrazol-4-ol, 1-Ethyl-3-methyl-5-propyl-pyrazol-4-ol, 1-Propyl-3-methyl-5-propyl-pyrazol-4-ol, 1-Butyl-3-methyl-5-propyl-pyrazol-4-ol, 1-Isobutyl- 3-methyl-5-propyl-pyrazol-4-ol, 1-tert-Butyl-3-methyl-5- propyl-pyrazol-4-ol, 1-Phenyl-3-methyl-5-propyl-pyrazol-4-ol, 1-(4-methylphenyl)-3-methyl-5-propyl-pyrazol-4-ol, 1-(2-methylphenyl)-3-methyl-5-propyl-pyrazol-4-ol, 1-(3-methylphenyl)-3-methyl-5-propyl-pyrazol-4-ol, 1-(4-methoxyphenyl)-3-methyl-5-propyl-pyrazol-4-ol, 1-Methyl-3-methyl-5-isopropyl-pyrazol-4-ol, 1-Ethyl-3-methyl-5-isopropyl-pyrazol-4-ol, 1-Propyl-3-methyl-5-isopropyl -pyrazol-4-ol, 1-Isopropyl-3-methyl-5-isopropyl -pyrazol-4-ol, 1-Butyl-3-methyl-5-isopropyl -pyrazol-4-ol, 1-Isobutyl- 3-methyl-5-isopropyl-pyrazol-4-ol, 1-tert-Butyl-3-methyl-5-isopropyl -pyrazol-4-ol, 1-Phenyl-3-methyl-5-isopropyl-pyrazol-4-ol, 1-(4-methylphenyl)-3-methyl-5- isopropyl -pyrazol-4-ol, 1-(2-methylphenyl)-3-methyl-5- isopropyl-pyrazol-4-ol, 1-(3-methylphenyl)-3-methyl-5-isopropyl-pyrazol-4-ol, 1-(4-methoxyphenyl)-3-methyl-5-isopropyl-pyrazol-4-ol, 1-Methyl-3-methyl-5- butyl-pyrazol-4-ol, 1-Ethyl-3-methyl-5- butyl-pyrazol-4-ol, 1-Propyl-3-methyl-5-butyl-pyrazol-4-ol, 1-Isopropyl-3-methyl-5-butyl-pyrazol-4-ol, 1-Butyl-3-methyl-5-butyl-pyrazol-4-ol, 1-Isobutyl-3-methyl-5-butyl-pyrazol-4-ol, 1-tert-Butyl-3-methyl-5-butyl-pyrazol-4-ol, 1-Phenyl-3-methyl-5-butyl-pyrazol-4-ol, 1-(4-methylphenyl)-3-methyl-5-butyl-pyrazol-4-ol, 1-(2-methylphenyl)-3-methyl-5-butyl-pyrazol-4-ol, 1-(3-methylphenyl)-3-methyl-5-butyl-pyrazol-4-ol, 1-(4-methoxyphenyl)-3-methyl-5-butyl-pyrazol-4-ol, 1-Methyl-3-methyl-5-isobutyl-pyrazol-4-ol, 1-Ethyl-3-methyl-5-isobutyl-pyrazol-4-ol, 1-Propyl-3-methyl-5-isobutyl-pyrazol-4-ol, 1-Isopropyl-3-methyl-5-isobutyl-pyrazol-4-ol, 1-Butyl-3-methyl-5-isobutyl-pyrazol-4-ol, 1-Isobutyl-3-methyl-5-isobutyl-pyrazol-4-ol, 1-tert-Butyl-3-methyl-5-isobutyl-pyrazol-4-ol, 1-Phenyl-3-methyl-5-isobutyl-pyrazol-4-ol, 1-(4-methylphenyl)-3-methyl-5-isobutyl-pyrazol-4-ol, 1-(2-methyl-phenyl)-3-methyl-5-isobutyl-pyrazol-4-ol, 1-(3-methylphenyl)-3-methyl-5-isobutyl-pyrazol-4-ol, 1-(4-methoxyphenyl)-3-methyl-5-isobutyl-pyrazol-4-ol, 1-Methyl-3-methyl-5-tert-butyl-pyrazol-4-ol, 1-Ethyl-3-methyl-5-tert-butyl-pyrazol-4-ol, 1-Propyl-3-methyl-5-tert-butyl-pyrazol-4-ol, 1-Isopropyl-3-methyl-5-tert-butyl-pyrazol-4-ol, 1-Butyl-3-methyl-5-tert-butyl-pyrazol-4-ol, 1-Isobutyl-3-methyl-5- tert-butyl-pyrazol-4-ol, 1-tert-Butyl-3-methyl-5-tert-butyl -pyrazol-4-ol, 1-Phenyl-3-methyl-5-tert-butyl-pyrazol-4-ol, 1-(4-methylphenyl)-3-methyl-5-tert-butyl-pyrazol-4-ol, 1-(2-methylphenyl)-3-methyl-5-tert-butyl-pyrazol-4-ol, 1-(3-methylphenyl)-3-methyl-5-tert-butyl-pyrazol-4-ol, 1-(4-methoxyphenyl)-3-methyl-5-phenyl-pyrazol-4-ol, 1-Methyl-3-methyl-5-phenyl-pyrazol-4-ol, 1-Ethyl-3-methyl-5-phenyl-pyrazol-4-ol, 1-Propyl-3-methyl-5-phenyl-pyrazol-4-ol, 1-Isopropyl-3-methyl-5-phenyl-pyrazol-4-ol, 1-Butyl-3-methyl-5-phenyl-pyrazol-4-ol, 1-Isobutyl-3-methyl-5-phenyl-pyrazol-4-ol, 1-tert-Butyl-3-methyl-5-phenyl-pyrazol-4-ol, 1-(4-methylphenyl)-3-methyl-5-phenyl-pyrazol-4-ol, 1-(2-methylphenyl)-3-methyl-5-phenyl-pyrazol-4-ol, 1-(3-methylphenyl)-3-methyl-5-phenyl-pyrazol-4-ol, 1-(4-methoxyphenyl)-3-methyl-5-(2-methylphenyl)-pyrazol-4-ol, 1-Methyl-3-methyl-5-(2-methylphenyl)-pyrazol-4-ol, 1-Ethyl-3-methyl-5-(2-methylphenyl)-pyrazol-4-ol, 1-Propyl-3-methyl-5-(2-methylphenyl)-pyrazol-4-ol, 1-Isopropyl-3-methyl-5-(2-methylphenyl)-pyrazol-4-ol, 1-Butyl-3-methyl-5-(2-methylphenyl)-pyrazol-4-ol, 1-Isobutyl- 3-methyl-5-(2-methylphenyl)-pyrazol-4-ol, 1-tert-Butyl-3-methyl-5-(2-methylphenyl)-pyrazol-4-ol, 1-Phenyl-3-methyl-5-(2-methylphenyl)-pyrazol-4-ol, 1-(4-methylphenyl)-3-methyl-5-(2-methylphenyl)-pyrazol-4-ol, 1-(2-methylphenyl)-3-methyl-5-(2-methylphenyl)-pyrazol-4-ol, 1-(3-methylphenyl)-3-methyl-5-(2-methylphenyl)-pyrazol-4-ol, 1-(4-methoxyphenyl)-3-methyl-5-(3-methylphenyl)-pyrazol-4-ol, 1-Methyl-3-methyl-5-(3-methylphenyl)-pyrazol-4-ol, 1-Ethyl-3-methyl-5-(3-methylphenyl)-pyrazol-4-ol, 1-Propyl-3-methyl-5-(3-methylphenyl) -pyrazol-4-ol, 1-Isopropyl-3-methyl-5-(3-methylphenyl)-pyrazol-4-ol, 1-Butyl-3-methyl-5-(3-methylphenyl)-pyrazol-4-ol, 1-Isobutyl-3-methyl-5-(3-methylphenyl)-pyrazol-4-ol, 1-tert-Butyl-3-methyl-5-(3-methylphenyl)-pyrazol-4-ol, 1-Phenyl-3-methyl-5-(3-methylphenyl)-pyrazol-4-ol, 1-(4-methylphenyl)-3-methyl-5-(3-methylphenyl)-pyrazol-4-ol, 1-(2-methyl-phenyl)-3-methyl-5-(3-methylphenyl)-pyrazol-4-ol, 1-(3-methylphenyl)-3-methyl-5-(3-methylphenyl)-pyrazol-4-ol, 1-(4-methoxyphenyl)-3-methyl-5-(3-methylphenyl)-pyrazol-4-ol, 1-Methyl-3-methyl-5-(3-methylphenyl)-pyrazol-4-ol, 1-Ethyl-3-methyl-5-(3-methylphenyl)-pyrazol-4-ol, 1-Propyl-3-methyl-5-(3-methylphenyl) -pyrazol-4-ol, 1-Isopropyl-3-methyl-5- (3-methylphenyl)-pyrazol-4-ol, 1-Butyl-3-methyl-5-(3-methylphenyl)-pyrazol-4-ol, 1-Isobutyl- 3-methyl-5-(3-methylphenyl)-pyrazol-4-ol, 1-tert-Butyl-3-methyl-5-butyl-pyrazol-4-ol, 1-Phenyl-3-methyl-5-(3-methylphenyl)-pyrazol-4-ol, 1-(4-methylphenyl)-3-methyl-5-(3-methylphenyl)-pyrazol-4-ol, 1-(2-methylphenyl)-3-methyl-5-(3-methylphenyl)-pyrazol-4-ol, 1-(3-methylphenyl)-3-methyl-5-(3-methylphenyl)-pyrazol-4-ol, 1-(4-methoxyphenyl)-3-methyl-5-butyl-pyrazol-4-ol, 1-Methyl-3-methyl-5-(4-methylphenyl)-pyrazol-4-ol, 1-Ethyl-3-methyl-5-(4-methylphenyl)-pyrazol-4-ol, 1-Propyl-3-methyl-5-(4-methylphenyl)-pyrazol-4-ol, 1-Isopropyl-3-methyl-5-(4-methylphenyl)-pyrazol-4-ol, 1-Butyl-3-methyl-5-(4-methylphenyl) -pyrazol-4-ol, 1-Isobutyl-3-methyl-5-(4-methylphenyl)-pyrazol-4-ol, 1-tert-Butyl-3-methyl-5-(4-methylphenyl)-pyrazol-4-ol, 1-Phenyl-3-methyl-5-(4-methylphenyl)-pyrazol-4-ol, 1-(4-methylphenyl)-3-methyl-5-(4-methylphenyl)-pyrazol-4-ol, 1-(2-methylphenyl)-3-methyl-5-(4-methylphenyl)-pyrazol-4-ol, 1-(3-methylphenyl)-3-methyl-5-(4-methylphenyl)-pyrazol-4-ol, 1-(4-methoxyphenyl)-3-methyl-5-(4-methylphenyl)-pyrazol-4-ol, 1-Methyl-3-ethyl-5-phenyl-pyrazol-4-ol, 1-Ethyl-3-ethyl5-phenyl-pyrazol-4-ol, 1-Propyl-3-ethyl-5-phenyl-pyrazol-4-ol, 1-Isopropyl-3-ethyl-5-phenyl-pyrazol-4-ol, 1-Butyl-3-ethyl-5-phenyl-pyrazol-4-ol, 1-Isobutyl- 3-ethyl-5-phenyl-pyrazol-4-ol, 1-tert-Butyl-3-ethyl-5-phenyl-pyrazol-4-ol, 1-Phenyl-3-ethyl-5-phenyl-pyrazol-4-ol, 1-(4-methylphenyl)-3-ethyl-5-phenyl-pyrazol-4-ol, 1-(2-methylphenyl)-3-ethyl-5-phenyl-pyrazol-4-ol, 1-(3-methylphenyl)-3-ethyl-5-phenyl-pyrazol-4-ol, 1-(4-methoxyphenyl)-3-ethyl-5-phenyl-pyrazol-4-ol,1-Methyl-3-ethyl-5-methyl-pyrazol-4-ol, 1-Ethyl-3-ethyl-5-methyl-pyrazol-4-ol, 1-Propyl-3-ethyl-5-methyl-pyrazol-4-ol, 1-Isopropyl-3-ethyl-5-methyl-pyrazol-4-ol, 1-Butyl-3-ethyl-5-methyl-pyrazol-4-ol, 1-Isobutyl- 3-ethyl-5-methyl-pyrazol-4-ol, 1-tert-Butyl-3-ethyl-5-methyl-pyrazol-4-ol, 1-Phenyl-3-ethyl-5-methyl-pyrazol-4-ol, 1-(4-methylphenyl)-3-ethyl-5-methyl-pyrazol-4-ol, 1-(2-methylphenyl)-3-ethyl-5-methyl -pyrazol-4-ol, 1-(3-methylphenyl)-3-ethyl-5-methyl-pyrazol-4-ol, 1-(4-methoxyphenyl)-3-ethyl-5-methyl-pyrazol-4-ol,1-methyl-3-ethyl-5-ethyl-pyrazol-4-ol, 1-ethyl-3-ethyl-5-ethyl-pyrazol-4-ol, 1-propyl-3-ethyl-5-ethyl-pyrazol-4-ol, 1-isopropyl-3-ethyl-5-ethyl-pyrazol-4-ol, 1-butyl-3-ethyl-5-ethyl-pyrazol-4-ol, 1-Isobutyl-3-ethyl-5-ethyl-pyrazol-4-ol, 1-tert-butyl-3-ethyl-5-ethyl-pyrazol-4-ol, 1-Phenyl-3-ethyl-5-ethyl-pyrazol-4-ol, 1-(4-methylphenyl)-3-ethyl-5-ethyl-pyrazol-4-ol, 1-(2-methylphenyl)-3-thyl-5-ethyl-pyrazol-4-ol, 1-(3-methylphenyl)-3-ethyl-5-ethyl-pyrazol-4-ol, 1-(4-methoxyphenyl)-3-ethyl-5-ethyl-pyrazol-4-ol, 1-Methyl-3-ethyl-5-propyl-pyrazol-4-ol, 1-Ethyl-3-ethyl-5- propyl-pyrazol-4-ol, 1-Propyl-3-ethyl-5-propyl-pyrazol-4-ol, 1-Isopropyl-3-ethyl-5-methyl-pyrazol-4-ol, 1-Butyl-3-ethyl-5-propyl-pyrazol-4-ol, 1-Isobutyl-3-ethyl-5-propyl-pyrazol-4-ol, 1-tert-Butyl-3-ethyl-5-propyl-pyrazol-4-ol, 1-Phenyl-3-ethyl-5-propyl-pyrazol-4-ol, 1-(4-methylphenyl)-3-ethyl-5-propyl-pyrazol-4-ol, 1-(2-methylphenyl)-3-ethyl-5-propyl-pyrazol-4-ol, 1-(3-methylphenyl)-3-ethyl-5-propyl-pyrazol-4-ol, 1-(4-methoxyphenyl)-3-ethyl-5-propyl -pyrazol-4-ol, 1-Methyl-3-ethyl-5-isopropyl-pyrazol-4-ol, 1-Ethyl-3-ethyl-5-isopropyl-pyrazol-4-ol, 1-Propyl-3-ethyl-5-isopropyl-pyrazol-4-ol, 1-Isopropyl-3-ethyl-5-isopropyl-pyrazol-4-ol, 1-Butyl-3-ethyl-5-isopropyl-pyrazol-4-ol, 1-Isobutyl- 3-ethyl-5-isopropyl-pyrazol-4-ol, 1-tert-Butyl-3-ethyl-5-isopropyl-pyrazol-4-ol, 1-Phenyl-3-ethyl-5-isopropyl-pyrazol-4-ol, 1-(4-methylphenyl)-3-ethyl-5-isopropyl-pyrazol-4-ol, 1-(2-methyl-phenyl)-3-ethyl-5-isopropyl-pyrazol-4-ol, 1-(3-methylphenyl)-3-ethyl-5-isopropyl-pyrazol-4-ol, 1-(4-methoxyphenyl)-3-ethyl-5- isopropyl-pyrazol-4-ol, 1-Methyl-3-ethyl-5- butyl-pyrazol-4-ol, 1-Ethyl-3-ethyl-5-butyl-pyrazol-4-ol, 1-Propyl-3-ethyl-5-butyl-pyrazol-4-ol, 1-Isopropyl-3-ethyl-5-butyl-pyrazol-4-ol, 1-Butyl-3-ethyl-5-butyl-pyrazol-4-ol, 1-Isobutyl-3-ethyl-5- butyl-pyrazol-4-ol, 1-tert-Butyl-3-ethyl-5-butyl-pyrazol-4-ol, 1-Phenyl-3-ethyl-5-butyl-pyrazol-4-ol, 1-(4-methylphenyl)-3-ethyl-5- butyl-pyrazol-4-ol, 1-(2-methylphenyl)-3-ethyl-5- butyl-pyrazol-4-ol, 1-(3-methylphenyl)-3-ethyl-5-butyl-pyrazol-4-ol, 1-(4-methoxyphenyl)-3-ethyl-5- butyl-pyrazol-4-ol,
1-Methyl-3-ethyl-5- isobutyl-pyrazol-4-ol, 1-Ethyl-3-ethyl-5-isobutyl-pyrazol-4-ol, 1-Propyl-3-ethyl-5-isobutyl-pyrazol-4-ol, 1-Isopropyl-3-ethyl-5-isobutyl-pyrazol-4-ol, 1-Butyl-3-ethyl-5-isobutyl-pyrazol-4-ol, 1-Isobutyl-3-ethyl-5isobutyl-pyrazol-4-ol, 1-tert-Butyl-3-ethyl-5-isobutyl-pyrazol-4-ol, 1-Phenyl-3-ethyl-5-isobutyl-pyrazol-4-ol, 1-(4-methylphenyl)-3-ethyl-5-isobutyl-pyrazol-4-ol, 1-(2-methylphenyl)-3-ethyl-5-isobutyl-pyrazol-4-ol, 1-(3-methylphenyl)-3-ethyl-5-isobutyl-pyrazol-4-ol, 1-(4-methoxyphenyl)-3-ethyl-5-isobutyl-pyrazol-4-ol, 1-Methyl-3-ethyl-5- tert-butyl-pyrazol-4-ol, 1-Ethyl-3-ethyl-5-tert-butyl-pyrazol-4-ol, 1-Propyl-3-ethyl-5- tert-butyl-pyrazol-4-ol, 1-Isopropyl-3-ethyl-5-tert-butyl-pyrazol-4-ol, 1-Butyl-3-ethyl-5- tert-butyl-pyrazol-4-ol, 1-Isobutyl-3-ethyl-5- tert-butyl-pyrazol-4-ol, 1-tert-Butyl-3-ethyl-5-tert-butyl-pyrazol-4-ol, 1-Phenyl-3-ethyl-5- tert-butyl-pyrazol-4-ol, 1-(4-methylphenyl)-3-ethyl-5- tert-butyl-pyrazol-4-ol, 1-(2-methylphenyl)-3-ethyl-5- tert-butyl-pyrazol-4-ol, 1-(3-methylphenyl)-3-ethyl-5- tert-butyl-pyrazol-4-ol, 1-(4-methoxyphenyl)-3-ethyl-5- phenyl-pyrazol-4-ol, 1-Methyl-3-ethyl-5-phenyl-pyrazol-4-ol, 1-Ethyl-3-ethyl-5-phenyl-pyrazol-4-ol, 1-Propyl-3-ethyl-5-phenyl-pyrazol-4-ol, 1-Isopropyl-3-ethyl-5-phenyl-pyrazol-4-ol, 1-Butyl-3-ethyl-5-phenyl-pyrazol-4-ol, 1-Isobutyl-3-ethyl-5-phenyl-pyrazol-4-ol, 1-tert-Butyl-3-ethyl-5-phenyl-pyrazol-4-ol, 1-Phenyl-3-ethyl-5-phenyl-pyrazol-4-ol, 1-(4-methylphenyl)-3-ethyl-5-phenyl-pyrazol-4-ol, 1-(2-methylphenyl)-3-ethyl-5-phenyl-pyrazol-4-ol, 1-(3-methylphenyl)-3-ethyl-5-phenyl-pyrazol-4-ol, 1-(4-methoxyphenyl)-3-ethyl-5- (2-methylphenyl)-pyrazol-4-ol, 1-Methyl-3-ethyl-5- (2-methylphenyl)-pyrazol-4-ol, 1-Ethyl-3-ethyl-5- (2-methylphenyl) -pyrazol-4-ol, 1-Propyl-3-ethyl-5- (2-methylphenyl)-pyrazol-4-ol, 1-Isopropyl-3-ethyl-5-(2-methyl-phenyl)-pyrazol-4-ol, 1-Butyl-3-ethyl-5- (2-methylphenyl)-pyrazol-4-ol, 1-Isobutyl-3-ethyl-5-(2-methylphenyl)-pyrazol-4-ol, 1-tert-Butyl-3-ethyl-5- (2-methylphenyl)-pyrazol-4-ol, 1-Phenyl-3-ethyl-5- (2-methylphenyl)-pyrazol-4-ol, 1-(4-methylphenyl)-3-ethyl-5- (2-methyl-phenyl)-pyrazol-4-ol, 1-(2-methylphenyl)-3-ethyl-5- (2-methylphenyl)-pyrazol-4-ol, 1-(3-methylphenyl)-3-ethyl-5-(2-methylphenyl)-pyrazol-4-ol, 1-(4-methoxyphenyl)-3-ethyl-5- (3-methylphenyl)-pyrazol-4-ol, 1-Methyl-3-ethyl-5-(3-methylphenyl)-pyrazol-4-ol, 1-Ethyl-3-ethyl-5-(3-methylphenyl)-pyrazol-4-ol, 1-Propyl-3-ethyl-5-(3-methylphenyl)-pyrazol-4-ol, 1-Isopropyl-3-ethyl-5-(3-methylphenyl)-pyrazol-4-ol, 1-Butyl-3-ethyl-5- (3-methylphenyl)-pyrazol-4-ol, 1-Isobutyl-3-ethyl-5-(3-methylphenyl)-pyrazol-4-ol, 1-tert-Butyl-3-ethyl-5- (3-methylphenyl) -pyrazol-4-ol, 1-Phenyl-3-ethyl-5-(3-methylphenyl)-pyrazol-4-ol, 1-(4-methylphenyl)-3-ethyl-5-(3-methylphenyl)-pyrazol-4-ol, 1-(2-methylphenyl)-3-ethyl-5- (3-methylphenyl)-pyrazol-4-ol, 1-(3-methylphenyl)-3-ethyl-5-(3-methylphenyl)-pyrazol-4-ol, 1-(4-methoxyphenyl)-3-ethyl-5-(3-methylphenyl)-pyrazol-4-ol, 1-Methyl-3-ethyl-5- (3-methyl-phenyl)-pyrazol-4-ol, 1-Ethyl-3-ethyl-5-(3-methylphenyl)-pyrazol-4-ol, 1-Propyl-3-ethyl-5- (3-methylphenyl) -pyrazol-4-ol, 1-Isopropyl-3-ethyl-5-(3-methylphenyl)-pyrazol-4-ol, 1-Butyl-3-ethyl-5-(3-methylphenyl)-pyrazol-4-ol, 1-Isobutyl-3-ethyl-5-(3-methylphenyl)-pyrazol-4-ol, 1-tert-Butyl-3-ethyl-5-butyl-pyrazol-4-ol, 1-Phenyl-3-ethyl-5-(3-methylphenyl)-pyrazol-4-ol, 1-(4-methylphenyl)-3-ethyl-5-(3-methylphenyl)-pyrazol-4-ol, 1-(2-methylphenyl)-3-ethyl-5- (3-methylphenyl)-pyrazol-4-ol, 1-(3-methylphenyl)-3-ethyl-5-(3-methylphenyl)-pyrazol-4-ol, 1-(4-methoxyphenyl)-3-ethyl-5-butyl-pyrazol-4-ol, 1-Methyl-3-ethyl-5- (4-methylphenyl)-pyrazol-4-ol, 1-Ethyl-3-ethyl-5-(4-methylphenyl)-pyrazol-4-ol, 1-Propyl-3-ethyl-5-(4-methylphenyl)-pyrazol-4-ol, 1-Isopropyl-3-ethyl-5-(4-methylphenyl)-pyrazol-4-ol, 1-Butyl-3-ethyl-5-(4-methylphenyl)-pyrazol-4-ol, 1-Isobutyl-3-ethyl-5-(4-methylphenyl)-pyrazol-4-ol, 1-tert-Butyl-3-ethyl-5-(4-methylphenyl)-pyrazol-4-ol, 1-Phenyl-3-ethyl-5- (4-methylphenyl)-pyrazol-4-ol, 1-(4-methylphenyl)-3-ethyl-5-(4-methylphenyl)-pyrazol-4-ol, 1-(2-methyl-phenyl)-3-ethyl-5-(4-methylphenyl)-pyrazol-4-ol, 1-(3-methylphenyl)-3-ethyl-5- (4-methyl-phenyl)-pyrazol-4-ol, 1-(4-methoxyphenyl)-3-ethyl-5-(4-methylphenyl)-pyrazol-4-ol, 1-Methyl-3-propyl-5-phenyl-pyrazol-4-ol, 1-Ethyl-3-propyl5-phenyl-pyrazol-4-ol, 1-Propyl-3-propyl-5-phenyl-pyrazol-4-ol, 1-Isopropyl-3-propyl-5-phenyl-pyrazol-4-ol, 1-Butyl-3-propyl-5-phenyl-pyrazol-4-ol, 1-Isobutyl-3-propyl-5-phenyl-pyrazol-4-ol, 1-tert-Butyl-3-propyl-5-phenyl-pyrazol-4-ol, 1-Phenyl-3-propyl-5-phenyl-pyrazol-4-ol, 1-(4-methylphenyl)-3-propyl-5-phenyl-pyrazol-4-ol, 1-(2-methylphenyl)-3-propyl-5-phenyl-pyrazol-4-ol, 1-(3-methylphenyl)-3-propyl-5-phenyl-pyrazol-4-ol, 1-(4-methoxyphenyl)-3-propyl-5-phenyl-pyrazol-4-ol, 1-Methyl-3-propyl-5-methyl-pyrazol-4-ol, 1-Ethyl-3-propyl-5-methyl-pyrazol-4-ol, 1-Propyl-3-propyl-5-methyl-pyrazol-4-ol, 1-Isopropyl-3-propyl-5-methyl-pyrazol-4-ol, 1-Butyl-3-propyl-5-methyl-pyrazol-4-ol, 1-Isobutyl-3-propyl-5-methyl-pyrazol-4-ol, 1-tert-Butyl-3-propyl-5-methyl-pyrazol-4-ol, 1-Phenyl-3-propyl-5-methyl-pyrazol-4-ol, 1-(4-methylphenyl)-3-propyl-5- me-thyl-pyrazol-4-ol, 1-(2-methylphenyl)-3-propyl-5-methyl-pyrazol-4-ol, 1-(3-methylphenyl)-3-propyl-5-methyl-pyrazol-4-ol, 1-(4-methoxyphenyl)-3-propyl-5-methyl-pyrazol-4-ol, 1-methyl-3-propyl-5-ethyl-pyrazol-4-ol, 1-ethyl-3-propyl-5-ethyl-pyrazol-4-ol, 1-propyl-3-propyl-5-ethyl-pyrazol-4-ol, 1-isopropyl-3-propyl-5-ethyl-pyrazol-4-ol, 1-butyl-3-propyl-5-ethyl-pyrazol-4-ol, 1-Isobutyl-3-propyl-5-ethyl-pyrazol-4-ol, 1-tert-butyl-3-propyl-5-ethyl-pyrazol-4-ol, 1-Phenyl-3-propyl-5-ethyl-pyrazol-4-ol, 1-(4-methylphenyl)-3-propyl-5-ethyl-pyrazol-4-ol, 1-(2-methylphenyl)-3-propyl-5-ethyl-pyrazol-4-ol, 1-(3-methylphenyl)-3-propyl-5-ethyl-pyrazol-4-ol, 1-(4-methoxyphenyl)-3-propyl-5-ethyl-pyrazol-4-ol, 1-Methyl-3-propyl-5-propyl-pyrazol-4-ol, 1-Ethyl-3-propyl-5-propyl-pyrazol-4-ol, 1-Propyl-3-propyl-5-propyl-pyrazol-4-ol, 1-Isopropyl-3-propyl-5-methyl-pyrazol-4-ol, 1-Butyl-3-propyl-5-propyl-pyrazol-4-ol, 1-Isobutyl-3-propyl-5-propyl-pyrazol-4-ol, 1-tert-Butyl-3-propyl-5-propyl-pyrazol-4-ol, 1-Phenyl-3-propyl-5-propyl-pyrazol-4-ol, 1-(4-methylphenyl)-3-propyl-5-propyl-pyrazol-4-ol, 1-(2-methyl-phenyl)-3-propyl-5-propyl-pyrazol-4-ol, 1-(3-methylphenyl)-3-propyl-5-propyl-pyrazol-4-ol, 1-(4-methoxyphenyl)-3-propyl-5-propyl-pyrazol-4-ol, 1-Methyl-3-propyl-5-isopropyl-pyrazol-4-ol, 1-Ethyl-3-propyl-5-isopropyl-pyrazol-4-ol, 1-Propyl-3-propyl-5-isopropyl-pyrazol-4-ol, 1-Isopropyl-3-propyl-5-isopropyl-pyrazol-4-ol, 1-Butyl-3-propyl-5-isopropyl-pyrazol-4-ol, 1-Isobutyl-3-propyl-5-isopropyl-pyrazol-4-ol, 1-tert-Butyl-3-propyl-5-isopropyl-pyrazol-4-ol, 1-Phenyl-3-propyl-5-isopropyl-pyrazol-4-ol, 1-(4-methylphenyl)-3-propyl-5-isopropyl -pyrazol-4-ol, 1-(2-methylphenyl)-3-propyl-5-isopropyl-pyrazol-4-ol, 1-(3-methylphenyl)-3-propyl-5-isopropyl-pyrazol-4-ol, 1-(4-methoxyphenyl)-3-propyl-5-isopropyl-pyrazol-4-ol, 1-Methyl-3-propyl-5-butyl-pyrazol-4-ol, 1-Ethyl-3-propyl-5-butyl-pyrazol-4-ol, 1-Propyl-3-propyl-5-butyl-pyrazol-4-ol, 1-Isopropyl-3-propyl-5-butyl-pyrazol-4-ol, 1-Butyl-3-propyl-5-butyl-pyrazol-4-ol, 1-Isobutyl-3-propyl-5-butyl-pyrazol-4-ol, 1-tert-Butyl-3-propyl-5-butyl-pyrazol-4-ol, 1-Phenyl-3-propyl-5-butyl-pyrazol-4-ol, 1-(4-methylphenyl)-3-propyl-5-butyl-pyrazol-4-ol, 1-(2-methyl-phenyl)-3-propyl-5-butyl-pyrazol-4-ol, 1-(3-methylphenyl)-3-propyl-5-butyl-pyrazol-4-ol, 1-(4-methoxyphenyl)-3-propyl-5-butyl-pyrazol-4-ol, 1-Methyl-3-propyl-5-isobutyl-pyrazol-4-ol, 1-Ethyl-3-propyl-5-isobutyl-pyrazol-4-ol, 1-Propyl-3-propyl-5-isobutyl-pyrazol-4-ol, 1-Isopropyl-3-propyl-5-isobutyl-pyrazol-4-ol, 1-Butyl-3-propyl-5-isobutyl-pyrazol-4-ol, 1-Isobutyl-3-propyl-5-isobutyl-pyrazol-4-ol, 1-tert-Butyl-3-propyl-5-isobutyl-pyrazol-4-ol, 1-Phenyl-3-propyl-5-isobutyl-pyrazol-4-ol, 1-(4-methylphenyl)-3-propyl-5-isobutyl-pyrazol-4-ol, 1-(2-methylphenyl)-3-propyl-5-isobutyl-pyrazol-4-ol, 1-(3-methylphenyl)-3-propyl-5- isobutyl-pyrazol-4-ol, 1-(4-methoxyphenyl)-3-propyl-5-isobutyl-pyrazol-4-ol, 1-Methyl-3-propyl-5- tert-butyl-pyrazol-4-ol, 1-Ethyl-3-propyl-5-tert-butyl-pyrazol-4-ol, 1-Propyl-3-propyl-5- tert-butyl-pyrazol-4-ol, 1-Isopropyl-3-propyl-5-tert-butyl-pyrazol-4-ol, 1-Butyl-3-propyl-5-tert-butyl-pyrazol-4-ol, 1-Isobutyl-3-propyl-5-tert-butyl-pyrazol-4-ol, 1-tert-Butyl-3-propyl-5- tert-butyl-pyrazol-4-ol, 1-Phenyl-3-propyl-5-tert-butyl-pyrazol-4-ol, 1-(4-methylphenyl)-3-propyl-5- tert-butyl-pyrazol-4-ol, 1-(2-methylphenyl)-3-propyl-5-tert-butyl-pyrazol-4-ol, 1-(3-methylphenyl)-3-propyl-5-tert-butyl-pyrazol-4-ol, 1-(4-methoxyphenyl)-3-propyl-5-phenyl-pyrazol-4-ol, 1-Methyl-3-propyl-5-phenyl-pyrazol-4-ol, 1-Ethyl-3-propyl-5-phenyl-pyrazol-4-ol, 1-Propyl-3-propyl-5-phenyl-pyrazol-4-ol, 1-Isopropyl-3-propyl-5-phenyl-pyrazol-4-ol, 1-Butyl-3-propyl-5-phenyl-pyrazol-4-ol, 1-Isobutyl-3-propyl-5-phenyl-pyrazol-4-ol, 1-tert-Butyl-3-propyl-5-phenyl-pyrazol-4-ol, 1-Phenyl-3-propyl-5-phenyl-pyrazol-4-ol, 1-(4-methylphenyl)-3-propyl-5-phenyl-pyrazol-4-ol, 1-(2-methylphenyl)-3-propyl-5-phenyl-pyrazol-4-ol, 1-(3-methyl-phenyl)-3-propyl-5-phenyl-pyrazol-4-ol, 1-(4-methoxyphenyl)-3-propyl-5- (2-methylphenyl)-pyrazol-4-ol, 1-Methyl-3-propyl-5-(2-methylphenyl)-pyrazol-4-ol, 1-Ethyl-3-propyl-5- (2-methylphenyl)-pyrazol-4-ol, 1-Propyl-3-propyl-5-(2-methylphenyl)-pyrazol-4-ol, 1-Isopropyl-3-propyl-5-(2-methylphenyl)-pyrazol-4-ol, 1-Butyl-3-propyl-5-(2-methylphenyl)-pyrazol-4-ol, 1-Isobutyl-3-propyl-5-(2-methylphenyl)-pyrazol-4-ol, 1-tert-Butyl-3-propyl-5- (2-methyl-phenyl)-pyrazol-4-ol, 1-Phenyl-3-propyl-5-(2-methylphenyl)-pyrazol-4-ol, 1-(4-methyl-phenyl)-3-propyl-5-(2-methylphenyl)-pyrazol-4-ol, 1-(2-methylphenyl)-3-propyl-5- (2-methylphenyl)-pyrazol-4-ol, 1-(3-methylphenyl)-3-propyl-5-(2-methylphenyl)-pyrazol-4-ol, 1-(4-methoxyphenyl)-3-propyl-5-(3-methylphenyl)-pyrazol-4-ol, 1-Methyl-3-propyl-5- (3-methylphenyl)-pyrazol-4-ol, 1-Ethyl-3-propyl-5-(3-methylphenyl)-pyrazol-4-ol, 1-Propyl-3-propyl-5-(3-methylphenyl)-pyrazol-4-ol, 1-Isopropyl-3-propyl-5-(3-methylphenyl) -pyrazol-4-ol, 1-Butyl-3-propyl-5-(3-methylphenyl)-pyrazol-4-ol, 1-Isobutyl-3-propyl-5- (3-methylphenyl) -pyrazol-4-ol, 1-tert-Butyl-3-propyl-5-(3-methylphenyl)-pyrazol-4-ol, 1-Phenyl-3-propyl-5- (3-methylphenyl)-pyrazol-4-ol, 1-(4-methylphenyl)-3-propyl-5-(3-methylphenyl)-pyrazol-4-ol, 1-(2-methylphenyl)-3-propyl-5-(3-methylphenyl)-pyrazol-4-ol, 1-(3-methylphenyl)-3-propyl-5-(3-methylphenyl)-pyrazol-4-ol, 1-(4-methoxyphenyl)-3-propyl-5- (3-methylphenyl) -pyrazol-4-ol, 1-Methyl-3-propyl-5-(3-methylphenyl)-pyrazol-4-ol, 1-Ethyl-3-propyl-5- (3-methylphenyl)-pyrazol-4-ol, 1-Propyl-3-propyl-5-(3-methylphenyl)-pyrazol-4-ol, 1-Isopropyl-3-propyl-5-(3-methylphenyl)-pyrazol-4-ol, 1-Butyl-3-propyl-5-(3-methylphenyl)-pyrazol-4-ol, 1-Isobutyl-3-propyl-5-(3-methylphenyl)-pyrazol-4-ol, 1-tert-Butyl-3-propyl-5-butyl-pyrazol-4-ol, 1-Phenyl-3-propyl-5-(3-methylphenyl)-pyrazol-4-ol, 1-(4-methylphenyl)-3-propyl-5- (3-methylphenyl)-pyrazol-4-ol, 1-(2-methylphenyl)-3-propyl-5-(3-methylphenyl)-pyrazol-4-ol, 1-(3-methyl-phenyl)-3-propyl-5-(3-methylphenyl)-pyrazol-4-ol, 1-(4-methoxyphenyl)-3-propyl-5-butyl-pyrazol-4-ol, 1-Methyl-3-propyl-5-(4-methylphenyl)-pyrazol-4-ol, 1-Ethyl-3-propyl-5- (4-methylphenyl)-pyrazol-4-ol, 1-Propyl-3-propyl-5-(4-methylphenyl)-pyrazol-4-ol, 1-Isopropyl-3-propyl-5-(4-methylphenyl)-pyrazol-4-ol, 1-Butyl-3-propyl-5-(4-methylphenyl)-pyrazol-4-ol, 1-Isobutyl-3-propyl-5-(4-methylphenyl)-pyrazol-4-ol, 1-tert-Butyl-3-propyl-5- (4-methyl-phenyl)-pyrazol-4-ol, 1-Phenyl-3-propyl-5-(4-methylphenyl)-pyrazol-4-ol, 1-(4-methyl-phenyl)-3-propyl-5-(4-methylphenyl)-pyrazol-4-ol, 1-(2-methylphenyl)-3-propyl-5- (4-methylphenyl)-pyrazol-4-ol, 1-(3-methylphenyl)-3-propyl-5-(4-methylphenyl)-pyrazol-4-ol, 1-(4-methoxyphenyl)-3-propyl-5-(4-methylphenyl)-pyrazol-4-ol, 1-Methyl-3-butyl-5-phenyl-pyrazol-4-ol, 1-Ethyl-3-butyl5-phenyl-pyrazol-4-ol, 1-Propyl-3-butyl-5-phenyl-pyrazol-4-ol, 1-Isopropyl-3-butyl-5-phenyl-pyrazol-4-ol, 1-Butyl-3-butyl-5-phenyl-pyrazol-4-ol, 1-Isobutyl-3-butyl-5-phenyl-pyrazol-4-ol, 1-tert-Butyl-3-butyl-5-phenyl-pyrazol-4-ol, 1-Phenyl-3-butyl-5-phenyl-pyrazol-4-ol, 1-(4-methylphenyl)-3-butyl-5-phenyl-pyrazol-4-ol, 1-(2-methylphenyl)-3-butyl-5-phenyl-pyrazol-4-ol, 1-(3-methylphenyl)-3-butyl-5-phenyl-pyrazol-4-ol, 1-(4-methoxy-phenyl)-3-butyl-5-phenyl-pyrazol-4-ol, 1-Methyl-3-butyl-5-methyl-pyrazol-4-ol, 1-Ethyl-3-butyl-5-methyl-pyrazol-4-ol, 1-Propyl-3-butyl-5-methyl-pyrazol-4-ol, 1-Isopropyl-3-butyl-5-methyl-pyrazol-4-ol, 1-Butyl-3-butyl-5-methyl-pyrazol-4-ol, 1-Isobutyl-3-butyl-5-methyl-pyrazol-4-ol, 1-tert-Butyl-3-butyl-5-methyl-pyrazol-4-ol, 1-Phenyl-3-butyl-5-methyl-pyrazol-4-ol, 1-(4-methylphenyl)-3-butyl-5-methyl-pyrazol-4-ol, 1-(2-methylphenyl)-3-butyl-5-methyl -pyrazol-4-ol, 1-(3-methylphenyl)-3-butyl-5-methyl-pyrazol-4-ol, 1-(4-methoxyphenyl)-3-butyl-5-methyl-pyrazol-4-ol, 1-methyl-3-butyl-5-ethyl-pyrazol-4-ol, 1-ethyl-3-butyl-5-ethyl-pyrazol-4-ol, 1-propyl-3-butyl-5-ethyl-pyrazol-4-ol, 1-isopropyl-3-butyl-5-ethyl-pyrazol-4-ol, 1-butyl-3-butyl-5-ethyl-pyrazol-4-ol, 1-Isobutyl-3-butyl-5-ethyl-pyrazol-4-ol, 1-tert-butyl-3-butyl-5-ethyl-pyrazol-4-ol, 1-Phenyl-3-butyl-5-ethyl-pyrazol-4-ol, 1-(4-methylphenyl)-3-butyl-5-ethyl-pyrazol-4-ol, 1-(2-methylphenyl)-3-butyl-5-ethyl-pyrazol-4-ol, 1-(3-methylphenyl)-3-butyl-5-ethyl-pyrazol-4-ol, 1-(4-methoxyphenyl)-3-butyl-5-ethyl-pyrazol-4-ol, 1-Methyl-3-butyl-5-propyl-pyrazol-4-ol, 1-Ethyl-3-butyl-5-propyl-pyrazol-4-ol, 1-Propyl-3-butyl-5-propyl-pyrazol-4-ol, 1-Isopropyl-3-butyl-5-methyl-pyrazol-4-ol, 1-Butyl-3-butyl-5-propyl-pyrazol-4-ol, 1-Isobutyl-3-butyl-5-propyl-pyrazol-4-ol, 1-tert-Butyl-3-butyl-5-propyl-pyrazol-4-ol, 1-Phenyl-3-butyl-5-propyl-pyrazol-4-ol, 1-(4-methylphenyl)-3-butyl-5-propyl-pyrazol-4-ol, 1-(2-methylphenyl)-3-butyl-5-propyl-pyrazol-4-ol, 1-(3-methylphenyl)-3-butyl-5-propyl-pyrazol-4-ol, 1-(4-methoxyphenyl)-3-butyl-5-propyl-pyrazol-4-ol, 1-Methyl-3-butyl-5-isopropyl-pyrazol-4-ol, 1-Ethyl-3-butyl-5-isopropyl-pyrazol-4-ol, 1-Propyl-3-butyl-5-isopropyl-pyrazol-4-ol, 1-Isopropyl-3-butyl-5-isopropyl-pyrazol-4-ol, 1-Butyl-3-butyl-5-isopropyl-pyrazol-4-ol, 1-Isobutyl-3-butyl-5-isopropyl-pyrazol-4-ol, 1-tert-Butyl-3-butyl-5-isopropyl-pyrazol-4-ol, 1-Phenyl-3-butyl-5-isopropyl-pyrazol-4-ol, 1-(4-methylphenyl)-3-butyl-5-isopropyl-pyrazol-4-ol, 1-(2-methyl-phenyl)-3-butyl-5-isopropyl-pyrazol-4-ol, 1-(3-methylphenyl)-3-butyl-5-isopropyl -pyrazol-4-ol, 1-(4-methoxyphenyl)-3-butyl-5-isopropyl-pyrazol-4-ol, 1-Methyl-3-butyl-5-butyl -pyrazol-4-ol, 1-Ethyl-3-butyl-5-butyl-pyrazol-4-ol, 1-Propyl-3-butyl-5-butyl-pyrazol-4-ol, 1-Isopropyl-3-butyl-5-butyl-pyrazol-4-ol, 1-Butyl-3-butyl-5-butyl-pyrazol-4-ol, 1-Isobutyl-3-butyl-5- butyl-pyrazol-4-ol, 1-tert-Butyl-3-butyl-5-butyl-pyrazol-4-ol, 1-Phenyl-3-butyl-5-butyl-pyrazol-4-ol, 1-(4-methylphenyl)-3-butyl-5-butyl-pyrazol-4-ol, 1-(2-methylphenyl)-3-butyl-5- butyl-pyrazol-4-ol, 1-(3-methylphenyl)-3-butyl-5-butyl-pyrazol-4-ol, 1-(4-methoxyphenyl)-3-butyl-5- butyl-pyrazol-4-ol, 1-Methyl-3-butyl-5-isobutyl-pyrazol-4-ol, 1-Ethyl-3-butyl-5-isobutyl-pyrazol-4-ol, 1-Propyl-3-butyl-5-isobutyl-pyrazol-4-ol, 1-Isopropyl-3-butyl-5-isobutyl-pyrazol-4-ol, 1-Butyl-3-butyl-5-isobutyl-pyrazol-4-ol, 1-Isobutyl-3-butyl-5-isobutyl-pyrazol-4-ol, 1-tert-Butyl-3-butyl-5-isobutyl-pyrazol-4-ol, 1-Phenyl-3-butyl-5-isobutyl-pyrazol-4-ol, 1-(4-methylphenyl)-3-butyl-5-isobutyl-pyrazol-4-ol, 1-(2-methylphenyl)-3-butyl-5-isobutyl-pyrazol-4-ol, 1-(3-methylphenyl)-3-butyl-5-isobutyl-pyrazol-4-ol, 1-(4-methoxyphenyl)-3-butyl-5-isobutyl-pyrazol-4-ol, 1-Methyl-3-butyl-5-tert-butyl-pyrazol-4-ol, , 1-Ethyl-3-butyl-5-tert-butyl-pyrazol-4-ol, 1-Propyl-3-butyl-5-tert-butyl-pyrazol-4-ol, 1-Isopropyl-3-butyl-5-tert-butyl-pyrazol-4-ol, 1-Butyl-3-butyl-5-tert-butyl-pyrazol-4-ol, 1-Isobutyl-3-butyl-5- tert-butyl-pyrazol-4-ol, 1-tert-Butyl-3-butyl-5-tert-butyl-pyrazol-4-ol, 1-Phenyl-3-butyl-5- tert-butyl-pyrazol-4-ol, 1-(4-methylphenyl)-3-butyl-5-tert-butyl-pyrazol-4-ol, 1-(2-methylphenyl)-3-butyl-5- tert-butyl -pyrazol-4-ol, 1-(3-methylphenyl)-3-butyl-5-tert-butyl-pyrazol-4-ol, 1-(4-methoxyphenyl)-3-butyl-5-phenyl-pyrazol-4-ol, 1-Methyl-3-butyl-5- phenyl-pyrazol-4-ol, 1-Ethyl-3-butyl-5-phenyl-pyrazol-4-ol, 1-Propyl-3-butyl-5-phenyl-pyrazol-4-ol, 1-Isopropyl-3-butyl-5-phenyl-pyrazol-4-ol, 1-Butyl-3-butyl-5-phenyl-pyrazol-4-ol, 1-Isobutyl-3-butyl-5-phenyl-pyrazol-4-ol, 1-tert-Butyl-3-butyl-5-phenyl-pyrazol-4-ol, 1-Phenyl-3-butyl-5-phenyl -pyrazol-4-ol, 1-(4-methylphenyl)-3-butyl-5-phenyl-pyrazol-4-ol, 1-(2-methylphenyl)-3-butyl-5-phenyl-pyrazol-4-ol, 1-(3-methylphenyl)-3-butyl-5-phenyl-pyrazol-4-ol, 1-(4-methoxyphenyl)-3-butyl-5-(2-methylphenyl)-pyrazol-4-ol, 1-Methyl-3-butyl-5- (2-methylphenyl)-pyrazol-4-ol, , 1-Ethyl-3-butyl-5-(2-methylphenyl)-pyrazol-4-ol, 1-Propyl-3-butyl-5-(2-methylphenyl)-pyrazol-4-ol, 1-Isopropyl-3-butyl-5-(2-methylphenyl)-pyrazol-4-ol, 1-Butyl-3-butyl-5-(2-methylphenyl)-pyrazol-4-ol, 1-Isobutyl-3-butyl-5-(2-methylphenyl)-pyrazol-4-ol, 1-tert-Butyl-3-butyl-5- (2-methylphenyl)-pyrazol-4-ol, 1-Phenyl-3-butyl-5- (2-methylphenyl)-pyrazol-4-ol, 1-(4-methylphenyl)-3-butyl-5-(2-methylphenyl)-pyrazol-4-ol, 1-(2-methylphenyl)-3-butyl-5- (2-methylphenyl)-pyrazol-4-ol, 1-(3-methylphenyl)-3-butyl-5-(2-methylphenyl-pyrazol-4-ol, 1-(4-methoxyphenyl)-3-butyl-5-(3-methylphenyl)-pyrazol-4-ol, 1-Methyl-3-butyl-5- (3-methylphenyl)-pyrazol-4-ol, , 1-Ethyl-3-butyl-5-(3-methylphenyl)-pyrazol-4-ol, 1-Propyl-3-butyl-5-(3-methylphenyl)-pyrazol-4-ol, 1-Isopropyl-3-butyl-5-(3-methylphenyl)-pyrazol-4-ol, 1-Butyl-3-butyl-5-(3-methylphenyl)-pyrazol-4-ol, 1-Isobutyl-3-butyl-5- (3-methylphenyl)-pyrazol-4-ol, 1-tert-Butyl-3-butyl-5-(3-methylphenyl)-pyrazol-4-ol, 1-Phenyl-3-butyl-5- (3-methylphenyl)-pyrazol-4-ol, 1-(4-methylphenyl)-3-butyl-5-(3-methylphenyl)-pyrazol-4-ol, 1-(2-methylphenyl)-3-butyl-5-(3-methylphenyl)-pyrazol-4-ol, 1-(3-methylphenyl)-3-butyl-5- (3-methylphenyl)-pyrazol-4-ol, 1-(4-methoxyphenyl)-3-butyl-5-(3-methylphenyl)-pyrazol-4-ol, 1-Methyl-3-butyl-5-(3-methylphenyl)-pyrazol-4-ol, , 1-Ethyl-3-butyl-5-(3-methylphenyl)-pyrazol-4-ol, 1-Propyl-3-butyl-5-(3-methylphenyl)-pyrazol-4-ol, 1-Isopropyl-3-butyl-5- (3-methylphenyl)-pyrazol-4-ol, 1-Butyl-3-butyl-5-(3-methylphenyl)-pyrazol-4-ol,
1-Isobutyl-3-butyl-5-(3-methylphenyl)-pyrazol-4-ol, 1-tert-Butyl-3-butyl-5-butyl-pyrazol-4-ol, 1-Phenyl-3-butyl-5-(3-methylphenyl)-pyrazol-4-ol, 1-(4-methylphenyl)-3-butyl-5- (3-methylphenyl)-pyrazol-4-ol, 1-(2-methylphenyl)-3-butyl-5-(3-methylphenyl)-pyrazol-4-ol, 1-(3-methylphenyl)-3-butyl-5-(3-methylphenyl)-pyrazol-4-ol, 1-(4-methoxyphenyl)-3-butyl-5-butyl-pyrazol-4-ol, 1-Methyl-3-butyl-5-(4-methylphenyl)-pyrazol-4-ol, 1-Ethyl-3-butyl-5- (4-methylphenyl)-pyrazol-4-ol, 1-Propyl-3-butyl-5-(4-methylphenyl)-pyrazol-4-ol, 1-Isopropyl-3-butyl-5-(4-methylphenyl)-pyrazol-4-ol, 1-Butyl-3-butyl-5-(4-methylphenyl)-pyrazol-4-ol, 1-Isobutyl- 3-butyl-5-(4-methylphenyl)-pyrazol-4-ol, 1-tert-Butyl-3-butyl-5- (4-methylphenyl) - pyrazol-4-ol, 1-Phenyl-3-butyl-5-(4-methylphenyl)-pyrazol-4-ol, 1-(4-methylphenyl)-3-butyl-5-(4-methylphenyl)-pyrazol-4-ol, 1-(2-methylphenyl)-3-butyl-5- (4-methylphenyl) -pyrazol-4-ol, 1-(3-methylphenyl)-3-butyl-5-(4-methylphenyl)-pyrazol-4-ol, 1-(4-methoxyphenyl)-3-butyl-5-(4-methylphenyl)-pyrazol-4-ol, 1-Methyl-3-isobutyl-5-phenyl-pyrazol-4-ol, , 1-Ethyl-3-isobutyl5-phenyl-pyrazol-4-ol, 1-Propyl-3-isobutyl-5-phenyl-pyrazol-4-ol, 1-Isopropyl-3-isobutyl-5-phenyl-pyrazol-4-ol, 1-Butyl-3-isobutyl-5-phenyl-pyrazol-4-ol, 1-Isobutyl-3-isobutyl-5-phenyl-pyrazol-4-ol, 1-tert-Butyl-3-isobutyl-5-phenyl-pyrazol-4-ol, 1-Phenyl-3-isobutyl-5-phenyl-pyrazol-4-ol, 1-(4-methylphenyl)-3-isobutyl-5-phenyl-pyrazol-4-ol, 1-(2-methylphenyl)-3-isobutyl-5-phenyl-pyrazol-4-ol, 1-(3-methylphenyl)-3-isobutyl-5-phenyl-pyrazol-4-ol, 1-(4-methoxyphenyl)-3-isobutyl-5-phenyl-pyrazol-4-ol, 1-Methyl-3-isobutyl-5-methyl-pyrazol-4-ol, , 1-Ethyl-3-isobutyl-5-methyl-pyrazol-4-ol, 1-Propyl-3-isobutyl-5- methyl-pyrazol-4-ol, 1-Isopropyl-3-isobutyl-5-methyl-pyrazol-4-ol, 1-Butyl-3-isobutyl-5- methyl-pyrazol-4-ol, 1-Isobutyl-3-isobutyl-5-methyl-pyrazol-4-ol, 1-tert-Butyl-3-isobutyl-5-methyl-pyrazol-4-ol, 1-Phenyl-3-isobutyl-5-methyl-pyrazol-4-ol, 1-(4-methylphenyl)-3-isobutyl-5-methyl-pyrazol-4-ol, 1-(2-methylphenyl)-3-isobutyl-5-methyl-pyrazol-4-ol, 1-(3-methylphenyl)-3-isobutyl-5-methyl-pyrazol-4-ol, 1-(4-methoxyphenyl)-3-isobutyl-5-methyl-pyrazol-4-ol, 1-methyl-3-isobutyl-5-ethyl-pyrazol-4-ol, , 1-ethyl-3-isobutyl-5-ethyl-pyrazol-4-ol, 1-propyl-3-isobutyl-5-thyl-pyrazol-4-ol, 1-isopropyl-3-isobutyl-5-ethyl-pyrazol-4-ol, 1-butyl-3-isobutyl-5-ethyl-pyrazol-4-ol, 1-Isobutyl-3-isobutyl-5-ethyl-pyrazol-4-ol, 1-tert-butyl-3-isobutyl-5-ethyl-pyrazol-4-ol, 1-Phenyl-3-isobutyl-5-ethyl-pyrazol-4-ol, 1-(4-methylphenyl)-3-isobutyl-5-ethyl-pyrazol-4-ol, 1-(2-methylphenyl)-3-isobutyl-5- ethyl-pyrazol-4-ol, 1-(3-methylphenyl)-3-isobutyl-5-ethyl-pyrazol-4-ol, 1-(4-methoxyphenyl)-3-isobutyl-5-ethyl-pyrazol-4-ol, 1-Methyl-3-isobutyl-5-propyl-pyrazol-4-ol, , 1-Ethyl-3-isobutyl-5-propyl-pyrazol-4-ol, 1-Propyl-3-isobutyl-5-propyl-pyrazol-4-ol,
1-Isopropyl-3-isobutyl-5-methyl-pyrazol-4-ol, 1-Butyl-3-isobutyl-5-propyl-pyrazol-4-ol, 1-Isobutyl- 3-isobutyl-5-propyl-pyrazol-4-ol, 1-tert-Butyl-3-isobutyl-5-propyl-pyrazol-4-ol, 1-Phenyl-3-isobutyl-5-propyl-pyrazol-4-ol, 1-(4-methylphenyl)-3-isobutyl-5- propyl -pyrazol-4-ol, 1-(2-methylphenyl)-3-isobutyl-5-propyl-pyrazol-4-ol, 1-(3-methylphenyl)-3-isobutyl-5-propyl-pyrazol-4-ol, 1-(4-methoxyphenyl)-3-isobutyl-5-propyl-pyrazol-4-ol, 1-Methyl-3-isobutyl-5-isopropyl-pyrazol-4-ol, , 1-Ethyl-3-isobutyl-5- isopropyl-pyrazol-4-ol, 1-Propyl-3-isobutyl-5-isopropyl-pyrazol-4-ol, 1-Isopropyl-3-isobutyl-5-isopropyl-pyrazol-4-ol, 1-Butyl-3-isobutyl-5-isopropyl-pyrazol-4-ol, 1-Isobutyl-3-isobutyl-5-isopropyl-pyrazol-4-ol, 1-tert-Butyl-3-isobutyl-5-isopropyl-pyrazol-4-ol, 1-Phenyl-3-isobutyl-5- isopropyl -pyrazol-4-ol, 1-(4-methylphenyl)-3-isobutyl-5-isopropyl-pyrazol-4-ol, 1-(2-methylphenyl)-3-isobutyl-5-isopropyl-pyrazol-4-ol, 1-(3-methylphenyl)-3-isobutyl-5-isopropyl-pyrazol-4-ol, 1-(4-methoxyphenyl)-3-isobutyl-5-isopropyl-pyrazol-4-ol, 1-Methyl-3-isobutyl-5- butyl -pyrazol-4-ol, , 1-Ethyl-3-isobutyl-5-butyl-pyrazol-4-ol, 1-Propyl-3-isobutyl-5-butyl-pyrazol-4-ol, 1-Isopropyl-3-isobutyl-5-butyl-pyrazol-4-ol, 1-Butyl-3-isobutyl-5-butyl-pyrazol-4-ol, 1-Isobutyl-3-isobutyl-5-butyl-pyrazol-4-ol, 1-tert-Butyl-3-isobutyl-5-butyl-pyrazol-4-ol, 1-Phenyl-3-isobutyl-5-butyl-pyrazol-4-ol, 1-(4-methylphenyl)-3-isobutyl-5-butyl-pyrazol-4-ol, 1-(2-methylphenyl)-3-isobutyl-5-butyl-pyrazol-4-ol, 1-(3-methylphenyl)-3-isobutyl-5-butyl-pyrazol-4-ol, 1-(4-methoxyphenyl)-3-isobutyl-5-butyl-pyrazol-4-ol, 1-Methyl-3-isobutyl-5-isobutyl-pyrazol-4-ol, , 1-Ethyl-3-isobutyl-5-isobutyl-pyrazol-4-ol, 1-Propyl-3-isobutyl-5-isobutyl-pyrazol-4-ol, 1-Isopropyl-3-isobutyl-5-isobutyl-pyrazol-4-ol, 1-Butyl-3-isobutyl-5-isobutyl-pyrazol-4-ol, 1-Isobutyl-3-isobutyl-5- isobutyl-pyrazol-4-ol, 1-tert-Butyl-3-isobutyl-5-isobutyl-pyrazol-4-ol, 1-Phenyl-3-isobutyl-5-isobutyl-pyrazol-4-ol, 1-(4-methylphenyl)-3-isobutyl-5-isobutyl-pyrazol-4-ol, 1-(2-methylphenyl)-3-isobutyl-5-isobutyl-pyrazol-4-ol, 1-(3-methyl-phenyl)-3-isobutyl-5- isobutyl-pyrazol-4-ol, 1-(4-methoxyphenyl)-3-isobutyl-5- isobutyl-pyrazol-4-ol, 1-Methyl-3-isobutyl-5- tert-butyl-pyrazol-4-ol, , 1-Ethyl-3-isobutyl-5- tert-butyl-pyrazol-4-ol, 1-Propyl-3-isobutyl-5- tert-butyl-pyrazol-4-ol, 1-Isopropyl-3-isobutyl-5-tert-butyl -pyrazol-4-ol, 1-Butyl-3-isobutyl-5-tert-butyl-pyrazol-4-ol, 1-Isobutyl-3-isobutyl-5- tert-butyl-pyrazol-4-ol, 1-tert-Butyl-3-isobutyl-5-tert-butyl-pyrazol-4-ol, 1-Phenyl-3-isobutyl-5- tert-butyl -pyrazol-4-ol, 1-(4-methylphenyl)-3-isobutyl-5-tert-butyl-pyrazol-4-ol, 1-(2-methylphenyl)-3-isobutyl-5-tert-butyl-pyrazol-4-ol, 1-(3-methylphenyl)-3-isobutyl-5-tert-butyl-pyrazol-4-ol, 1-(4-methoxyphenyl)-3-isobutyl-5-phenyl-pyrazol-4-ol, 1-Methyl-3-isobutyl-5- phenyl -pyrazol-4-ol, , 1-Ethyl-3-isobutyl-5-phenyl-pyrazol-4-ol, 1-Propyl-3-isobutyl-5-phenyl-pyrazol-4-ol, 1-Isopropyl-3-isobutyl-5-phenyl-pyrazol-4-ol, 1-Butyl-3-isobutyl-5-phenyl-pyrazol-4-ol, 1-Isobutyl- 3-isobutyl-5-phenyl-pyrazol-4-ol, 1-tert-Butyl-3-isobutyl-5-phenyl-pyrazol-4-ol, 1-Phenyl-3-isobutyl-5-phenyl-pyrazol-4-ol, 1-(4-methylphenyl)-3-isobutyl-5- phenyl -pyrazol-4-ol, 1-(2-methylphenyl)-3-isobutyl-5-phenyl-pyrazol-4-ol, 1-(3-methylphenyl)-3-isobutyl-5-phenyl-pyrazol-4-ol, 1-(4-methoxyphenyl)-3-isobutyl-5-(2-methylphenyl)-pyrazol-4-ol, 1-Methyl-3-isobutyl-5-(2-methylphenyl)-pyrazol-4-ol, , 1-Ethyl-3-isobutyl-5- (2-methylphenyl)-pyrazol-4-ol, 1-Propyl-3-isobutyl-5-(2-methylphenyl)-pyrazol-4-ol, 1-Isopropyl-3-isobutyl-5-(2-methylphenyl)-pyrazol-4-ol, 1-Butyl-3-isobutyl-5- (2-methylphenyl)-pyrazol-4-ol, 1-Isobutyl- 3-isobutyl-5-(2-methylphenyl)-pyrazol-4-ol, 1-tert-Butyl-3-isobutyl-5- (2-methyl-phenyl)-pyrazol-4-ol, 1-Phenyl-3-isobutyl-5-(2-methylphenyl)-pyrazol-4-ol, 1-(4-methyl-phenyl)-3-isobutyl-5-(2-methylphenyl)-pyrazol-4-ol, 1-(2-methylphenyl)-3-isobutyl-5- (2-methylphenyl)-pyrazol-4-ol, 1-(3-methylphenyl)-3-isobutyl-5-(2-methylphenyl)-pyrazol-4-ol, 1-(4-methoxyphenyl)-3-isobutyl-5-(3-methylphenyl)-pyrazol-4-ol, 1-Methyl-3-isobutyl-5- (3-methylphenyl)-pyrazol-4-ol, 1-Ethyl-3-isobutyl-5-(3-methylphenyl)-pyrazol-4-ol, 1-Propyl-3-isobutyl-5-(3-methylphenyl)-pyrazol-4-ol, 1-Isopropyl-3-isobutyl-5-(3-methylphenyl) - pyrazol-4-ol, 1-Butyl-3-isobutyl-5-(3-methylphenyl)-pyrazol-4-ol, 1-Isobutyl-3-isobutyl-5- (3-methylphenyl)-pyrazol-4-ol, 1-tert-Butyl-3-isobutyl-5-(3-methylphenyl)-pyrazol-4-ol, 1-Phenyl-3-isobutyl-5-(3-methylphenyl)-pyrazol-4-ol, 1-(4-methylphenyl)-3-isobutyl-5- (3-methylphenyl)-pyrazol-4-ol, 1-(2-methylphenyl)-3-isobutyl-5-(3-methylphenyl)-pyrazol-4-ol, 1-(3-methylphenyl)-3-isobutyl-5-(3-methylphenyl)-pyrazol-4-ol, 1-(4-methoxyphenyl)-3-isobutyl-5-(3-methylphenyl)-pyrazol-4-ol, 1-Methyl-3-isobutyl-5- (3-methylphenyl) -pyrazol-4-ol, , 1-Ethyl-3-isobutyl-5- (3-methylphenyl) -pyrazol-4-ol, 1-Propyl-3-isobutyl-5- (3-methyl-phenyl)-pyrazol-4-ol, 1-Isopropyl-3-isobutyl-5-(3-methylphenyl)-pyrazol-4-ol, 1-Butyl-3-isobutyl-5-(3-methylphenyl)-pyrazol-4-ol, 1-Isobutyl-3-isobutyl-5- (3-methylphenyl) -pyrazol-4-ol, 1-tert-Butyl-3-isobutyl-5-butyl-pyrazol-4-ol, 1-Phenyl-3-isobutyl-5- (3-methylphenyl)-pyrazol-4-ol, 1-(4-methylphenyl)-3-isobutyl-5-(3-methylphenyl)-pyrazol-4-ol, 1-(2-methyl-phenyl)-3-isobutyl-5-(3-methylphenyl)-pyrazol-4-ol, 1-(3-methylphenyl)-3-isobutyl-5- (3-methylphenyl)-pyrazol-4-ol, 1-(4-methoxyphenyl)-3-isobutyl-5-butyl-pyrazol-4-ol, 1-Methyl-3-isobutyl-5-(4-methylphenyl)-pyrazol-4-ol, , 1-Ethyl-3-isobutyl-5- (4-methylphenyl) -pyrazol-4-ol, 1-Propyl-3-isobutyl-5-(4-methylphenyl)-pyrazol-4-ol, 1-Isopropyl-3-isobutyl-5-(4-methylphenyl)-pyrazol-4-ol, 1-Butyl-3-isobutyl-5-(4-methylphenyl)-pyrazol-4-ol, 1-Isobutyl-3-isobutyl-5-(4-methylphenyl)-pyrazol-4-ol, 1-tert-Butyl-3-isobutyl-5-(4-methylphenyl)-pyrazol-4-ol, 1-Phenyl-3-isobutyl-5-(4-methylphenyl)-pyrazol-4-ol, 1-(4-methylphenyl)-3-isobutyl-5-(4-methylphenyl)-pyrazol-4-ol, 1-(2-methylphenyl)-3-isobutyl-5- (4-methylphenyl)-pyrazol-4-ol, 1-(3-methylphenyl)-3-isobutyl-5-(4-methylphenyl)-pyrazol-4-ol, 1-(4-methoxy-phenyl)-3-isobutyl-5-(4-methylphenyl)-pyrazol-4-ol, 1-Methyl-3-tert-butyl-5-phenyl-pyrazol-4-ol, , 1-Ethyl-3-tert-butyl5-phenyl-pyrazol-4-ol, 1-Propyl-3-tert-butyl-5-phenyl-pyrazol-4-ol, 1-Isopropyl-3-tert-butyl-5-phenyl-pyrazol-4-ol, 1-Butyl-3-tert-butyl-5-phenyl-pyrazol-4-ol, 1-Isobutyl-3-tert-butyl-5-phenyl-pyrazol-4-ol, 1-tert-Butyl-3-tert-butyl-5-phenyl-pyrazol-4-ol, 1-Phenyl-3-tert-butyl-5-phenyl-pyrazol-4-ol, 1-(4-methylphenyl)-3-tert-butyl-5-phenyl-pyrazol-4-ol, 1-(2-methylphenyl)-3-tert-butyl-5-phenyl-pyrazol-4-ol, 1-(3-methylphenyl)-3-tert-butyl-5-phenyl-pyrazol-4-ol, 1-(4-methoxyphenyl)-3-tert-butyl-5-phenyl-pyrazol-4-ol, 1-Methyl-3-tert-butyl-5-methyl-pyrazol-4-ol, , 1-Ethyl-3-tert-butyl-5-methyl-pyrazol-4-ol, 1-Propyl-3-tert-butyl-5-methyl-pyrazol-4-ol, 1-Isopropyl-3-tert-butyl-5-methyl-pyrazol-4-ol, 1-Butyl-3-tert-butyl-5-methyl-pyrazol-4-ol, 1-Isobutyl-3-tert-butyl-5- methyl-pyrazol-4-ol, 1-tert-Butyl-3-tert-butyl-5-methyl-pyrazol-4-ol, 1-Phenyl-3-tert-butyl-5-methyl-pyrazol-4-ol, 1-(4-methylphenyl)-3-tert-butyl-5-methyl-pyrazol-4-ol, 1-(2-methylphenyl)-3-tert-butyl-5-methyl-pyrazol-4-ol, 1-(3-methylphenyl)-3-tert-butyl-5-methyl-pyrazol-4-ol, 1-(4-methoxyphenyl)-3-tert-butyl-5- methyl -pyrazol-4-ol, 1-methyl-3-tert-butyl-5-ethyl-pyrazol-4-ol, 1-ethyl-3-tert-butyl-5-ethyl-pyrazol-4-ol, 1-propyl-3-tert-butyl-5-ethyl-pyrazol-4-ol, 1-isopropyl-3-tert-butyl-5-ethyl-pyrazol-4-ol, 1-butyl-3-tert-butyl-5-ethyl-pyrazol-4-ol, 1-Isobutyl-3-tert-butyl-5-ethyl-pyrazol-4-ol, 1-tert-butyl-3-tert-butyl-5-ethyl-pyrazol-4-ol, 1-Phenyl-3-tert-butyl-5-ethyl-pyrazol-4-ol, 1-(4-methylphenyl)-3-tert-butyl-5-ethyl-pyrazol-4-ol, 1-(2-methylphenyl)-3-tert-butyl-5- ethyl-pyrazol-4-ol, 1-(3-methylphenyl)-3-tert-butyl-5-ethyl-pyrazol-4-ol, 1-(4-methoxyphenyl)-3-tert-butyl-5-ethyl-pyrazol-4-ol, 1-Methyl-3-tert-butyl-5-propyl-pyrazol-4-ol, , 1-Ethyl-3-tert-butyl-5-propyl-pyrazol-4-ol, 1-Propyl-3-tert-butyl-5-propyl-pyrazol-4-ol, 1-Isopropyl-3-tert-butyl-5-methyl-pyrazol-4-ol, 1-Butyl-3-tert-butyl-5-propyl-pyrazol-4-ol, 1-Isobutyl-3-tert-butyl-5-propyl-pyrazol-4-ol, 1-tert-Butyl-3-tert-butyl-5-propyl-pyrazol-4-ol, 1-Phenyl-3-tert-butyl-5-propyl-pyrazol-4-ol, 1-(4-methylphenyl)-3-tert-butyl-5-propyl-pyrazol-4-ol, 1-(2-methylphenyl)-3-tert-butyl-5-propyl-pyrazol-4-ol, 1-(3-methylphenyl)-3-tert-butyl-5- propyl-pyrazol-4-ol, 1-(4-methoxyphenyl)-3-tert-butyl-5-propyl-pyrazol-4-ol, 1-Methyl-3-tert-butyl-5-isopropyl-pyrazol-4-ol, , 1-Ethyl-3-tert-butyl-5-isopropyl-pyrazol-4-ol, 1-Propyl-3-tert-butyl-5-isopropyl-pyrazol-4-ol, 1-Isopropyl-3-tert-butyl-5-isopropy-pyrazol-4-ol, 1-Butyl-3-tert-butyl-5-isopropyl-pyrazol-4-ol, 1-Isobutyl-3-tert-butyl-5-isopropyl-pyrazol-4-ol, 1-tert-Butyl-3-tert-butyl-5-isopropyl-pyrazol-4-ol, 1-Phenyl-3-tert-butyl-5-isoprop-pyrazol-4-ol, 1-(4-methyl-phenyl)-3-tert-butyl-5-isopropyl-pyrazol-4-ol, 1-(2-methylphenyl)-3-tert-butyl-5-isopropyl-pyrazol-4-ol, 1-(3-methylphenyl)-3-tert-butyl-5-isopropyl-pyrazol-4-ol, 1-(4-methoxyphenyl)-3-tert-butyl-5-isopropyl-pyrazol-4-ol, 1-Methyl-3-tert-butyl-5-butyl-pyrazol-4-ol, , 1-Ethyl-3-tert-butyl-5-butyl-pyrazol-4-ol, 1-Propyl-3-tert-butyl-5-butyl-pyrazol-4-ol, 1-Isopropyl-3-tert-butyl-5-butyl-pyrazol-4-ol, 1-Butyl-3-tert-butyl-5-butyl-pyrazol-4-ol, 1-Isobutyl-3-tert-butyl-5-butyl-pyrazol-4-ol, 1-tert-Butyl-3-tert-butyl-5-butyl-pyrazol-4-ol, 1-Phenyl-3-tert-butyl-5-butyl-pyrazol-4-ol, 1-(4-methylphenyl)-3-tert-butyl-5-butyl-pyrazol-4-ol, 1-(2-methylphenyl)-3-tert-butyl-5-butyl-pyrazol-4-ol, 1-(3-methylphenyl)-3-tert-butyl-5butyl-pyrazol-4-ol, 1-(4-methoxyphenyl)-3-tert-butyl-5-butyl-pyrazol-4-ol, 1-Methyl-3-tert-butyl-5-isobutyl-pyrazol-4-ol, , 1-Ethyl-3-tert-butyl-5-isobutyl-pyrazol-4-ol, 1-Propyl-3-tert-butyl-5-isobutyl-pyrazol-4-ol, 1-Isopropyl-3-tert-butyl-5-isobutyl-pyrazol-4-ol, 1-Butyl-3-tert-butyl-5-isobutyl-pyrazol-4-ol, 1-Isobutyl-3-tert-butyl-5-isobutyl-pyrazol-4-ol, 1-tert-Butyl-3-tert-butyl-5-isobutyl-pyrazol-4-ol, 1-Phenyl-3-tert-butyl-5-isobutyl-pyrazol-4-ol, 1-(4-methylphenyl)-3-tert-butyl-5-isobutyl-pyrazol-4-ol, 1-(2-methylphenyl)-3-tert-butyl-5-isobutyl-pyrazol-4-ol, 1-(3-methylphenyl)-3-tert-butyl-5-isobutyl-pyrazol-4-ol, 1-(4-methoxyphenyl)-3-tert-butyl-5-isobutyl-pyrazol-4-ol, 1-Methyl-3-tert-butyl-5-tert-butyl-pyrazol-4-ol, , 1-Ethyl-3-tert-butyl-5-tert-butyl-pyrazol-4-ol, 1-Propyl-3-tert-butyl-5-tert-butyl-pyrazol-4-ol, 1-Isopropyl-3-tert-butyl-5-tert-butyl-pyrazol-4-ol, 1-Butyl-3-tert-butyl-5-tert-butyl-pyrazol-4-ol, 1-Isobutyl-3-tert-butyl-5-tert-butyl -pyrazol-4-ol, 1-tert-Butyl-3-tert-butyl-5-tert-butyl-pyrazol-4-ol, 1-Phenyl-3-tert-butyl-5- tert-butyl-pyrazol-4-ol, 1-(4-methylphenyl)-3-tert-butyl-5-tert-butyl-pyrazol-4-ol, 1-(2-methylphenyl)-3-tert-butyl-5-tert-butyl-pyrazol-4-ol, 1-(3-methylphenyl)-3-tert-butyl-5-tert-butyl-pyrazol-4-ol,
1-(4-methoxyphenyl)-3-tert-butyl-5-phenyl-pyrazol-4-ol, 1-Methyl-3-tert-butyl-5- phenyl-pyrazol-4-ol, , 1-Ethyl-3-tert-butyl-5- phenyl -pyrazol-4-ol, 1-Propyl-3-tert-butyl-5-phenyl-pyrazol-4-ol, 1-Isopropyl-3-tert-butyl-5-phenyl-pyrazol-4-ol, 1-Butyl-3-tert-butyl-5- phenyl-pyrazol-4-ol, 1-Isobutyl-3-tert-butyl-5-phenyl-pyrazol-4-ol, 1-tert-Butyl-3-tert-butyl-5- phenyl -pyrazol-4-ol, 1-Phenyl-3-tert-butyl-5- phenyl-pyrazol-4-ol, 1-(4-methylphenyl)-3-tert-butyl-5-phenyl-pyrazol-4-ol, 1-(2-methylphenyl)-3-tert-butyl-5-phenyl-pyrazol-4-ol, 1-(3-methyl-phenyl)-3-tert-butyl-5-phenyl-pyrazol-4-ol, 1-(4-methoxyphenyl)-3-tert-butyl-5-(2-methyl-phenyl)-pyrazol-4-ol, 1-Methyl-3-tert-butyl-5-(2-methylphenyl)-pyrazol-4-ol, 1-Ethyl-3-tert-butyl-5-(2-methylphenyl)-pyrazol-4-ol, 1-Propyl-3-tert-butyl-5- (2-methylphenyl) -pyrazol-4-ol, 1-Isopropyl-3-tert-butyl-5-(2-methylphenyl)-pyrazol-4-ol, 1-Butyl-3-tert-butyl-5-(2-methyl-phenyl)-pyrazol-4-ol, 1-Isobutyl-3-tert-butyl-5-(2-methylphenyl)-pyrazol-4-ol, 1-tert-Butyl-3-tert-butyl-5-(2-methylphenyl)-pyrazol-4-ol, 1-Phenyl-3-tert-butyl-5- (2-methylphenyl)-pyrazol-4-ol, 1-(4-methylphenyl)-3-tert-butyl-5-(2-methylphenyl)-pyrazol-4-ol, 1-(2-methyl-phenyl)-3-tert-butyl-5-(2-methylphenyl)-pyrazol-4-ol, 1-(3-methylphenyl)-3-tert-butyl-5- (2-methylphenyl)-pyrazol-4-ol, 1-(4-methoxyphenyl)-3-tert-butyl-5- (3-methylphenyl) -pyrazol-4-ol, 1-Methyl-3-tert-butyl-5-(3-methylphenyl)-pyrazol-4-ol, , 1-Ethyl-3-tert-butyl-5- (3-methyl-phenyl)-pyrazol-4-ol, 1-Propyl-3-tert-butyl-5-(3-methylphenyl)-pyrazol-4-ol, 1-Isopropyl-3-tert-butyl-5-(3-methylphenyl)-pyrazol-4-ol, 1-Butyl-3-tert-butyl-5- (3-methylphenyl) -pyrazol-4-ol, 1-Isobutyl-3-tert-butyl-5-(3-methylphenyl)-pyrazol-4-ol, 1-tert-Butyl-3-tert-butyl-5- (3-methylphenyl)-pyrazol-4-ol, 1-Phenyl-3-tert-butyl-5-(3-methylphenyl)-pyrazol-4-ol, 1-(4-methylphenyl)-3-tert-butyl-5-(3-methylphenyl)-pyrazol-4-ol, 1-(2-methylphenyl)-3-tert-butyl-5-(3-methylphenyl)-pyrazol-4-ol, 1-(3-methylphenyl)-3-tert-butyl-5- (3-methylphenyl) - pyrazol-4-ol, 1-(4-methoxyphenyl)-3-tert-butyl-5-(3-methylphenyl)-pyrazol-4-ol, 1-Methyl-3-tert-butyl-5-(3-methylphenyl)-pyrazol-4-ol, , 1-Ethyl-3-tert-butyl-5- (3-methylphenyl) -pyrazol-4-ol, 1-Propyl-3-tert-butyl-5-(3-methylphenyl)-pyrazol-4-ol, 1-Isopropyl-3-tert-butyl-5-(3-methylphenyl)-pyrazol-4-ol, 1-Butyl-3-tert-butyl-5-(3-methylphenyl)-pyrazol-4-ol, 1-Isobutyl-3-tert-butyl-5-(3-methylphenyl)-pyrazol-4-ol, 1-tert-Butyl-3-tert-butyl-5-butyl-pyrazol-4-ol, 1-Phenyl-3-tert-butyl-5-(3-methylphenyl)-pyrazol-4-ol, 1-(4-methylphenyl)-3-tert-butyl-5- (3-methylphenyl)-pyrazol-4-ol, 1-(2-methylphenyl)-3-tert-butyl-5-(3-methylphenyl)-pyrazol-4-ol, 1-(3-methylphenyl)-3-tert-butyl-5- (3-methylphenyl) -pyrazol-4-ol, 1-(4-methoxyphenyl)-3-tert-butyl-5-butyl-pyrazol-4-ol, 1-Methyl-3-tert-butyl-5-(4-methylphenyl)-pyrazol-4-ol, , 1-Ethyl-3-tert-butyl-5-(4-methylphenyl)-pyrazol-4-ol, 1-Propyl-3-tert-butyl-5- (4-methylphenyl)-pyrazol-4-ol, 1-Isopropyl-3-tert-butyl-5-(4-methylphenyl)-pyrazol-4-ol, 1-Butyl-3-tert-butyl-5-(4-methylphenyl)-pyrazol-4-ol, 1-Isobutyl-3-tert-butyl-5-(4-methylphenyl)-pyrazol-4-ol, 1-tert-Butyl-3-tert-butyl-5-(4-methylphenyl)-pyrazol-4-ol, 1-Phenyl-3-tert-butyl-5- (4-methylphenyl)-pyrazol-4-ol, 1-(4-methylphenyl)-3-tert-butyl-5-(4-methylphenyl)-pyrazol-4-ol, 1-(2-methylphenyl)-3-tert-butyl-5-(4-methylphenyl)-pyrazol-4-ol, 1-(3-methylphenyl)-3-tert-butyl-5-(4-methylphenyl)-pyrazol-4-ol, 1-(4-methoxyphenyl)-3-tert-butyl-5- (4-methylphenyl)-pyrazol-4-ol, 1-Methyl-3-phenyl-5-phenyl-pyrazol-4-ol, 1-Ethyl-3-phenyl-5-phenyl-pyrazol-4-ol, 1-Propyl-3-phenyl-5-phenyl-pyrazol-4-ol, 1-Isopropyl-3-phenyl-5-phenyl-pyrazol-4-ol, 1-Butyl-3-phenyl-5-phenyl-pyrazol-4-ol, 1-Isobutyl-3-phenyl-5-phenyl-pyrazol-4-ol, 1-tert-Butyl-3-phenyl-5-phenyl-pyrazol-4-ol, 1-Phenyl-3-phenyl-5-phenyl-pyrazol-4-ol, 1-(4-methylphenyl)-3-phenyl-5-phenyl-pyrazol-4-ol, 1-(2-methylphenyl)-3-phenyl-5-phenyl-pyrazol-4-ol, 1-(3-methylphenyl)-3-phenyl-5-phenyl-pyrazol-4-ol, 1-(4-methoxyphenyl)-3-phenyl-5-phenyl-pyrazol-4-ol, 1-Methyl-3-phenyl-5-methyl-pyrazol-4-ol, , 1-Ethyl-3-phenyl-5-methyl-pyrazol-4-ol, 1-Propyl-3-phenyl-5-methyl-pyrazol-4-ol, 1-Isopropyl-3-phenyl-5-methyl-pyrazol-4-ol, 1-Butyl-3-phenyl-5-methyl-pyrazol-4-ol, 1-Isobutyl-3-phenyl-5-methyl-pyrazol-4-ol, 1-tert-Butyl-3-phenyl-5-methyl-pyrazol-4-ol, 1-Phenyl-3-phenyl-5-methyl-pyrazol-4-ol, 1-(4-methylphenyl)-3-phenyl-5-methyl-pyrazol-4-ol, 1-(2-methylphenyl)-3-phenyl-5- methyl-pyrazol-4-ol, 1-(3-methylphenyl)-3-phenyl-5-methyl-pyrazol-4-ol, 1-(4-methoxyphenyl)-3-phenyl-5-methyl-pyrazol-4-ol, 1-methyl-3-phenyl-5-ethyl-pyrazol-4-ol, , 1-ethyl-3-phenyl-5-ethyl-pyrazol-4-ol, 1-propyl-3-phenyl-5-ethyl-pyrazol-4-ol, 1-isopropyl-3-phenyl-5- ethyl-pyrazol-4-ol, 1-butyl-3-phenyl-5-ethyl-pyrazol-4-ol, 1-Isobutyl-3-phenyl-5- ethyl -pyrazol-4-ol, 1-tert-butyl-3-phenyl-5-ethyl-pyrazol-4-ol, 1-Phenyl-3-phenyl-5-ethyl-pyrazol-4-ol, 1-(4-methylphenyl)-3-phenyl-5-ethyl-pyrazol-4-ol, 1-(2-methylphenyl)-3-phenyl-5- ethyl -pyrazol-4-ol, 1-(3-methylphenyl)-3-phenyl-5-ethyl-pyrazol-4-ol, 1-(4-methoxyphenyl)-3-phenyl-5-ethyl-pyrazol-4-ol, 1-Methyl-3-phenyl-5-propyl-pyrazol-4-ol, , 1-Ethyl-3-phenyl-5- propyl-pyrazol-4-ol, 1-Propyl-3-phenyl-5-propyl-pyrazol-4-ol, 1-Isopropyl-3-phenyl-5-methyl-pyrazol-4-ol, 1-Butyl-3-phenyl-5-propyl-pyrazol-4-ol, 1-Isobutyl-3-phenyl-5- propyl -pyrazol-4-ol, 1-tert-Butyl-3-phenyl-5-propyl-pyrazol-4-ol, 1-Phenyl-3-phenyl-5-propyl-pyrazol-4-ol, 1-(4-methylphenyl)-3-phenyl-5-propyl-pyrazol-4-ol, 1-(2-methylphenyl)-3-phenyl-5- propyl-pyrazol-4-ol, 1-(3-methylphenyl)-3-phenyl-5-propyl-pyrazol-4-ol, 1-(4-methoxyphenyl)-3-phenyl-5-propyl-pyrazol-4-ol, 1-Methyl-3-phenyl-5-isopropyl-pyrazol-4-ol, , 1-Ethyl-3-phenyl-5-isopropyl-pyrazol-4-ol, 1-Propyl-3-phenyl-5-isopropyl-pyrazol-4-ol, 1-Isopropyl-3-phenyl-5-isopropyl-pyrazol-4-ol, 1-Butyl-3-phenyl-5-isopropyl-pyrazol-4-ol, 1-Isobutyl-3-phenyl-5-isopropyl-pyrazol-4-ol, 1-tert-Butyl-3-phenyl-5-isopropyl-pyrazol-4-ol, 1-Phenyl-3-phenyl-5-isopropyl-pyrazol-4-ol, 1-(4-methylphenyl)-3-phenyl-5-isopropyl-pyrazol-4-ol, 1-(2-methyl-phenyl)-3-phenyl-5-isopropyl-pyrazol-4-ol, 1-(3-methylphenyl)-3-phenyl-5- isopropyl-pyrazol-4-ol, 1-(4-methoxyphenyl)-3-phenyl-5-isopropyl-pyrazol-4-ol, 1-Methyl-3-phenyl-5-butyl-pyrazol-4-ol, , 1-Ethyl-3-phenyl-5-butyl-pyrazol-4-ol, 1-Propyl-3-phenyl-5- butyl-pyrazol-4-ol, 1-Isopropyl-3-phenyl-5-butyl-pyrazol-4-ol, 1-Butyl-3-phenyl-5- butyl -pyrazol-4-ol, 1-Isobutyl-3-phenyl-5-butyl-pyrazol-4-ol, 1-tert-Butyl-3-phenyl-5-butyl-pyrazol-4-ol, 1-Phenyl-3-phenyl-5-butyl-pyrazol-4-ol, 1-(4-methylphenyl)-3-phenyl-5-butyl-pyrazol-4-ol, 1-(2-methylphenyl)-3-phenyl-5-butyl-pyrazol-4-ol, 1-(3-methylphenyl)-3-phenyl-5- butyl-pyrazol-4-ol, 1-(4-methoxyphenyl)-3-phenyl-5-butyl-pyrazol-4-ol, 1-Methyl-3-phenyl-5-isobutyl-pyrazol-4-ol, 1-Ethyl-3-phenyl-5-isobutyl-pyrazol-4-ol, 1-Propyl-3-phenyl-5- isobutyl -pyrazol-4-ol, 1-Isopropyl-3-phenyl-5-isobutyl-pyrazol-4-ol, 1-Butyl-3-phenyl-5- isobutyl-pyrazol-4-ol, 1-Isobutyl-3-phenyl-5-isobutyl-pyrazol-4-ol, 1-tert-Butyl-3-phenyl-5- isobutyl-pyrazol-4-ol, 1-Phenyl-3-phenyl-5-isobutyl-pyrazol-4-ol, 1-(4-methylphenyl)-3-phenyl-5-isobutyl-pyrazol-4-ol, 1-(2-methylphenyl)-3-phenyl-5-isobutyl-pyrazol-4-ol, 1-(3-methyl-phenyl)-3-phenyl-5-isobutyl-pyrazol-4-ol, 1-(4-methoxyphenyl)-3-phenyl-5- isobutyl -pyrazol-4-ol, 1-Methyl-3-phenyl-5-tert-butyl-pyrazol-4-ol, , 1-Ethyl-3-phenyl-5- tert-butyl-pyrazol-4-ol, 1-Propyl-3-phenyl-5-tert-butyl-pyrazol-4-ol, 1-Isopropyl-3-phenyl-5-tert-butyl-pyrazol-4-ol, 1-Butyl-3-phenyl-5-tert-butyl-pyrazol-4-ol, 1-Isobutyl-3-phenyl-5-tert-butyl-pyrazol-4-ol, 1-tert-Butyl-3-phenyl-5-tert-butyl-pyrazol-4-ol, 1-Phenyl-3-phenyl-5- tert-butyl-pyrazol-4-ol, 1-(4-methylphenyl)-3-phenyl-5- tert-butyl -pyrazol-4-ol, 1-(2-methylphenyl)-3-phenyl-5-tert-butyl-pyrazol-4-ol, 1-(3-methylphenyl)-3-phenyl-5-tert-butyl-pyrazol-4-ol, 1-(4-methoxyphenyl)-3-phenyl-5-phenyl-pyrazol-4-ol, 1-Methyl-3-phenyl-5-phenyl-pyrazol-4-ol, 1-Ethyl-3-phenyl-5-phenyl-pyrazol-4-ol, 1-Propyl-3-phenyl-5-phenyl-pyrazol-4-ol,
1-Isopropyl-3-phenyl-5-phenyl-pyrazol-4-ol, 1-Butyl-3-phenyl-5- phenyl -pyrazol-4-ol, 1-Isobutyl-3-phenyl-5-phenyl-pyrazol-4-ol, 1-tert-Butyl-3-phenyl-5- phenyl-pyrazol-4-ol, 1-Phenyl-3-phenyl-5-phenyl-pyrazol-4-ol, 1-(4-methylphenyl)-3-phenyl-5-phenyl-pyrazol-4-ol, 1-(2-methylphenyl)-3-phenyl-5-phenyl-pyrazol-4-ol, 1-(3-methylphenyl)-3-phenyl-5-phenyl-pyrazol-4-ol, 1-(4-methoxyphenyl)-3-phenyl-5-(2-methylphenyl)-pyrazol-4-ol, 1-Methyl-3-phenyl-5-(2-methylphenyl)-pyrazol-4-ol, , 1-Ethyl-3-phenyl-5-(2-methylphenyl)-pyrazol-4-ol, 1-Propyl-3-phenyl-5-(2-methylphenyl)-pyrazol-4-ol, 1-Isopropyl-3-phenyl-5-(2-methylphenyl)-pyrazol-4-ol, 1-Butyl-3-phenyl-5-(2-methylphenyl)-pyrazol-4-ol, 1-Isobutyl-3-phenyl-5-(2-methylphenyl)-pyrazol-4-ol, 1-tert-Butyl-3-phenyl-5- (2-methylphenyl)-pyrazol-4-ol, 1-Phenyl-3-phenyl-5-(2-methylphenyl) -pyrazol-4-ol, 1-(4-methylphenyl)-3-phenyl-5-(2-methylphenyl)-pyrazol-4-ol, 1-(2-methylphenyl)-3-phenyl-5-(2-methylphenyl)-pyrazol-4-ol, 1-(3-methylphenyl)-3-phenyl-5-(2-methylphenyl)-pyrazol-4-ol, 1-(4-methoxyphenyl)-3-phenyl-5- (3-methylphenyl)-pyrazol-4-ol, 1-Methyl-3-phenyl-5-(3-methylphenyl)-pyrazol-4-ol, 1-Ethyl-3-phenyl-5-(3-methylphenyl)-pyrazol-4-ol, 1-Propyl-3-phenyl-5-(3-methylphenyl) -pyrazol-4-ol, 1-Isopropyl-3-phenyl-5-(3-methylphenyl)-pyrazol-4-ol, 1-Butyl-3-phenyl-5-(3-methylphenyl)-pyrazol-4-ol, 1-Isobutyl-3-phenyl-5-(3-methylphenyl)-pyrazol-4-ol, 1-tert-Butyl-3-phenyl-5-(3-methylphenyl)-pyrazol-4-ol, 1-Phenyl-3-phenyl-5-(3-methylphenyl)-pyrazol-4-ol, 1-(4-methylphenyl)-3-phenyl-5- (3-methylphenyl) -pyrazol-4-ol, 1-(2-methylphenyl)-3-phenyl-5-(3-methylphenyl)-pyrazol-4-ol, 1-(3-methylphenyl)-3-phenyl-5-(3-methylphenyl)-pyrazol-4-ol, 1-(4-methoxyphenyl)-3-phenyl-5-(3-methylphenyl)-pyrazol-4-ol, 1-Methyl-3-phenyl-5-(3-methylphenyl)-pyrazol-4-ol, , 1-Ethyl-3-phenyl-5-(3-methylphenyl)-pyrazol-4-ol, 1-Propyl-3-phenyl-5-(3-methylphenyl)-pyrazol-4-ol, 1-Osopropyl-3-phenyl-5-(3-methylphenyl) -pyrazol-4-ol, 1-Butyl-3-phenyl-5-(3-methylphenyl)-pyrazol-4-ol, 1-Isobutyl-3-phenyl-5-(3-methylphenyl)-pyrazol-4-ol, 1-tert-Butyl-3-phenyl-5- butyl -pyrazol-4-ol, 1-Phenyl-3-phenyl-5- (3-methylphenyl) -pyrazol-4-ol, 1-(4-methylphenyl)-3-phenyl-5- (3-methylphenyl) -pyrazol-4-ol, 1-(2-methylphenyl)-3-phenyl-5- (3-methylphenyl) -pyrazol-4-ol, 1-(3-methylphenyl)-3-phenyl-5- (3-methylphenyl) -pyrazol-4-ol, 1-(4-methoxyphenyl)-3-phenyl-5- butyl -pyrazol-4-ol,
1-Methyl-3-phenyl-5-(4-methylphenyl)-pyrazol-4-ol, 1-Ethyl-3-phenyl-5-(4-methylphenyl)-pyrazol-4-ol, 1-Propyl-3-phenyl-5-(4-methylphenyl)-pyrazol-4-ol, 1-Isopropyl-3-phenyl-5-(4-methylphenyl)-pyrazol-4-ol, 1-Butyl-3-phenyl-5-(4-methylphenyl)-pyrazol-4-ol, 1-Isobutyl-3-phenyl-5-(4-methylphenyl)-pyrazol-4-ol, 1-tert-Butyl-3-phenyl-5-(4-methylphenyl)-pyrazol-4-ol, 1-Phenyl-3-phenyl-5-(4-methylphenyl)-pyrazol-4-ol, 1-(4-methylphenyl)-3-phenyl-5-(4-methylphenyl)-pyrazol-4-ol, 1-(2-methylphenyl)-3-phenyl-5-(4-methylphenyl)-pyrazol-4-ol, 1-(3-methylphenyl)-3-phenyl-5-(4-methylphenyl)-pyrazol-4-ol, 1-(4-methoxyphenyl)-3-phenyl-5-(4-methylphenyl)-pyrazol-4-ol, 1-Methyl-3-(4-methylphenyl)-5-phenyl-pyrazol-4-ol, , 1-Ethyl-3-(4-methylphenyl)5-phenyl-pyrazol-4-ol, 1-Propyl-3-(4-methylphenyl)-5-phenyl-pyrazol-4-ol, 1-Isopropyl-3-(4-methylphenyl)-5-phenyl-pyrazol-4-ol, 1-Butyl-3-(4-methylphenyl)-5-phenyl-pyrazol-4-ol, 1-Isobutyl-3-(4-methylphenyl)-5-phenyl-pyrazol-4-ol, 1-tert-Butyl-3-(4-methylphenyl)-5-phenyl-pyrazol-4-ol, 1-Phenyl-3-(4-methylphenyl)-5-phenyl-pyrazol-4-ol, 1-(4-methylphenyl)-3-(4-methylphenyl)-5-phenyl-pyrazol-4-ol, 1-(2-methylphenyl)-3-(4-methylphenyl)-5-phenyl-pyrazol-4-ol, 1-(3-methylphenyl)-3-(4-methylphenyl)-5-phenyl-pyrazol-4-ol, 1-(4-methoxyphenyl)-3-(4-methylphenyl)-5-phenyl-pyrazol-4-ol,
1-Methyl-3-(4-methylphenyl)-5-methyl-pyrazol-4-ol, 1-Ethyl-3-(4-methylphenyl)-5-methyl-pyrazol-4-ol, 1-propyl-3-(4-methylphenyl)-5-methyl-pyrazol-4-ol, 1-Isopropyl-3-(4-methyl-phenyl)-5-methyl-pyrazol-4-ol, 1-Butyl-3-(4-methylphenyl)-5-methyl-pyrazol-4-ol, 1-Isobutyl-3-(4-methylphenyl)-5-methyl-pyrazol-4-ol, 1-tert-Butyl-3-(4-methylphenyl)-5-methyl-pyrazol-4-ol, 1-Phenyl-3-(4-methylphenyl)-5-methyl-pyrazol-4-ol, 1-(4-methylphenyl)-3-(4-methylphenyl)-5-methyl-pyrazol-4-ol, 1-(2-methylphenyl)-3-(4-methylphenyl)-5-methyl-pyrazol-4-ol, 1-(3-methylphenyl)-3-(4-methylphenyl)-5-methyl-pyrazol-4-ol, 1-(4-methoxyphenyl)-3-(4-methylphenyl)-5-methyl-pyrazol-4-ol, 1-methyl-3-(4-methylphenyl)-5-ethyl-pyrazol-4-ol, 1-ethyl-3-(4-methylphenyl)-5-ethyl-pyrazol-4-ol, 1-propyl-3-(4-methylphenyl)-5-ethyl-pyrazol-4-ol, 1-isopropyl-3-(4-methylphenyl)-5-ethyl-pyrazol-4-ol, 1-butyl-3-(4-methylphenyl)-5-ethyl-pyrazol-4-ol, 1-Isobutyl-3-(4-methylphenyl)-5-ethyl-pyrazol-4-ol, 1-tert-butyl-3-(4-methylphenyl)-5-ethyl-pyrazol-4-ol, 1-Phenyl-3-(4-methylphenyl)-5- ethyl-pyrazol-4-ol, 1-(4-methylphenyl)-3-(4-methylphenyl)-5-ethyl-pyrazol-4-ol, 1-(2-methylphenyl)-3-(4-methylphenyl)-5-ethyl-pyrazol-4-ol, 1-(3-methylphenyl)-3-(4-methylphenyl)-5-ethyl-pyrazol-4-ol, 1-(4-methoxyphenyl)-3-(4-methylphenyl)-5-ethyl-pyrazol-4-ol, 1-Methyl-3-(4-methylphenyl)-5-propyl-pyrazol-4-ol, 1-Ethyl-3-(4-methylphenyl)-5-propyl-pyrazol-4-ol, 1-Propyl-3-(4-methylphenyl)-5-propyl-pyrazol-4-ol, 1-Isopropyl-3-(4-methylphenyl)-5-methyl-pyrazol-4-ol, 1-Butyl-3-(4-methylphenyl)-5-propyl-pyrazol-4-ol, 1-Isobutyl-3-(4-methylphenyl)-5-propyl-pyrazol-4-ol,
1-tert-Butyl-3-(4-methylphenyl)-5-propyl-pyrazol-4-ol, 1-Phenyl-3-(4-methylphenyl)-5-propyl -pyrazol-4-ol, 1-(4-methylphenyl)-3-(4-methylphenyl)-5-propyl-pyrazol-4-ol, 1-(2-methylphenyl)-3-(4-methylphenyl)-5-propyl-pyrazol-4-ol, 1-(3-methylphenyl)-3-(4-methylphenyl)-5-propyl-pyrazol-4-ol, 1-(4-methoxyphenyl)-3-(4-methylphenyl)-5-propyl-pyrazol-4-ol, 1-Methyl-3-(4-methylphenyl)-5-isopropyl-pyrazol-4-ol, 1-Ethyl-3-(4-methylphenyl)-5-isopropyl-pyrazol-4-ol, 1-Propyl-3-(4-methylphenyl)-5-isopropyl-pyrazol-4-ol, 1-Isopropyl-3-(4-methylphenyl)-5-isopropyl-pyrazol-4-ol, 1-Butyl-3-(4-methylphenyl)-5-isopropyl-pyrazol-4-ol, 1-Isobutyl-3-(4-methylphenyl)-5-isopropyl-pyrazol-4-ol, 1-tert-Butyl-3-(4-methylphenyl)-5-isopropyl-pyrazol-4-ol, 1-Phenyl-3-(4-methylphenyl)-5-isopropyl-pyrazol-4-ol, 1-(4-methylphenyl)-3-(4-methylphenyl)-5-isopropyl-pyrazol-4-ol, 1-(2-methylphenyl)-3-(4-methylphenyl)-5-isopropyl-pyrazol-4-ol, 1-(3-methylphenyl)-3-(4-methylphenyl)-5-isopropyl-pyrazol-4-ol, 1-(4-methoxyphenyl)-3-(4-methylphenyl)-5-isopropyl-pyrazol-4-ol, 1-Methyl-3-(4-methylphenyl)-5-butyl-pyrazol-4-ol, 1-Ethyl-3-(4-methylphenyl)-5-butyl-pyrazol-4-ol, 1-Propyl-3-(4-methylphenyl)-5-butyl-pyrazol-4-ol, 1-Isopropyl-3-(4-methylphenyl)-5-butyl-pyrazol-4-ol, 1-Butyl-3-(4-methylphenyl)-5-butyl-pyrazol-4-ol, 1-Isobutyl-3-(4-methylphenyl)-5-butyl-pyrazol-4-ol, 1-tert-Butyl-3-(4-methylphenyl)-5-butyl-pyrazol-4-ol, 1-Phenyl-3-(4-methylphenyl)-5-butyl-pyrazol-4-ol, 1-(4-methylphenyl)-3-(4-methylphenyl)-5-butyl-pyrazol-4-ol, 1-(2-methylphenyl)-3-(4-methylphenyl)-5-butyl-pyrazol-4-ol, 1-(3-methylphenyl)-3-(4-methylphenyl)-5-butyl-pyrazol-4-ol, 1-(4-methoxyphenyl)-3-(4-methylphenyl)-5-butyl-pyrazol-4-ol, 1-Methyl-3-(4-methylphenyl)-5-isobutyl-pyrazol-4-ol,1-Ethyl-3-(4-methylphenyl)-5-isobutyl-pyrazol-4-ol, 1-Propyl-3-(4-methylphenyl)-5-isobutyl-pyrazol-4-ol, 1-Isopropyl-3-(4-methylphenyl)-5-isobutyl-pyrazol-4-ol, 1-Butyl-3-(4-methylphenyl)-5-isobutyl-pyrazol-4-ol, 1-Isobutyl-3-(4-methylphenyl)-5-isobutyl -pyrazol-4-ol, 1-tert-Butyl-3-(4-methylphenyl)-5-isobutyl-pyrazol-4-ol, 1-Phenyl-3-(4-methylphenyl)-5-isobutyl-pyrazol-4-ol,
1-(4-methylphenyl)-3-(4-methylphenyl)-5-isobutyl -pyrazol-4-ol, 1-(2-methylphenyl)-3-(4-methylphenyl)-5-isobutyl-pyrazol-4-ol,
1-(3-methylphenyl)-3-(4-methylphenyl)-5-isobutyl-pyrazol-4-ol, 1-(4-methoxyphenyl)-3-(4-methylphenyl)-5-isobutyl-pyrazol-4-ol, 1-Methyl-3-(4-methylphenyl)-5-tert-butyl-pyrazol-4-ol, 1-Ethyl-3-(4-methylphenyl)-5- tert-butyl -pyrazol-4-ol, 1-Propyl-3-(4-methylphenyl)-5- tert-butyl-pyrazol-4-ol, 1-Isopropyl-3-(4-methylphenyl)-5-tert-butyl-pyrazol-4-ol,
1-Butyl-3-(4-methylphenyl)-5-tert-butyl -pyrazol-4-ol, 1-Isobutyl-3-(4-methylphenyl)-5-tert-butyl-pyrazol-4-ol,
1-tert-Butyl-3-(4-methylphenyl)-5-tert-butyl-pyrazol-4-ol, 1-Phenyl-3-(4-methylphenyl)-5-tert-butyl-pyrazol-4-ol, 1-(4-methylphenyl)-3-(4-methylphenyl)-5-tert-butyl-pyrazol-4-ol, 1-(2-methylphenyl)-3-(4-methylphenyl)-5- tert-butyl-pyrazol-4-ol,1-(3-methylphenyl)-3-(4-methylphenyl)-5-tert-butyl-pyrazol-4-ol, 1-(4-methoxyphenyl)-3-(4-methylphenyl)-5-phenyl-pyrazol-4-ol, 1-Methyl-3-(4-methylphenyl)-5-phenyl-pyrazol-4-ol, 1-Ethyl-3-(4-methylphenyl)-5-phenyl-pyrazol-4-ol, 1-Propyl-3-(4-methylphenyl)-5-phenyl-pyrazol-4-ol, 1-Isopropyl-3-(4-methylphenyl)-5-phenyl-pyrazol-4-ol, 1-Butyl-3-(4-methylphenyl)-5-phenyl-pyrazol-4-ol, 1-Isobutyl-3-(4-methylphenyl)-5-phenyl-pyrazol-4-ol, 1-tert-Butyl-3-(4-methylphenyl)-5-phenyl -pyrazol-4-ol, 1-Phenyl-3-(4-methylphenyl)-5-phenyl-pyrazol-4-ol, 1-(4-methylphenyl)-3-(4-methylphenyl)-5-phenyl-pyrazol-4-ol, 1-(2-methylphenyl)-3-(4-methylphenyl)-5-phenyl-pyrazol-4-ol, 1-(3-methylphenyl)-3-(4-methylphenyl)-5-phenyl-pyrazol-4-ol, 1-(4-methoxyphenyl)-3-(4-methylphenyl)-5- (2-methylphenyl)-pyrazol-4-ol, 1-Methyl-3-(4-methylphenyl)-5-(2-methylphenyl)-pyrazol-4-ol, 1-Ethyl-3-(4-methylphenyl)-5-(2-methylphenyl)-pyrazol-4-ol, 1-Propyl-3-(4-methylphenyl)-5-(2-methylphenyl)-pyrazol-4-ol, 1-Isopropyl-3-(4-methylphenyl)-5-(2-methylphenyl)-pyrazol-4-ol, 1-Butyl-3-(4-methylphenyl)-5-(2-methylphenyl)-pyrazol-4-ol,1-Isobutyl-3-(4-methylphenyl)-5-(2-methylphenyl)-pyrazol-4-ol, 1-tert-Butyl-3-(4-methylphenyl)-5-(2-methylphenyl)-pyrazol-4-ol, 1-Phenyl-3-(4-methylphenyl)-5-(2-methylphenyl)-pyrazol-4-ol, 1-(4-methylphenyl)-3-(4-methylphenyl)-5-(2-methylphenyl)-pyrazol-4-ol, 1-(2-methylphenyl)-3-(4-methylphenyl)-5-(2-methylphenyl)-pyrazol-4-ol, 1-(3-methylphenyl)-3-(4-methylphenyl)-5-(2-methylphenyl)-pyrazol-4-ol, 1-(4-methoxyphenyl)-3-(4-methylphenyl)-5-(3-methylphenyl)-pyrazol-4-ol, 1-Methyl-3-(4-methylphenyl)-5-(3-methylphenyl)-pyrazol-4-ol, 1-Ethyl-3-(4-methylphenyl)-5-(3-methylphenyl)-pyrazol-4-ol, 1-Propyl-3-(4-methylphenyl)-5- (3-methylphenyl)-pyrazol-4-ol, 1-Isopropyl-3-(4-methylphenyl)-5-(3-methylphenyl) -pyrazol-4-ol, 1-Butyl-3-(4-methylphenyl)-5-(3-methylphenyl)-pyrazol-4-ol, 1-Isobutyl-3-(4-methylphenyl)-5- (3-methylphenyl)-pyrazol-4-ol, 1-tert-Butyl-3-(4-methylphenyl)-5-(3-methylphenyl)-pyrazol-4-ol, 1-Phenyl-3-(4-methylphenyl)-5- (3-methylphenyl)-pyrazol-4-ol, 1-(4-methylphenyl)-3-(4-methylphenyl)-5-(3-methylphenyl)-pyrazol-4-ol, 1-(2-methylphenyl)-3-(4-methylphenyl)-5-(3-methylphenyl)-pyrazol-4-ol, 1-(3-methylphenyl)-3-(4-methylphenyl)-5-(3-methylphenyl)-pyrazol-4-ol, 1-(4-methoxyphenyl)-3-(4-methylphenyl)-5-(3-methylphenyl)-pyrazol-4-ol, 1-Methyl-3-(4-methylphenyl)-5-(3-methylphenyl)-pyrazol-4-ol, 1-Ethyl-3-(4-methylphenyl)-5- (3-methylphenyl) -pyrazol-4-ol, 1-Propyl-3-(4-methylphenyl)-5-(3-methylphenyl) -pyrazol-4-ol, 1-Isopropyl-3-(4-methylphenyl)-5-(3-methylphenyl)-pyrazol-4-ol, 1-Butyl-3-(4-methylphenyl)-5-(3-methylphenyl)-pyrazol-4-ol, 1-Isobutyl-3-(4-methylphenyl)-5-(3-methylphenyl)-pyrazol-4-ol, 1-tert-Butyl-3-(4-methylphenyl)-5-butyl-pyrazol-4-ol, 1-Phenyl-3-(4-methylphenyl)-5-(3-methylphenyl)-pyrazol-4-ol, 1-(4-methylphenyl)-3-(4-methylphenyl)-5-(3-methylphenyl)-pyrazol-4-ol, 1-(2-methylphenyl)-3-(4-methylphenyl)-5-(3-methylphenyl)-pyrazol-4-ol, 1-(3-methylphenyl)-3-(4-methylphenyl)-5-(3-methylphenyl)-pyrazol-4-ol, 1-(4-methoxyphenyl)-3-(4-methylphenyl)-5-butyl-pyrazol-4-ol, 1-Methyl-3-(4-methylphenyl)-5-(4-methylphenyl)-pyrazol-4-ol, 1-Ethyl-3-(4-methylphenyl)-5-(4-methylphenyl)-pyrazol-4-ol, 1-Propyl-3-(4-methylphenyl)-5-(4-methylphenyl)-pyrazol-4-ol, 1-Isopropyl-3-(4-methylphenyl)-5-(4-methylphenyl)-pyrazol-4-ol, 1-Butyl-3-(4-methylphenyl)-5-(4-methylphenyl)-pyrazol-4-ol, 1-Isobutyl-3-(4-methylphenyl)-5-(4-methylphenyl) -pyrazol-4-ol, 1-tert-Butyl-3-(4-methylphenyl)-5-(4-methylphenyl)-pyrazol-4-ol, 1-Phenyl-3-(4-methylphenyl)-5-(4-methylphenyl)-pyrazol-4-ol, 1-(4-methylphenyl)-3-(4-methylphenyl)-5-(4-methylphenyl)-pyrazol-4-ol, 1-(2-methylphenyl)-3-(4-methylphenyl)-5-(4-methylphenyl)-pyrazol-4-ol,1-(3-methylphenyl)-3-(4-methylphenyl)-5-(4-methylphenyl) -pyrazol-4-ol, 1-(4-methoxyphenyl)-3-(4-methylphenyl)-5-(4-methylphenyl)-pyrazol-4-ol, 1-(4-Hydroxy-2-(4-methoxyphenyl)-5-methyl-pyrazol-3-yl)ethanon, 1-(4-Hydroxy-2-(4-methylphenyl)-5-methyl-pyrazol-3-yl)ethanon, 1-(4-Hydroxy-2-(2-methoxyphenyl)-5-methyl-pyrazol-3-yl)ethanon, 1-(4-Hydroxy-2-(2-methylphenyl)-5-methyl-pyrazol-3-yl)ethanon, 1-(4-Hydroxy-2-phenyl-5-methyl-pyrazol-3-yl)ethanon, 1-(4-Hydroxy-2-methyl-5-methyl-pyrazol-3-yl)ethanon, 1-(4-Hydroxy-2-ethyl-5-methyl-pyrazol-3-yl)ethanon, 1-(4-Hydroxy-2-propyl-5-methyl-pyrazol-3-yl)ethanon, 1-(4-Hydroxy-2-isopropyl-5-methyl-pyrazol-3-yl)ethanon, 1-(4-Hydroxy-2-butyl-5-methyl-pyrazol-3-yl)ethanon, 1-(4-Hydroxy-2-isobutyl-5-methyl-pyrazol-3-yl)ethanon, 1-(4-Hydroxy-2-tert-butyl-5-methyl-pyrazol-3-yl)ethanon, 1-(4-Hydroxy-2-(4-methoxyphenyl)-5-phenyl-pyrazol-3-yl)ethanon, 1-(4-Hydroxy-2-(4-methylphenyl)-5-phenyl-pyrazol-3-yl)ethanon, 1-(4-Hydroxy-2-(2-methoxyphenyl)-5-phenyl-pyrazol-3-yl)ethanon, 1-(4-Hydroxy-2-(2-methylphenyl)-5-phenyl-pyrazol-3-yl)ethanon, 1-(4-Hydroxy-2-phenyl-5-methyl-phenyl-3-yl)ethanon, 1-(4-Hydroxy-2-methyl-5-methyl-phenyl-3-yl)ethanon, 1-(4-Hydroxy-2-ethyl-5-methyl-phenyl-3-yl)ethanon, 1-(4-Hydroxy-2-propyl-5-methyl-phenyl-3-yl)ethanon, 1-(4-Hydroxy-2-isopropyl-5-phenyl-pyrazol-3-yl)ethanon, 1-(4-Hydroxy-2-butyl-5-phenyl-pyrazol-3-yl)ethanon, 1-(4-Hydroxy-2-isobutyl-5-phenyl-pyrazol-3-yl)ethanon, 1-(4-Hydroxy-2-tert-butyl-5-phenyl-pyrazol-3-yl)ethanon, 1-(4-Hydroxy-2-(4-methoxyphenyl)-5-methyl-pyrazol-3-yl)-phenyl-methanon, 1-(4-Hydroxy-2-(4-methylphenyl)-5-methyl-pyrazol-3-yl)-phenyl-methanon, 1-(4-Hydroxy-2-(2-methoxyphenyl)-5-methyl-pyrazol-3-yl)-phenyl-methanon, 1-(4-Hydroxy-2-(2-methylphenyl)-5-methyl-pyrazol-3-yl)-phenyl-methanon, 1-(4-Hydroxy-2-phenyl-5-methyl-pyrazol-3-yl)-phenyl-methanon, 1-(4-Hydroxy-2-methyl-5-methyl-pyrazol-3-yl)-phenyl-methanon, 1-(4-Hydroxy-2-ethyl-5-methyl-pyrazol-3-yl)-phenyl-methanon, 1-(4-Hydroxy-2-propyl-5-methyl-pyrazol-3-yl)-phenyl-methanon, 1-(4-Hydroxy-2-isopropyl-5-methyl-pyrazol-3-yl)-phenyl-methanon, 1-(4-Hydroxy-2-butyl-5-methyl-pyrazol-3-yl)-phenyl-methanon, 1-(4-Hydroxy-2-isobutyl-5-methyl-pyrazol-3-yl)-phenyl-methanon, 1-(4-Hydroxy-2-tert-butyl-5-methyl-pyrazol-3-yl)-phenyl-methanon, 1-(4-Hydroxy-2-(4-methoxyphenyl)-5-phenyl-pyrazol-3-yl)-phenyl-methanon, 1-(4-Hydroxy-2-(4-methylphenyl)-5-phenyl-pyrazol-3-yl)-phenyl-methanon, 1-(4-Hydroxy-2-(2-methoxyphenyl)-5-phenyl-pyrazol-3-yl)-phenyl-methanon, 1-(4-Hydroxy-2-(2-methylphenyl)-5-phenyl-pyrazol-3-yl)-phenyl-methanon, 1-(4-Hydroxy-2-phenyl-5-methyl-phenyl-3-yl)-phenyl-methanon, 1-(4-Hydroxy-2-methyl-5-methyl-phenyl-3-yl)-phenyl-methanon, 1-(4-Hydroxy-2-ethyl-5-methyl-phenyl-3-yl)-phenyl-methanon, 1-(4-Hydroxy-2-propyl-5-methyl-phenyl-3-yl)-phenyl-methanon, 1-(4-Hydroxy-2-isopropyl-5-phenyl-pyrazol-3-yl)-phenyl-methanon, 1-(4-Hydroxy-2-butyl-5-phenyl-pyrazol-3-yl)-phenyl-methanon, 1-(4-Hydroxy-2-isobutyl-5-phenyl-pyrazol-3-yl)-phenyl-methanon, 1-(4-Hydroxy-2-tert-butyl-5-phenyl-pyrazol-3-yl)-phenyl-methanon, 1-(4-Hydroxy-1-(4-methoxyphenyl)-5-methyl-pyrazol-3-yl)ethanon, 1-(4-Hydroxy-1-(4-methylphenyl)-5-methyl-pyrazol-3-yl)ethanon, 1-(4-Hydroxy-1-(2-methoxyphenyl)-5-methyl-pyrazol-3-yl)ethanon, 1-(4-Hydroxy-1-(2-methylphenyl)-5-methyl-pyrazol-3-yl)ethanon, 1-(4-Hydroxy-1-phenyl-5-methyl-pyrazol-3-yl)ethanon, 1-(4-Hydroxy-1-methyl-5-methyl-pyrazol-3-I)ethanon, 1-(4-Hydroxy-1-ethyl-5-methyl-pyrazol-3-yl)ethanon, 1-(4-Hydroxy-1-propyl-5-methyl-yrazol-3-yl)ethanon, 1-(4-Hydroxy-1-isopropyl-5-methyl-pyrazol-3-yl)ethanon, 1-(4-Hydroxy-1-butyl-5-methyl-pyrazol-3-yl)ethanon, 1-(4-Hydroxy-1-isobutyl-5-methyl-pyrazol-3-yl)ethanon, 1-(4-hydroxy-1-tert-butyl-5-methyl-pyrazol-3-yl)ethanon, 1-(4-Hydroxy-1-(4-methoxyphenyl)-5-phenyl-pyrazol-3-yl)ethanon, 1-(4-Hydroxy-1-(4-methylphenyl)-5-phenyl-pyrazol-3-yl)ethanon, 1-(4-hydroxy-1-(2-methoxyphenyl)-5-phenyl-pyrazol-3-yl)ethanon, 1-(4-Hydroxy-1-(2-methylphenyl)-5-phenyl-pyrazol-3-yl)ethanon, 1-(4-Hydroxy-1-phenyl-5-methyl-phenyl-3-yl)ethanon, 1-(4-Hydroxy-1-methyl-5-methyl-phenyl-3-yl)ethanon, 1-(4-Hydroxy-1-ethyl-5-methyl-phenyl-3-yl)ethanon, 1-(4-hydroxy-1-propyl-5-methyl-phenyl-3-yl)ethanon, 1-(4-Hydroxy-1-isopropyl-5-phenyl-pyrazol-3-yl)ethanon, 1-(4-Hydroxy-1-butyl-5-phenyl-pyrazol-3-yl)ethanon, 1-(4-Hydroxy-1-isobutyl-5-phenyl-pyrazol-3-yl)ethanon, 1-(4-Hydroxy-1-tert-butyl-5-phenyl-pyrazol-3-yl)ethanon, 1-(4-Hydroxy-1-(4-methoxyphenyl)-5-methyl-pyrazol-3-yl)-phenyl-methanon, 1-(4-Hydroxy-1-(4-methylphenyl)-5-methyl-pyrazol-3-yl)-phenyl-methanon, 1-(4-Hydroxy-1-(2-methoxyphenyl)-5-methyl-pyrazol-3-yl)-phenyl-methanon, 1-(4-Hydroxy-1-(2-methylphenyl)-5-methyl-pyrazol-3-yl)-phenyl-methanon, 1-(4-Hydroxy-1-phenyl-5-methyl-pyrazol-3-yl)-phenyl-methanon, 1-(4-Hydroxy-1-methyl-5-methyl-pyrazol-3-yl)-phenyl-methanon, 1-(4-Hydroxy-1-ethyl-5-methyl-pyrazol-3-yl)-phenyl-methanon, 1-(4-Hydroxy-1-propyl-5-methyl-pyrazol-3-yl)-phenyl-methanon, 1-(4-Hydroxy-1-isopropyl-5-methyl-pyrazol-3-yl)-phenyl-methanon, 1-(4-Hydroxy-1-butyl-5-methyl-pyrazol-3-yl)-phenyl-methanon, 1-(4-Hydroxy-1-isobutyl-5-methyl-pyrazol-3-yl)-phenyl-methanon, 1-(4-Hydroxy-1-tert-butyl-5-methyl-pyrazol-3-yl)-phenyl-methanon, 1-(4-Hydroxy-1-(4-methoxyphenyl)-5-phenyl-pyrazol-3-yl)-phenyl-methanon, 1-(4-Hydroxy-1-(4-methylphenyl)-5-phenyl-pyrazol-3-yl)-phenyl-methanon, 1-(4-Hydroxy-1-(2-methoxyphenyl)-5-phenyl-pyrazol-3-yl)-phenyl-methanon, 1-(4-Hydroxy-1-(2-methylphenyl)-5-phenyl-pyrazol-3-yl)-phenyl-methanon, 1-(4-Hydroxy-1-phenyl-5-methyl-phenyl-3-yl)-phenyl-methanon, 1-(4-Hydroxy-1-methyl-5-methyl-phenyl-3-yl)-phenyl-methanon, 1-(4-Hydroxy-1-ethyl-5-methyl-phenyl-3-yl)-phenyl-methanon, 1-(4-Hydroxy-1-propyl-5-methyl-phenyl-3-yl)-phenyl-methanon, 1-(4-Hydroxy-2-isopropyl-5-phenyl-pyrazol-3-yl)-phenyl-methanon, 1-(4-Hydroxy-1-butyl-5-phenyl-pyrazol-3-yl)-phenyl-methanon, 1-(4-Hydroxy-1-isobutyl-5-phenyl-pyrazol-3-yl)-phenyl-methanon oder 1-(4-Hydroxy-1-tert-butyl-5-phenyl-pyrazol-3-yl)-phenyl-methanon.

Sofern nicht näher spezifiziert steht Butyl für iso-Butyl oder n-Butyl. Insbesonders sind solche Verbindungen bevorzugt, die keinen Pyridyl-Substituenten aufweisen.

In bevorzugten Verbindungen der allgemeinen Formel (III) sind
R⁹ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Alkanoyl mit C₁- bis C₆-Alkyl, oder 4-iso-Propoxyphenyl,
R¹⁰ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl, oder 4-iso-Propoxyphenyl,
R¹¹ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁- bis C₆-Alkyl verestert sein kann, (N,N-Dimethylsulfonamid)-phenyl, Sulfonamidphenyl, Carboxylester mit C₁- bis C₈-Alkyl wie -COOMe, Alkyanoyl mit C₁- bis C₆-Alkyl, oder 4-iso-Propoxyphenyl.

Besonders bevorzugte Verbindungen der allgemeinen Formel (III) sind
1-Methyl-pyrazol-3-ol, 1-Ethyl-pyrazol-3-ol, 1-Propyl-pyrazol-3-ol, 1-Isopropyl-pyrazol-3-ol, 1-Butyl-pyrazol-3-ol, 1-Isobutyl-1H-pyrazol-3-ol,1-tert-Butyl-pyrazol-3-ol, 1-Phenyl-pyrazol-3-ol, 1-(4-Methoxyphenyl)-pyrazol-3-ol, 1-(2-Methoxyphenyl)-pyrazol-3-ol, 1-(4-Methylphenyl)-pyrazol-3-ol, 1-(4-Methylphenyl)-pyrazol-3-ol, 1-(2-methylphenyl)-pyrazol-3-ol, 1-(2-methylphenyl)-pyrazol-3-ol, 1-(3-methylphenyl)-pyrazol-3-ol, 1-(4-Methoxyphenyl)-pyrazol-3-ol, 1-Methyl-4-phenyl-pyrazol-3-ol, 1-Ethyl-4-phenyl-pyrazol-3-ol, 1-Propyl-4-phenyl-pyrazol-3-ol, 1-Isopropyl-4-phenyl-pyrazol-3-ol, 1-Butyl-4-phenyl-pyrazol-3-ol, 1-Isobutyl-4-phenyl-pyrazol-3-ol, 1-tert-Butyl-4-phenyl-pyrazol-3-ol, 1-Phenyl-4-phenyl-pyrazol-3-ol, 1-(4-methylphenyl)-4-phenyl-pyrazol-3-ol, 1-(2-methylphenyl)-4-phenyl-pyrazol-3-ol, 1-(3-methylphenyl)-4-phenyl-pyrazol-3-ol, 1-(4-Methoxyphenyl)-4-phenyl-pyrazol-3-ol, 1-Methyl-4-methyl-pyrazol-3-ol, 1-Ethyl-4-methyl-pyrazol-3-ol, 1-Propyl-4-methyl-pyrazol-3-ol, 1-Isopropyl-4-methyl-pyrazol-3-ol, 1-Butyl-4-methyl-pyrazol-3-ol, 1-Isobutyl-4-methyl-pyrazol-3-ol, 1-tert-Butyl-4-methyl -pyrazol-3-ol, 1-Phenyl-4-methyl-pyrazol-3-ol, 1-(4-methylphenyl)-4- methyl -pyrazol-3-ol, 1-(2-methylphenyl)-4-methyl -pyrazol-3-ol, 1-(3-methylphenyl)-4-methyl-pyrazol-3-ol, 1-(4-Methoxyphenyl)-4-methyl -pyrazol-3-ol, 1-methyl-4-ethyl-pyrazol-3-ol, 1-ethyl-4-ethyl-pyrazol-3-ol, 1-propyl-4-ethyl-pyrazol-3-ol, 1-isopropyl-4-ethyl-pyrazol-3-ol, 1-butyl-4-ethyl -pyrazol-3-ol, 1-Isobutyl-4-ethyl-pyrazol-3-ol, 1-tert-butyl-4-ethyl-pyrazol-3-ol, 1-Phenyl-4-ethyl-pyrazol-3-ol, 1-(4-methylphenyl)-4-ethyl-pyrazol-3-ol, 1-(2-methylphenyl)-4-ethyl-pyrazol-3-ol, 1-(3-methylphenyl)-4-ethyl-pyrazol-3-ol, 1-(4-Methoxyphenyl)-4-ethyl -pyrazol-3-ol, 1-Methyl-4-propyl-pyrazol-3-ol, 1-Ethyl-4-propyl -pyrazol-3-ol, 1-Propyl-4-propyl-pyrazol-3-ol, 1-Isopropyl-4-methyl-pyrazol-3-ol, 1-Butyl-4-propyl -pyrazol-3-ol, 1-Isobutyl-4-propyl-pyrazol-3-ol, 1-tert-Butyl-4-propyl-pyrazol-3-ol, 1-Phenyl-4- propyl-pyrazol-3-ol, 1-(4-methylphenyl)-4-propyl-pyrazol-3-ol, 1-(2-methylphenyl)-4- propyl-pyrazol-3-ol, 1-(3-methylphenyl)-4-propyl-pyrazol-3-ol, 1-(4-Methoxyphenyl)-4-propyl-pyrazol-3-ol, 1-Methyl-4-isopropyl-pyrazol-3-ol, 1-Ethyl-4-isopropyl-pyrazol-3-ol, 1-Propyl-4-isopropyl-pyrazol-3-ol, 1-lsopropyl-4-isopropyl-pyrazol-3-ol, 1-Butyl-4-isopropyl-pyrazol-3-ol, 1-Isobutyl-4-isopropyl-pyrazol-3-ol, 1-tert-Butyl-4-isopropyl-pyrazol-3-ol, 1-Phenyl-4-isopropyl-pyrazol-3-ol, 1-(4-methylphenyl)-4-isopropyl-pyrazol-3-ol, 1-(2-methylphenyl)-4-isopropyl -pyrazol-3-ol, 1-(3-methylphenyl)-4-isopropyl-pyrazol-3-ol, 1-(4-Methoxyphenyl)-4-isopropyl -pyrazol-3-ol, 1-Methyl-4- butyl -pyrazol-3-ol, 1-Ethyl-4-butyl-pyrazol-3-ol, 1-Propyl-4-butyl -pyrazol-3-ol, 1-Isopropyl-4-butyl-pyrazol-3-ol, 1-Butyl-4-butyl-pyrazol-3-ol, 1-Isobutyl-4-butyl-pyrazol-3-ol, 1-tert-Butyl-4-butyl-pyrazol-3-ol, 1-Phenyl-4-butyl-pyrazol-3-ol, 1-(4-methylphenyl)-4-butyl-pyrazol-3-ol, 1-(2-methylphenyl)-4-butyl-pyrazol-3-ol, 1-(3-methylphenyl)-4-butyl-pyrazol-3-ol, 1-(4-Methoxyphenyl)-4-butyl-pyrazol-3-ol, 1-Methyl-4-isobutyl-pyrazol-3-ol, 1-Ethyl-4-isobutyl-pyrazol-3-ol, 1-Propyl-4-isobutyl-pyrazol-3-ol, 1-Isopropyl-4-isobutyl-pyrazol-3-ol, 1-Butyl-4-isobutyl-pyrazol-3-ol, 1-Isobutyl-4-isobutyl-pyrazol-3-ol, 1-tert-Butyl-4-isobutyl-pyrazol-3-ol, 1-Phenyl-4-isobutyl-pyrazol-3-ol, 1-(4-methylphenyl)-4-isobutyl-pyrazol-3-ol, 1-(2-methylphenyl)-4-isobutyl-pyrazol-3-ol, 1-(3-methylphenyl)-4-isobutyl-pyrazol-3-ol, 1-(4-Methoxyphenyl)-4-isobutyl-pyrazol-3-ol, 1-Methyl-4-tert-butyl-pyrazol-3-ol, 1-Ethyl-4-tert-butyl-pyrazol-3-ol,1-Propyl-4-tert-butyl-pyrazol-3-ol, 1-Isopropyl-4-tert-butyl-pyrazol-3-ol, 1-Butyl-4- tert-butyl-pyrazol-3-ol, 1-Isobutyl-4-tert-butyl-pyrazol-3-ol, 1-tert-Butyl-4-tert-butyl -pyrazol-3-ol, 1-Phenyl-4-tert-butyl-pyrazol-3-ol, 1-(4-methylphenyl)-4-tert-butyl-pyrazol-3-ol, 1-(2-methylphenyl)-4-tert-butyl-pyrazol-3-ol, 1-(3-methylphenyl)-4-tert-butyl-pyrazol-3-ol, 1-(4-Methoxyphenyl)-4-phenyl-pyrazol-3-ol, 1-Methyl-4-phenyl-pyrazol-3-ol, 1-Ethyl-4-phenyl-pyrazol-3-ol, 1-Propyl-4-phenyl-pyrazol-3-ol, 1-Isopropyl-4-phenyl-pyrazol-3-ol, 1-Butyl-4-phenyl-pyrazol-3-ol, 1-Isobutyl-4-phenyl-pyrazol-3-ol, 1-tert-Butyl-4-phenyl-pyrazol-3-ol, 1-Phenyl-4-phenyl-pyrazol-3-ol, 1-(4-methylphenyl)-4-phenyl-pyrazol-3-ol, 1-(2-methylphenyl)-4-phenyl-pyrazol-3-ol, 1-(3-methylphenyl)-4-phenyl-pyrazol-3-ol, 1-(4-Methoxyphenyl)-4-(2-methylphenyl)-pyrazol-3-ol, 1-Methyl-4-(2-methylphenyl)-pyrazol-3-ol, 1-Ethyl-4-(2-methylphenyl) -pyrazol-3-ol, 1-Propyl-4-(2-methylphenyl)-pyrazol-3-ol, 1-Isopropyl-4-(2-methylphenyl)-pyrazol-3-ol, 1-Butyl-4-(2-methylphenyl)-pyrazol-3-ol, 1-Isobutyl-4-(2-methylphenyl)-pyrazol-3-ol, 1-tert-Butyl-4-(2-methylphenyl)-pyrazol-3-ol, 1-Phenyl-4-(2-methylphenyl)-pyrazol-3-ol, 1-(4-methylphenyl)-4-(2-methylphenyl)-pyrazol-3-ol, 1-(2-methylphenyl)-4-(2-methylphenyl)-pyrazol-3-ol, 1-(3-methylphenyl)-4-(2-methylphenyl)-pyrazol-3-ol, 1-(4-Methoxyphenyl)-4-(3-methylphenyl)-pyrazol-3-ol, 1-Methyl-4-(3-methylphenyl)-pyrazol-3-ol, 1-Ethyl-4-(3-methylphenyl)-pyrazol-3-ol, 1-Propyl-4-(3-methylphenyl)-pyrazol-3-ol, 1-Isopropyl-4-(3-methylphenyl)-pyrazol-3-ol , 1-Butyl-4-(3-methylphenyl)-pyrazol-3-ol, 1-Isobutyl-4-(3-methylphenyl)-pyrazol-3-ol, 1-tert-Butyl-4-(3-methylphenyl)-pyrazol-3-ol, 1-Phenyl-4-(3-methylphenyl)-pyrazol-3-ol, 1-(4-methylphenyl)-4-(3-methylphenyl)-pyrazol-3-ol, 1-(2-methylphenyl)-4-(3-methylphenyl)-pyrazol-3-ol, 1-(3-methylphenyl)-4-(3-methylphenyl)-pyrazol-3-ol, 1-(4-Methoxyphenyl)-4-(3-methylphenyl) -pyrazol-3-ol, 1-Methyl-4-(3-methylphenyl)-pyrazol-3-ol, 1-Ethyl-4-(3-methylphenyl) -pyrazol-3-ol, 1-Propyl-4-(3-methylphenyl)-pyrazol-3-ol, 1-Isopropyl-4-(3-methylphenyl)-pyrazol-3-ol, 1-Butyl-4-(3-methylphenyl)-pyrazol-3-ol, 1-Isobutyl-4-(3-methylphenyl)-pyrazol-3-ol, 1-tert-Butyl-4-butyl-pyrazol-3-ol, 1-Phenyl-4-(3-methylphenyl)-pyrazol-3-ol, 1-(4-methylphenyl)-4- (3-methylphenyl)-pyrazol-3-ol, 1-(2-methylphenyl)-4-(3-methylphenyl)-pyrazol-3-ol, 1-(3-methylphenyl)-4-(3-methylphenyl)-pyrazol-3-ol, 1-(4-Methoxyphenyl)-4-butyl-pyrazol-3-ol, 1-Methyl-4-(4-methylphenyl)-pyrazol-3-ol, 1-Ethyl-4-(4-methylphenyl)-pyrazol-3-ol, 1-Propyl-4-(4-methylphenyl)-pyrazol-3-ol, 1-Isopropyl-4-(4-methylphenyl)-pyrazol-3-ol, 1-Butyl-4-(4-methylphenyl)-pyrazol-3-ol, 1-Isobutyl-4-(4-methylphenyl)-pyrazol-3-ol, 1-tert-Butyl-4-(4-methylphenyl)-pyrazol-3-ol, 1-Phenyl-4-(4-methylphenyl)-pyrazol-3-ol, 1-(4-methylphenyl)-4-(4-methylphenyl)-pyrazol-3-ol, 1-(2-methylphenyl)-4-(4-methylphenyl)-pyrazol-3-ol, 1-(3-methylphenyl)-4-(4-methylphenyl)-pyrazol-3-ol, 1-(4-Methoxyphenyl)-4-(4-methylphenyl)-pyrazol-3-ol, 1-Methyl-5-phenyl-pyrazol-3-ol, 1-Ethyl-5-phenyl-pyrazol-3-ol, 1-Propyl-5-phenyl-pyrazol-3-ol, 1-Isopropyl-5-phenyl-pyrazol-3-ol, 1-Butyl-5-phenyl-pyrazol-3-ol, 1-Isobutyl-5-phenyl-pyrazol-3-ol, 1-tert-Butyl-5-phenyl-pyrazol-3-ol, 1-Phenyl-5-phenyl-pyrazol-3-ol, 1-(4-methylphenyl)-5-phenyl-pyrazol-3-ol, 1-(2-methylphenyl)-5-phenyl-pyrazol-3-ol, 1-(3-methylphenyl)-5-phenyl-pyrazol-3-ol, 1-(4-Methoxyphenyl)-5-phenyl-pyrazol-3-ol, 1-Methyl-5-methyl-pyrazol-3-ol, 1-Ethyl-5-methyl-pyrazol-3-ol, 1-Propyl-5-methyl-pyrazol-3-ol, 1-Isopropyl-5-methyl-pyrazol-3-ol, 1-Butyl-5-methyl-pyrazol-3-ol, 1-Isobutyl-5-methyl-pyrazol-3-ol, 1-tert-Butyl-5-methyl-pyrazol-3-ol, 1-Phenyl-5-methyl-pyrazol-3-ol, 1-(4-methylphenyl)-5-methyl-pyrazol-3-ol, 1-(2-methylphenyl)-5-methyl-pyrazol-3-ol, 1-(3-methylphenyl)-5-methyl-pyrazol-3-ol, 1-(4-Methoxyphenyl)-5-methyl-pyrazol-3-ol, 1-methyl-5-ethyl-pyrazol-3-ol, 1-ethyl-5-ethyl-pyrazol-3-ol, 1-propyl-5-ethyl-pyrazol-3-ol, 1-isopropyl-5-ethyl-pyrazol-3-ol, 1-butyl-5-ethyl -pyrazol-3-ol, 1-Isobutyl-5-ethyl-pyrazol-3-ol, 1-tert-butyl-5-ethyl-pyrazol-3-ol, 1-Phenyl-5-ethyl -pyrazol-3-ol, 1-(4-methylphenyl)-5-ethyl-pyrazol-3-ol, 1-(2-methylphenyl)-5-ethyl-pyrazol-3-ol, 1-(3-methylphenyl)-5- ethyl -pyrazol-3-ol, 1-(4-Methoxyphenyl)-5-ethyl -pyrazol-3-ol, 1-Methyl-5-propyl-pyrazol-3-ol, 1-Ethyl-5-propyl-pyrazol-3-ol, 1-Propyl-5-propyl-pyrazol-3-ol, 1-Isopropyl-5-methyl-pyrazol-3-ol, 1-Butyl-5-propyl-pyrazol-3-ol, 1-Isobutyl-5-propyl-pyrazol-3-ol, 1-tert-Butyl-5-propyl-pyrazol-3-ol, 1-Phenyl-5-propyl-pyrazol-3-ol, 1-(4-methylphenyl)-5-propyl -pyrazol-3-ol, 1-(2-methylphenyl)-5-propyl-pyrazol-3-ol, 1-(3-methylphenyl)-5-propyl -pyrazol-3-ol, 1-(4-Methoxyphenyl)-5-propyl-pyrazol-3-ol, 1-Methyl-5-isopropyl-pyrazol-3-ol, 1-Ethyl-5- isopropyl -pyrazol-3-ol, 1-Propyl-5- isopropyl -pyrazol-3-ol, 1-Isopropyl-5-isopropyl-pyrazol-3-ol, 1-Butyl-5-isopropyl-pyrazol-3-ol, 1-Isobutyl-5-isopropyl-pyrazol-3-ol, 1-tert-Butyl-5-isopropyl-pyrazol-3-ol, 1-Phenyl-5-isopropyl-pyrazol-3-ol, 1-(4-methylphenyl)-5-isopropyl-pyrazol-3-ol, 1-(2-methylphenyl)-5- isopropyl-pyrazol-3-ol, 1-(3-methylphenyl)-5-isopropyl-pyrazol-3-ol, 1-(4-Methoxyphenyl)-5- isopropyl-pyrazol-3-ol, 1-Methyl-5-butyl-pyrazol-3-ol, 1-Ethyl-5-butyl-pyrazol-3-ol, 1-Propyl-5-butyl-pyrazol-3-ol, 1-Isopropyl-5-butyl-pyrazol-3-ol, 1-Butyl-5-butyl-pyrazol-3-ol, 1-Isobutyl-5-butyl-pyrazol-3-ol, 1-tert-Butyl-5-butyl-pyrazol-3-ol, 1-Phenyl-5-butyl-pyrazol-3-ol, 1-(4-methylphenyl)-5-butyl-pyrazol-3-ol, 1-(2-methylphenyl)-5-butyl-pyrazol-3-ol, 1-(3-methylphenyl)-5-butyl-pyrazol-3-ol, 1-(4-Methoxyphenyl)-5-butyl-pyrazol-3-ol, 1-Methyl-5-isobutyl-pyrazol-3-ol, 1-Ethyl-5-isobutyl-pyrazol-3-ol, 1-Propyl-5-isobutyl-pyrazol-3-ol, 1-Isopropyl-5-isobutyl-pyrazol-3-ol, 1-Butyl-5-isobutyl-pyrazol-3-ol, 1-Isobutyl-5-isobutyl-pyrazol-3-ol, 1-tert-Butyl-5-isobutyl-pyrazol-3-ol, 1-Phenyl-5-isobutyl -pyrazol-3-ol, 1-(4-methylphenyl)-5-isobutyl-pyrazol-3-ol, 1-(2-methylphenyl)-5-isobutyl-pyrazol-3-ol, 1-(3-methylphenyl)-5-isobutyl-pyrazol-3-ol, 1-(4-Methoxyphenyl)-5-isobutyl-pyrazol-3-ol, 1-Methyl-5-tert-butyl-pyrazol-3-ol, 1-Ethyl-5-tert-butyl-pyrazol-3-ol, 1-Propyl-5-tert-butyl-pyrazol-3-ol, 1-Isopropyl-5-tert-butyl-pyrazol-3-ol, 1-Butyl-5-tert-butyl-pyrazol-3-ol, 1-Isobutyl-5-tert-butyl-pyrazol-3-ol, 1-tert-Butyl-5- tert-butyl -pyrazol-3-ol, 1-Phenyl-5- tert-butyl -pyrazol-3-ol, 1-(4-methylphenyl)-5-tert-butyl -pyrazol-3-ol, 1-(2-methylphenyl)-5- tert-butyl -pyrazol-3-ol, 1-(3-methylphenyl)-5-tert-butyl-pyrazol-3-ol, 1-(4-Methoxyphenyl)-5-phenyl-pyrazol-3-ol, 1-Methyl-5-phenyl-pyrazol-3-ol, 1-Ethyl-5-phenyl-pyrazol-3-ol, 1-Propyl-5-phenyl-pyrazol-3-ol, 1-Isopropyl-5-phenyl-pyrazol-3-ol, 1-Butyl-5-phenyl-pyrazol-3-ol, 1-Isobutyl-5-phenyl-pyrazol-3-ol, 1-tert-Butyl-5-phenyl-pyrazol-3-ol, 1-Phenyl-5- phenyl -pyrazol-3-ol, 1-(4-methylphenyl)-5- phenyl -pyrazol-3-ol, 1-(2-methylphenyl)-5-phenyl-pyrazol-3-ol, 1-(3-methylphenyl)-5-phenyl-pyrazol-3-ol, 1-(4-Methoxyphenyl)-5-(2-methylphenyl)-pyrazol-3-ol, 1-Methyl-5-(2-methylphenyl)-pyrazol-3-ol, 1-Ethyl-5- (2-methylphenyl) -pyrazol-3-ol, 1-Propyl-5- (2-methylphenyl)-pyrazol-3-ol, 1-Isopropyl-5-(2-methylphenyl)-pyrazol-3-ol, 1-Butyl-5-(2-methylphenyl)-pyrazol-3-ol, 1-Isobutyl-5-(2-methylphenyl)-pyrazol-3-ol, 1-tert-Butyl-5-(2-methylphenyl)-pyrazol-3-ol, 1-Phenyl-5-(2-methylphenyl)-pyrazol-3-ol, 1-(4-methylphenyl)-5-(2-methylphenyl)-pyrazol-3-ol, 1-(2-methylphenyl)-5-(2-methylphenyl)-pyrazol-3-ol, 1-(3-methylphenyl)-5-(2-methylphenyl)-pyrazol-3-ol, 1-(4-Methoxyphenyl)-5-(3-methylphenyl)-pyrazol-3-ol, 1-Methyl-5-(3-methylphenyl)-pyrazol-3-ol, 1-Ethyl-5-(3-methylphenyl)-pyrazol-3-ol, 1-Propyl-5-(3-methylphenyl)-pyrazol-3-ol, 1-Isopropyl-5-(3-methylphenyl)-pyrazol-3-ol, 1-Butyl-5-(3-methylphenyl)-pyrazol-3-ol, 1-Isobutyl-5-(3-methylphenyl)-pyrazol-3-ol, 1-tert-Butyl-5-(3-methylphenyl)-pyrazol-3-ol, 1-Phenyl-5-(3-methylphenyl)-pyrazol-3-ol, 1-(4-methylphenyl)-5-(3-methylphenyl)-pyrazol-3-ol, 1-(2-methylphenyl)-5-(3-methylphenyl)-pyrazol-3-ol, 1-(3-methylphenyl)-5-(3-methylphenyl)-pyrazol-3-ol, 1-(4-Methoxyphenyl)-5-(3-methylphenyl)-pyrazol-3-ol, 1-Methyl-5-(3-methylphenyl)-pyrazol-3-ol, 1-Ethyl-5-(3-methylphenyl)-pyrazol-3-ol, 1-Propyl-5-(3-methylphenyl)-pyrazol-3-ol, 1-Isopropyl-5-(3-methylphenyl)-pyrazol-3-ol, 1-Butyl-5-(3-methylphenyl)-pyrazol-3-ol, 1-Isobutyl-5-(3-methylphenyl)-pyrazol-3-ol, 1-tert-Butyl-5-butyl-pyrazol-3-ol, 1-Phenyl-5-(3-methylphenyl)-pyrazol-3-ol, 1-(4-methylphenyl)-5- (3-methylphenyl) -pyrazol-3-ol, 1-(2-methylphenyl)-5- (3-methylphenyl)-pyrazol-3-ol, 1-(3-methylphenyl)-5-(3-methylphenyl)-pyrazol-3-ol, 1-(4-Methoxyphenyl)-5-butyl-pyrazol-3-ol, 1-Methyl-5-(4-methylphenyl)-pyrazol-3-ol, 1-Ethyl-5-(4-methylphenyl)-pyrazol-3-ol, 1-Propyl-5-(4-methylphenyl)-pyrazol-3-ol, 1-Isopropyl-5-(4-methylphenyl)-pyrazol-3-ol, 1-Butyl-5-(4-methylphenyl)-pyrazol-3-ol, 1-Isobutyl-5-(4-methylphenyl)-pyrazol-3-ol, 1-tert-Butyl-5-(4-methylphenyl)-pyrazol-3-ol, 1-Phenyl-5-(4-methylphenyl) -pyrazol-3-ol, 1-(4-methylphenyl)-5- (4-methylphenyl) -pyrazol-3-ol, 1-(2-methylphenyl)-5-(4-methylphenyl)-pyrazol-3-ol, 1-(3-methylphenyl)-5-(4-methylphenyl)-pyrazol-3-ol, 1-(4-Methoxyphenyl)-5-(4-methylphenyl)-pyrazol-3-ol,1-Methyl-4-methyl-5-phenyl-pyrazol-3-ol, 1-Ethyl-4-methyl5-phenyl-pyrazol-3-ol, 1-Propyl-4-methyl-5-phenyl-pyrazol-3-ol, 1-Isopropyl-4-methyl-5-phenyl-pyrazol-3-ol, 1-Butyl-4-methyl-5-phenyl-pyrazol-3-ol, 1-Isobutyl-4-methyl-5-phenyl-pyrazol-3-ol, 1-tert-Butyl-4-methyl-5-phenyl-pyrazol-3-ol, 1-Phenyl-4-methyl-5-phenyl-pyrazol-3-ol, 1-(4-methylphenyl)-4-methyl-5-phenyl-pyrazol-3-ol, 1-(2-methylphenyl)-4-methyl-5-phenyl-pyrazol-3-ol, 1-(3-methylphenyl)-4-methyl-5-phenyl-pyrazol-3-ol, 1-(4-Methoxyphenyl)-4-methyl-5-phenyl-pyrazol-3-ol, 1-Methyl-4-methyl-5-methyl-pyrazol-3-ol, 1-Ethyl-4-methyl-5-methyl-pyrazol-3-ol, 1-Propyl-4-methyl-5-methyl-pyrazol-3-ol, 1-Isopropyl-4-methyl-5-methyl-pyrazol-3-ol, 1-Butyl-4-methyl-5-methyl-pyrazol-3-ol, 1-Isobutyl-4-methyl-5-methyl-pyrazol-3-ol, 1-tert-Butyl-4-methyl-5-methyl-pyrazol-3-ol, 1-Phenyl-4-methyl-5-methyl-pyrazol-3-ol, 1-(4-methylphenyl)-4-methyl-5-methyl -pyrazol-3-ol, 1-(2-methylphenyl)-4-methyl-5-methyl-pyrazol-3-ol, 1-(3-methylphenyl)-4-methyl-5-methyl-pyrazol-3-ol, 1-(4-Methoxyphenyl)-4-methyl-5-methyl-pyrazol-3-ol, 1-methyl-4-methyl-5-ethyl-pyrazol-3-ol, 1-ethyl-4-methyl-5-ethyl-pyrazol-3-ol, 1-propyl-4-methyl-5-ethyl-pyrazol-3-ol, 1-isopropyl-4-methyl-5- ethyl -pyrazol-3-ol, 1-butyl-4-methyl-5-ethyl-pyrazol-3-ol, 1-Isobutyl-4-methyl-5-ethyl-pyrazol-3-ol, 1-tert-butyl-4-methyl-5-ethyl-pyrazol-3-ol, 1-Phenyl-4-methyl-5-ethyl-pyrazol-3-ol, 1-(4-methylphenyl)-4-methyl-5-ethyl-pyrazol-3-ol, 1-(2-methylphenyl)-4-methyl-5-ethyl-pyrazol-3-ol, 1-(3-methylphenyl)-4-methyl-5-ethyl-pyrazol-3-ol, 1-(4-Methoxyphenyl)-4-methyl-5-ethyl-pyrazol-3-ol, 1-Methyl-4-methyl-5-propyl-pyrazol-3-ol, 1-Ethyl-4-methyl-5-propyl-pyrazol-3-ol, 1-Propyl-4-methyl-5-propyl-pyrazol-3-ol, 1-Isopropyl-4-methyl-5-methyl-pyrazol-3-ol, 1-Butyl-4-methyl-5-propyl-pyrazol-3-ol, 1-Isobutyl-4-methyl-5-propyl-pyrazol-3-ol, 1-tert-Butyl-4-methyl-5-propyl-pyrazol-3-ol, 1-Phenyl-4-methyl-5- propyl-pyrazol-3-ol, 1-(4-methylphenyl)-4-methyl-5-propyl-pyrazol-3-ol, 1-(2-methylphenyl)-4-methyl-5- propyl -pyrazol-3-ol, 1-(3-methylphenyl)-4-methyl-5- propyl -pyrazol-3-ol, 1-(4-Methoxyphenyl)-4-methyl-5-propyl-pyrazol-3-ol, 1-Methyl-4-methyl-5-isopropyl-pyrazol-3-ol, 1-Ethyl-4-methyl-5-isopropyl-pyrazol-3-ol, 1-Propyl-4-methyl-5-isopropyl-pyrazol-3-ol, 1-Isopropyl-4-methyl-5-isopropyl-pyrazol-3-ol, 1-Butyl-4-methyl-5-isopropyl-pyrazol-3-ol, 1-Isobutyl-4-methyl-5-isopropyl-pyrazol-3-ol, 1-tert-Butyl-4-methyl-5-isopropyl-pyrazol-3-ol, 1-Phenyl-4-methyl-5-isopropyl-pyrazol-3-ol, 1-(4-methylphenyl)-4-methyl-5-isopropyl -pyrazol-3-ol, 1-(2-methylphenyl)-4-methyl-5-isopropyl-pyrazol-3-ol, 1-(3-methylphenyl)-4-methyl-5-isopropyl-pyrazol-3-ol, 1-(4-Methoxyphenyl)-4-methyl-5-isopropyl-pyrazol-3-ol, 1-Methyl-4-methyl-5-butyl-pyrazol-3-ol, 1-Ethyl-4-methyl-5-butyl-pyrazol-3-ol, 1-Propyl-4-methyl-5-butyl-pyrazol-3-ol, 1-Isopropyl-4-methyl-5-butyl-pyrazol-3-ol, 1-Butyl-4-methyl-5- butyl -pyrazol-3-ol, 1-Isobutyl-4-methyl-5-butyl-pyrazol-3-ol, 1-tert-Butyl-4-methyl-5-butyl -pyrazol-3-ol, 1-Phenyl-4-methyl-5-butyl-pyrazol-3-ol, 1-(4-methylphenyl)-4-methyl-5-butyl-pyrazol-3-ol, 1-(2-methylphenyl)-4-methyl-5- butyl -pyrazol-3-ol, 1-(3-methylphenyl)-4-methyl-5-butyl-pyrazol-3-ol, 1-(4-Methoxyphenyl)-4-methyl-5-butyl-pyrazol-3-ol,
1-Methyl-4-methyl-5-isobutyl-pyrazol-3-ol, 1-Ethyl-4-methyl-5-isobutyl-pyrazol-3-ol, 1-Propyl-4-methyl-5-isobutyl-pyrazol-3-ol, 1-Isopropyl-4-methyl-5-isobutyl-pyrazol-3-ol, 1-Butyl-4-methyl-5-isobutyl-pyrazol-3-ol, 1-Isobutyl-4-methyl-5-isobutyl-pyrazol-3-ol, 1-tert-Butyl-4-methyl-5-isobutyl-pyrazol-3-ol, 1-Phenyl-4-methyl-5-isobutyl-pyrazol-3-ol, 1-(4-methylphenyl)-4-methyl-5-isobutyl-pyrazol-3-ol, 1-(2-methylphenyl)-4-methyl-5- isobutyl-pyrazol-3-ol, 1-(3-methylphenyl)-4-methyl-5-isobutyl-pyrazol-3-ol, 1-(4-Methoxyphenyl)-4-methyl-5-isobutyl-pyrazol-3-ol, 1-Methyl-4-methyl-5-tert-butyl-pyrazol-3-ol, 1-Ethyl-4-methyl-5-tert-butyl-pyrazol-3-ol, 1-Propyl-4-methyl-5-tert-butyl-pyrazol-3-ol, 1-Isopropyl-4-methyl-5-tert-butyl-pyrazol-3-ol, 1-Butyl-4-methyl-5-tert-butyl-pyrazol-3-ol, 1-Isobutyl-4-methyl-5-tert-butyl-pyrazol-3-ol, 1-tert-Butyl-4-methyl-5-tert-butyl-pyrazol-3-ol, 1-Phenyl-4-methyl-5-tert-butyl-pyrazol-3-ol, 1-(4-methylphenyl)-4-methyl-5- tert-butyl -pyrazol-3-ol, 1-(2-methylphenyl)-4-methyl-5-tert-butyl -pyrazol-3-ol, 1-(3-methylphenyl)-4-methyl-5- tert-butyl-pyrazol-3-ol, 1-(4-Methoxyphenyl)-4-methyl-5- phenyl-pyrazol-3-ol, 1-Methyl-4-methyl-5-phenyl-pyrazol-3-ol, 1-Ethyl-4-methyl-5-phenyl -pyrazol-3-ol, 1-Propyl-4-methyl-5-phenyl-pyrazol-3-ol, 1-Isopropyl-4-methyl-5-phenyl-pyrazol-3-ol, 1-Butyl-4-methyl-5-phenyl-pyrazol-3-ol, 1-Isobutyl-4-methyl-5-phenyl-pyrazol-3-ol, 1-tert-Butyl-4-methyl-5-phenyl-pyrazol-3-ol, 1-Phenyl-4-methyl-5-phenyl-pyrazol-3-ol, 1-(4-methylphenyl)-4-methyl-5-phenyl-pyrazol-3-ol, 1-(2-methylphenyl)-4-methyl-5-phenyl-pyrazol-3-ol, 1-(3-methylphenyl)-4-methyl-5-phenyl -pyrazol-3-ol, 1-(4-Methoxyphenyl)-4-methyl-5-(2-methylphenyl)-pyrazol-3-ol, 1-Methyl-4-methyl-5-(2-methylphenyl)-pyrazol-3-ol, 1-Ethyl-4-methyl-5-(2-methylphenyl)-pyrazol-3-ol, 1-Propyl-4-methyl-5- (2-methylphenyl) -pyrazol-3-ol, 1-Isopropyl-4-methyl-5-(2-methylphenyl)-pyrazol-3-ol, 1-Butyl-4-methyl-5-(2-methylphenyl)-pyrazol-3-ol, 1-Isobutyl-4-methyl-5-(2-methylphenyl)-pyrazol-3-ol, 1-tert-Butyl-4-methyl-5-(2-methylphenyl)-pyrazol-3-ol, 1-Phenyl-4-methyl-5- (2-methylphenyl) -pyrazol-3-ol, 1-(4-methylphenyl)-4-methyl-5- (2-methylphenyl)-pyrazol-3-ol, 1-(2-methylphenyl)-4-methyl-5-(2-methylphenyl)-pyrazol-3-ol, 1-(3-methylphenyl)-4-methyl-5- (2-methylphenyl) -pyrazol-3-ol, 1-(4-Methoxyphenyl)-4-methyl-5- (3-methylphenyl) -pyrazol-3-ol, 1-Methyl-4-methyl-5- (3-methylphenyl) -pyrazol-3-ol, 1-Ethyl-4-methyl-5- (3-methylphenyl) -pyrazol-3-ol, 1-Propyl-4-methyl-5- (3-methylphenyl) -pyrazol-3-ol, 1-lsopropyl-4-methyl-5- (3-methylphenyl) -pyrazol-3-ol, 1-Butyl-4-methyl-5- (3-methylphenyl) -pyrazol-3-ol, 1-Isobutyl-4-methyl-5-(3-methylphenyl)-pyrazol-3-ol, 1-tert-Butyl-4-methyl-5-(3-methylphenyl)-pyrazol-3-ol, 1-Phenyl-4-methyl-5-(3-methylphenyl)-pyrazol-3-ol, 1-(4-methylphenyl)-4-methyl-5-(3-methylphenyl)-pyrazol-3-ol, 1-(2-methylphenyl)-4-methyl-5-(3-methylphenyl)-pyrazol-3-ol, 1-(3-methylphenyl)-4-methyl-5-(3-methylphenyl)-pyrazol-3-ol, 1-(4-Methoxyphenyl)-4-methyl-5-(3-methylphenyl)-pyrazol-3-ol, 1-Methyl-4-methyl-5-(3-methylphenyl)-pyrazol-3-ol, 1-Ethyl-4-methyl-5-(3-methylphenyl)-pyrazol-3-ol, 1-Propyl-4-methyl-5-(3-methylphenyl)-pyrazol-3-ol, 1-Isopropyl-4-methyl-5-(3-methylphenyl)-pyrazol-3-ol, 1-Butyl-4-methyl-5-(3-methylphenyl)-pyrazol-3-ol, 1-Isobutyl-4-methyl-5- (3-methylphenyl) -pyrazol-3-ol, 1-tert-Butyl-4-methyl-5- butyl -pyrazol-3-ol, 1-Phenyl-4-methyl-5- (3-methylphenyl) -pyrazol-3-ol, 1-(4-methylphenyl)-4-methyl-5- (3-methylphenyl)-pyrazol-3-ol, 1-(2-methylphenyl)-4-methyl-5-(3-methylphenyl)-pyrazol-3-ol, 1-(3-methylphenyl)-4-methyl-5-(3-methylphenyl)-pyrazol-3-ol, 1-(4-Methoxyphenyl)-4-methyl-5-butyl-pyrazol-3-ol, 1-Methyl-4-methyl-5-(4-methylphenyl)-pyrazol-3-ol, 1-Ethyl-4-methyl-5-(4-methylphenyl)-pyrazol-3-ol, 1-Propyl-4-methyl-5-(4-methylphenyl)-pyrazol-3-ol, 1-Isopropyl-4-methyl-5-(4-methylphenyl)-pyrazol-3-ol, 1-Butyl-4-methyl-5-(4-methylphenyl) -pyrazol-3-ol, 1-Isobutyl-4-methyl-5-(4-methylphenyl)-pyrazol-3-ol, 1-tert-Butyl-4-methyl-5-(4-methylphenyl)-pyrazol-3-ol, 1-Phenyl-4-methyl-5-(4-methylphenyl)-pyrazol-3-ol, 1-(4-methylphenyl)-4-methyl-5-(4-methylphenyl)-pyrazol-3-ol, 1-(2-methylphenyl)-4-methyl-5-(4-methylphenyl)-pyrazol-3-ol, 1-(3-methylphenyl)-4-methyl-5-(4-methylphenyl)-pyrazol-3-ol, 1-(4-Methoxyphenyl)-4-methyl-5- (4-methylphenyl) -pyrazol-3-ol, 1-Methyl-4-ethyl-5-phenyl-pyrazol-3-ol, 1-Ethyl-4-ethyl5-phenyl-pyrazol-3-ol, 1-Propyl-4-ethyl-5-phenyl-pyrazol-3-ol, 1-Isopropyl-4-ethyl-5-phenyl-pyrazol-3-ol, 1-Butyl-4-ethyl-5-phenyl-pyrazol-3-ol, 1-Isobutyl-4-ethyl-5-phenyl-pyrazol-3-ol, 1-tert-Butyl-4-ethyl-5-phenyl-pyrazol-3-ol, 1-Phenyl-4-ethyl-5-phenyl-pyrazol-3-ol, 1-(4-methylphenyl)-4-ethyl-5-phenyl-pyrazol-3-ol, 1-(2-methylphenyl)-4-ethyl-5-phenyl-pyrazol-3-ol, 1-(3-methylphenyl)-4-ethyl-5-phenyl-pyrazol-3-ol, 1-(4-Methoxyphenyl)-4-ethyl-5-phenyl-pyrazol-3-ol,1-Methyl-4-ethyl-5-methyl-pyrazol-3-ol, 1-Ethyl-4-ethyl-5-methyl-pyrazol-3-ol, 1-Propyl-4-ethyl-5-methy-pyrazol-3-ol, 1-Isopropyl-4-ethyl-5-methyl - pyrazol-3-ol, 1-Butyl-4-ethyl-5-methyl-pyrazol-3-ol, 1-Isobutyl-4-ethyl-5-methyl-pyrazol-3-ol, 1-tert-Butyl-4-ethyl-5-methyl-pyrazol-3-ol, 1-Phenyl-4-ethyl-5-methyl-pyrazol-3-ol, 1-(4-methylphenyl)-4-ethyl-5-methyl-pyrazol-3-ol, 1-(2-methylphenyl)-4-ethyl-5-methyl-pyrazol-3-ol, 1-(3-methylphenyl)-4-ethyl-5-methyl-pyrazol-3-ol, 1-(4-Methoxyphenyl)-4-ethyl-5-methyl-pyrazol-3-ol,1-methyl-4-ethyl-5-ethyl-pyrazol-3-ol, 1-ethyl-4-ethyl-5-ethyl-pyrazol-3-ol, 1-propyl-4-ethyl-5- ethyl -pyrazol-3-ol, 1-isopropyl-4-ethyl-5-ethyl-pyrazol-3-ol, 1-butyl-4-ethyl-5-ethyl-pyrazol-3-ol, 1-Isobutyl-4-ethyl-5-ethyl-pyrazol-3-ol, 1-tert-butyl-4-ethyl-5-ethyl-pyrazol-3-ol, 1-Phenyl-4-ethyl-5-ethyl-pyrazol-3-ol, 1-(4-methylphenyl)-4-ethyl-5-ethyl-pyrazol-3-ol, 1-(2-methylphenyl)-4-ethyl-5-ethyl -pyrazol-3-ol, 1-(3-methylphenyl)-4-ethyl-5-ethyl-pyrazol-3-ol, 1-(4-Methoxyphenyl)-4-ethyl-5- ethyl-pyrazol-3-ol, 1-Methyl-4-ethyl-5-propyl-pyrazol-3-ol, 1-Ethyl-4-ethyl-5-propyl-pyrazol-3-ol, 1-Propyl-4-ethyl-5-propyl-pyrazol-3-ol, 1-Isopropyl-4-ethyl-5-methyl-pyrazol-3-ol, 1-Butyl-4-ethyl-5-propyl-pyrazol-3-ol, 1-Isobutyl-4-ethyl-5-propyl-pyrazol-3-ol, 1-tert-Butyl-4-ethyl-5-propyl-pyrazol-3-ol, 1-Phenyl-4-ethyl-5-propyl-pyrazol-3-ol, 1-(4-methylphenyl)-4-ethyl-5-propyl-pyrazol-3-ol, 1-(2-methylphenyl)-4-ethyl-5-propyl-pyrazol-3-ol, 1-(3-methylphenyl)-4-ethyl-5-propyl-pyrazol-3-ol, 1-(4-Methoxyphenyl)-4-ethyl-5- propyl -pyrazol-3-ol, 1-Methyl-4-ethyl-5-isopropyl-pyrazol-3-ol, 1-Ethyl-4-ethyl-5-isopropyl-pyrazol-3-ol, 1-Propyl-4-ethyl-5-isopropyl -pyrazol-3-ol, 1-Isopropyl-4-ethyl-5-isopropyl-pyrazol-3-ol, 1-Butyl-4-ethyl-5-isopropyl-pyrazol-3-ol, 1-Isobutyl-4-ethyl-5-isopropyl-pyrazol-3-ol, 1-tert-Butyl-4-ethyl-5-isopropyl-pyrazol-3-ol, 1-Phenyl-4-ethyl-5-isopropyl-pyrazol-3-ol, 1-(4-methylphenyl)-4-ethyl-5-isop-ropyl-pyrazol-3-ol, 1-(2-methylphenyl)-4-ethyl-5-isopropyl-pyrazol-3-ol, 1-(3-methylphenyl)-4-ethyl-5-isopropyl-pyrazol-3-ol, 1-(4-Methoxyphenyl)-4-ethyl-5-isopropyl-pyrazol-3-ol, 1-Methyl-4-ethyl-5-butyl-pyrazol-3-ol, 1-Ethyl-4-ethyl-5-butyl-pyrazol-3-ol, 1-Propyl-4-ethyl-5-butyl-pyrazol-3-ol, 1-Isopropyl-4-ethyl-5-butyl-pyrazol-3-ol, 1-Butyl-4-ethyl-5- butyl -pyrazol-3-ol, 1-Isobutyl-4-ethyl-5-butyl-pyrazol-3-ol, 1-tert-Butyl-4-ethyl-5-butyl-pyrazol-3-ol, 1-Phenyl-4-ethyl-5-butyl-pyrazol-3-ol, 1-(4-methylphenyl)-4-ethyl-5-butyl-pyrazol-3-ol, 1-(2-methylphenyl)-4-ethyl-5-butyl-pyrazol-3-ol, 1-(3-methylphenyl)-4-ethyl-5-butyl-pyrazol-3-ol, 1-(4-Methoxyphenyl)-4-ethyl-5-butyl-pyrazol-3-ol,
1-Methyl-4-ethyl-5-isobutyl-pyrazol-3-ol, 1-Ethyl-4-ethyl-5-isobutyl-pyrazol-3-ol, 1-Propyl-4-ethyl-5-isobutyl-pyrazol-3-ol, 1-Isopropyl-4-ethyl-5-isobuty-pyrazol-3-ol, 1-Butyl-4-ethyl-5-isobutyl-pyrazol-3-ol, 1-Isobutyl-4-ethyl-5-isobutyl-pyrazol-3-ol, 1-tert-Butyl-4-ethyl-5-isobutyl-pyrazol-3-ol, 1-Phenyl-4-ethyl-5-isobutyl-pyrazol-3-ol, 1-(4-methylphenyl)-4-ethyl-5-isobutyl-pyrazol-3-ol, 1-(2-methylphenyl)-4-ethyl-5-isobutyl-pyrazol-3-ol, 1-(3-methylphenyl)-4-ethyl-5-isobutyl-pyrazol-3-ol, 1-(4-Methoxyphenyl)-4-ethyl-5- isobutyl -pyrazol-3-ol, 1-Methyl-4-ethyl-5-tert-butyl-pyrazol-3-ol, 1-Ethyl-4-ethyl-5-tert-butyl-pyrazol-3-ol, 1-Propyl-4-ethyl-5-tert-butyl-pyrazol-3-ol, 1-Isopropyl-4-ethyl-5-tert-butyl-pyrazol-3-ol, 1-Butyl-4-ethyl-5-tert-butyl-pyrazol-3-ol, 1-Isobutyl-4-ethyl-5-tert-butyl-pyrazol-3-ol, 1-tert-Butyl-4-ethyl-5-tert-butyl-pyrazol-3-ol, 1-Phenyl-4-ethyl-5-tert-butyl-pyrazol-3-ol, 1-(4-methylphenyl)-4-ethyl-5-tert-butyl-pyrazol-3-ol, 1-(2-methylphenyl)-4-ethyl-5-tert-butyl-pyrazol-3-ol, 1-(3-methylphenyl)-4-ethyl-5-tert-butyl-pyrazol-3-ol, 1-(4-Methoxyphenyl)-4-ethyl-5-phenyl-pyrazol-3-ol, 1-Methyl-4-ethyl-5-phenyl-pyrazol-3-ol, 1-Ethyl-4-ethyl-5-phenyl-pyrazol-3-ol, 1-Propyl-4-ethyl-5-phenyl-pyrazol-3-ol, 1-Isopropyl-4-ethyl-5-phenyl-pyrazol-3-ol, 1-Butyl-4-ethyl-5-phenyl-pyrazol-3-ol, 1-Isobutyl-4-ethyl-5- phenyl -pyrazol-3-ol, 1-tert-Butyl-4-ethyl-5-phenyl-pyrazol-3-ol, 1-Phenyl-4-ethyl-5-phenyl-pyrazol-3-ol, 1-(4-methylphenyl)-4-ethyl-5-phenyl-pyrazol-3-ol, 1-(2-methylphenyl)-4-ethyl-5- phenyl -pyrazol-3-ol, 1-(3-methylphenyl)-4-ethyl-5-phenyl-pyrazol-3-ol, 1-(4-Methoxyphenyl)-4-ethyl-5- (2-methylphenyl)-pyrazol-3-ol, 1-Methyl-4-ethyl-5-(2-methylphenyl)-pyrazol-3-ol, 1-Ethyl-4-ethyl-5-(2-methylphenyl)-pyrazol-3-ol, 1-Propyl-4-ethyl-5-(2-methylphenyl)-pyrazol-3-ol, 1-Isopropyl-4-ethyl-5-(2-methylphenyl)-pyrazol-3-ol, 1-Butyl-4-ethyl-5-(2-methylphenyl)-pyrazol-3-ol, 1-Isobutyl-4-ethyl-5-(2-methylphenyl)-pyrazol-3-ol, 1-tert-Butyl-4-ethyl-5- (2-methylphenyl)-pyrazol-3-ol, 1-Phenyl-4-ethyl-5-(2-methylphenyl)-pyrazol-3-ol, 1-(4-methylphenyl)-4-ethyl-5-(2-methylphenyl)-pyrazol-3-ol, 1-(2-methylphenyl)-4-ethyl-5-(2-methylphenyl)-pyrazol-3-ol, 1-(3-methylphenyl)-4-ethyl-5-(2-methylphenyl)-pyrazol-3-ol, 1-(4-Methoxyphenyl)-4-ethyl-5-(3-methylphenyl)-pyrazol-3-ol, 1-Methyl-4-ethyl-5-(3-methylphenyl)-pyrazol-3-ol, 1-Ethyl-4-ethyl-5-(3-methylphenyl)-pyrazol-3-ol, 1-Propyl-4-ethyl-5- (3-methylphenyl)-pyrazol-3-ol, 1-Isopropyl-4-ethyl-5-(3-methylphenyl)-pyrazol-3-ol, 1-Butyl-4-ethyl-5-(3-methylphenyl)-pyrazol-3-ol, 1-Isobutyl-4-ethyl-5-(3-methylphenyl)-pyrazol-3-ol, 1-tert-Butyl-4-ethyl-5-(3-methylphenyl)-pyrazol-3-ol, 1-Phenyl-4-ethyl-5-(3-methylphenyl)-pyrazol-3-ol, 1-(4-methylphenyl)-4-ethyl-5-(3-methylphenyl)-pyrazol-3-ol, 1-(2-methylphenyl)-4-ethyl-5-(3-methylphenyl)-pyrazol-3-ol, 1-(3-methylphenyl)-4-ethyl-5-(3-methylphenyl)-pyrazol-3-ol, 1-(4-Methoxyphenyl)-4-ethyl-5-(3-methylphenyl)-pyrazol-3-ol, 1-Methyl-4-ethyl-5-(3-methylphenyl)-pyrazol-3-ol, 1-Ethyl-4-ethyl-5- (3-methylphenyl) -pyrazol-3-ol, 1-Propyl-4-ethyl-5-(3-methylphenyl)-pyrazol-3-ol, 1-Isopropyl-4-ethyl-5-(3-methylphenyl)-pyrazol-3-ol, 1-Butyl-4-ethyl-5-(3-methylphenyl)-pyrazol-3-ol, 1-Isobutyl-4-ethyl-5-(3-methylphenyl)-pyrazol-3-ol, 1-tert-Butyl-4-ethyl-5-butyl-pyrazol-3-ol, 1-Phenyl-4-ethyl-5-(3-methylphenyl)-pyrazol-3-ol, 1-(4-methylphenyl)-4-ethyl-5-(3-methylphenyl)-pyrazol-3-ol, 1-(2-methylphenyl)-4-ethyl-5-(3-methylphenyl)-pyrazol-3-ol, 1-(3-methylphenyl)-4-ethyl-5-(3-methylphenyl)-pyrazol-3-ol, 1-(4-Methoxyphenyl)-4-ethyl-5-butyl-pyrazol-3-ol, 1-Methyl-4-ethyl-5-(4-methylphenyl)-pyrazol-3-ol, 1-Ethyl-4-ethyl-5-(4-methylphenyl)-pyrazol-3-ol, 1-Propyl-4-ethyl-5- (4-methylphenyl)-pyrazol-3-ol, 1-Isopropyl-4-ethyl-5-(4-methylphenyl)-pyrazol-3-ol, 1-Butyl-4-ethyl-5-(4-methylphenyl)-pyrazol-3-ol, 1-Isobutyl-4-ethyl-5- (4-methylphenyl)-pyrazol-3-ol, 1-tert-Butyl-4-ethyl-5-(4-methylphenyl)-pyrazol-3-ol, 1-Phenyl-4-ethyl-5-(4-methylphenyl)-pyrazol-3-ol, 1-(4-methylphenyl)-4-ethyl-5-(4-methylphenyl)-pyrazol-3-ol, 1-(2-methylphenyl)-4-ethyl-5-(4-methylphenyl)-pyrazol-3-ol, 1-(3-methylphenyl)-4-ethyl-5-(4-methylphenyl)-pyrazol-3-ol, 1-(4-Methoxyphenyl)-4-ethyl-5-(4-methylphenyl)-pyrazol-3-ol, 1-Methyl-4-propyl-5-phenyl-pyrazol-3-ol, 1-Ethyl-4-propyl5-phenyl-pyrazol-3-ol, 1-Propyl-4-propyl-5-phenyl-pyrazol-3-ol, 1-Isopropyl-4-propyl-5-phenyl-pyrazol-3-ol, 1-Butyl-4-propyl-5-phenyl-pyrazol-3-ol, 1-Isobutyl-4-propyl-5-phenyl-pyrazol-3-ol, 1-tert-Butyl-4-propyl-5-phenyl-pyrazol-3-ol, 1-Phenyl-4-propyl-5-phenyl-pyrazol-3-ol, 1-(4-methylphenyl)-4-propyl-5-phenyl-pyrazol-3-ol, 1-(2-methylphenyl)-4-propyl-5-phenyl-pyrazol-3-ol, 1-(3-methylphenyl)-4-propyl-5-phenyl-pyrazol-3-ol, 1-(4-Methoxyphenyl)-4-propyl-5-phenyl-pyrazol-3-ol, 1-Methyl-4-propyl-5-methyl-pyrazol-3-ol, 1-Ethyl-4-propyl-5-methyl-pyrazol-3-ol, 1-Propyl-4-propyl-5-methyl-pyrazol-3-ol, 1-Isopropyl-4-propyl-5-methyl-pyrazol-3-ol, 1-Butyl-4-propyl-5-methyl-pyrazol-3-ol, 1-Isobutyl-4-propyl-5-methyl-pyrazol-3-ol, 1-tert-Butyl-4-propyl-5-methyl-pyrazol-3-ol, 1-Phenyl-4-propyl-5-methyl-pyrazol-3-ol, 1-(4-methylphenyl)-4-propyl-5-methyl-pyrazol-3-ol, 1-(2-methylphenyl)-4-propyl-5-methyl-pyrazol-3-ol, 1-(3-methylphenyl)-4-propyl-5-methyl-pyrazol-3-ol, 1-(4-Methoxyphenyl)-4-propyl-5-methyl-pyrazol-3-ol, 1-methyl-4-propyl-5-ethyl-pyrazol-3-ol, 1-ethyl-4-propyl-5-ethyl-pyrazol-3-ol, 1-propyl-4-propyl-5-ethyl-pyrazol-3-ol, 1-isopropyl-4-propyl-5-ethyl-pyrazol-3-ol, 1-butyl-4-propyl-5-ethyl-pyrazol-3-ol, 1-Isobutyl-4-propyl-5-ethyl-pyrazol-3-ol, 1-tert-butyl-4-propyl-5-ethyl-pyrazol-3-ol, 1-Phenyl-4-propyl-5-ethyl-pyrazol-3-ol, 1-(4-methylphenyl)-4-propyl-5-ethyl-pyrazol-3-ol, 1-(2-methylphenyl)-4-propyl-5- ethyl -pyrazol-3-ol, 1-(3-methylphenyl)-4-propyl-5-ethyl-pyrazol-3-ol, 1-(4-Methoxyphenyl)-4-propyl-5-ethyl-pyrazol-3-ol, 1-Methyl-4-propyl-5-propyl-pyrazol-3-ol, 1-Ethyl-4-propyl-5-propyl-pyrazol-3-ol, 1-Propyl-4-propyl-5-propyl-pyrazol-3-ol, 1-Isopropyl-4-propyl-5-methyl-pyrazol-3-ol, 1-Butyl-4-propyl-5-propyl-pyrazol-3-ol, 1-Isobutyl-4-propyl-5-propyl-pyrazol-3-ol, 1-tert-Butyl-4-propyl-5-propyl-pyrazol-3-ol, 1-Phenyl-4-propyl-5-propyl-pyrazol-3-ol, 1-(4-methylphenyl)-4-propyl-5-propyl-pyrazol-3-ol, 1-(2-methylphenyl)-4-propyl-5-propyl-pyrazol-3-ol, 1-(3-methylphenyl)-4-propyl-5-propyl-pyrazol-3-ol, 1-(4-Methoxyphenyl)-4-propyl-5-propyl-pyrazol-3-ol, 1-Methyl-4-propyl-5-isopropyl-pyrazol-3-ol, 1-Ethyl-4-propyl-5-isopropyl-pyrazol-3-ol, 1-Propyl-4-propyl-5-isopropyl-pyrazol-3-ol, 1-Isopropyl-4-propyl-5-isopropyl-pyrazol-3-ol, 1-Butyl-4-propyl-5-isopropyl-pyrazol-3-ol, 1-Isobutyl-4-propyl-5-isopropyl-pyrazol-3-ol, 1-tert-Butyl-4-propyl-5-isopropyl-pyrazol-3-ol, 1-Phenyl-4-propyl-5-isopropyl-pyrazol-3-ol, 1-(4-methylphenyl)-4-propyl-5-isopropyl-pyrazol-3-ol, 1-(2-methylphenyl)-4-propyl-5-isopropyl-pyrazol-3-ol, 1-(3-methylphenyl)-4-propyl-5-isopropyl-pyrazol-3-ol, 1-(4-Methoxyphenyl)-4-propyl-5-isopropyl-pyrazol-3-ol, 1-Methyl-4-propyl-5-butyl-pyrazol-3-ol, 1-Ethyl-4-propyl-5-butyl-pyrazol-3-ol, 1-Propyl-4-propyl-5-butyl-pyrazol-3-ol, 1-Isopropyl-4-propyl-5-butyl-pyrazol-3-ol, 1-Butyl-4-propyl-5-butyl-pyrazol-3-ol, 1-Isobutyl-4-propyl-5-butyl-pyrazol-3-ol, 1-tert-Butyl-4-propyl-5-butyl-pyrazol-3-ol, 1-Phenyl-4-propyl-5-butyl-pyrazol-3-ol, 1-(4-methylphenyl)-4-propyl-5-butyl-pyrazol-3-ol, 1-(2-methylphenyl)-4-propyl-5- butyl-pyrazol-3-ol, 1-(3-methylphenyl)-4-propyl-5-butyl-pyrazol-3-ol, 1-(4-Methoxyphenyl)-4-propyl-5-butyl-pyrazol-3-ol, 1-Methyl-4-propyl-5- isobutyl-pyrazol-3-ol, 1-Ethyl-4-propyl-5-isobutyl-pyrazol-3-ol, 1-Propyl-4-propyl-5-isobutyl-pyrazol-3-ol, 1-Isopropyl-4-propyl-5- isobutyl-pyrazol-3-ol, 1-Butyl-4-propyl-5-isobutyl-pyrazol-3-ol, 1-Isobutyl-4-propyl-5- isobutyl-pyrazol-3-ol, 1-tert-Butyl-4-propyl-5-isobutyl-pyrazol-3-ol, 1-Phenyl-4-propyl-5-isobutyl-pyrazol-3-ol, 1-(4-methylphenyl)-4-propyl-5-isobutyl-pyrazol-3-ol, 1-(2-methylphenyl)-4-propyl-5-isobutyl-pyrazol-3-ol, 1-(3-methylphenyl)-4-propyl-5-isobutyl -pyrazol-3-ol, 1-(4-Methoxyphenyl)-4-propyl-5- isobutyl-pyrazol-3-ol, 1-Methyl-4-propyl-5-tert-butyl-pyrazol-3-ol, 1-Ethyl-4-propyl-5-tert-butyl-pyrazol-3-ol, 1-Propyl-4-propyl-5-tert-butyl-pyrazol-3-ol, 1-Isopropyl-4-propyl-5-tert-butyl-pyrazol-3-ol, 1-Butyl-4-propyl-5-tert-butyl-pyrazol-3-ol, 1-Isobutyl-4-propyl-5- tert-butyl-pyrazol-3-ol, 1-tert-Butyl-4-propyl-5-tert-butyl-pyrazol-3-ol, 1-Phenyl-4-propyl-5-tert-butyl-pyrazol-3-ol, 1-(4-methylphenyl)-4-propyl-5- tert-butyl-pyrazol-3-ol, 1-(2-methylphenyl)-4-propyl-5- tert-butyl-pyrazol-3-ol, 1-(3-methylphenyl)-4-propyl-5-tert-butyl-pyrazol-3-ol, 1-(4-Methoxyphenyl)-4-propyl-5-phenyl-pyrazol-3-ol, 1-Methyl-4-propyl-5-phenyl-pyrazol-3-ol, 1-Ethyl-4-propyl-5-phenyl-pyrazol-3-ol, 1-Propyl-4-propyl-5-phenyl-pyrazol-3-ol, 1-Isopropyl-4-propyl-5-phenyl-pyrazol-3-ol, 1-Butyl-4-propyl-5-phenyl-pyrazol-3-ol, 1-Isobutyl-4-propyl-5-phenyl-pyrazol-3-ol, 1-tert-Butyl-4-propyl-5-phenyl-pyrazol-3-ol, 1-Phenyl-4-propyl-5-phenyl-pyrazol-3-ol, 1-(4-methylphenyl)-4-propyl-5-phenyl-pyrazol-3-ol, 1-(2-methylphenyl)-4-propyl-5-phenyl-pyrazol-3-ol, 1-(3-methylphenyl)-4-propyl-5-phenyl-pyrazol-3-ol, 1-(4-Methoxyphenyl)-4-propyl-5-(2-methylphenyl)-pyrazol-3-ol, 1-Methyl-4-propyl-5-(2-methylphenyl)-pyrazol-3-ol, 1-Ethyl-4-propyl-5-(2-methylphenyl)-pyrazol-3-ol, 1-Propyl-4-propyl-5-(2-methylphenyl)-pyrazol-3-ol, 1-Isopropyl-4-propyl-5-(2-methylphenyl)-pyrazol-3-ol, 1-Butyl-4-propyl-5-(2-methylphenyl)-pyrazol-3-ol, 1-Isobutyl-4-propyl-5-(2-methylphenyl)-pyrazol-3-ol, 1-tert-Butyl-4-propyl-5- (2-methylphenyl)-pyrazol-3-ol, 1-Phenyl-4-propyl-5-(2-methylphenyl)-pyrazol-3-ol, 1-(4-methylphenyl)-4-propyl-5-(2-methylphenyl)-pyrazol-3-ol, 1-(2-methylphenyl)-4-propyl-5- (2-methylphenyl)-pyrazol-3-ol, 1-(3-methylphenyl)-4-propyl-5-(2-methylphenyl)-pyrazol-3-ol, 1-(4-Methoxyphenyl)-4-propyl-5-(3-methylphenyl)-pyrazol-3-ol, 1-Methyl-4-propyl-5- (3-methylphenyl)-pyrazol-3-ol, 1-Ethyl-4-propyl-5-(3-methylphenyl)-pyrazol-3-ol, 1-Propyl-4-propyl-5-(3-methylphenyl)-pyrazol-3-ol, 1-Isopropyl-4-propyl-5-(3-methylphenyl) -pyrazol-3-ol, 1-Butyl-4-propyl-5-(3-methylphenyl)-pyrazol-3-ol, 1-Isobutyl-4-propyl-5- (3-methylphenyl) -pyrazol-3-ol, 1-tert-Butyl-4-propyl-5-(3-methylphenyl)-pyrazol-3-ol, 1-Phenyl-4-propyl-5- (3-methylphenyl)-pyrazol-3-ol, 1-(4-methylphenyl)-4-propyl-5-(3-methylphenyl)-pyrazol-3-ol, 1-(2-methylphenyl)-4-propyl-5-(3-methylphenyl)-pyrazol-3-ol, 1-(3-methylphenyl)-4-propyl-5-(3-methylphenyl)-pyrazol-3-ol, 1-(4-Methoxyphenyl)-4-propyl-5- (3-methylphenyl) -pyrazol-3-ol, 1-Methyl-4-propyl-5-(3-methylphenyl)-pyrazol-3-ol, 1-Ethyl-4-propyl-5-(3-methylphenyl)-pyrazol-3-ol, 1-Propyl-4-propyl-5- (3-methylphenyl)-pyrazol-3-ol, 1-Isopropyl-4-propyl-5-(3-methylphenyl)-pyrazol-3-ol, 1-Butyl-4-propyl-5-(3-methylphenyl)-pyrazol-3-ol, 1-Isobutyl-4-propyl-5-(3-methylphenyl)-pyrazol-3-ol, 1-tert-Butyl-4-propyl-5-butyl-pyrazol-3-ol, 1-Phenyl-4-propyl-5-(3-methylphenyl)-pyrazol-3-ol, 1-(4-methylphenyl)-4-propyl-5-(3-methylphenyl)-pyrazol-3-ol, 1-(2-methylphenyl)-4-propyl-5-(3-methylphenyl)-pyrazol-3-ol, 1-(3-methylphenyl)-4-propyl-5-(3-methylphenyl)-pyrazol-3-ol, 1-(4-Methoxyphenyl)-4-propyl-5-butyl-pyrazol-3-ol, 1-Methyl-4-propyl-5-(4-methylphenyl)-pyrazol-3-ol, 1-Ethyl-4-propyl-5- (4-methylphenyl)-pyrazol-3-ol, 1-Propyl-4-propyl-5-(4-methylphenyl)-pyrazol-3-ol, 1-Isopropyl-4-propyl-5-(4-methylphenyl)-pyrazol-3-ol, 1-Butyl-4-propyl-5-(4-methylphenyl)-pyrazol-3-ol, 1-Isobutyl-4-propyl-5-(4-methylphenyl)-pyrazol-3-ol, 1-tert-Butyl-4-propyl-5- (4-methylphenyl)-pyrazol-3-ol, 1-Phenyl-4-propyl-5-(4-methylphenyl)-pyrazol-3-ol, 1-(4-methylphenyl)-4-propyl-5-(4-methylphenyl)-pyrazol-3-ol, 1-(2-methylphenyl)-4-propyl-5- (4-methylphenyl)-pyrazol-3-ol, 1-(3-methylphenyl)-4-propyl-5-(4-methylphenyl)-pyrazol-3-ol, 1-(4-Methoxyphenyl)-4-propyl-5-(4-methylphenyl)-pyrazol-3-ol, 1-Methyl-4-butyl-5-phenyl-pyrazol-3-ol, 1-Ethyl-4-butyl5-phenyl-pyrazol-3-ol, 1-Propyl-4-butyl-5-phenyl-pyrazol-3-ol, 1-Isopropyl-4-butyl-5-phenyl-pyrazol-3-ol, 1-Butyl-4-butyl-5-phenyl-pyrazol-3-ol, 1-Isobutyl-4-butyl-5-phenyl-pyrazol-3-ol, 1-tert-Butyl-4-butyl-5-phenyl-pyrazol-3-ol, 1-Phenyl-4-butyl-5-phenyl-pyrazol-3-ol, 1-(4-methylphenyl)-4-butyl-5-phenyl-pyrazol-3-ol, 1-(2-methylphenyl)-4-butyl-5-phenyl-pyrazol-3-ol, 1-(3-methylphenyl)-4-butyl-5-phenyl-pyrazol-3-ol, 1-(4-Methoxyphenyl)-4-butyl-5-phenyl-pyrazol-3-ol, 1-Methyl-4-butyl-5-methyl-pyrazol-3-ol, 1-Ethyl-4-butyl-5-methyl-pyrazol-3-ol, 1-Propyl-4-butyl-5-methyl-pyrazol-3-ol, 1-Isopropyl-4-butyl-5-methyl-pyrazol-3-ol, 1-Butyl-4-butyl-5-methyl-pyrazol-3-ol, 1-Isobutyl-4-butyl-5-methyl-pyrazol-3-ol, 1-tert-Butyl-4-butyl-5-methyl-pyrazol-3-ol, 1-Phenyl-4-butyl-5-methyl-pyrazol-3-ol, 1-(4-methylphenyl)-4-butyl-5-methyl-pyrazol-3-ol, 1-(2-methylphenyl)-4-butyl-5-methyl-pyrazol-3-ol, 1-(3-methylphenyl)-4-butyl-5-methyl-pyrazol-3-ol, 1-(4-Methoxyphenyl)-4-butyl-5- methyl-pyrazol-3-ol, 1-methyl-4-butyl-5-ethyl-pyrazol-3-ol, 1-ethyl-4-butyl-5-ethyl-pyrazol-3-ol, 1-propyl-4-butyl-5-ethyl-pyrazol-3-ol, 1-isopropyl-4-butyl-5-ethyl-pyrazol-3-ol, 1-butyl-4-butyl-5- ethyl-pyrazol-3-ol, 1-Isobutyl-4-butyl-5-ethyl-pyrazol-3-ol, 1-tert-butyl-4-butyl-5-ethyl-pyrazol-3-ol, 1-Phenyl-4-butyl-5-ethyl-pyrazol-3-ol, 1-(4-methylphenyl)-4-butyl-5-ethyl-pyrazol-3-ol, 1-(2-methylphenyl)-4-butyl-5-ethyl-pyrazol-3-ol, 1-(3-methylphenyl)-4-butyl-5-ethyl-pyrazol-3-ol, 1-(4-Methoxyphenyl)-4-butyl-5-ethyl-pyrazol-3-ol, 1-Methyl-4-butyl-5-propyl-pyrazol-3-ol, 1-Ethyl-4-butyl-5-propyl-pyrazol-3-ol, 1-Propyl-4-butyl-5-propyl-pyrazol-3-ol, 1-Isopropyl-4-butyl-5-methyl-pyrazol-3-ol, 1-Butyl-4-butyl-5-propyl-pyrazol-3-ol, 1-Isobutyl-4-butyl-5-propyl-pyrazol-3-ol, 1-tert-Butyl-4-butyl-5-propyl-pyrazol-3-ol, 1-Phenyl-4-butyl-5-propyl-pyrazol-3-ol, 1-(4-methylphenyl)-4-butyl-5-propyl-pyrazol-3-ol, 1-(2-methylphenyl)-4-butyl-5-propyl-pyrazol-3-ol, 1-(3-methylphenyl)-4-butyl-5-propyl-pyrazol-3-ol, 1-(4-Methoxyphenyl)-4-butyl-5- propyl-pyrazol-3-ol, 1-Methyl-4-butyl-5-isopropyl-pyrazol-3-ol, 1-Ethyl-4-butyl-5- isopropyl-pyrazol-3-ol, 1-Propyl-4-butyl-5-isopropyl -pyrazol-3-ol, 1-Isopropyl-4-butyl-5-isopropyl-pyrazol-3-ol, 1-Butyl-4-butyl-5-isopropyl-pyrazol-3-ol, 1-Isobutyl-4-butyl-5- isopropyl-pyrazol-3-ol, 1-tert-Butyl-4-butyl-5-isopropyl-pyrazol-3-ol, 1-Phenyl-4-butyl-5-isopropyl-pyrazol-3-ol, 1-(4-methylphenyl)-4-butyl-5-isopropyl-pyrazol-3-ol, 1-(2-methylphenyl)-4-butyl-5-isopropyl-pyrazol-3-ol, 1-(3-methylphenyl)-4-butyl-5-isopropyl-pyrazol-3-ol, 1-(4-Methoxyphenyl)-4-butyl-5-isopropyl-pyrazol-3-ol, 1-Methyl-4-butyl-5-butyl-pyrazol-3-ol, 1-Ethyl-4-butyl-5-butyl-pyrazol-3-ol ,1-Propyl-4-butyl-5-butyl-pyrazol-3-ol, 1-Isopropyl-4-butyl-5-butyl-pyrazol-3-ol, 1-Butyl-4-butyl-5-butyl-pyrazol-3-ol, 1-Isobutyl-4-butyl-5-butyl-pyrazol-3-ol, 1-tert-Butyl-4-butyl-5-butyl-pyrazol-3-ol, 1-Phenyl-4-butyl-5-butyl-pyrazol-3-ol, 1-(4-methylphenyl)-4-butyl-5-butyl-pyrazol-3-ol, 1-(2-methylphenyl)-4-butyl-5-butyl-pyrazol-3-ol, 1-(3-methylphenyl)-4-butyl-5-butyl-pyrazol-3-ol, 1-(4-Methoxyphenyl)-4-butyl-5-butyl-pyrazol-3-ol, 1-Methyl-4-butyl-5-isobutyl-pyrazol-3-ol, 1-Ethyl-4-butyl-5-isobutyl-pyrazol-3-ol, 1-Propyl-4-butyl-5-isobutyl-pyrazol-3-ol, 1-Isopropyl-4-butyl-5-isobutyl-pyrazol-3-ol, 1-Butyl-4-butyl-5-isobutyl-pyrazol-3-ol, 1-Isobutyl-4-butyl-5-isobutyl-pyrazol-3-ol, 1-tert-Butyl-4-butyl-5-isobutyl-pyrazol-3-ol, 1-Phenyl-4-butyl-5-isobutyl-pyrazol-3-ol, 1-(4-methylphenyl)-4-butyl-5-isobutyl-pyrazol-3-ol, 1-(2-methylphenyl)-4-butyl-5-isobutyl-pyrazol-3-ol, 1-(3-methylphenyl)-4-butyl-5-isobutyl-pyrazol-3-ol, 1-(4-Methoxyphenyl)-4-butyl-5-isobutyl-pyrazol-3-ol, 1-Methyl-4-butyl-5- tert-butyl-pyrazol-3-ol, , 1-Ethyl-4-butyl-5-tert-butyl-pyrazol-3-ol, 1-Propyl-4-butyl-5-tert-butyl-pyrazol-3-ol, 1-Isopropyl-4-butyl-5-tert-butyl-pyrazol-3-ol, 1-Butyl-4-butyl-5-tert-butyl-pyrazol-3-ol, 1-Isobutyl-4-butyl-5-tert-butyl-pyrazol-3-ol, 1-tert-Butyl-4-butyl-5-tert-butyl-pyrazol-3-ol, 1-Phenyl-4-butyl-5-tert-butyl-pyrazol-3-ol, 1-(4-methylphenyl)-4-butyl-5- tert-butyl-pyrazol-3-ol, 1-(2-methylphenyl)-4-butyl-5-tert-butyl-pyrazol-3-ol, 1-(3-methylphenyl)-4-butyl-5-tert-butyl-pyrazol-3-ol, 1-(4-Methoxyphenyl)-4-butyl-5-phenyl-pyrazol-3-ol, 1-Methyl-4-butyl-5-phenyl-pyrazol-3-ol, , 1-Ethyl-4-butyl-5-phenyl-pyrazol-3-ol, 1-Propyl-4-butyl-5-phenyl-pyrazol-3-ol, 1-Isopropyl-4-butyl-5-phenyl-pyrazol-3-ol, 1-Butyl-4-butyl-5-phenyl-pyrazol-3-ol, 1-Isobutyl-4-butyl-5-phenyl-pyrazol-3-ol, 1-tert-Butyl-4-butyl-5- phenyl -pyrazol-3-ol, 1-Phenyl-4-butyl-5-phenyl-pyrazol-3-ol, 1-(4-methylphenyl)-4-butyl-5-phenyl-pyrazol-3-ol, 1-(2-methylphenyl)-4-butyl-5- phenyl -pyrazol-3-ol, 1-(3-methylphenyl)-4-butyl-5-phenyl -pyrazol-3-ol, 1-(4-Methoxyphenyl)-4-butyl-5-(2-methylphenyl)-pyrazol-3-ol, 1-Methyl-4-butyl-5-(2-methylphenyl)-pyrazol-3-ol, , 1-Ethyl-4-butyl-5- (2-methylphenyl)-pyrazol-3-ol, 1-Propyl-4-butyl-5- (2-methylphenyl)-pyrazol-3-ol, 1-Isopropyl-4-butyl-5-(2-methylphenyl)-pyrazol-3-ol, 1-Butyl-4-butyl-5- (2-methylphenyl)-pyrazol-3-ol, 1-Isobutyl-4-butyl-5-(2-methylphenyl)-pyrazol-3-ol, 1-tert-Butyl-4-butyl-5- (2-methylphenyl)-pyrazol-3-ol, 1-Phenyl-4-butyl-5- (2-methylphenyl)-pyrazol-3-ol, 1-(4-methylphenyl)-4-butyl-5-(2-methylphenyl)-pyrazol-3-ol, 1-(2-methylphenyl)-4-butyl-5-(2-methylphenyl)-pyrazol-3-ol, 1-(3-methylphenyl)-4-butyl-5- (2-methylphenyl)-pyrazol-3-ol, 1-(4-Methoxyphenyl)-4-butyl-5-(3-methylphenyl)-pyrazol-3-ol, 1-Methyl-4-butyl-5-(3-methylphenyl)-pyrazol-3-ol, , 1-Ethyl-4-butyl-5- (3-methylphenyl)-pyrazol-3-ol, 1-Propyl-4-butyl-5-(3-methylphenyl)-pyrazol-3-ol, 1-Isopropyl-4-butyl-5-(3-methylphenyl)-pyrazol-3-ol, 1-Butyl-4-butyl-5-(3-methylphenyl)-pyrazol-3-ol, 1-Isobutyl-4-butyl-5- (3-methylphenyl)-pyrazol-3-ol, 1-tert-Butyl-4-butyl-5- (3-methylphenyl)-pyrazol-3-ol, 1-Phenyl-4-butyl-5- (3-methylphenyl)-pyrazol-3-ol, 1-(4-methylphenyl)-4-butyl-5- (3-methylphenyl)-pyrazol-3-ol, 1-(2-methylphenyl)-4-butyl-5-(3-methylphenyl)-pyrazol-3-ol, 1-(3-methylphenyl)-4-butyl-5-(3-methylphenyl)-pyrazol-3-ol, 1-(4-Methoxyphenyl)-4-butyl-5-(3-methylphenyl)-pyrazol-3-ol, 1-Methyl-4-butyl-5-(3-methylphenyl)-pyrazol-3-ol, , 1-Ethyl-4-butyl-5-(3-methylphenyl)-pyrazol-3-ol, 1-Propyl-4-butyl-5-(3-methylphenyl)-pyrazol-3-ol, 1-Isopropyl-4-butyl-5-(3-methylphenyl)-pyrazol-3-ol, 1-Butyl-4-butyl-5- (3-methylphenyl)-pyrazol-3-ol,
1-Isobutyl-4-butyl-5-(3-methylphenyl)-pyrazol-3-ol, 1-tert-Butyl-4-butyl-5-butyl-pyrazol-3-ol, 1-Phenyl-4-butyl-5-(3-methylphenyl)-pyrazol-3-ol, 1-(4-methylphenyl)-4-butyl-5-(3-methylphenyl)-pyrazol-3-ol, 1-(2-methylphenyl)-4-butyl-5-(3-methylphenyl)-pyrazol-3-ol, 1-(3-methylphenyl)-4-butyl-5-(3-methylphenyl)-pyrazol-3-ol, 1-(4-Methoxyphenyl)-4-butyl-5-butyl -pyrazol-3-ol, 1-Methyl-4-butyl-5-(4-methylphenyl)-pyrazol-3-ol, 1-Ethyl-4-butyl-5-(4-methylphenyl)-pyrazol-3-ol, 1-Propyl-4-butyl-5-(4-methylphenyl)-pyrazol-3-ol, 1-Isopropyl-4-butyl-5-(4-methylphenyl)-pyrazol-3-ol, 1-Butyl-4-butyl-5- (4-methylphenyl)-pyrazol-3-ol, 1-Isobutyl- 4-butyl-5-(4-methylphenyl)-pyrazol-3-ol, 1-tert-Butyl-4-butyl-5-(4-methylphenyl)-pyrazol-3-ol, 1-Phenyl-4-butyl-5-(4-methylphenyl)-pyrazol-3-ol, 1-(4-methylphenyl)-4-butyl-5- (4-methylphenyl)-pyrazol-3-ol, 1-(2-methylphenyl)-4-butyl-5- (4-methylphenyl) -pyrazol-3-ol, 1-(3-methylphenyl)-4-butyl-5-(4-methylphenyl)-pyrazol-3-ol, 1-(4-Methoxyphenyl)-4-butyl-5-(4-methylphenyl)-pyrazol-3-ol, 1-Methyl-4-isobutyl-5-phenyl-pyrazol-3-ol, , 1-Ethyl-4-isobutyl5-phenyl-pyrazol-3-ol, 1-Propyl-4-isobutyl-5-phenyl-pyrazol-3-ol, 1-Isopropyl-4-isobutyl-5-phenyl-pyrazol-3-ol, 1-Butyl-4-isobutyl-5-phenyl-pyrazol-3-ol, 1-Isobutyl-4-isobutyl-5-phenyl-pyrazol-3-ol, 1-tert-Butyl-4-isobutyl-5-phenyl-pyrazol-3-ol, 1-Phenyl-4-isobutyl-5-phenyl-pyrazol-3-ol, 1-(4-methylphenyl)-4-isobutyl-5-phenyl-pyrazol-3-ol, 1-(2-methylphenyl)-4-isobutyl-5-phenyl-pyrazol-3-ol, 1-(3-methylphenyl)-4-isobutyl-5-phenyl-pyrazol-3-ol, 1-(4-Methoxyphenyl)-4-isobutyl-5-phenyl-pyrazol-3-ol, 1-Methyl-4-isobutyl-5-methyl-pyrazol-3-ol, , 1-Ethyl-4-isobutyl-5-methyl-pyrazol-3-ol, 1-Propyl-4-isobutyl-5-methyl-pyrazol-3-ol, 1-Isopropyl-4-isobutyl-5-methyl-pyrazol-3-ol, 1-Butyl-4-isobutyl-5-methyl-pyrazol-3-ol, 1-Isobutyl-4-isobutyl-5-methyl-pyrazol-3-ol, 1-tert-Butyl-4-isobutyl-5-methyl-pyrazol-3-ol, 1-Phenyl-4-isobutyl-5-methyl-pyrazol-3-ol, 1-(4-methylphenyl)-4-isobutyl-5-methyl-pyrazol-3-ol, 1-(2-methylphenyl)-4-isobutyl-5-methyl-pyrazol-3-ol, 1-(3-methylphenyl)-4-isobutyl-5-methyl-pyrazol-3-ol, 1-(4-Methoxyphenyl)-4-isobutyl-5-methyl-pyrazol-3-ol, 1-methyl-4-isobutyl-5-ethyl-pyrazol-3-ol, , 1-ethyl-4-isobutyl-5-ethyl-pyrazol-3-ol, 1-propyl-4-isobutyl-5-ethyl-pyrazol-3-ol, 1-isopropyl-4-isobutyl-5-ethyl-pyrazol-3-ol, 1-butyl-4-isobutyl-5-ethyl-pyrazol-3-ol, 1-Isobutyl-4-isobutyl-5-ethyl-pyrazol-3-ol, 1-tert-butyl-4-isobutyl-5-ethyl-pyrazol-3-ol, 1-Phenyl-4-isobutyl-5-ethyl-pyrazol-3-ol, 1-(4-methylphenyl)-4-isobutyl-5- ethyl -pyrazol-3-ol, 1-(2-methylphenyl)-4-isobutyl-5-ethyl-pyrazol-3-ol, 1-(3-methylphenyl)-4-isobutyl-5-ethyl-pyrazol-3-ol, 1-(4-Methoxyphenyl)-4-isobutyl-5-ethyl-pyrazol-3-ol, 1-Methyl-4-isobutyl-5-propyl-pyrazol-3-ol, , 1-Ethyl-4-isobutyl-5-propyl-pyrazol-3-ol, 1-Propyl-4-isobutyl-5-propyl-pyrazol-3-ol,
1-Isopropyl-4-isobutyl-5-methyl-pyrazol-3-ol, 1-Butyl-4-isobutyl-5-propyl-pyrazol-3-ol, 1-Isobutyl- 4-isobutyl-5-propyl-pyrazol-3-ol, 1-tert-Butyl-4-isobutyl-5-propyl-pyrazol-3-ol, 1-Phenyl-4-isobutyl-5-propyl-pyrazol-3-ol, 1-(4-methylphenyl)-4-isobutyl-5- propyl -pyrazol-3-ol, 1-(2-methylphenyl)-4-isobutyl-5-propyl-pyrazol-3-ol, 1-(3-methylphenyl)-4-isobutyl-5-propyl-pyrazol-3-ol, 1-(4-Methoxyphenyl)-4-isobutyl-5-propyl-pyrazol-3-ol, 1-Methyl-4-isobutyl-5-isopropyl-pyrazol-3-ol, , 1-Ethyl-4-isobutyl-5-isopropyl-pyrazol-3-ol, 1-Propyl-4-isobutyl-5-isopropyl-pyrazol-3-ol, 1-Isopropyl-4-isobutyl-5-isopropyl-pyrazol-3-ol, 1-Butyl-4-isobutyl-5-isopropyl-pyrazol-3-ol, 1-Isobutyl-4-isobutyl-5-isopropyl-pyrazol-3-ol,
1-tert-Butyl-4-isobutyl-5- isopropyl -pyrazol-3-ol, 1-Phenyl-4-isobutyl-5-isopropyl-pyrazol-3-ol, 1-(4-methylphenyl)-4-isobutyl-5-isopropyl-pyrazol-3-ol, 1-(2-methylphenyl)-4-isobutyl-5-isopropyl-pyrazol-3-ol, 1-(3-methylphenyl)-4-isobutyl-5-isopropyl-pyrazol-3-ol, 1-(4-Methoxyphenyl)-4-isobutyl-5-isopropyl-pyrazol-3-ol, 1-Methyl-4-isobutyl-5-butyl-pyrazol-3-ol, , 1-Ethyl-4-isobutyl-5-butyl-pyrazol-3-ol, 1-Propyl-4-isobutyl-5-butyl-pyrazol-3-ol, 1-Isopropyl-4-isobutyl-5-butyl-pyrazol-3-ol, 1-Butyl-4-isobutyl-5-butyl-pyrazol-3-ol, 1-Isobutyl-4-isobutyl-5-butyl-pyrazol-3-ol, 1-tert-Butyl-4-isobutyl-5-butyl-pyrazol-3-ol, 1-Phenyl-4-isobutyl-5-butyl-pyrazol-3-ol, 1-(4-methylphenyl)-4-isobutyl-5-butyl-pyrazol-3-ol, 1-(2-methylphenyl)-4-isobutyl-5-butyl-pyrazol-3-ol, 1-(3-methylphenyl)-4-isobutyl-5-butyl -pyrazol-3-ol, 1-(4-Methoxyphenyl)-4-isobutyl-5-butyl-pyrazol-3-ol, 1-Methyl-4-isobutyl-5-isobutyl-pyrazol-3-ol, , 1-Ethyl-4-isobutyl-5-isobutyl-pyrazol-3-ol, 1-Propyl-4-isobutyl-5-isobutyl-pyrazol-3-ol, 1-Isopropyl-4-isobutyl-5-isobutyl-pyrazol-3-ol, 1-Butyl-4-isobutyl-5-isobutyl-pyrazol-3-ol, 1-Isobutyl-4-isobutyl-5-isobutyl-pyrazol-3-ol, 1-tert-Butyl-4-isobutyl-5-isobutyl-pyrazol-3-ol, 1-Phenyl-4-isobutyl-5-isobutyl-pyrazol-3-ol, 1-(4-methylphenyl)-4-isobutyl-5-isobutyl-pyrazol-3-ol, 1-(2-methylphenyl)-4-isobutyl-5-isobutyl-pyrazol-3-ol, 1-(3-methylphenyl)-4-isobutyl-5- isobutyl-pyrazol-3-ol, 1-(4-Methoxyphenyl)-4-isobutyl-5-isobutyl-pyrazol-3-ol, 1-Methyl-4-isobutyl-5-tert-butyl-pyrazol-3-ol, , 1-Ethyl-4-isobutyl-5-tert-butyl-pyrazol-3-ol, 1-Propyl-4-isobutyl-5-tert-butyl-pyrazol-3-ol, 1-Isopropyl-4-isobutyl-5-tert-butyl -pyrazol-3-ol, 1-Butyl-4-isobutyl-5-tert-butyl-pyrazol-3-ol, 1-Isobutyl-4-isobutyl-5-tert-butyl-pyrazol-3-ol, 1-tert-Butyl-4-isobutyl-5-tert-butyl-pyrazol-3-ol, 1-Phenyl-4-isobutyl-5-tert-butyl -pyrazol-3-ol, 1-(4-methylphenyl)-4-isobutyl-5-tert-butyl-pyrazol-3-ol, 1-(2-methylphenyl)-4-isobutyl-5- tert-butyl-pyrazol-3-ol, 1-(3-methylphenyl)-4-isobutyl-5-tert-butyl-pyrazol-3-ol, 1-(4-Methoxyphenyl)-4-isobutyl-5-phenyl-pyrazol-3-ol, 1-Methyl-4-isobutyl-5- phenyl -pyrazol-3-ol, , 1-Ethyl-4-isobutyl-5-phenyl-pyrazol-3-ol, 1-Propyl-4-isobutyl-5-phenyl-pyrazol-3-ol, 1-Isopropyl-4-isobutyl-5-phenyl-pyrazol-3-ol, 1-Butyl-4-isobutyl-5-phenyl-pyrazol-3-ol, 1-Isobutyl-4-isobutyl-5-phenyl-pyrazol-3-ol, 1-tert-Butyl-4-isobutyl-5-phenyl-pyrazol-3-ol, 1-Phenyl-4-isobutyl-5-phenyl-pyrazol-3-ol, 1-(4-methylphenyl)-4-isobutyl-5-phenyl-pyrazol-3-ol, 1-(2-methylphenyl)-4-isobutyl-5-phenyl-pyrazol-3-ol, 1-(3-methylphenyl)-4-isobutyl-5-phenyl-pyrazol-3-ol, 1-(4-Methoxyphenyl)-4-isobutyl-5-(2-methylphenyl)-pyrazol-3-ol, 1-Methyl-4-isobutyl-5-(2-methylphenyl)-pyrazol-3-ol, , 1-Ethyl-4-isobutyl-5-(2-methylphenyl)-pyrazol-3-ol, 1-Propyl-4-isobutyl-5-(2-methylphenyl)-pyrazol-3-ol, 1-Isopropyl-4-isobutyl-5-(2-methylphenyl)-pyrazol-3-ol, 1-Butyl-4-isobutyl-5-(2-methylphenyl)-pyrazol-3-ol, 1-Isobutyl- 4-isobutyl-5-(2-methylphenyl)-pyrazol-3-ol, 1-tert-Butyl-4-isobutyl-5- (2-methylphenyl)-pyrazol-3-ol, 1-Phenyl-4-isobutyl-5-(2-methylphenyl)-pyrazol-3-ol, 1-(4-methylphenyl)-4-isobutyl-5-(2-methylphenyl)-pyrazol-3-ol, 1-(2-methylphenyl)-4-isobutyl-5- (2-methylphenyl)-pyrazol-3-ol, 1-(3-methylphenyl)-4-isobutyl-5-(2-methylphenyl)-pyrazol-3-ol, 1-(4-Methoxyphenyl)-4-isobutyl-5-(3-methylphenyl)-pyrazol-3-ol, 1-Methyl-4-isobutyl-5- (3-methylphenyl)-pyrazol-3-ol, , 1-Ethyl-4-isobutyl-5-(3-methylphenyl)-pyrazol-3-ol, 1-Propyl-4-isobutyl-5-(3-methylphenyl)-pyrazol-3-ol, 1-Isopropyl-4-isobutyl-5-(3-methylphenyl)-pyrazol-3-ol, 1-Butyl-4-isobutyl-5-(3-methylphenyl)-pyrazol-3-ol, 1-Isobutyl-4-isobutyl-5-(3-methylphenyl)-pyrazol-3-ol,1-tert-Butyl-4-isobutyl-5-(3-methylphenyl)-pyrazol-3-ol, 1-Phenyl-4-isobutyl-5-(3-methylphenyl)-pyrazol-3-ol, 1-(4-methylphenyl)-4-isobutyl-5- (3-methylphenyl)-pyrazol-3-ol, 1-(2-methylphenyl)-4-isobutyl-5-(3-methylphenyl)-pyrazol-3-ol, 1-(3-methylphenyl)-4-isobutyl-5-(3-methylphenyl)-pyrazol-3-ol, 1-(4-Methoxyphenyl)-4-isobutyl-5-(3-methylphenyl)-pyrazol-3-ol, 1-Methyl-4-isobutyl-5- (3-methylphenyl) -pyrazol-3-ol, 1-Ethyl-4-isobutyl-5-(3-methylphenyl)-pyrazol-3-ol, 1-Propyl-4-isobutyl-5- (3-methylphenyl)-pyrazol-3-ol, 1-Isopropyl-4-isobutyl-5-(3-methylphenyl)-pyrazol-3-ol, 1-Butyl-4-isobutyl-5-(3-methylphenyl)-pyrazol-3-ol, 1-Isobutyl-4-isobutyl-5-(3-methylphenyl) -pyrazol-3-ol, 1-tert-Butyl-4-isobutyl-5-butyl-pyrazol-3-ol, 1-Phenyl-4-isobutyl-5-(3-methylphenyl)-pyrazol-3-ol, 1-(4-methylphenyl)-4-isobutyl-5-(3-methylphenyl)-pyrazol-3-ol, 1-(2-methylphenyl)-4-isobutyl-5-(3-methylphenyl)-pyrazol-3-ol, 1-(3-methylphenyl)-4-isobutyl-5- (3-methylphenyl)-pyrazol-3-ol, 1-(4-Methoxyphenyl)-4-isobutyl-5-butyl-pyrazol-3-ol, 1-Methyl-4-isobutyl-5-(4-methylphenyl)-pyrazol-3-ol, , 1-Ethyl-4-isobutyl-5-(4-methylphenyl) -pyrazol-3-ol, 1-Propyl-4-isobutyl-5-(4-methylphenyl)-pyrazol-3-ol, 1-Isopropyl-4-isobutyl-5-(4-methylphenyl)-pyrazol-3-ol, 1-Butyl-4-isobutyl-5-(4-methylphenyl)-pyrazol-3-ol, 1-Isobutyl-4-isobutyl-5-(4-methylphenyl)-pyrazol-3-ol, 1-tert-Butyl-4-isobutyl-5-(4-methylphenyl)-pyrazol-3-ol, 1-Phenyl-4-isobutyl-5-(4-methylphenyl)-pyrazol-3-ol, 1-(4-methylphenyl)-4-isobutyl-5-(4-methylphenyl)-pyrazol-3-ol, 1-(2-methylphenyl)-4-isobutyl-5-(4-methylphenyl)-pyrazol-3-ol, 1-(3-methylphenyl)-4-isobutyl-5-(4-methylphenyl)-pyrazol-3-ol, 1-(4-Methoxyphenyl)-4-isobutyl-5-(4-methylphenyl)-pyrazol-3-ol, 1-Methyl-4-tert-butyl-5-phenyl-pyrazol-3-ol, , 1-Ethyl-4-tert-butyl5-phenyl-pyrazol-3-ol, 1-Propyl-4-tert-butyl-5-phenyl-pyrazol-3-ol, 1-Isopropyl-4-tert-butyl-5-phenyl-pyrazol-3-ol, 1-Butyl-4-tert-butyl-5-phenyl-pyrazol-3-ol, 1-Isobutyl-4-tert-butyl-5-phenyl-pyrazol-3-ol, 1-tert-Butyl-4-tert-butyl-5-phenyl-pyrazol-3-ol, 1-Phenyl-4-tert-butyl-5-phenyl-pyrazol-3-ol, 1-(4-methylphenyl)-4-tert-butyl-5-phenyl-pyrazol-3-ol, 1-(2-methylphenyl)-4-tert-butyl-5-phenyl-pyrazol-3-ol, 1-(3-methylphenyl)-4-tert-butyl-5-phenyl-pyrazol-3-ol, 1-(4-Methoxyphenyl)-4-tert-butyl-5-phenyl-pyrazol-3-ol, 1-Methyl-4-tert-butyl-5-methyl-pyrazol-3-ol, , 1-Ethyl-4-tert-butyl-5-methyl-pyrazol-3-ol, 1-Propyl-4-tert-butyl-5- methyl-pyrazol-3-ol, 1-Isopropyl-4-tert-butyl-5-methyl-pyrazol-3-ol, 1-Butyl-4-tert-butyl-5-methyl-pyrazol-3-ol, 1-Isobutyl-4-tert-butyl-5-methyl-pyrazol-3-ol, 1-tert-Butyl-4-tert-butyl-5-methyl-pyrazol-3-ol, 1-Phenyl-4-tert-butyl-5-methyl-pyrazol-3-ol, 1-(4-methylphenyl)-4-tert-butyl-5-methyl-pyrazol-3-ol, 1-(2-methylphenyl)-4-tert-butyl-5-methyl-pyrazol-3-ol, 1-(3-methylphenyl)-4-tert-butyl-5-methyl-pyrazol-3-ol, 1-(4-Methoxyphenyl)-4-tert-butyl-5- methyl -pyrazol-3-ol, 1-methyl-4-tert-butyl-5-ethyl-pyrazol-3-ol, 1-ethyl-4-tert-butyl-5-ethyl-pyrazol-3-ol, 1-propyl-4-tert-butyl-5-ethyl-pyrazol-3-ol, 1-isopropyl-4-tert-butyl-5-ethyl-pyrazol-3-ol, 1-butyl-4-tert-butyl-5-ethyl-pyrazol-3-ol, 1-Isobutyl-4-tert-butyl-5-ethyl-pyrazol-3-ol, 1-tert-butyl-4-tert-butyl-5-ethyl-pyrazol-3-ol, 1-Phenyl-4-tert-butyl-5-ethyl-pyrazol-3-ol, 1-(4-methylphenyl)-4-tert-butyl-5-ethyl-pyrazol-3-ol, 1-(2-methylphenyl)-4-tert-butyl-5-ethyl-pyrazol-3-ol, 1-(3-methylphenyl)-4-tert-butyl-5-ethyl-pyrazol-3-ol, 1-(4-Methoxyphenyl)-4-tert-butyl-5- ethyl -pyrazol-3-ol, 1-Methyl-4-tert-butyl-5-propyl-pyrazol-3-ol, , 1-Ethyl-4-tert-butyl-5-propyl-pyrazol-3-ol, 1-Propyl-4-tert-butyl-5-propyl-pyrazol-3-ol, 1-Isopropyl-4-tert-butyl-5-methyl-pyrazol-3-ol, 1-Butyl-4-tert-butyl-5-propyl-pyrazol-3-ol, 1-Isobutyl-4-tert-butyl-5-propyl-pyrazol-3-ol, 1-tert-Butyl-4-tert-butyl-5-propyl-pyrazol-3-ol, 1-Phenyl-4-tert-butyl-5-propyl-pyrazol-3-ol, 1-(4-methylphenyl)-4-tert-butyl-5-propyl-pyrazol-3-ol, 1-(2-methylphenyl)-4-tert-butyl-5-propyl-pyrazol-3-ol, 1-(3-methylphenyl)-4-tert-butyl-5-propyl-pyrazol-3-ol, 1-(4-Methoxyphenyl)-4-tert-butyl-5-propyl-pyrazol-3-ol, 1-Methyl-4-tert-butyl-5-isopropyl-pyrazol-3-ol, , 1-Ethyl-4-tert-butyl-5-isopropyl-pyrazol-3-ol, 1-Propyl-4-tert-butyl-5-isopropyl-pyrazol-3-ol, 1-Isopropyl-4-tert-butyl-5-isopropyl-pyrazol-3-ol, 1-Butyl-4-tert-butyl-5-isopropyl-pyrazol-3-ol, 1-Isobutyl-4-tert-butyl-5-isopropyl-pyrazol-3-ol, 1-tert-Butyl-4-tert-butyl-5-isopropyl-pyrazol-3-ol, 1-Phenyl-4-tert-butyl-5-isopropyl-pyrazol-3-ol, 1-(4-methylphenyl)-4-tert-butyl-5-isopropyl-pyrazol-3-ol, 1-(2-methylphenyl)-4-tert-butyl-5-isopropyl-pyrazol-3-ol, 1-(3-methylphenyl)-4-tert-butyl-5-isopropyl-pyrazol-3-ol, 1-(4-Methoxyphenyl)-4-tert-butyl-5- isopropyl-pyrazol-3-ol, 1-Methyl-4-tert-butyl-5-butyl-pyrazol-3-ol, , 1-Ethyl-4-tert-butyl-5-butyl-pyrazol-3-ol, 1-Propyl-4-tert-butyl-5-butyl-pyrazol-3-ol, 1-Isopropyl-4-tert-butyl-5-butyl-pyrazol-3-ol, 1-Butyl-4-tert-butyl-5-butyl-pyrazol-3-ol, 1-Isobutyl-4-tert-butyl-5-butyl-pyrazol-3-ol, 1-tert-Butyl-4-tert-butyl-5-butyl-pyrazol-3-ol, 1-Phenyl-4-tert-butyl-5-butyl-pyrazol-3-ol, 1-(4-methylphenyl)-4-tert-butyl-5-butyl-pyrazol-3-ol, 1-(2-methylphenyl)-4-tert-butyl-5-butyl-pyrazol-3-ol, 1-(3-methylphenyl)-4-tert-butyl-5-butyl-pyrazol-3-ol, 1-(4-Methoxyphenyl)-4-tert-butyl-5-butyl-pyrazol-3-ol, 1-Methyl-4-tert-butyl-5- isobutyl -pyrazol-3-ol, , 1-Ethyl-4-tert-butyl-5-isobutyl-pyrazol-3-ol, 1-Propyl-4-tert-butyl-5-isobutyl-pyrazol-3-ol, 1-Isopropyl-4-tert-butyl-5-isobutyl-pyrazol-3-ol, 1-Butyl-4-tert-butyl-5-isobutyl-pyrazol-3-ol, 1-Isobutyl-4-tert-butyl-5-isobutyl-pyrazol-3-ol, 1-tert-Butyl-4-tert-butyl-5-isobutyl-pyrazol-3-ol, 1-Phenyl-4-tert-butyl-5-isobutyl-pyrazol-3-ol, 1-(4-methylphenyl)-4-tert-butyl-5-isobutyl-pyrazol-3-ol, 1-(2-methylphenyl)-4-tert-butyl-5-isobutyl-pyrazol-3-ol, 1-(3-methylphenyl)-4-tert-butyl-5- isobutyl-pyrazol-3-ol, 1-(4-Methoxyphenyl)-4-tert-butyl-5- isobutyl-pyrazol-3-ol, 1-Methyl-4-tert-butyl-5-tert-butyl-pyrazol-3-ol, , 1-Ethyl-4-tert-butyl-5-tert-butyl-pyrazol-3-ol, 1-Propyl-4-tert-butyl-5-tert-butyl-pyrazol-3-ol, 1-Isopropyl-4-tert-butyl-5-tert-butyl -pyrazol-3-ol, 1-Butyl-4-tert-butyl-5-tert-butyl-pyrazol-3-ol, 1-Isobutyl-4-tert-butyl-5- tert-butyl -pyrazol-3-ol, 1-tert-Butyl-4-tert-butyl-5-tert-butyl-pyrazol-3-ol, 1-Phenyl-4-tert-butyl-5-tert-butyl-pyrazol-3-ol, 1-(4-methylphenyl)-4-tert-butyl-5-tert-butyl-pyrazol-3-ol, 1-(2-methylphenyl)-4-tert-butyl-5-tert-butyl-pyrazol-3-ol, 1-(3-methylphenyl)-4-tert-butyl-5-tert-butyl-pyrazol-3-ol,
1-(4-Methoxyphenyl)-4-tert-butyl-5-phenyl-pyrazol-3-ol, 1-Methyl-4-tert-butyl-5-phenyl-pyrazol-3-ol, , 1-Ethyl-4-tert-butyl-5-phenyl-pyrazol-3-ol, 1-Propyl-4-tert-butyl-5-phenyl-pyrazol-3-ol, 1-Isopropyl-4-tert-butyl-5-phenyl-pyrazol-3-ol, 1-Butyl-4-tert-butyl-5-phenyl-pyrazol-3-ol, 1-Isobutyl-4-tert-butyl-5-phenyl-pyrazol-3-ol, 1-tert-Butyl-4-tert-butyl-5-phenyl -pyrazol-3-ol, 1-Phenyl-4-tert-butyl-5-phenyl-pyrazol-3-ol, 1-(4-methylphenyl)-4-tert-butyl-5-phenyl-pyrazol-3-ol, 1-(2-methylphenyl)-4-tert-butyl-5-phenyl-pyrazol-3-ol, 1-(3-methylphenyl)-4-tert-butyl-5-phenyl-pyrazol-3-ol, 1-(4-Methoxyphenyl)-4-tert-butyl-5- (2-methylphenyl)-pyrazol-3-ol, 1-Methyl-4-tert-butyl-5-(2-methylphenyl)-pyrazol-3-ol, 1-Ethyl-4-tert-butyl-5-(2-methylphenyl)-pyrazol-3-ol, 1-Propyl-4-tert-butyl-5- (2-methylphenyl) -pyrazol-3-ol, 1-Isopropyl-4-tert-butyl-5-(2-methylphenyl)-pyrazol-3-ol, 1-Butyl-4-tert-butyl-5- (2-methylphenyl)-pyrazol-3-ol, 1-Isobutyl-4-tert-butyl-5-(2-methylphenyl)-pyrazol-3-ol, 1-tert-Butyl-4-tert-butyl-5-(2-methylphenyl)-pyrazol-3-ol, 1-Phenyl-4-tert-butyl-5-(2-methylphenyl)-pyrazol-3-ol, 1-(4-methylphenyl)-4-tert-butyl-5-(2-methylphenyl)-pyrazol-3-ol, 1-(2-methylphenyl)-4-tert-butyl-5-(2-methylphenyl-pyrazol-3-ol, 1-(3-methylphenyl)-4-tert-butyl-5- (2-methylphenyl)-pyrazol-3-ol, 1-(4-Methoxyphenyl)-4-tert-butyl-5- (3-methylphenyl) -pyrazol-3-ol, 1-Methyl-4-tert-butyl-5-(3-methylphenyl)-pyrazol-3-ol, , 1-Ethyl-4-tert-butyl-5- (3-methylphenyl)-pyrazol-3-ol, 1-Propyl-4-tert-butyl-5-(3-methylphenyl)-pyrazol-3-ol, 1-Isopropyl-4-tert-butyl-5-(3-methylphenyl)-pyrazol-3-ol, 1-Butyl-4-tert-butyl-5- (3-methylphenyl) -pyrazol-3-ol, 1-Isobutyl-4-tert-butyl-5-(3-methylphenyl)-pyrazol-3-ol, 1-tert-Butyl-4-tert-butyl-5- (3-methylphenyl)-pyrazol-3-ol, 1-Phenyl-4-tert-butyl-5-(3-methylphenyl)-pyrazol-3-ol, 1-(4-methylphenyl)-4-tert-butyl-5-(3-methylphenyl)-pyrazol-3-ol, 1-(2-methylphenyl)-4-tert-butyl-5-(3-methylphenyl)-pyrazol-3-ol, 1-(3-methylphenyl)-4-tert-butyl-5-(3-methylphenyl)- pyrazol-3-ol, 1-(4-Methoxyphenyl)-4-tert-butyl-5-(3-methylphenyl)-pyrazol-3-ol, 1-Methyl-4-tert-butyl-5- (3-methylphenyl)-pyrazol-3-ol, , 1-Ethyl-4-tert-butyl-5- (3-methylphenyl) -pyrazol-3-ol, 1-Propyl-4-tert-butyl-5-(3-methylphenyl)-pyrazol-3-ol, 1-Isopropyl-4-tert-butyl-5-(3-methylphenyl)-pyrazol-3-ol, 1-Butyl-4-tert-butyl-5-(3-methylphenyl)-pyrazol-3-ol, 1-Isobutyl-4-tert-butyl-5-(3-methylphenyl)-pyrazol-3-ol, 1-tert-Butyl-4-tert-butyl-5-butyl-pyrazol-3-ol, 1-Phenyl-4-tert-butyl-5-(3-methylphenyl)-pyrazol-3-ol, 1-(4-methylphenyl)-4-tert-butyl-5- (3-methylphenyl)-pyrazol-3-ol, 1-(2-methylphenyl)-4-tert-butyl-5-(3-methylphenyl)-pyrazol-3-ol, 1-(3-methylphenyl)-4-tert-butyl-5-(3-methylphenyl)-pyrazol-3-ol, 1-(4-Methoxyphenyl)-4-tert-butyl-5-butyl-pyrazol-3-ol, 1-Methyl-4-tert-butyl-5-(4-methylphenyl)-pyrazol-3-ol, , 1-Ethyl-4-tert-butyl-5-(4-methylphenyl)-pyrazol-3-ol, 1-Propyl-4-tert-butyl-5-(4-methylphenyl)-pyrazol-3-ol, 1-Isopropyl-4-tert-butyl-5-(4-methylphenyl)-pyrazol-3-ol, 1-Butyl-4-tert-butyl-5-(4-methylphenyl)-pyrazol-3-ol, 1-Isobutyl-4-tert-butyl-5-(4-methylphenyl)-pyrazol-3-ol, 1-tert-Butyl-4-tert-butyl-5- (4-methylphenyl)-pyrazol-3-ol, 1-Phenyl-4-tert-butyl-5- (4-methylphenyl)-pyrazol-3-ol, 1-(4-methylphenyl)-4-tert-butyl-5-(4-methylphenyl)-pyrazol-3-ol, 1-(2-methylphenyl)-4-tert-butyl-5-(4-methylphenyl)-pyrazol-3-ol, 1-(3-methylphenyl)-4-tert-butyl-5-(4-methylphenyl)-pyrazol-3-ol, 1-(4-Methoxyphenyl)-4-tert-butyl-5-(4-methylphenyl)-pyrazol-3-ol, 1-Methyl-4-phenyl-5-phenyl-pyrazol-3-ol, 1-Ethyl-4-phenyl-5-phenyl-pyrazol-3-ol, 1-Propyl-4-phenyl-5-phenyl-pyrazol-3-ol, 1-Isopropyl-4-phenyl-5-phenyl-pyrazol-3-ol, 1-Butyl-4-phenyl-5-phenyl-pyrazol-3-ol, 1-Isobutyl-4-phenyl-5-phenyl-pyrazol-3-ol, 1-tert-Butyl-4-phenyl-5-phenyl-pyrazol-3-ol, 1-Phenyl-4-phenyl-5-phenyl-pyrazol-3-ol, 1-(4-methylphenyl)-4-phenyl-5-phenyl-pyrazol-3-ol, 1-(2-methylphenyl)-4-phenyl-5-phenyl-pyrazol-3-ol, 1-(3-methylphenyl)-4-phenyl-5-phenyl-pyrazol-3-ol, 1-(4-Methoxyphenyl)-4-phenyl-5-phenyl-pyrazol-3-ol, 1-Methyl-4-phenyl-5-methyl-pyrazol-3-ol, , 1-Ethyl-4-phenyl-5-methyl-pyrazol-3-ol, 1-Propyl-4-phenyl-5-methyl-pyrazol-3-ol, 1-Isopropyl-4-phenyl-5-methyl-pyrazol-3-ol, 1-Butyl-4-phenyl-5-methyl-pyrazol-3-ol, 1-Isobutyl-4-phenyl-5-methyl-pyrazol-3-ol, 1-tert-Butyl-4-phenyl-5-methyl-pyrazol-3-ol, 1-Phenyl-4-phenyl-5-methyl-pyrazol-3-ol, 1-(4-methylphenyl)-4-phenyl-5-methyl-pyrazol-3-ol, 1-(2-methylphenyl)-4-phenyl-5-methyl-pyrazol-3-ol, 1-(3-methylphenyl)-4-phenyl-5-methyl-pyrazol-3-ol, 1-(4-Methoxyphenyl)-4-phenyl-5-methyl-pyrazol-3-ol, 1-methyl-4-phenyl-5-ethyl-pyrazol-3-ol, , 1-ethyl-4-phenyl-5-ethyl-pyrazol-3-ol, 1-propyl-4-phenyl-5-ethyl-pyrazol-3-ol, 1-isopropyl-4-phenyl-5-ethyl-pyrazol-3-ol, 1-butyl-4-phenyl-5-ethyl-pyrazol-3-ol, 1-Isobutyl-4-phenyl-5-ethyl-pyrazol-3-ol, 1-tert-butyl-4-phenyl-5-ethyl-pyrazol-3-ol, 1-Phenyl-4-phenyl-5-ethyl-pyrazol-3-ol, 1-(4-methylphenyl)-4-phenyl-5-ethyl-pyrazol-3-ol, 1-(2-methylphenyl)-4-phenyl-5- ethyl -pyrazol-3-ol, 1-(3-methylphenyl)-4-phenyl-5-ethyl-pyrazol-3-ol, 1-(4-Methoxyphenyl)-4-phenyl-5-ethyl-pyrazol-3-ol, 1-Methyl-4-phenyl-5-propyl-pyrazol-3-ol, , 1-Ethyl-4-phenyl-5-propyl-pyrazol-3-ol, 1-Propyl-4-phenyl-5-propyl-pyrazol-3-ol, 1-Isopropyl-4-phenyl-5-methyl-pyrazol-3-ol, 1-Butyl-4-phenyl-5-propyl-pyrazol-3-ol, 1-Isobutyl-4-phenyl-5- propyl -pyrazol-3-ol, 1-tert-Butyl-4-phenyl-5-propyl-pyrazol-3-ol, 1-Phenyl-4-phenyl-5-propyl-pyrazol-3-ol, 1-(4-methylphenyl)-4-phenyl-5-propyl-pyrazol-3-ol, 1-(2-methylphenyl)-4-phenyl-5-propyl-pyrazol-3-ol, 1-(3-methylphenyl)-4-phenyl-5-propyl-pyrazol-3-ol, 1-(4-Methoxyphenyl)-4-phenyl-5-propyl-pyrazol-3-ol, 1-Methyl-4-phenyl-5-isopropyl-pyrazol-3-ol, , 1-Ethyl-4-phenyl-5-isopropyl-pyrazol-3-ol, 1-Propyl-4-phenyl-5-isopropyl-pyrazol-3-ol, 1-Isopropyl-4-phenyl-5-isopropyl-pyrazol-3-ol, 1-Butyl-4-phenyl-5-isopropyl-pyrazol-3-ol, 1-Isobutyl-4-phenyl-5-isopropyl-pyrazol-3-ol, 1-tert-Butyl-4-phenyl-5-isopropyl-pyrazol-3-ol, 1-Phenyl-4-phenyl-5-isopropyl-pyrazol-3-ol, 1-(4-methylphenyl)-4-phenyl-5-isopropyl-pyrazol-3-ol, 1-(2-methylphenyl)-4-phenyl-5-isopropyl-pyrazol-3-ol, 1-(3-methylphenyl)-4-phenyl-5-isopropyl-pyrazol-3-ol, 1-(4-Methoxyphenyl)-4-phenyl-5- isopropyl-pyrazol-3-ol, 1-Methyl-4-phenyl-5-butyl-pyrazol-3-ol, , 1-Ethyl-4-phenyl-5-butyl-pyrazol-3-ol, 1-Propyl-4-phenyl-5-butyl-pyrazol-3-ol, 1-Isopropyl-4-phenyl-5-butyl-pyrazol-3-ol, 1-Butyl-4-phenyl-5- butyl -pyrazol-3-ol, 1-Isobutyl-4-phenyl-5-butyl-pyrazol-3-ol, 1-tert-Butyl-4-phenyl-5-butyl-pyrazol-3-ol, 1-Phenyl-4-phenyl-5-butyl-pyrazol-3-ol, 1-(4-methylphenyl)-4-phenyl-5-butyl-pyrazol-3-ol, 1-(2-methylphenyl)-4-phenyl-5-butyl-pyrazol-3-ol, 1-(3-methylphenyl)-4-phenyl-5-butyl-pyrazol-3-ol, 1-(4-Methoxyphenyl)-4-phenyl-5-butyl-pyrazol-3-ol, 1-Methyl-4-phenyl-5-isobutyl-pyrazol-3-ol, 1-Ethyl-4-phenyl-5-isobutyl-pyrazol-3-ol, 1-Propyl-4-phenyl-5- isobutyl -pyrazol-3-ol, 1-Isopropyl-4-phenyl-5-isobutyl-pyrazol-3-ol, 1-Butyl-4-phenyl-5-isobutyl-pyrazol-3-ol, 1-Isobutyl-4-phenyl-5-isobutyl-pyrazol-3-ol, 1-tert-Butyl-4-phenyl-5-isobutyl-pyrazol-3-ol, 1-Phenyl-4-phenyl-5-isobutyl-pyrazol-3-ol, 1-(4-methylphenyl)-4-phenyl-5-isobutyl-pyrazol-3-ol, 1-(2-methylphenyl)-4-phenyl-5-isobutyl-pyrazol-3-ol, 1-(3-methylphenyl)-4-phenyl-5-isobutyl-pyrazol-3-ol, 1-(4-Methoxyphenyl)-4-phenyl-5- isobutyl -pyrazol-3-ol, 1-Methyl-4-phenyl-5-tert-butyl-pyrazol-3-ol, , 1-Ethyl-4-phenyl-5-tert-butyl-pyrazol-3-ol, 1-Propyl-4-phenyl-5-tert-butyl-pyrazol-3-ol, 1-Isopropyl-4-phenyl-5-tert-buty-pyrazol-3-ol, 1-Butyl-4-phenyl-5-tert-butyl-pyrazol-3-ol, 1-Isobutyl-4-phenyl-5-tert-butyl-pyrazol-3-ol, 1-tert-Butyl-4-phenyl-5-tert-butyl-pyrazol-3-ol, 1-Phenyl-4-phenyl-5-tert-butyl-pyrazol-3-ol, 1-(4-methylphenyl)-4-phenyl-5-tert-butyl-pyrazol-3-ol, 1-(2-methylphenyl)-4-phenyl-5-tert-butyl-pyrazol-3-ol, 1-(3-methylphenyl)-4-phenyl-5-tert-butyl-pyrazol-3-ol, 1-(4-Methoxyphenyl)-4-phenyl-5-phenyl-pyrazol-3-ol, 1-Methyl-4-phenyl-5-phenyl-pyrazol-3-ol, , 1-Ethyl-4-phenyl-5-phenyl-pyrazol-3-ol, 1-Propyl-4-phenyl-5- phenyl-pyrazol-3-ol,
1-Isopropyl-4-phenyl-5-phenyl-pyrazol-3-ol, 1-Butyl-4-phenyl-5- phenyl-pyrazol-3-ol, 1-Isobutyl-4-phenyl-5-phenyl-pyrazol-3-ol, 1-tert-Butyl-4-phenyl-5-phenyl-pyrazol-3-ol, 1-Phenyl-4-phenyl-5-phenyl-pyrazol-3-ol, 1-(4-methylphenyl)-4-phenyl-5-phenyl-pyrazol-3-ol, 1-(2-methylphenyl)-4-phenyl-5-phenyl-pyrazol-3-ol, 1-(3-methylphenyl)-4-phenyl-5-phenyl-pyrazol-3-ol, 1-(4-Methoxyphenyl)-4-phenyl-5-(2-methylphenyl)-pyrazol-3-ol, 1-Methyl-4-phenyl-5-(2-methylphenyl)-pyrazol-3-ol, , 1-Ethyl-4-phenyl-5-(2-methylphenyl)-pyrazol-3-ol, 1-Propyl-4-phenyl-5-(2-methylphenyl)-pyrazol-3-ol, 1-Isopropyl-4-phenyl-5-(2-methylphenyl)-pyrazol-3-ol, 1-Butyl-4-phenyl-5-(2-methylphenyl)-pyrazol-3-ol, 1-Isobutyl-4-phenyl-5-(2-methylphenyl)-pyrazol-3-ol, 1-tert-Butyl-4-phenyl-5-(2-methylphenyl)-pyrazol-3-ol, 1-Phenyl-4-phenyl-5-(2-methylphenyl)-pyrazol-3-ol, 1-(4-methylphenyl)-4-phenyl-5-(2-methylphenyl)-pyrazol-3-ol, 1-(2-methylphenyl)-4-phenyl-5-(2-methylphenyl)-pyrazol-3-ol, 1-(3-methylphenyl)-4-phenyl-5-(2-methylphenyl)-pyrazol-3-ol, 1-(4-Methoxyphenyl)-4-phenyl-5-(3-methylphenyl)-pyrazol-3-ol, 1-Methyl-4-phenyl-5-(3-methylphenyl)-pyrazol-3-ol, 1-Ethyl-4-phenyl-5-(3-methylphenyl)-pyrazol-3-ol, 1-Propyl-4-phenyl-5-(3-methylphenyl) -pyrazol-3-ol, 1-Isopropyl-4-phenyl-5-(3-methylphenyl)-pyrazol-3-ol, 1-Butyl-4-phenyl-5- (3-methylphenyl)-pyrazol-3-ol, 1-Isobutyl-4-phenyl-5-(3-methylphenyl)-pyrazol-3-ol, 1-tert-Butyl-4-phenyl-5-(3-methylphenyl)-pyrazol-3-ol, 1-Phenyl-4-phenyl-5-(3-methylphenyl)-pyrazol-3-ol, 1-(4-methylphenyl)-4-phenyl-5-(3-methylphenyl)-pyrazol-3-ol, 1-(2-methylphenyl)-4-phenyl-5-(3-methylphenyl)-pyrazol-3-ol, 1-(3-methylphenyl)-4-phenyl-5-(3-methylphenyl)-pyrazol-3-ol, 1-(4-Methoxyphenyl)-4-phenyl-5-(3-methylphenyl)-pyrazol-3-ol, 1-Methyl-4-phenyl-5-(3-methylphenyl)-pyrazol-3-ol, , 1-Ethyl-4-phenyl-5-(3-methylphenyl)-pyrazol-3-ol, 1-Propyl-4-phenyl-5-(3-methylphenyl)-pyrazol-3-ol, 1-Isopropyl-4-phenyl-5-(3-methylphenyl)-pyrazol-3-ol, 1-Butyl-4-phenyl-5-(3-methylphenyl)-pyrazol-3-ol, 1-Isobutyl-4-phenyl-5- (3-methylphenyl)-pyrazol-3-ol, 1-tert-Butyl-4-phenyl-5-butyl-pyrazol-3-ol, 1-Phenyl-4-phenyl-5-(3-methylphenyl)-pyrazol-3-ol, 1-(4-methylphenyl)-4-phenyl-5-(3-methylphenyl)-pyrazol-3-ol, 1-(2-methylphenyl)-4-phenyl-5-(3-methylphenyl)-pyrazol-3-ol, 1-(3-methylphenyl)-4-phenyl-5-(3-methylphenyl)-pyrazol-3-ol, 1-(4-Methoxyphenyl)-4-phenyl-5-butyl-pyrazol-3-ol,
1-Methyl-4-phenyl-5-(4-methylphenyl)-pyrazol-3-ol, 1-Ethyl-4-phenyl-5-(4-methylphenyl)-pyrazol-3-ol, 1-Propyl-4-phenyl-5-(4-methylphenyl)-pyrazol-3-ol, 1-Isopropyl-4-phenyl-5-(4-methylphenyl)-pyrazol-3-ol, 1-Butyl-4-phenyl-5-(4-methylphenyl)-pyrazol-3-ol, 1-Isobutyl-4-phenyl-5-(4-methylphenyl)-pyrazol-3-ol, 1-tert-Butyl-4-phenyl-5-(4-methylphenyl)-pyrazol-3-ol, 1-Phenyl-4-phenyl-5-(4-methylphenyl)-pyrazol-3-ol, 1-(4-methylphenyl)-4-phenyl-5-(4-methylphenyl)-pyrazol-3-ol, 1-(2-methylphenyl)-4-phenyl-5-(4-methylphenyl)-pyrazol-3-ol, 1-(3-methylphenyl)-4-phenyl-5-(4-methylphenyl)-pyrazol-3-ol, 1-(4-Methoxyphenyl)-4-phenyl-5-(4-methylphenyl)-pyrazol-3-ol, 1-Methyl-4-(4-methylphenyl)-5-phenyl-pyrazol-3-ol, 1-Ethyl-4-(4-methylphenyl)5-phenyl-pyrazol-3-ol, 1-Propyl-4-(4-methylphenyl)-5-phenyl-pyrazol-3-ol, 1-Isopropyl-4-(4-methylphenyl)-5-phenyl-pyrazol-3-ol, 1-Butyl-4-(4-methyl-phenyl)-5-phenyl-pyrazol-3-ol, 1-Isobutyl-4-(4-methylphenyl)-5-phenyl-pyrazol-3-ol, 1-tert-Butyl-4-(4-methylphenyl)-5-phenyl-pyrazol-3-ol, 1-Phenyl-4-(4-methylphenyl)-5-phenyl-pyrazol-3-ol, 1-(4-methylphenyl)-4-(4-methylphenyl)-5-phenyl-pyrazol-3-ol, 1-(2-methyl-phenyl)-4-(4-methylphenyl)-5-phenyl-pyrazol-3-ol, 1-(3-methylphenyl)-4-(4-methylphenyl)-5-phenyl-pyrazol-3-ol, 1-(4-Methoxyphenyl)-4-(4-methylphenyl)-5-phenyl-pyrazol-3-ol, 1-Methyl-4-(4-methylphenyl)-5-methyl-pyrazol-3-ol, 1-Ethyl-4-(4-methylphenyl)-5-methyl-pyrazol-3-ol, 1-propyl-4-(4-methylphenyl)-5-methyl-pyrazol-3-ol, 1-Isopropyl-4-(4-methyl-phenyl)-5-methyl-pyrazol-3-ol, 1-Butyl-4-(4-methylphenyl)-5-methyl-pyrazol-3-ol, 1-Isobutyl-4-(4-methylphenyl)-5-methyl-pyrazol-3-ol, 1-tert-Butyl-4-(4-methylphenyl)-5-methyl -pyrazol-3-ol, 1-Phenyl-4-(4-methylphenyl)-5-methyl-pyrazol-3-ol, 1-(4-methylphenyl)-4-(4-methylphenyl)-5-methyl-pyrazol-3-ol, 1-(2-methylphenyl)-4-(4-methylphenyl)-5-methyl-pyrazol-3-ol, 1-(3-methylphenyl)-4-(4-methylphenyl)-5-methyl-pyrazol-3-ol, 1-(4-Methoxy-phenyl)-4-(4-methylphenyl)-5-methyl-pyrazol-3-ol, 1-methyl-4-(4-methylphenyl)-5-ethyl-pyrazol-3-ol, , 1-ethyl-4-(4-methylphenyl)-5-ethyl-pyrazol-3-ol, 1-propyl-4-(4-methylphenyl)-5-ethyl-pyrazol-3-ol, 1-isopropyl-4-(4-methylphenyl)-5-ethyl-pyrazol-3-ol, 1-butyl-4-(4-methyl-phenyl)-5-ethyl-pyrazol-3-ol, 1-Isobutyl-4-(4-methylphenyl)-5-ethyl-pyrazol-3-ol, 1-tert-butyl-4-(4-methylphenyl)-5-ethyl-pyrazol-3-ol, 1-Phenyl-4-(4-methylphenyl)-5-ethyl-pyrazol-3-ol, 1-(4-methylphenyl)-4-(4-methylphenyl)-5-ethyl-pyrazol-3-ol, 1-(2-methylphenyl)-4-(4-methylphenyl)-5-ethyl-pyrazol-3-ol, 1-(3-methylphenyl)-4-(4-methylphenyl)-5-ethyl-pyrazol-3-ol, 1-(4-Methoxyphenyl)-4-(4-methylphenyl)-5-ethyl-pyrazol-3-ol, 1-Methyl-4-(4-methylphenyl)-5-propyl-pyrazol-3-ol, 1-Ethyl-4-(4-methylphenyl)-5-propyl-pyrazol-3-ol, 1-Propyl-4-(4-methylphenyl)-5-propyl-pyrazol-3-ol, 1-Isopropyl-4-(4-methylphenyl)-5-methyl-pyrazol-3-ol, 1-Butyl-4-(4-methylphenyl)-5-propyl-pyrazol-3-ol, 1-Isobutyl-4-(4-methylphenyl)-5-propyl -pyrazol-3-ol,
1-tert-Butyl-4-(4-methylphenyl)-5-propyl-pyrazol-3-ol, 1-Phenyl-4-(4-methylphenyl)-5-propyl -pyrazol-3-ol, 1-(4-methylphenyl)-4-(4-methylphenyl)-5-propyl-pyrazol-3-ol, 1-(2-methyl-phenyl)-4-(4-methylphenyl)-5-propyl-pyrazol-3-ol, 1-(3-methylphenyl)-4-(4-methylphenyl)-5-propyl-pyrazol-3-ol, 1-(4-Methoxyphenyl)-4-(4-methylphenyl)-5- propyl-pyrazol-3-ol, 1-Methyl-4-(4-methylphenyl)-5-isopropyl-pyrazol-3-ol, 1-Ethyl-4-(4-methylphenyl)-5-isopropyl-pyrazol-3-ol, 1-Propyl-4-(4-methylphenyl)-5-isopropyl-pyrazol-3-ol, 1-Isopropyl-4-(4-methyl-phenyl)-5-isopropyl-pyrazol-3-ol, 1-Butyl-4-(4-methylphenyl)-5- isopropyl-pyrazol-3-ol, 1-Isobutyl-4-(4-methylphenyl)-5-isopropyl-pyrazol-3-ol, 1-tert-Butyl-4-(4-methylphenyl)-5-isopropyl-pyrazol-3-ol, 1-Phenyl-4-(4-methylphenyl)-5-isopropyl-pyrazol-3-ol, 1-(4-methyl-phenyl)-4-(4-methylphenyl)-5-isopropyl-pyrazol-3-ol, 1-(2-methylphenyl)-4-(4-methylphenyl)-5-isopropyl-pyrazol-3-ol, 1-(3-methylphenyl)-4-(4-methylphenyl)-5-isopropyl-pyrazol-3-ol, 1-(4-Methoxyphenyl)-4-(4-methylphenyl)-5-isopropyl-pyrazol-3-ol, 1-Methyl-4-(4-methyl-phenyl)-5-butyl-pyrazol-3-ol, 1-Ethyl-4-(4-methylphenyl)-5-butyl-pyrazol-3-ol, 1-Propyl-4-(4-methylphenyl)-5-butyl-pyrazol-3-ol, 1-Isopropyl-4-(4-methylphenyl)-5-butyl-pyrazol-3-ol, 1-Butyl-4-(4-methylphenyl)-5-butyl-pyrazol-3-ol, 1-Isobutyl-4-(4-methylphenyl)-5-butyl-pyrazol-3-ol, 1-tert-Butyl-4-(4-methylphenyl)-5-butyl-pyrazol-3-ol, 1-Phenyl-4-(4-methyl-phenyl)-5-butyl-pyrazol-3-ol, 1-(4-methylphenyl)-4-(4-methylphenyl)-5-butyl-pyrazol-3-ol, 1-(2-methylphenyl)-4-(4-methylphenyl)-5-butyl-pyrazol-3-ol, 1-(3-methylphenyl)-4-(4-methyl-phenyl)-5-butyl-pyrazol-3-ol, 1-(4-Methoxyphenyl)-4-(4-methylphenyl)-5-butyl-pyrazol-3-ol, 1-Methyl-4-(4-methylphenyl)-5-isobutyl-pyrazol-3-ol,1-Ethyl-4-(4-methylphenyl)-5-isobutyl-pyrazol-3-ol, 1-Propyl-4-(4-methylphenyl)-5-isobutyl-pyrazol-3-ol, 1-Isopropyl-4-(4-methyl-phenyl)-5-isobutyl-pyrazol-3-ol,
1-Butyl-4-(4-methylphenyl)-5-isobutyl-pyrazol-3-ol, 1-Isobutyl-4-(4-methylphenyl)-5-isobutyl-pyrazol-3-ol, 1-tert-Butyl-4-(4-methylphenyl)-5-isobutyl-pyrazol-3-ol, 1-Phenyl-4-(4-methylphenyl)-5-isobutyl-pyrazol-3-ol,
1-(4-methylphenyl)-4-(4-methylphenyl)-5-isobutyl-pyrazol-3-ol, 1-(2-methylphenyl)-4-(4-methylphenyl)-5-isobutyl-pyrazol-3-ol,
1-(3-methylphenyl)-4-(4-methylphenyl)-5-isobutyl-pyrazol-3-ol, 1-(4-Methoxyphenyl)-4-(4-methylphenyl)-5-isobutyl-pyrazol-3-ol, 1-Methyl-4-(4-methylphenyl)-5-tert-butyl-pyrazol-3-ol, 1-Ethyl-4-(4-methylphenyl)-5-tert-butyl-pyrazol-3-ol, 1-Propyl-4-(4-methylphenyl)-5-tert-butyl-pyrazol-3-ol, 1-Isopropyl-4-(4-methylphenyl)-5-tert-butyl-pyrazol-3-ol,
1-Butyl-4-(4-methylphenyl)-5-tert-butyl-pyrazol-3-ol, 1-Isobutyl-4-(4-methylphenyl)-5-tert-butyl-pyrazol-3-ol,
1-tert-Butyl-4-(4-methylphenyl)-5-tert-butyl-pyrazol-3-ol, 1-Phenyl-4-(4-methylphenyl)-5-tert-butyl-pyrazol-3-ol, 1-(4-methylphenyl)-4-(4-methylphenyl)-5- tert-butyl-pyrazol-3-ol, 1-(2-methylphenyl)-4-(4-methylphenyl)-5-tert-butyl-pyrazol-3-ol, 1-(3-methylphenyl)-4-(4-methylphenyl)-5-tert-butyl-pyrazol-3-ol, 1-(4-Methoxyphenyl)-4-(4-methylphenyl)-5-phenyl-pyrazol-3-ol, 1-Methyl-4-(4-methylphenyl)-5-phenyl-pyrazol-3-ol, 1-Ethyl-4-(4-methylphenyl)-5-phenyl-pyrazol-3-ol, 1-Propyl-4-(4-methylphenyl)-5-phenyl-pyrazol-3-ol, 1-Isopropyl-4-(4-methylphenyl)-5- phenyl-pyrazol-3-ol, 1-Butyl-4-(4-methylphenyl)-5- phenyl-pyrazol-3-ol, 1-Isobutyl- 4-(4-methylphenyl)-5-phenyl-pyrazol-3-ol, 1-tert-Butyl-4-(4-methylphenyl)-5- phenyl -pyrazol-3-ol, 1-Phenyl-4-(4-methylphenyl)-5- phenyl-pyrazol-3-ol, 1-(4-methylphenyl)-4-(4-methylphenyl)-5-phenyl-pyrazol-3-ol, 1-(2-methylphenyl)-4-(4-methylphenyl)-5-phenyl-pyrazol-3-ol, 1-(3-methylphenyl)-4-(4-methylphenyl)-5-phenyl-pyrazol-3-ol, 1-(4-Methoxy-phenyl)-4-(4-methylphenyl)-5-(2-methylphenyl)-pyrazol-3-ol, 1-Methyl-4-(4-methylphenyl)-5-(2-methylphenyl)-pyrazol-3-ol, 1-Ethyl-4-(4-methylphenyl)-5-(2-methylphenyl)-pyrazol-3-ol, 1-Propyl-4-(4-methylphenyl)-5-(2-methylphenyl)-pyrazol-3-ol, 1-Isopropyl-4-(4-methyl-phenyl)-5-(2-methylphenyl)-pyrazol-3-ol, 1-Butyl-4-(4-methylphenyl)-5-(2-methylphenyl)-pyrazol-3-ol,1-Isobutyl-4-(4-methylphenyl)-5-(2-methylphenyl)-pyrazol-3-ol, 1-tert-Butyl-4-(4-methylphenyl)-5-(2-methylphenyl)-pyrazol-3-ol, 1-Phenyl-4-(4-methylphenyl)-5-(2-methylphenyl)-pyrazol-3-ol, 1-(4-methylphenyl)-4-(4-methylphenyl)-5-(2-methylphenyl)-pyrazol-3-ol, 1-(2-methylphenyl)-4-(4-methylphenyl)-5-(2-methylphenyl)-pyrazol-3-ol, 1-(3-methylphenyl)-4-(4-methylphenyl)-5-(2-methylphenyl)-pyrazol-3-ol, 1-(4-Methoxyphenyl)-4-(4-methylphenyl)-5-(3-methylphenyl)-pyrazol-3-ol, 1-Methyl-4-(4-methylphenyl)-5-(3-methylphenyl)-pyrazol-3-ol, 1-Ethyl-4-(4-methylphenyl)-5-(3-methylphenyl)-pyrazol-3-ol, 1-Propyl-4-(4-methylphenyl)-5-(3-methylphenyl)-pyrazol-3-ol, 1-Isopropyl-4-(4-methylphenyl)-5-(3-methylphenyl)-pyrazol-3-ol, 1-Butyl-4-(4-methylphenyl)-5-(3-methylphenyl)-pyrazol-3-ol, 1-Isobutyl-4-(4-methylphenyl)-5-(3-methylphenyl)-pyrazol-3-ol, 1-tert-Butyl-4-(4-methyl-phenyl)-5-(3-methylphenyl)-pyrazol-3-ol, 1-Phenyl-4-(4-methylphenyl)-5-(3-methylphenyl)-pyrazol-3-ol, 1-(4-methylphenyl)-4-(4-methylphenyl)-5-(3-methylphenyl)-pyrazol-3-ol, 1-(2-methylphenyl)-4-(4-methylphenyl)-5-(3-methylphenyl)-pyrazol-3-ol, 1-(3-methylphenyl)-4-(4-methylphenyl)-5-(3-methylphenyl)-pyrazol-3-ol, 1-(4-Methoxyphenyl)-4-(4-methylphenyl)-5-(3-methylphenyl)-pyrazol-3-ol, 1-Methyl-4-(4-methylphenyl)-5-(3-methylphenyl)-pyrazol-3-ol, 1-Ethyl-4-(4-methylphenyl)-5-(3-methylphenyl)-pyrazol-3-ol, 1-Propyl-4-(4-methylphenyl)-5-(3-methylphenyl)-pyrazol-3-ol, 1-Isopropyl-4-(4-methylphenyl)-5-(3-methylphenyl)-pyrazol-3-ol, 1-Butyl-4-(4-methylphenyl)-5-(3-methylphenyl)-pyrazol-3-ol, 1-Isobutyl-4-(4-methylphenyl)-5-(3-methylphenyl)-pyrazol-3-ol, 1-tert-Butyl-4-(4-methylphenyl)-5-butyl-pyrazol-3-ol, 1-Phenyl-4-(4-methylphenyl)-5-(3-methylphenyl)-pyrazol-3-ol, 1-(4-methyl-phenyl)-4-(4-methylphenyl)-5-(3-methylphenyl)-pyrazol-3-ol, 1-(2-methylphenyl)-4-(4-methylphenyl)-5-(3-methylphenyl)-pyrazol-3-ol, 1-(3-methylphenyl)-4-(4-methylphenyl)-5-(3-methylphenyl)-pyrazol-3-ol, 1-(4-Methoxyphenyl)-4-(4-methylphenyl)-5-butyl-pyrazol-3-ol, 1-Methyl-4-(4-methylphenyl)-5-(4-methylphenyl)-pyrazol-3-ol, 1-Ethyl-4-(4-methyl-phenyl)-5-(4-methylphenyl)-pyrazol-3-ol, 1-Propyl-4-(4-methylphenyl)-5-(4-methylphenyl)-pyrazol-3-ol, 1-Isopropyl-4-(4-methylphenyl)-5-(4-methylphenyl)-pyrazol-3-ol, 1-Butyl-4-(4-methylphenyl)-5-(4-methylphenyl)-pyrazol-3-ol, 1-Isobutyl-4-(4-methylphenyl)-5-(4-methyl-phenyl)-pyrazol-3-ol, 1-tert-Butyl-4-(4-methylphenyl)-5-(4-methylphenyl)-pyrazol-3-ol, 1-Phenyl-4-(4-methylphenyl)-5-(4-methylphenyl)-pyrazol-3-ol, 1-(4-methylphenyl)-4-(4-methylphenyl)-5-(4-methylphenyl)-pyrazol-3-ol, 1-(2-methylphenyl)-4-(4-methylphenyl)-5-(4-methylphenyl)-pyrazol-3-ol, 1-(3-methylphenyl)-4-(4-methylphenyl)-5-(4-methylphenyl)-pyrazol-3-ol, 1-(4-Methoxyphenyl)-4-(4-methylphenyl)-5-(4-methylphenyl)-pyrazol-3-ol, 1-Benzyl-pyrazol-4-ol, 1-Benzyl-3-Methyl-pyrazol-4-ol, 1-Benzyl-5-Methyl-pyrazol-4-ol, 1-Benzyl-3,5-Dimethyl-pyrazol-4-ol, 1-Benzyl-3-ethyl-pyrazol-4-ol, 1-Benzyl-5-ethyl-pyrazol-4-ol, 1-Benzyl-3,5-Diethyl-pyrazol-4-ol, 1-Benzyl-3-Propyl-pyrazol-4-ol, 1-Benzyl-5-Propyl-pyrazol-4-ol, 1-Benzyl-3,5-Dipropyl-pyrazol-4-ol, 1-Benzyl-3-ethyl-5-methyl-pyrazol-4-ol, 1-Benzyl-3-methyl-5-ethylpyrazol-4-ol, 1-Benzyl-3-methyl-5-phenyl-pyrazol-4-ol, 1-Benzyl-3-phenyl-5-methyl-pyrazol-4-ol, 1-Isopropyl-pyrazol-4-ol, 1-Isopropyl-3-Methyl-pyrazol-4-ol, 1-Isopropyl-5-Methyl-pyrazol-4-ol, 1-Isopropyl-3,5-Dimethyl-pyrazol-4-ol, 1-Isopropyl-3-ethyl-pyrazol-4-ol, 1-Isopropyl-5-ethyl-pyrazol-4-ol, 1-Isopropyl-3,5-Diethyl-pyrazol-4-ol, 1-Isopropyl-3-Propyl-pyrazol-4-ol, 1-Isopropyl-5-Propyl-pyrazol-4-ol, 1-Isopropyl-3,5-Dipropyl-pyrazol-4-ol, 1-Isopropyl-3-ethyl-5-methyl-pyrazol-4-ol, 1-Isopropyl-3-methyl-5-ethylpyrazol-4-ol, 1-Isopropyl-3-methyl-5-phenyl-pyrazol-4-ol, 1-Isopropyl-3-phenyl-5-methyl-pyrazol-4-ol, 1-tert-Butyl-pyrazol-4-ol, 1-tert-Butyl-3-Methyl-pyrazol-4-ol, 1-tert-Butyl-5-Methyl-pyrazol-4-ol, 1-tert-Butyl-3,5-Dimethyl-pyrazol-4-ol, 1-tert-Butyl-3-ethyl-pyrazol-4-ol, 1-tert-Butyl-5-ethyl-pyrazol-4-ol, 1-tert-Butyl-3,5-Diethyl-pyrazol-4-ol, 1-tert-Butyl-3-Propyl-pyrazol-4-ol, 1-tert-Butyl-5-Propyl-pyrazol-4-ol, 1-tert-Butyl-3,5-Dipropyl-pyrazol-4-ol, 1-tert-Butyl-3-ethyl-5-methyl-pyrazol-4-ol, 1-tert-Butyl-3-methyl-5-ethylpyrazol-4-ol, 1-tert-Butyl-3-methyl-5-phenyl-pyrazol-4-ol oder 1-tert-Butyl-3-phenyl-5-methyl-pyrazol-4-ol.

Sofern nicht näher spezifiziert steht Butyl für iso-Butyl oder n-Butyl. Insbesonders sind solche Verbindungen bevorzugt, die keinen Pyridyl-Substituenten aufweisen.

In bevorzugten Verbindungen der allgemeinen Formel (IV) sind
R¹² H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁-bis C₆-Alkyl verestert sein kann, (N,N-Dimethylsulfonamid)-phenyl, Sulfonamidphenyl, Alkanoyl mit C₁- bis C₆-Alkyl, oder 4-iso-Propoxyphenyl,
R¹³ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁-bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl, oder 4-iso-Propoxyphenyl,
R¹⁴ H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁-bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl, oder 4-iso-Propoxyphenyl,
R¹⁵H, C₁- bis C₈-Alkyl, bevorzugt C₁- bis C₆-Alkyl, Phenyl, Cyanophenyl, 2-Chlorphenyl, 3-Chlorphenyl, 4-Chlorphenyl, 2,4-Dichlorphenyl, 3,4-Dichlorphenyl, 2-Bromphenyl, 3-Bromphenyl, 4-Bromphenyl, 2-Methylphenyl, 3-Methylphenyl, 4-Methylphenyl, Dimethylphenyl, 2-Methoxyphenyl, 3-Methoxyphenyl, 4-Methoxyphenyl, 2-Ethylphenyl, 3-Ethylphenyl, 4-Ethylphenyl, 2-Ethoxyphenyl, 3-Ethoxyphenyl, 4-Ethoxyphenyl, 4-iso-Propylphenyl, Benzyl, 2-Pyridyl, 3-Pyridyl, 4-Pyridyl, (Carboxymethyl)-phenyl wobei die Carboxygruppe auch mit C₁-bis C₆-Alkyl verestert sein kann, (N,N-Dimethylsulfonamid)-phenyl, Sulfonamidphenyl, Carboxylester mit C₁- bis C₈-Alkyl wie -COOMe, Alkanoyl mit C₁- bis C₆-Alkyl, 4-iso-Propoxyphenyl, NH₂, NH(CO)R¹⁶ wobei R¹⁶ H, C₁- bis C₆-Alkyl, Phenyl, Toluyl wie m- oder p-Toluyl, 4-Methoxyphenyl oder Dimethylphenyl, 3,4-Dimethylphenyl ist.

Besonders bevorzugt Verbindungen der allgemeinen Formel (IV) sind
2-Methyl-1H-pyrazol-3-on,2-Ethyl-1H-pyrazol-3-on, 2-Propyl-1H-pyrazol-3-on,2-Isopropyl-1H-pyrazol-3-on,2-Butyl-1H-pyrazol-3-on, 2-Isobutyl-1H-pyrazol-3-on, 2-tert-Butyl-1H-pyrazol-3-on, 2-Phenyl-1H-pyrazol-3-on, 2-(4-Methylphenyl)-1H-pyrazol-3-on, 2-(2-Methylphenyl)-1H-pyrazol-3-on, 2-(3-Methylphenyl)-1H-pyrazol-3-on, 2-(4-Methoxy-1H-pyrazol-3-on, 2-Methyl-4-phenyl-1H-pyrazol-3-on, 2-Ethyl-4-phenyl-1H-pyrazol-3-on, 2-Propyl-4-phenyl-1H-pyrazol-3-on, 2-Isopropyl-4-phenyl-1H-pyrazol-3-on, 2-Butyl-4-phenyl-1H-pyrazol-3-on, 2-Isobutyl-4-phenyl-1H-pyrazol-3-on, 2-tert-Butyl-4-phenyl-1H-pyrazol-3-on, 2-Phenyl-4-phenyl-1H-pyrazol-3-on, 2-(4-Methylphenyl)-4-phenyl-1H-pyrazol-3-on, 2-(2-Methylphenyl)-4-phenyl-1H-pyrazol-3-on, 2-(3-Methylphenyl)-4-phenyl-1H-pyrazol-3-on, 2-(4-Methoxy-4-phenyl-1H-pyrazol-3-on, 2-Methyl-4-methyl-1H-pyrazol-3-on, 2-Ethyl-4-methyl-1H-pyrazol-3-on, 2-Propyl-4-methyl-1H-pyrazol-3-on, 2-Isopropyl-4-methyl-1H-pyrazol-3-on, 2-Butyl-4-methyl-1H-pyrazol-3-on, 2-Isobutyl-4-methyl-1H-pyrazol-3-on, 2-tert-Butyl-4-methyl-1H-pyrazol-3-on, 2-Phenyl-4-methyl-1H-pyrazol-3-on, 2-(4-Methylphenyl)-4-methyl-1H-pyrazol-3-on, 2-(2-Methylphenyl)-4-methyl-1H-pyrazol-3-on, 2-(3-Methylphenyl)-4-methyl-1H-pyrazol-3-on, 2-(4-Methoxy-4-methyl-1H-pyrazol-3-on, 2-methyl-4-ethyl-1H-pyrazol-3-on, 2-ethyl-4-ethyl-1H-pyrazol-3-on, 2-propyl-4-ethyl-1H-pyrazol-3-on, 2-isopropyl-4-ethyl-1H-pyrazol-3-on, 2-butyl-4-ethyl-1H-pyrazol-3-on, 2-Isobutyl-4-ethyl-1H-pyrazol-3-on, 2-tert-butyl-4-ethyl-1H-pyrazol-3-on, 2-Phenyl-4-ethyl-1H-pyrazol-3-on, 2-(4-Methylphenyl)-4-ethyl-1H-pyrazol-3-on, 2-(2-Methylphenyl)-4-ethyl-1H-pyrazol-3-on, 2-(3-Methylphenyl)-4-ethyl-1H-pyrazol-3-on, 2-(4-Methoxy-4-ethyl-1H-pyrazol-3-on, 2-Methyl-4-propyl-1H-pyrazol-3-on, 2-Ethyl-4-propyl-1H-pyrazol-3-on, 2-Propyl-4-propyl-1H-pyrazol-3-on, 2-Isopropyl-4-methyl-1H-pyrazol-3-on, 2-Butyl-4-propyl-1H-pyrazol-3-on, 2-Isobutyl-4-propyl -1H-pyrazol-3-on, 2-tert-Butyl-4-propyl-1H-pyrazol-3-on, 2-Phenyl-4-propyl-1H-pyrazol-3-on, 2-(4-Methylphenyl)-4-propyl-1H-pyrazol-3-on, 2-(2-Methylphenyl)-4-propyl-1H-pyrazol-3-on, 2-(3-Methylphenyl)-4-propyl-1H-pyrazol-3-on, 2-(4-Methoxy-4-propyl-1H-pyrazol-3-on, 2-Methyl-4-isopropyl-1H-pyrazol-3-on, 2-Ethyl-4-isopropyl-1H-pyrazol-3-on, 2-Propyl-4-isopropyl-1H-pyrazol-3-on, 2-Isopropyl-4-isopropyl-1H-pyrazol-3-on, 2-Butyl-4-isopropyl-1H-pyrazol-3-on, 2-Isobutyl-4-isopropyl-1H-pyrazol-3-on, 2-tert-Butyl-4-isopropyl-1H-pyrazol-3-on, 2-Phenyl-4-isopropyl-1H-pyrazol-3-on, 2-(4-Methylphenyl)-4-isopropyl-1H-pyrazol-3-on, 2-(2-Methylphenyl)-4-isopropyl-1H-pyrazol-3-on, 2-(3-Methylphenyl)-4-isopropyl-1H-pyrazol-3-on, 2-(4-Methoxy-4-isopropyl-1H-pyrazol-3-on, 2-Methyl-4-butyl-1H-pyrazol-3-on, 2-Ethyl-4-butyl-1H-pyrazol-3-on, 2-Propyl-4-butyl-1H-pyrazol-3-on, 2-Isopropyl-4-butyl-1H-pyrazol-3-on, 2-Butyl-4-butyl-1H-pyrazol-3-on, 2-Isobutyl-4-butyl-1H-pyrazol-3-on, 2-tert-Butyl-4-butyl-1H-pyrazol-3-on, 2-Phenyl-4-butyl-1H-pyrazol-3-on, 2-(4-Methylphenyl)-4-butyl-1H-pyrazol-3-on, 2-(2-Methylphenyl)-4-butyl-1H-pyrazol-3-on, 2-(3-Methylphenyl)-4-butyl-1H-pyrazol-3-on, 2-(4-Methoxy-4-butyl-1H-pyrazol-3-on, 2-Methyl-4-isobutyl-1H-pyrazol-3-on, 2-Ethyl-4-isobutyl-1H-pyrazol-3-on, 2-Propyl-4-isobutyl-1H-pyrazol-3-on, 2-Iopropyl-4-isobutyl-1H-pyrazol-3-on, 2-Butyl-4- isobutyl-1H-pyrazol-3-on, 2-Isobutyl-4-isobutyl-1H-pyrazol-3-on, 2-tert-Butyl-4- isobutyl-1H-pyrazol-3-on, 2-Phenyl-4-isobutyl-1H-pyrazol-3-on, 2-(4-Methylphenyl)-4-isobutyl-1H-pyrazol-3-on, 2-(2-Methyl-phenyl)-4-isobutyl-1H-pyrazol-3-on, 2-(3-Methylphenyl)-4-isobutyl-1H-pyrazol-3-on, 2-(4-Methoxy-4-isobutyl-1H-pyrazol-3-on, 2-Methyl-4-tert-butyl-1H-pyrazol-3-on, 2-Ethyl-4-tert-butyl-1H-pyrazol-3-on, 2-Propyl-4-tert-butyl-1H-pyrazol-3-on, 2-Isopropyl-4-tert-butyl-1H-pyrazol-3-on, 2-Butyl-4-tert-butyl-1H-pyrazol-3-on, 2-Iobutyl-4-tert-butyl-1H-pyrazol-3-on, 2-tert-Butyl-4-tert-butyl-1H-pyrazol-3-on, 2-Phenyl-4-tert-butyl-1H-pyrazol-3-on, 2-(4-Methylphenyl)-4-tert-butyl-1H-pyrazol-3-on, 2-(2-Methylphenyl)-4-tert-butyl-1H-pyrazol-3-on, 2-(3-Methylphenyl)-4- tert-butyl-1H-pyrazol-3-on, 2-(4-Methoxy-4-phenyl-1H-pyrazol-3-on, 2-Methyl-4-phenyl-1H-pyrazol-3-on, 2-Ethyl-4-phenyl-1H-pyrazol-3-on, 2-Propyl-4-phenyl-1H-pyrazol-3-on, 2-Iopropyl-4-phenyl-1H-pyrazol-3-on, 2-Butyl-4-phenyl-1H-pyrazol-3-on, 2-Iobutyl-4-phenyl-1H-pyrazol-3-on, 2-tert-Butyl-4-phenyl-1H-pyrazol-3-on, 2-Phenyl-4-phenyl-1H-pyrazol-3-on, 2-(4-Methylphenyl)-4-phenyl-1H-pyrazol-3-on, 2-(2-Methylphenyl)-4-phenyl-1H-pyrazol-3-on, 2-(3-Methylphenyl)-4-phenyl-1H-pyrazol-3-on, 2-(4-Methoxy-4-(2-Methylphenyl)-1H-pyrazol-3-on, 2-Methyl-4-(2-Methylphenyl)-1H-pyrazol-3-on, 2-Ethyl-4-(2-Methylphenyl)-1H-pyrazol-3-on, 2-Propyl-4-(2-Methylphenyl)-1H-pyrazol-3-on, 2-Isopropyl-4-(2-Methylphenyl)-1H-pyrazol-3-on, 2-Butyl-4-(2-Methylphenyl)-1H-pyrazol-3-on, 2-Isobutyl-4-(2-Methylphenyl)-1H-pyrazol-3-on, 2-tert-Butyl-4-(2-Methyl-phenyl)-1H-pyrazol-3-on, 2-Phenyl-4-(2-Methylphenyl)-1H-pyrazol-3-on, 2-(4-Methyl-phenyl)-4-(2-Methylphenyl)-1H-pyrazol-3-on, 2-(2-Methylphenyl)-4-(2-Methylphenyl)-1H-pyrazol-3-on, 2-(3-Methylphenyl)-4-(2-Methylphenyl)-1H-pyrazol-3-on, 2-(4-Methoxy-4-(3-Methylphenyl)-1H-pyrazol-3-on, 2-Methyl-4-(3-Methylphenyl)-1H-pyrazol-3-on, 2-Ethyl-4-(3-Methylphenyl)-1H-pyrazol-3-on, 2-Propyl-4-(3-Methylphenyl)-1H-pyrazol-3-on, 2-Isopropyl-4-(3-Methylphenyl)-1H-pyrazol-3-on , 2-Butyl-4-(3-Methylphenyl)-1H-pyrazol-3-on, 2-Isobutyl-4-(3-Methylphenyl)-1H-pyrazol-3-on, 2-tert-Butyl-4-(3-Methylphenyl)-1H-pyrazol-3-on, 2-Phenyl-4-(3-Methylphenyl)-1H-pyrazol-3-on, 2-(4-Methylphenyl)-4-(3-Methylphenyl)-1H-pyrazol-3-on, 2-(2-Methylphenyl)-4-(3-Methylphenyl)-1H-pyrazol-3-on, 2-(3-Methylphenyl)-4-(3-Methylphenyl)-1H-pyrazol-3-on, 2-(4-Methoxy-4-(3-Methylphenyl)-1H-pyrazol-3-on, 2-Methyl-4-(3-Methylphenyl)-1H-pyrazol-3-on, 2-Ethyl-4-(3-Methylphenyl) -1H-pyrazol-3-on, 2-Propyl-4-(3-Methylphenyl)-1H-pyrazol-3-on, 2-Isopropyl-4-(3-Methyl-phenyl)-1H-pyrazol-3-on, 2-Butyl-4-(3-Methylphenyl)-1H-pyrazol-3-on, 2-Isobutyl-4-(3-Methylphenyl)-1H-pyrazol-3-on, 2-tert-Butyl-4-butyl-1H-pyrazol-3-on, 2-Phenyl-4-(3-Methylphenyl)-1H-pyrazol-3-on, 2-(4-Methylphenyl)-4-(3-Methylphenyl)-1H-pyrazol-3-on, 2-(2-Methylphenyl)-4-(3-Methylphenyl)-1H-pyrazol-3-on, 2-(3-Methylphenyl)-4-(3-Methyl-phenyl)-1H-pyrazol-3-on, 2-(4-Methoxy-4-butyl-1H-pyrazol-3-on, 2-Methyl-4-(4-Methyl-phenyl)-1H-pyrazol-3-on, 2-Ethyl-4-(4-Methylphenyl)-1H-pyrazol-3-on, 2-Propyl-4-(4-Methylphenyl)-1H-pyrazol-3-on, 2-Isopropyl-4-(4-Methylphenyl)-1H-pyrazol-3-on, 2-Butyl-4-(4-Methylphenyl)-1H-pyrazol-3-on, 2-Isobutyl-4-(4-Methylphenyl)-1H-pyrazol-3-on, 2-tert-Butyl-4-(4-Methylphenyl)-1H-pyrazol-3-on, 2-Phenyl-4-(4-Methylphenyl)-1H-pyrazol-3-on, 2-(4-Methylphenyl)-4-(4-Methylphenyl)-1H-pyrazol-3-on, 2-(2-Methylphenyl)-4-(4-Methylphenyl)-1H-pyrazol-3-on, 2-(3-Methylphenyl)-4-(4-Methylphenyl)-1H-pyrazol-3-on, 2-(4-Methoxy-4-(4-Methylphenyl)-1H-pyrazol-3-on, 2-Methyl-5-phenyl-1H-pyrazol-3-on, 2-Ethyl-5-phenyl-1H-pyrazol-3-on, 2-Propyl-5-phenyl-1H-pyrazol-3-on, 2-Isopropyl-5-phenyl-1H-pyrazol-3-on,2-Butyl-5-phenyl-1H-pyrazol-3-on, 2-Isobutyl-5-phenyl-1H-pyrazol-3-on, 2-tert-Butyl-5-phenyl-1H-pyrazol-3-on, 2-Phenyl-5-phenyl-1H-pyrazol-3-on, 2-(4-Methylphenyl)-5-phenyl-1H-pyrazol-3-on, 2-(2-Methylphenyl)-5-phenyl-1H-pyrazol-3-on, 2-(3-Methylphenyl)-5-phenyl-1H-pyrazol-3-on, 2-(4-Methoxy-5-phenyl-1H-pyrazol-3-on, 2-Methyl-5-methyl-1H-pyrazol-3-on, 2-Ethyl-5-methyl-1H-pyrazol-3-on, 2-Propyl-5-methyl-1H-pyrazol-3-on, 2-Isopropyl-5-methyl-1H-pyrazol-3-on, 2-Butyl-5-methyl-1H-pyrazol-3-on, 2-Isobutyl-5-methyl -1H-pyrazol-3-on, 2-tert-Butyl-5-methyl-1H-pyrazol-3-on, 2-Phenyl-5-methyl-1H-pyrazol-3-on, 2-(4-Methylphenyl)-5-methyl-1H-pyrazol-3-on, 2-(2-Methylphenyl)-5-methyl-1H-pyrazol-3-on, 2-(3-Methylphenyl)-5-methyl-1H-pyrazol-3-on, 2-(4-Methoxy-5-methyl-1H-pyrazol-3-on, 2-methyl-5-ethyl-1H-pyrazol-3-on, 2-ethyl-5-ethyl-1H-pyrazol-3-on, 2-propyl-5-ethyl-1H-pyrazol-3-on, 2-isopropyl-5-ethyl-1H-pyrazol-3-on, 2-butyl-5-ethyl-1H-pyrazol-3-on, 2-Isobutyl-5-ethyl-1H-pyrazol-3-on, 2-tert-butyl-5-ethyl-1H-pyrazol-3-on, 2-Phenyl-5-ethyl-1H-pyrazol-3-on, 2-(4-Methylphenyl)-5-ethyl-1H-pyrazol-3-on, 2-(2-Methylphenyl)-5-ethyl-1H-pyrazol-3-on, 2-(3-Methylphenyl)-5-ethyl-1H-pyrazol-3-on, 2-(4-Methoxy-5-ethyl-1H-pyrazol-3-on, 2-Methyl-5-propyl-1H-pyrazol-3-on, 2-Ethyl-5-propyl-1H-pyrazol-3-on,2-Propyl-5-propyl-1H-pyrazol-3-on, 2-Isopropyl-5-methy-1H-pyrazol-3-on, 2-Butyl-5-propyl-1H-pyrazol-3-on, 2-Isobutyl-5-propyl-1H-pyrazol-3-on, 2-tert-Butyl-5-propyl-1H-pyrazol-3-on, 2-Phenyl-5-propyl-1H-pyrazol-3-on, 2-(4-Methylphenyl)-5-propyl-1H-pyrazol-3-on, 2-(2-Methylphenyl)-5-propyl-1H-pyrazol-3-on, 2-(3-Methylphenyl)-5-propyl-1H-pyrazol-3-on, 2-(4-Methoxy-5-propyl-1H-pyrazol-3-on, 2-Methyl-5-isopropyl-1H-pyrazol-3-on, 2-Ethyl-5-isopropyl-1H-pyrazol-3-on, 2-Propyl-5-isopropyl-1H-pyrazol-3-on, 2-Isopropyl-5-isopropyl-1H-pyrazol-3-on, 2-Butyl-5-isopropyl-1H-pyrazol-3-on, 2-Isobutyl-5-isopropyl-1H-pyrazol-3-on, 2-tert-Butyl-5-isopropyl-1H-pyrazol-3-on, 2-Phenyl-5-isopropyl-1H-pyrazol-3-on, 2-(4-Methylphenyl)-5-isopropyl-1H-pyrazol-3-on, 2-(2-Methylphenyl)-5-isopropyl-1H-pyrazol-3-on, 2-(3-Methylphenyl)-5-isopropyl-1H-pyrazol-3-on, 2-(4-Methoxy-5-isopropyl-1H-pyrazol-3-on,
2-Methyl-5-butyl-1H-pyrazol-3-on, 2-Ethyl-5-butyl-1H-pyrazol-3-on, 2-Propyl-5-butyl-1H-pyrazol-3-on, 2-Isopropyl-5-butyl-1H-pyrazol-3-on, 2-Butyl-5-butyl-1H-pyrazol-3-on, 2-Isobutyl-5-butyl-1H-pyrazol-3-on, 2-tert-Butyl-5-butyl-1H-pyrazol-3-on, 2-Phenyl-5-butyl-1H-pyrazol-3-on, 2-(4-Methylphenyl)-5-butyl-1H-pyrazol-3-on, 2-(2-Methylphenyl)-5-butyl-1H-pyrazol-3-on, 2-(3-Methylphenyl)-5-butyl-1H-pyrazol-3-on, 2-(4-Methoxy-5-butyl-1H-pyrazol-3-on, 2-Methyl-5-isobutyl-1H-pyrazol-3-on, 2-Ethyl-5-isobutyl-1H-pyrazol-3-on, 2-Propyl-5-isobutyl-1H-pyrazol-3-on, 2-Isopropyl-5-isobutyl-1H-pyrazol-3-on, 2-Butyl-5-isobutyl-1H-pyrazol-3-on, 2-Isobutyl-5-isobutyl-1H-pyrazol-3-on, 2-tert-Butyl-5-isobutyl-1H-pyrazol-3-on, 2-Phenyl-5-isobutyl-1H-pyrazol-3-on, 2-(4-Methylphenyl)-5-isobutyl-1H-pyrazol-3-on, 2-(2-Methylphenyl)-5-isobutyl-1H-pyrazol-3-on, 2-(3-Methylphenyl)-5-isobutyl -1H-pyrazol-3-on, 2-(4-Methoxy-5-isobutyl-1H-pyrazol-3-on, 2-Methyl-5-tert-butyl-1H-pyrazol-3-on, 2-Ethyl-5-tert-butyl-1H-pyrazol-3-on, 2-Propyl-5-tert-butyl-1H-pyrazol-3-on, 2-Isopropyl-5-tert-butyl-1H-pyrazol-3-on, 2-Butyl-5-tert-butyl-1H-pyrazol-3-on, 2-Isobutyl-5-tert-butyl-1H-pyrazol-3-on, 2-tert-Butyl-5-tert-butyl-1H-pyrazol-3-on, 2-Phenyl-5-tert-butyl-1H-pyrazol-3-on, 2-(4-Methylphenyl)-5-tert-butyl-1H-pyrazol-3-on, 2-(2-Methylphenyl)-5-tert-butyl-1H-pyrazol-3-on, 2-(3-Methylphenyl)-5-tert-butyl-1H-pyrazol-3-on, 2-(4-Methoxy-5-phenyl-1H-pyrazol-3-on, 2-Methyl-5-phenyl-1H-pyrazol-3-on, 2-Ethyl-5-phenyl-1H-pyrazol-3-on, 2-Propyl-5-phenyl-1H-pyrazol-3-on, 2-Isopropyl-5-phenyl-1H-pyrazol-3-on, 2-Butyl-5-phenyl-1H-pyrazol-3-on, 2-Isobutyl-5-phenyl-1H-pyrazol-3-on, 2-tert-Butyl-5-phenyl-1H-pyrazol-3-on, 2-Phenyl-5-phenyl-1H-pyrazol-3-on, 2-(4-Methylphenyl)-5-phenyl -1H-pyrazol-3-on, 2-(2-Methylphenyl)-5-phenyl-1H-pyrazol-3-on, 2-(3-Methylphenyl)-5-phenyl-1H-pyrazol-3-on, 2-(4-Methoxy-5-(2-Methylphenyl)-1H-pyrazol-3-on, 2-Methyl-5-(2-Methylphenyl)-1H-pyrazol-3-on, 2-Ethyl-5-(2-Methylphenyl)-1H-pyrazol-3-on, 2-Propyl-5-(2-Methylphenyl)-1H-pyrazol-3-on, 2-Isopropyl-5-(2-Methylphenyl)-1H-pyrazol-3-on, 2-Butyl-5-(2-Methylphenyl)-1H-pyrazol-3-on, 2-Isobutyl-5-(2-Methylphenyl)-1H-pyrazol-3-on, 2-tert-Butyl-5-(2-Methylphenyl)-1H-pyrazol-3-on, 2-Phenyl-5-(2-Methylphenyl)-1H-pyrazol-3-on, 2-(4-Methylphenyl)-5-(2-Methylphenyl)-1H-pyrazol-3-on, 2-(2-Methylphenyl)-5-(2-Methylphenyl)-1H-pyrazol-3-on, 2-(3-Methylphenyl)-5-(2-Methylphenyl)-1H-pyrazol-3-on, 2-(4-Methoxy-5-(3-Methylphenyl)-1H-pyrazol-3-on, 2-Methyl-5-(3-Methylphenyl)-1H-pyrazol-3-on, 2-Ethyl-5-(3-Methylphenyl)-1H-pyrazol-3-on, 2-Propyl-5-(3-Methylphenyl)-1H-pyrazol-3-on, 2-Isopropyl-5-(3-Methylphenyl)-1H-pyrazol-3-on, 2-Butyl-5-(3-Methylphenyl)-1H-pyrazol-3-on, 2-Isobutyl-5-(3-Methylphenyl)-1H-pyrazol-3-on, 2-tert-Butyl-5-(3-Methyl-phenyl)-1H-pyrazol-3-on, 2-Phenyl-5-(3-Methylphenyl)-1H-pyrazol-3-on, 2-(4-Methyl-phenyl)-5-(3-Methylphenyl)-1H-pyrazol-3-on, 2-(2-Methylphenyl)-5-(3-Methylphenyl)-1H-pyrazol-3-on, 2-(3-Methylphenyl)-5-(3-Methylphenyl)-1H-pyrazol-3-on
2-(4-Methoxy-5-(3-Methylphenyl)-1H-pyrazol-3-on, 2-Methyl-5-(3-Methylphenyl)-1H-pyrazol-3-on, 2-Ethyl-5-(3-Methylphenyl)-1H-pyrazol-3-on, 2-Propyl-5-(3-Methylphenyl)-1H-pyrazol-3-on, 2-Isopropyl-5-(3-Methylphenyl)-1H-pyrazol-3-on, 2-Butyl-5-(3-Methylphenyl)-1H-pyrazol-3-on, 2-Isobutyl-5-(3-Methylphenyl)-1H-pyrazol-3-on,
2-tert-Butyl-5-butyl-1H-pyrazol-3-on, 2-Phenyl-5-(3-Methylphenyl)-1H-pyrazol-3-on, 2-(4-Methylphenyl)-5-(3-Methylphenyl)-1H-pyrazol-3-on, 2-(2-Methylphenyl)-5-(3-Methylphenyl) -1H-pyrazol-3-on, 2-(3-Methylphenyl)-5-(3-Methylphenyl)-1H-pyrazol-3-on, 2-(4-Methoxy-5-butyl-1H-pyrazol-3-on, 2-Methyl-5-(4-Methylphenyl)-1H-pyrazol-3-on, 2-Ethyl-5-(4-Methylphenyl)-1H-pyrazol-3-on, 2-Propyl-5-(4-Methylphenyl)-1H-pyrazol-3-on, 2-Isopropyl-5-(4-Methylphenyl)-1H-pyrazol-3-on, 2-Butyl-5-(4-Methylphenyl)-1H-pyrazol-3-on, 2-Isobutyl-5-(4-Methylphenyl)-1H-pyrazol-3-on, 2-tert-Butyl-5-(4-Methylphenyl)-1H-pyrazol-3-on, 2-Phenyl-5-(4-Methylphenyl)-1H-pyrazol-3-on, 2-(4-Methylphenyl)-5-(4-Methylphenyl)-1H-pyrazol-3-on, 2-(2-Methylphenyl)-5-(4-Methylphenyl)-1H-pyrazol-3-on, 2-(3-Methylphenyl)-5-(4-Methylphenyl)-1H-pyrazol-3-on, 2-(4-Methoxy-5-(4-Methylphenyl)-1H-pyrazol-3-on, 2-Methyl-4-methyl-5-phenyl-1H-pyrazol-3-on, 2-Ethyl-4-methyl5-phenyl-1H-pyrazol-3-on, 2-Propyl-4-methyl-5-phenyl-1H-pyrazol-3-on, 2-Isopropyl-4-methyl-5-phenyl-1H-pyrazol-3-on, 2-Butyl-4-methyl-5-phenyl-1H-pyrazol-3-on, 2-Isobutyl-4-methyl-5-phenyl-1H-pyrazol-3-on, 2-tert-Butyl-4-methyl-5-phenyl-1H-pyrazol-3-on, 2-Phenyl-4-methyl-5-phenyl-1H-pyrazol-3-on, 2-(4-Methylphenyl)-4-methyl-5-phenyl-1H-pyrazol-3-on, 2-(2-Methylphenyl)-4-methyl-5-phenyl-1H-pyrazol-3-on, 2-(3-Methylphenyl)-4-methyl-5-phenyl-1H-pyrazol-3-on, 2-(4-Methoxy-4-methyl-5-phenyl-1H-pyrazol-3-on, 2-Methyl-4-methyl-5-methyl-1H-pyrazol-3-on, 2-Ethyl-4-methyl-5-methyl-1H-pyrazol-3-on, 2-Propyl-4-methyl-5-methyl-1H-pyrazol-3-on, 2-Isopropyl-4-methyl-5-methyl-1H-pyrazol-3-on, 2-Butyl-4-methyl-5-methyl-1H-pyrazol-3-on, 2-Isobutyl-4-methyl-5-methyl-1H-pyrazol-3-on, 2-tert-Butyl-4-methyl-5-methyl-1H-pyrazol-3-on, 2-Phenyl-4-methyl-5-methyl-1H-pyrazol-3-on, 2-(4-Methylphenyl)-4-methyl-5-methyl-1H-pyrazol-3-on, 2-(2-Methylphenyl)-4-methyl-5-methyl-1H-pyrazol-3-on, 2-(3-Methyl-phenyl)-4-methyl-5-methyl-1H-pyrazol-3-on, 2-(4-Methoxy-4-methyl-5-methyl-1H-pyrazol-3-on, 2-methyl-4-methyl-5-ethyl-1H-pyrazol-3-on, 2-ethyl-4-methyl-5-ethyl-1H-pyrazol-3-on, 2-propyl-4-methyl-5-ethyl-1H-pyrazol-3-on, 2-isopropyl-4-methyl-5-ethyl-1H-pyrazol-3-on, 2-butyl-4-methyl-5-ethyl-1H-pyrazol-3-on, 2-Isobutyl-4-methyl-5-ethyl-1H-pyrazol-3-on, 2-tert-butyl-4-methyl-5-ethyl-1H-pyrazol-3-on, 2-Phenyl-4-methyl-5-ethyl-1H-pyrazol-3-on, 2-(4-Methylphenyl)-4-methyl-5-ethyl-1H-pyrazol-3-on, 2-(2-Methylphenyl)-4-methyl-5-ethyl-1H-pyrazol-3-on, 2-(3-Methylphenyl)-4-methyl-5-ethyl-1H-pyrazol-3-on, 2-(4-Methoxy-4-methyl-5-ethyl-1H-pyrazol-3-on, 2-Methyl-4-methyl-5-propyl-1H-pyrazol-3-on, 2-Ethyl-4-methyl-5-propyl-1H-pyrazol-3-on, 2-Propyl-4-methyl-5-propyl-1H-pyrazol-3-on, 2-Isopropyl-4-methyl-5-methyl-1H-pyrazol-3-on, 2-Butyl-4-methyl-5-propyl-1H-pyrazol-3-on, 2-Isobutyl-4-methyl-5-propyl-1H-pyrazol-3-on, 2-tert-Butyl-4-methyl-5-propyl-1H-pyrazol-3-on, 2-Phenyl-4-methyl-5-propyl-1H-pyrazol-3-on, 2-(4-Methylphenyl)-4-methyl-5-propyl-1H-pyrazol-3-on, 2-(2-Methylphenyl)-4-methyl-5-propyl-1H-pyrazol-3-on, 2-(3-Methylphenyl)-4-methyl-5-propyl-1H-pyrazol-3-on, 2-(4-Methoxy-4-methyl-5-propyl-1H-pyrazol-3-on, 2-Methyl-4-methyl-5-isopropyl-1H-pyrazol-3-on, 2-Ethyl-4-methyl-5-isopropyl-1H-pyrazol-3-on, 2-Propyl-4-methyl-5-isopropyl-1H-pyrazol-3-on, 2-Isopropyl-4-methyl-5-isopropyl-1H-pyrazol-3-on, 2-Butyl-4-methyl-5-isopropyl-1H-pyrazol-3-on, 2-Isobutyl-4-methyl-5-isopropyl-1H-pyrazol-3-on, 2-tert-Butyl-4-methyl-5-isopropyl-1H-pyrazol-3-on, 2-Phenyl-4-methyl-5-isopropyl-1H-pyrazol-3-on, 2-(4-Methylphenyl)-4-methyl-5-isopropyl-1H-pyrazol-3-on, 2-(2-Methylphenyl)-4-methyl-5-isopropyl-1H-pyrazol-3-on, 2-(3-Methylphenyl)-4-methyl-5-isopropyl-1H-pyrazol-3-on, 2-(4-Methoxy-4-methyl-5-isopropyl-1H-pyrazol-3-on, 2-Methyl-4-methyl-5-butyl-1H-pyrazol-3-on, 2-Ethyl-4-methyl-5-butyl-1H-pyrazol-3-on, 2-Propyl-4-methyl-5-butyl-1H-pyrazol-3-on, 2-Isopropyl-4-methyl-5-butyl-1H-pyrazol-3-on, 2-Butyl-4-methyl-5-butyl-1H-pyrazol-3-on, 2-Isobutyl-4-methyl-5-butyl-1H-pyrazol-3-on, 2-tert-Butyl-4-methyl-5-butyl-1H-pyrazol-3-on, 2-Phenyl-4-methyl-5-butyl-1H-pyrazol-3-on, 2-(4-Methylphenyl)-4-methyl-5-butyl-1H-pyrazol-3-on, 2-(2-Methylphenyl)-4-methyl-5-butyl-1H-pyrazol-3-on, 2-(3-Methylphenyl)-4-methyl-5-butyl-1H-pyrazol-3-on, 2-(4-Methoxy-4-methyl-5-butyl-1H-pyrazol-3-on,
2-Methyl-4-methyl-5-isobutyl-1H-pyrazol-3-on, 2-Ethyl-4-methyl-5-isobutyl-1H-pyrazol-3-on, 2-Propyl-4-methyl-5-isobutyl-1H-pyrazol-3-on, 2-Isopropyl-4-methyl-5-isobutyl-1H-pyrazol-3-on, 2-Butyl-4-methyl-5-isobutyl-1H-pyrazol-3-on, 2-Isobutyl-4-methyl-5-isobutyl-1H-pyrazol-3-on, 2-tert-Butyl-4-methyl-5-isobutyl-1H-pyrazol-3-on, 2-Phenyl-4-methyl-5-isobutyl-1H-pyrazol-3-on, 2-(4-Methylphenyl)-4-methyl-5-isobutyl-1H-pyrazol-3-on, 2-(2-Methylphenyl)-4-methyl-5-isobutyl-1H-pyrazol-3-on, 2-(3-Methylphenyl)-4-methyl-5-isobutyl -1H-pyrazol-3-on, 2-(4-Methoxy-4-methyl-5-isobutyl-1H-pyrazol-3-on, 2-Methyl-4-methyl-5-tert-butyl-1H-pyrazol-3-on, 2-Ethyl-4-methyl-5-tert-butyl-1H-pyrazol-3-on, 2-Propyl-4-methyl-5-tert-butyl-1H-pyrazol-3-on, 2-Isopropyl-4-methyl-5-tert-butyl-1H-pyrazol-3-on, 2-Butyl-4-methyl-5-tert-butyl-1H-pyrazol-3-on, 2-Isobutyl-4-methyl-5-tert-butyl-1H-pyrazol-3-on, 2-tert-Butyl-4-methyl-5-tert-butyl-1H-pyrazol-3-on, 2-Phenyl-4-methyl-5-tert-butyl-1H-pyrazol-3-on, 2-(4-Methylphenyl)-4-methyl-5-tert-butyl-1H-pyrazol-3-on, 2-(2-Methylphenyl)-4-methyl-5-tert-butyl-1H-pyrazol-3-on, 2-(3-Methylphenyl)-4-methyl-5-tert-butyl-1H-pyrazol-3-on,
2-(4-Methoxy-4-methyl-5-phenyl-1H-pyrazol-3-on, 2-Methyl-4-methyl-5-phenyl-1H-pyrazol-3-on, 2-Ethyl-4-methyl-5-phenyl-1H-pyrazol-3-on, 2-Propyl-4-methyl-5-phenyl-1H-pyrazol-3-on, 2-Isopropyl-4-methyl-5-phenyl-1H-pyrazol-3-on, 2-Butyl-4-methyl-5-phenyl-1H-pyrazol-3-on, 2-Isobutyl-4-methyl-5-phenyl-1H-pyrazol-3-on, 2-tert-Butyl-4-methyl-5-phenyl -1H-pyrazol-3-on, 2-Phenyl-4-methyl-5-phenyl-1H-pyrazol-3-on, 2-(4-Methylphenyl)-4-methyl-5-phenyl-1H-pyrazol-3-on, 2-(2-Methylphenyl)-4-methyl-5-phenyl-1H-pyrazol-3-on, 2-(3-Methylphenyl)-4-methyl-5-phenyl-1H-pyrazol-3-on, 2-(4-Methoxy-4-methyl-5-(2-Methylphenyl)-1H-pyrazol-3-on, 2-Methyl-4-methyl-5-(2-Methylphenyl)-1H-pyrazol-3-on, 2-Ethyl-4-methyl-5-(2-Methylphenyl)-1H-pyrazol-3-on, 2-Propyl-4-methyl-5-(2-Methylphenyl)-1H-pyrazol-3-on, 2-Isopropyl-4-methyl-5-(2-Methylphenyl)-1H-pyrazol-3-on, 2-Butyl-4-methyl-5-(2-Methylphenyl)-1H-pyrazol-3-on, 2-Isobutyl-4-methyl-5-(2-Methylphenyl)-1H-pyrazol-3-on, 2-tert-Butyl-4-methyl-5-(2-Methylphenyl)-1H-pyrazol-3-on, 2-Phenyl-4-methyl-5-(2-Methylphenyl)-1H-pyrazol-3-on, 2-(4-Methylphenyl)-4-methyl-5-(2-Methylphenyl)-1H-pyrazol-3-on, 2-(2-Methylphenyl)-4-methyl-5-(2-Methylphenyl)-1H-pyrazol-3-on, 2-(3-Methylphenyl)-4-methyl-5-(2-Methylphenyl)-1H-pyrazol-3-on, 2-(4-Methoxy-4-methyl-5-(3-Methylphenyl)-1H-pyrazol-3-on, 2-Methyl-4-methyl-5-(3-Methylphenyl)-1H-pyrazol-3-on, 2-Ethyl-4-methyl-5-(3-Methylphenyl)-1H-pyrazol-3-on, 2-Propyl-4-methyl-5-(3-Methylphenyl)-1H-pyrazol-3-on, 2-Isopropyl-4-methyl-5-(3-Methylphenyl)-1H-pyrazol-3-on, 2-Butyl-4-methyl-5-(3-Methylphenyl)-1H-pyrazol-3-on, 2-Isobutyl-4-methyl-5-(3-Methylphenyl)-1H-pyrazol-3-on, 2-tert-Butyl-4-methyl-5-(3-Methylphenyl)-1H-pyrazol-3-on, 2-Phenyl-4-methyl-5-(3-Methylphenyl)-1H-pyrazol-3-on, 2-(4-Methylphenyl)-4-methyl-5-(3-Methylphenyl)-1H-pyrazol-3-on, 2-(2-Methylphenyl)-4-methyl-5-(3-Methylphenyl)-1H-pyrazol-3-on, 2-(3-Methylphenyl)-4-methyl-5-(3-Methylphenyl)-1H-pyrazol-3-on, 2-(4-Methoxy-4-methyl-5-(3-Methylphenyl)-1H-pyrazol-3-on, 2-Methyl-4-methyl-5-(3-Methylphenyl)-1H-pyrazol-3-on, 2-Ethyl-4-methyl-5-(3-Methylphenyl)-1H-pyrazol-3-on, 2-Propyl-4-methyl-5-(3-Methylphenyl)-1H-pyrazol-3-on, 2-Isopropyl-4-methyl-5-(3-Methylphenyl)-1H-pyrazol-3-on, 2-Butyl-4-methyl-5-(3-Methylphenyl)-1H-pyrazol-3-on, 2-Isobutyl-4-methyl-5-(3-Methylphenyl)-1H-pyrazol-3-on, 2-tert-Butyl-4-methyl-5-butyl-1H-pyrazol-3-on, 2-Phenyl-4-methyl-5-(3-Methyl-phenyl)-1H-pyrazol-3-on, 2-(4-Methylphenyl)-4-methyl-5-(3-Methylphenyl)-1H-pyrazol-3-on, 2-(2-Methylphenyl)-4-methyl-5-(3-Methylphenyl)-1H-pyrazol-3-on, 2-(3-Methylphenyl)-4-methyl-5-(3-Methylphenyl)-1H-pyrazol-3-on, 2-(4-Methoxy-4-methyl-5-butyl-1H-pyrazol-3-on, 2-Methyl-4-methyl-5-(4-Methylphenyl)-1H-pyrazol-3-on, 2-Ethyl-4-methyl-5-(4-Methyl-phenyl)-1H-pyrazol-3-on, 2-Propyl-4-methyl-5-(4-Methylphenyl)-1H-pyrazol-3-on, 2-Isopropyl-4-methyl-5-(4-Methylphenyl)-1H-pyrazol-3-on, 2-Butyl-4-methyl-5-(4-Methyl-phenyl)-1H-pyrazol-3-on, 2-Isobutyl-4-methyl-5-(4-Methylphenyl)-1H-pyrazol-3-on, 2-tert-Butyl-4-methyl-5-(4-Methylphenyl)-1H-pyrazol-3-on, 2-Phenyl-4-methyl-5-(4-Methylphenyl) -1H-pyrazol-3-on, 2-(4-Methylphenyl)-4-methyl-5-(4-Methylphenyl)-1H-pyrazol-3-on, 2-(2-Methylphenyl)-4-methyl-5-(4-Methylphenyl)-1H-pyrazol-3-on, 2-(3-Methylphenyl)-4-methyl-5-(4-Methylphenyl)-1H-pyrazol-3-on, 2-(4-Methoxy-4-methyl-5-(4-Methylphenyl)-1H-pyrazol-3-on, 2-Methyl-4-ethyl-5-phenyl-1H-pyrazol-3-on, 2-Ethyl-4-ethyl5-phenyl-1H-pyrazol-3-on, 2-Propyl-4-ethyl-5-phenyl-1H-pyrazol-3-on, 2-Isopropyl-4-ethyl-5-phenyl-1H-pyrazol-3-on, 2-Butyl-4-ethyl-5-phenyl-1H-pyrazol-3-on, 2-Isobutyl-4-ethyl-5-phenyl-1H-pyrazol-3-on, 2-tert-Butyl-4-ethyl-5-phenyl-1H-pyrazol-3-on, 2-Phenyl-4-ethyl-5-phenyl-1H-pyrazol-3-on, 2-(4-Methylphenyl)-4-ethyl-5-phenyl-1H-pyrazol-3-on, 2-(2-Methylphenyl)-4-ethyl-5-phenyl-1H-pyrazol-3-on, 2-(3-Methylphenyl)-4-ethyl-5-phenyl-1H-pyrazol-3-on, 2-(4-Methoxy-4-ethyl-5-phenyl-1H-pyrazol-3-on, 2-Methyl-4-ethyl-5-methyl-1H-pyrazol-3-on, 2-Ethyl-4-ethyl-5-methyl-1H-pyrazol-3-on, 2-Propyl-4-ethyl-5-methyl-1H-pyrazol-3-on, 2-Isopropyl-4-ethyl-5-methyl-1H-pyrazol-3-on, 2-Butyl-4-ethyl-5-methyl-1H-pyrazol-3-on, 2-Isobutyl-4-ethyl-5-methyl-1H-pyrazol-3-on, 2-tert-Butyl-4-ethyl-5-methyl-1H-pyrazol-3-on, 2-Phenyl-4-ethyl-5-methyl-1H-pyrazol-3-on, 2-(4-Methylphenyl)-4-ethyl-5-methyl-1H-pyrazol-3-on, 2-(2-Methylphenyl)-4-ethyl-5-methyl-1H-pyrazol-3-on, 2-(3-Methylphenyl)-4-ethyl-5-methyl-1H-pyrazol-3-on, 2-(4-Methoxy-4-ethyl-5-methyl-1H-pyrazol-3-on, 2-methyl-4-ethyl-5-ethyl-1H-pyrazol-3-on, 2-ethyl-4-ethyl-5-ethyl-1H-pyrazol-3-on, 2-propyl-4-ethyl-5-ethyl-1H-pyrazol-3-on, 2-isopropyl-4-ethyl-5-ethyl-1H-pyrazol-3-on, 2-butyl-4-ethyl-5-ethyl-1H-pyrazol-3-on, 2-Isobutyl-4-ethyl-5-ethyl-1H-pyrazol-3-on, 2-tert-butyl-4-ethyl-5-ethyl-1H-pyrazol-3-on, 2-Phenyl-4-ethyl-5-ethyl-1H-pyrazol-3-on, 2-(4-Methylphenyl)-4-ethyl-5-ethyl-1H-pyrazol-3-on, 2-(2-Methylphenyl)-4-ethyl-5-ethyl-1H-pyrazol-3-on, 2-(3-Methyl-phenyl)-4-ethyl-5-ethyl-1H-pyrazol-3-on, 2-(4-Methoxy-4-ethyl-5-ethyl-1H-pyrazol-3-on, 2-Methyl-4-ethyl-5-propyl-1H-pyrazol-3-on, 2-Ethyl-4-ethyl-5-propyl-1H-pyrazol-3-on, 2-Propyl-4-ethyl-5-propyl-1H-pyrazol-3-on, 2-Isopropyl-4-ethyl-5-methyl-1H-pyrazol-3-on, 2-Butyl-4-ethyl-5-propyl-1H-pyrazol-3-on, 2-Isobutyl-4-ethyl-5-propyl-1H-pyrazol-3-on, 2-tert-Butyl-4-ethyl-5-propyl-1H-pyrazol-3-on, 2-Phenyl-4-ethyl-5-propyl-1H-pyrazol-3-on, 2-(4-Methylphenyl)-4-ethyl-5-propyl-1H-pyrazol-3-on, 2-(2-Methylphenyl)-4-ethyl-5-propyl-1H-pyrazol-3-on, 2-(3-Methylphenyl)-4-ethyl-5-propyl-1H-pyrazol-3-on, 2-(4-Methoxy-4-ethyl-5-propyl-1H-pyrazol-3-on, 2-Methyl-4-ethyl-5-isopropyl-1H-pyrazol-3-on, 2-Ethyl-4-ethyl-5-isopropyl-1H-pyrazol-3-on, 2-Propyl-4-ethyl-5-isopropyl-1H-pyrazol-3-on, 2-Isopropyl-4-ethyl-5-isopropyl-1H-pyrazol-3-on, 2-Butyl-4-ethyl-5-isopropyl-1H-pyrazol-3-on, 2-Isobutyl-4-ethyl-5-isopropyl-1H-pyrazol-3-on, 2-tert-Butyl-4-ethyl-5-isopropyl-1H-pyrazol-3-on, 2-Phenyl-4-ethyl-5-isopropyl-1H-pyrazol-3-on, 2-(4-Methylphenyl)-4-ethyl-5-isopropyl-1H-pyrazol-3-on, 2-(2-Methylphenyl)-4-ethyl-5-isopropyl-1H-pyrazol-3-on, 2-(3-Methylphenyl)-4-ethyl-5-isopropyl-1H-pyrazol-3-on, 2-(4-Methoxy-4-ethyl-5-isopropyl-1H-pyrazol-3-on, 2-Methyl-4-ethyl-5-butyl-1H-pyrazol-3-on, 2-Ethyl-4-ethyl-5-butyl-1H-pyrazol-3-on, 2-Propyl-4-ethyl-5-butyl-1H-pyrazol-3-on, 2-Isopropyl-4-ethyl-5-butyl-1H-pyrazol-3-on, 2-Butyl-4-ethyl-5-butyl-1H-pyrazol-3-on, 2-Isobutyl-4-ethyl-5-butyl-1H-pyrazol-3-on, 2-tert-Butyl-4-ethyl-5-butyl-1H-pyrazol-3-on, 2-Phenyl-4-ethyl-5-butyl-1H-pyrazol-3-on, 2-(4-Methylphenyl)-4-ethyl-5-butyl-1H-pyrazol-3-on, 2-(2-Methylphenyl)-4-ethyl-5-butyl-1H-pyrazol-3-on, 2-(3-Methylphenyl)-4-ethyl-5-butyl-1H-pyrazol-3-on, 2-(4-Methoxy-4-ethyl-5-butyl-1H-pyrazol-3-on,
2-Methyl-4-ethyl-5-isobutyl-1H-pyrazol-3-on, 2-Ethyl-4-ethyl-5-isobutyl-1H-pyrazol-3-on, 2-Propyl-4-ethyl-5-isobutyl-1H-pyrazol-3-on, 2-Isopropyl-4-ethyl-5-isobutyl-1H-pyrazol-3-on, 2-Butyl-4-ethyl-5-isobutyl-1H-pyrazol-3-on, 2-Isobutyl-4-ethyl-5-isobutyl-1H-pyrazol-3-on,
2-tert-Butyl-4-ethyl-5-isobutyl-1H-pyrazol-3-on, 2-Phenyl-4-ethyl-5-isobutyl-1H-pyrazol-3-on, 2-(4-Methylphenyl)-4-ethyl-5-isobutyl-1H-pyrazol-3-on, 2-(2-Methylphenyl)-4-ethyl-5-isobutyl-1H-pyrazol-3-on, 2-(3-Methylphenyl)-4-ethyl-5-isobutyl-1H-pyrazol-3-on, 2-(4-Methoxy-4-ethyl-5-isobutyl-1H-pyrazol-3-on, 2-Methyl-4-ethyl-5-tert-butyl-1H-pyrazol-3-on, 2-Ethyl-4-ethyl-5-tert-butyl-1H-pyrazol-3-on, 2-Propyl-4-ethyl-5-tert-butyl-1H-pyrazol-3-on, 2-Isopropyl-4-ethyl-5-tert-butyl-1H-pyrazol-3-on, 2-Butyl-4-ethyl-5-tert-butyl-1H-pyrazol-3-on, 2-Isobutyl-4-ethyl-5-tert-butyl-1H-pyrazol-3-on, 2-tert-Butyl-4-ethyl-5-tert-butyl-1H-pyrazol-3-on, 2-Phenyl-4-ethyl-5-tert-butyl-1H-pyrazol-3-on, 2-(4-Methylphenyl)-4-ethyl-5-tert-butyl-1H-pyrazol-3-on, 2-(2-Methylphenyl)-4-ethyl-5-tert-butyl-1H-pyrazol-3-on, 2-(3-Methylphenyl)-4-ethyl-5-tert-butyl-1H-pyrazol-3-on, 2-(4-Methoxy-4-ethyl-5-phenyl-1H-pyrazol-3-on, 2-Methyl-4-ethyl-5-phenyl-1H-pyrazol-3-on, 2-Ethyl-4-ethyl-5-phenyl-1H-pyrazol-3-on, 2-Propyl-4-ethyl-5-phenyl-1H-pyrazol-3-on, 2-Isopropyl-4-ethyl-5-phenyl-1H-pyrazol-3-on, 2-Butyl-4-ethyl-5-phenyl-1H-pyrazol-3-on, 2-Isobutyl-4-ethyl-5-phenyl-1H-pyrazol-3-on, 2-tert-Butyl-4-ethyl-5-phenyl-1H-pyrazol-3-on, 2-Phenyl-4-ethyl-5-phenyl-1H-pyrazol-3-on, 2-(4-Methylphenyl)-4-ethyl-5-phenyl-1H-pyrazol-3-on, 2-(2-Methylphenyl)-4-ethyl-5-phenyl-1H-pyrazol-3-on, 2-(3-Methylphenyl)-4-ethyl-5-phenyl-1H-pyrazol-3-on, 2-(4-Methoxy-4-ethyl-5-(2-Methylphenyl)-1H-pyrazol-3-on,
2-Methyl-4-ethyl-5-(2-Methylphenyl)-1H-pyrazol-3-on, 2-Ethyl-4-ethyl-5-(2-Methylphenyl)-1H-pyrazol-3-on, 2-Propyl-4-ethyl-5-(2-Methylphenyl)-1H-pyrazol-3-on, 2-Isopropyl-4-ethyl-5-(2-Methylphenyl)-1H-pyrazol-3-on, 2-Butyl-4-ethyl-5-(2-Methylphenyl)-1H-pyrazol-3-on, 2-Isobutyl-4-ethyl-5-(2-Methylphenyl)-1H-pyrazol-3-on, 2-tert-Butyl-4-ethyl-5-(2-Methyl-phenyl)-1H-pyrazol-3-on, 2-Phenyl-4-ethyl-5-(2-Methylphenyl)-1H-pyrazol-3-on,2-(4-Methylphenyl)-4-ethyl-5-(2-Methylphenyl)-1H-pyrazol-3-on, 2-(2-Methylphenyl)-4-ethyl-5-(2-Methylphenyl)-1H-pyrazol-3-on, 2-(3-Methylphenyl)-4-ethyl-5-(2-Methylphenyl)-1H-pyrazol-3-on, 2-(4-Methoxy-4-ethyl-5-(3-Methylphenyl)-1H-pyrazol-3-on, 2-Methyl-4-ethyl-5-(3-Methylphenyl)-1H-pyrazol-3-on, 2-Ethyl-4-ethyl-5-(3-Methylphenyl)-1H-pyrazol-3-on, 2-Propyl-4-ethyl-5-(3-Methylphenyl)-1H-pyrazol-3-on, 2-Isopropyl-4-ethyl-5-(3-Methylphenyl) -1H-pyrazol-3-on, 2-Butyl-4-ethyl-5-(3-Methylphenyl)-1H-pyrazol-3-on, 2-Isobutyl-4-ethyl-5-(3-Methylphenyl)-1H-pyrazol-3-on, 2-tert-Butyl-4-ethyl-5-(3-Methylphenyl)-1H-pyrazol-3-on, 2-Phenyl-4-ethyl-5-(3-Methylphenyl)-1H-pyrazol-3-on, 2-(4-Methylphenyl)-4-ethyl-5-(3-Methylphenyl)-1H-pyrazol-3-on, 2-(2-Methylphenyl)-4-ethyl-5-(3-Methylphenyl)-1H-pyrazol-3-on, 2-(3-Methylphenyl)-4-ethyl-5-(3-Methylphenyl)-1H-pyrazol-3-on, 2-(4-Methoxy-4-ethyl-5-(3-Methylphenyl)-1H-pyrazol-3-on, 2-Methyl-4-ethyl-5-(3-Methylphenyl)-1H-pyrazol-3-on, 2-Ethyl-4-ethyl-5-(3-Methylphenyl)-1H-pyrazol-3-on, 2-Propyl-4-ethyl-5-(3-Methylphenyl)-1H-pyrazol-3-on, 2-Isopropyl-4-ethyl-5-(3-Methylphenyl)-1H-pyrazol-3-on, 2-Butyl-4-ethyl-5-(3-Methylphenyl)-1H-pyrazol-3-on, 2-Isobutyl-4-ethyl-5-(3-Methylphenyl)-1H-pyrazol-3-on, 2-tert-Butyl-4-ethyl-5-butyl-1H-pyrazol-3-on, 2-Phenyl-4-ethyl-5-(3-Methylphenyl)-1H-pyrazol-3-on, 2-(4-Methylphenyl)-4-ethyl-5-(3-Methylphenyl)-1H-pyrazol-3-on, 2-(2-Methyl-phenyl)-4-ethyl-5-(3-Methylphenyl)-1H-pyrazol-3-on, 2-(3-Methylphenyl)-4-ethyl-5-(3-Methylphenyl)-1H-pyrazol-3-on, 2-(4-Methoxy-4-ethyl-5-butyl-1H-pyrazol-3-on, 2-Methyl-4-ethyl-5-(4-Methylphenyl)-1H-pyrazol-3-on, 2-Ethyl-4-ethyl-5-(4-Methylphenyl)-1H-pyrazol-3-on, 2-Propyl-4-ethyl-5-(4-Methylphenyl)-1H-pyrazol-3-on, 2-Isopropyl-4-ethyl-5-(4-Methyl-phenyl)-1H-pyrazol-3-on, 2-Butyl-4-ethyl-5-(4-Methylphenyl)-1H-pyrazol-3-on, 2-Isobutyl-4-ethyl-5-(4-Methylphenyl)-1H-pyrazol-3-on, 2-tert-Butyl-4-ethyl-5-(4-Methylphenyl)-1H-pyrazol-3-on, 2-Phenyl-4-ethyl-5-(4-Methylphenyl)-1H-pyrazol-3-on, 2-(4-Methylphenyl)-4-ethyl-5-(4-Methylphenyl)-1H-pyrazol-3-on, 2-(2-Methylphenyl)-4-ethyl-5-(4-Methylphenyl)-1H-pyrazol-3-on, 2-(3-Methylphenyl)-4-ethyl-5-(4-Methylphenyl)-1H-pyrazol-3-on, 2-(4-Methoxy-4-ethyl-5-(4-Methylphenyl)-1H-pyrazol-3-on, 2-Methyl-4-propyl-5-phenyl-1H-pyrazol-3-on, 2-Ethyl-4-propyl5-phenyl-1H-pyrazol-3-on, 2-Propyl-4-propyl-5-phenyl-1H-pyrazol-3-on, 2-Isopropyl-4-propyl-5-phenyl-1H-pyrazol-3-on, 2-Butyl-4-propyl-5-phenyl-1H-pyrazol-3-on, 2-Isobutyl-4-propyl-5-phenyl-1H-pyrazol-3-on, 2-tert-Butyl-4-propyl-5-phenyl-1H-pyrazol-3-on, 2-Phenyl-4-propyl-5-phenyl-1H-pyrazol-3-on, 2-(4-Methylphenyl)-4-propyl-5-phenyl-1H-pyrazol-3-on, 2-(2-Methylphenyl)-4-propyl-5-phenyl-1H-pyrazol-3-on, 2-(3-Methylphenyl)-4-propyl-5-phenyl-1H-pyrazol-3-on, 2-(4-Methoxy-4-propyl-5-phenyl-1H-pyrazol-3-on, 2-Methyl-4-propyl-5-methyl-1H-pyrazol-3-on, 2-Ethyl-4-propyl-5-methyl-1H-pyrazol-3-on, 2-Propyl-4-propyl-5-methyl-1H-pyrazol-3-on, 2-Isopropyl-4-propyl-5-methyl-1H-pyrazol-3-on, 2-Butyl-4-propyl-5-methyl-1H-pyrazol-3-on, 2-Isobutyl-4-propyl-5-methyl-1H-pyrazol-3-on, 2-tert-Butyl-4-propyl-5-methyl-1H-pyrazol-3-on, 2-Phenyl-4-propyl-5-methyl-1H-pyrazol-3-on,2-(4-Methylphenyl)-4-propyl-5-methyl-1H-pyrazol-3-on,2-(2-Methylphenyl)-4-propyl-5-methyl-1H-pyrazol-3-on, 2-(3-Methylphenyl)-4-propyl-5-methyl-1H-pyrazol-3-on, 2-(4-Methoxy-4-propyl-5-methyl-1H-pyrazol-3-on, 2-methyl-4-propyl-5-ethyl-1H-pyrazol-3-on, 2-ethyl-4-propyl-5-ethyl-1H-pyrazol-3-on, 2-propyl-4-propyl-5-ethyl-1H-pyrazol-3-on, 2-isopropyl-4-propyl-5-ethyl-1H-pyrazol-3-on, 2-butyl-4-propyl-5-ethyl-1H-pyrazol-3-on, 2-Isobutyl-4-propyl-5-ethyl-1H-pyrazol-3-on, 2-tert-butyl-4-propyl-5-ethyl-1H-pyrazol-3-on, 2-Phenyl-4-propyl-5-ethyl-1H-pyrazol-3-on, 2-(4-Methylphenyl)-4-propyl-5-ethyl-1H-pyrazol-3-on, 2-(2-Methylphenyl)-4-propyl-5-ethyl-1H-pyrazol-3-on, 2-(3-Methyl-phenyl)-4-propyl-5-ethyl-1H-pyrazol-3-on, 2-(4-Methoxy-4-propyl-5-ethyl-1H-pyrazol-3-on, 2-Methyl-4-propyl-5-propyl-1H-pyrazol-3-on, 2-Ethyl-4-propyl-5-propyl-1H-pyrazol-3-on,2-Propyl-4-propyl-5-propyl-1H-pyrazol-3-on, 2-Isopropyl-4-propyl-5-methyl-1H-pyrazol-3-on, 2-Butyl-4-propyl-5-propyl-1H-pyrazol-3-on, 2-Isobutyl-4-propyl-5-propyl-1H-pyrazol-3-on, 2-tert-Butyl-4-propyl-5-propyl-1H-pyrazol-3-on, 2-Phenyl-4-propyl-5-propyl-1H-pyrazol-3-on, 2-(4-Methylphenyl)-4-propyl-5-propyl-1H-pyrazol-3-on, 2-(2-Methylphenyl)-4-propyl-5-propyl-1H-pyrazol-3-on, 2-(3-Methylphenyl)-4-propyl-5-propyl-1H-pyrazol-3-on, 2-(4-Methoxy-4-propyl-5-propyl-1H-pyrazol-3-on, 2-Methyl-4-propyl-5-isopropyl-1H-pyrazol-3-on, 2-Ethyl-4-propyl-5-isopropyl-1H-pyrazol-3-on, 2-Propyl-4-propyl-5-isopropyl-1H-pyrazol-3-on, 2-Isopropyl-4-propyl-5-isopropyl-1H-pyrazol-3-on, 2-Butyl-4-propyl-5-isopropyl-1H-pyrazol-3-on, 2-Isobutyl-4-propyl-5-isopropyl-1H-pyrazol-3-on, 2-tert-Butyl-4-propyl-5-isopropyl-1H-pyrazol-3-on, 2-Phenyl-4-propyl-5-isopropyl-1H-pyrazol-3-on, 2-(4-Methylphenyl)-4-propyl-5-isopropyl-1H-pyrazol-3-on, 2-(2-Methylphenyl)-4-propyl-5-isopropyl-1H-pyrazol-3-on, 2-(3-Methylphenyl)-4-propyl-5-isopropyl-1H-pyrazol-3-on, 2-(4-Methoxy-4-propyl-5-isopropyl-1H-pyrazol-3-on,
2-Methyl-4-propyl-5-butyl-1H-pyrazol-3-on, 2-Ethyl-4-propyl-5-butyl-1H-pyrazol-3-on, 2-Propyl-4-propyl-5-butyl-1H-pyrazol-3-on, 2-Isopropyl-4-propyl-5-butyl-1H-pyrazol-3-on, 2-Butyl-4-propyl-5-butyl-1H-pyrazol-3-on, 2-Isobutyl-4-propyl-5-butyl-1H-pyrazol-3-on, 2-tert-Butyl-4-propyl-5-butyl-1H-pyrazol-3-on, 2-Phenyl-4-propyl-5-butyl-1H-pyrazol-3-on, 2-(4-Methylphenyl)-4-propyl-5-butyl-1H-pyrazol-3-on, 2-(2-Methylphenyl)-4-propyl-5-butyl-1H-pyrazol-3-on, 2-(3-Methylphenyl)-4-propyl-5-butyl-1H-pyrazol-3-on, 2-(4-Methoxy-4-propyl-5-butyl-1H-pyrazol-3-on, 2-Methyl-4-propyl-5-isobutyl-1H-pyrazol-3-on, 2-Ethyl-4-propyl-5-isobutyl-1H-pyrazol-3-on, 2-Propyl-4-propyl-5-isobutyl-1H-pyrazol-3-on, 2-Isopropyl-4-propyl-5-isobutyl-1H-pyrazol-3-on, 2-Butyl-4-propyl-5-isobutyl-1H-pyrazol-3-on, 2-Isobutyl-4-propyl-5-isobutyl-1H-pyrazol-3-on, 2-tert-Butyl-4-propyl-5-isobutyl-1H-pyrazol-3-on, 2-Phenyl-4-propyl-5-isobutyl-1H-pyrazol-3-on, 2-(4-Methylphenyl)-4-propyl-5-isobutyl-1H-pyrazol-3-on, 2-(2-Methylphenyl)-4-propyl-5-isobutyl-1H-pyrazol-3-on, 2-(3-Methylphenyl)-4-propyl-5-isobutyl-1H-pyrazol-3-on, 2-(4-Methoxy-4-propyl-5-isobutyl-1H-pyrazol-3-on, 2-Methyl-4-propyl-5-tert-butyl-1H-pyrazol-3-on, 2-Ethyl-4-propyl-5-tert-butyl-1H-pyrazol-3-on, 2-Propyl-4-propyl-5-tert-butyl-1H-pyrazol-3-on, 2-Isopropyl-4-propyl-5-tert-butyl-1H-pyrazol-3-on, 2-Butyl-4-propyl-5-tert-butyl-1H-pyrazol-3-on, 2-Isobutyl-4-propyl-5-tert-butyl -1H-pyrazol-3-on, 2-tert-Butyl-4-propyl-5-tert-butyl-1H-pyrazol-3-on, 2-Phenyl-4-propyl-5-tert-butyl-1H-pyrazol-3-on, 2-(4-Methylphenyl)-4-propyl-5-tert-butyl-1H-pyrazol-3-on, 2-(2-Methylphenyl)-4-propyl-5-tert-butyl-1H-pyrazol-3-on, 2-(3-Methylphenyl)-4-propyl-5-tert-butyl-1H-pyrazol-3-on, 2-(4-Methoxy-4-propyl-5-phenyl-1H-pyrazol-3-on, 2-Methyl-4-propyl-5-phenyl-1H-pyrazol-3-on, 2-Ethyl-4-propyl-5-phenyl-1H-pyrazol-3-on, 2-Propyl-4-propyl-5-phenyl-1H-pyrazol-3-on, 2-Isopropyl-4-propyl-5-phenyl-1H-pyrazol-3-on, 2-Butyl-4-propyl-5-phenyl-1H-pyrazol-3-on, 2-Isobutyl-4-propyl-5-phenyl-1H-pyrazol-3-on, 2-tert-Butyl-4-propyl-5-phenyl-1H-pyrazol-3-on, 2-Phenyl-4-propyl-5-phenyl-1H-pyrazol-3-on, 2-(4-Methylphenyl)-4-propyl-5-phenyl-1H-pyrazol-3-on, 2-(2-Methylphenyl)-4-propyl-5-phenyl-1H-pyrazol-3-on, 2-(3-Methylphenyl)-4-propyl-5-phenyl-1H-pyrazol-3-on, 2-(4-Methoxy-4-propyl-5-(2-Methylphenyl)-1H-pyrazol-3-on, 2-Methyl-4-propyl-5-(2-Methylphenyl)-1H-pyrazol-3-on, 2-Ethyl-4-propyl-5-(2-Methylphenyl)-1H-pyrazol-3-on, 2-Propyl-4-propyl-5-(2-Methylphenyl)-1H-pyrazol-3-on, 2-Isopropyl-4-propyl-5-(2-Methylphenyl)-1H-pyrazol-3-on, 2-Butyl-4-propyl-5-(2-Methylphenyl)-1H-pyrazol-3-on, 2-Isobutyl-4-propyl-5-(2-Methyl-phenyl)-1H-pyrazol-3-on, 2-tert-Butyl-4-propyl-5-(2-Methylphenyl)-1H-pyrazol-3-on, 2-Phenyl-4-propyl-5-(2-Methylphenyl)-1H-pyrazol-3-on, 2-(4-Methylphenyl)-4-propyl-5-(2-Methylphenyl)-1H-pyrazol-3-on, 2-(2-Methylphenyl)-4-propyl-5-(2-Methylphenyl)-1H-pyrazol-3-on, 2-(3-Methylphenyl)-4-propyl-5-(2-Methylphenyl)-1H-pyrazol-3-on, 2-(4-Methoxy-4-propyl-5-(3-Methylphenyl)-1H-pyrazol-3-on, 2-Methyl-4-propyl-5-(3-Methyl-phenyl)-1H-pyrazol-3-on, 2-Ethyl-4-propyl-5-(3-Methylphenyl)-1H-pyrazol-3-on, 2-Propyl-4-propyl-5-(3-Methylphenyl)-1H-pyrazol-3-on, 2-Isopropyl-4-propyl-5-(3-Methylphenyl)-1H-pyrazol-3-on, 2-Butyl-4-propyl-5-(3-Methylphenyl)-1H-pyrazol-3-on,2-Isobutyl-4-propyl-5-(3-Methylphenyl)-1H-pyrazol-3-on, 2-tert-Butyl-4-propyl-5-(3-Methylphenyl)-1H-pyrazol-3-on, 2-Phenyl-4-propyl-5-(3-Methylphenyl)-1H-pyrazol-3-on, 2-(4-Methylphenyl)-4-propyl-5-(3-Methylphenyl)-1H-pyrazol-3-on, 2-(2-Methylphenyl)-4-propyl-5-(3-Methylphenyl)-1H-pyrazol-3-on, 2-(3-Methylphenyl)-4-propyl-5-(3-Methylphenyl)-1H-pyrazol-3-on, 2-(4-Methoxy-4-propyl-5-(3-Methylphenyl)-1H-pyrazol-3-on, 2-Methyl-4-propyl-5-(3-Methylphenyl)-1H-pyrazol-3-on, 2-Ethyl-4-propyl-5-(3-Methylphenyl)-1H-pyrazol-3-on, 2-Propyl-4-propyl-5-(3-Methylphenyl)-1H-pyrazol-3-on, 2-Isopropyl-4-propyl-5-(3-Methyl-phenyl)-1H-pyrazol-3-on,2-Butyl-4-propyl-5-(3-Methylphenyl)-1H-pyrazol-3-on, 2-Isobutyl-4-propyl-5-(3-Methylphenyl)-1H-pyrazol-3-on, 2-tert-Butyl-4-propyl-5-butyl-1H-pyrazol-3-on, 2-Phenyl-4-propyl-5-(3-Methylphenyl)-1H-pyrazol-3-on, 2-(4-Methylphenyl)-4-propyl-5-(3-Methylphenyl)-1H-pyrazol-3-on, 2-(2-Methylphenyl)-4-propyl-5-(3-Methylphenyl)-1H-pyrazol-3-on, 2-(3-Methylphenyl)-4-propyl-5-(3-Methylphenyl)-1H-pyrazol-3-on, 2-(4-Methoxy-4-propyl-5-butyl-1H-pyrazol-3-on, 2-Methyl-4-propyl-5-(4-Methylphenyl)-1H-pyrazol-3-on, 2-Ethyl-4-propyl-5-(4-Methylphenyl)-1H-pyrazol-3-on, 2-Propyl-4-propyl-5-(4-Methylphenyl)-1H-pyrazol-3-on, 2-Isopropyl-4-propyl-5-(4-Methylphenyl)-1H-pyrazol-3-on, 2-Butyl-4-propyl-5-(4-Methylphenyl)-1H-pyrazol-3-on, 2-Isobutyl-4-propyl-5-(4-Methyl-phenyl)-1H-pyrazol-3-on, 2-tert-Butyl-4-propyl-5-(4-Methylphenyl)-1H-pyrazol-3-on, 2-Phenyl-4-propyl-5-(4-Methylphenyl)-1H-pyrazol-3-on, 2-(4-Methylphenyl)-4-propyl-5-(4-Methylphenyl)-1H-pyrazol-3-on, 2-(2-Methylphenyl)-4-propyl-5-(4-Methylphenyl)-1H-pyrazol-3-on, 2-(3-Methylphenyl)-4-propyl-5-(4-Methylphenyl)-1H-pyrazol-3-on, 2-(4-Methoxy-4-propyl-5-(4-Methylphenyl)-1H-pyrazol-3-on, 2-Methyl-4-butyl-5-phenyl-1H-pyrazol-3-on, 2-Ethyl-4-butyl5-phenyl-1H-pyrazol-3-on, 2-Propyl-4-butyl-5-phenyl-1H-pyrazol-3-on, 2-Isopropyl-4-butyl-5-phenyl-1H-pyrazol-3-on, 2-Butyl-4-butyl-5-phenyl-1H-pyrazol-3-on, 2-Isobutyl-4-butyl-5-phenyl-1H-pyrazol-3-on, 2-tert-Butyl-4-butyl-5-phenyl-1H-pyrazol-3-on,2-Phenyl-4-butyl-5-phenyl-1H-pyrazol-3-on, 2-(4-Methylphenyl)-4-butyl-5-phenyl-1H-pyrazol-3-on, 2-(2-Methylphenyl)-4-butyl-5-phenyl-1H-pyrazol-3-on, 2-(3-Methylphenyl)-4-butyl-5-phenyl-1H-pyrazol-3-on, 2-(4-Methoxy-4-butyl-5-phenyl-1H-pyrazol-3-on, 2-Methyl-4-butyl-5-methyl-1H-pyrazol-3-on, 2-Ethyl-4-butyl-5-methyl-1H-pyrazol-3-on, 2-Propyl-4-butyl-5-methyl-1H-pyrazol-3-on, 2-Isopropyl-4-butyl-5-methyl-1H-pyrazol-3-on, 2-Butyl-4-butyl-5-methyl-1H-pyrazol-3-on, 2-Isobutyl-4-butyl-5-methyl-1H-pyrazol-3-on, 2-tert-Butyl-4-butyl-5-methyl-1H-pyrazol-3-on, 2-Phenyl-4-butyl-5-methyl-1H-pyrazol-3-on, 2-(4-Methylphenyl)-4-butyl-5-methyl-1H-pyrazol-3-on, 2-(2-Methylphenyl)-4-butyl-5-methyl-1H-pyrazol-3-on, 2-(3-Methylphenyl)-4-butyl-5-methyl-1H-pyrazol-3-on, 2-(4-Methoxy-4-butyl-5-methyl-1H-pyrazol-3-on, 2-methyl-4-butyl-5-ethyl-1H-pyrazol-3-on, 2-ethyl-4-butyl-5-ethyl-1H-pyrazol-3-on, 2-propyl-4-butyl-5-ethyl-1H-pyrazol-3-on, 2-isopropyl-4-butyl-5-ethyl-1H-pyrazol-3-on, 2-butyl-4-butyl-5-ethyl-1H-pyrazol-3-on, 2-Isobutyl-4-butyl-5-ethyl-1H-pyrazol-3-on, 2-tert-butyl-4-butyl-5-ethyl-1H-pyrazol-3-on, 2-Phenyl-4-butyl-5-ethyl-1H-pyrazol-3-on, 2-(4-Methylphenyl)-4-butyl-5-ethyl-1H-pyrazol-3-on, 2-(2-Methylphenyl)-4-butyl-5-ethyl-1H-pyrazol-3-on, 2-(3-Methylphenyl)-4-butyl-5-ethyl-1H-pyrazol-3-on, 2-(4-Methoxy-4-butyl-5-ethyl-1H-pyrazol-3-on, 2-Methyl-4-butyl-5-propyl-1H-pyrazol-3-on, 2-Ethyl-4-butyl-5-propyl-1H-pyrazol-3-on, 2-Propyl-4-butyl-5-propyl-1H-pyrazol-3-on, 2-Isopropyl-4-butyl-5-methyl-1H-pyrazol-3-on, 2-Butyl-4-butyl-5-propyl-1H-pyrazol-3-on, 2-Isobutyl-4-butyl-5-propyl-1H-pyrazol-3-on, 2-tert-Butyl-4-butyl-5-propyl-1H-pyrazol-3-on, 2-Phenyl-4-butyl-5-propyl-1H-pyrazol-3-on, 2-(4-Methylphenyl)-4-butyl-5-propyl-1H-pyrazol-3-on, 2-(2-Methylphenyl)-4-butyl-5-propyl-1H-pyrazol-3-on, 2-(3-Methylphenyl)-4-butyl-5-propyl-1H-pyrazol-3-on, 2-(4-Methoxy-4-butyl-5-propyl-1H-pyrazol-3-on, 2-Methyl-4-butyl-5-isopropyl-1H-pyrazol-3-on, 2-Ethyl-4-butyl-5-isopropyl-1H-pyrazol-3-on, 2-Propyl-4-butyl-5-isopropyl-1H-pyrazol-3-on, 2-Isopropyl-4-butyl-5-isopropyl-1H-pyrazol-3-on, 2-Butyl-4-butyl-5-isopropyl-1H-pyrazol-3-on, 2-Isobutyl-4-butyl-5-isopropyl-1H-pyrazol-3-on, 2-tert-Butyl-4-butyl-5-isopropyl-1H-pyrazol-3-on, 2-Phenyl-4-butyl-5-isopropyl-1H-pyrazol-3-on, 2-(4-Methylphenyl)-4-butyl-5-isopropyl-1H-pyrazol-3-on, 2-(2-Methylphenyl)-4-butyl-5-isopropyl-1H-pyrazol-3-on, 2-(3-Methylphenyl)-4-butyl-5-isopropyl-1H-pyrazol-3-on, 2-(4-Methoxy-4-butyl-5-isopropyl-1H-pyrazol-3-on, 2-Methyl-4-butyl-5-butyl-1H-pyrazol-3-on, 2-Ethyl-4-butyl-5-butyl-1H-pyrazol-3-on, 2-Propyl-4-butyl-5-butyl-1H-pyrazol-3-on, 2-Isopropyl-4-butyl-5-butyl-1H-pyrazol-3-on, 2-Butyl-4-butyl-5-butyl-1H-pyrazol-3-on, 2-Isobutyl-4-butyl-5-butyl-1H-pyrazol-3-on, 2-tert-Butyl-4-butyl-5-butyl-1H-pyrazol-3-on, 2-Phenyl-4-butyl-5-butyl-1H-pyrazol-3-on, 2-(4-Methylphenyl)-4-butyl-5-butyl-1H-pyrazol-3-on, 2-(2-Methylphenyl)-4-butyl-5-butyl-1H-pyrazol-3-on, 2-(3-Methylphenyl)-4-butyl-5-butyl-1H-pyrazol-3-on, 2-(4-Methoxy-4-butyl-5-butyl-1H-pyrazol-3-on, 2-Methyl-4-butyl-5-isobutyl-1H-pyrazol-3-on, 2-Ethyl-4-butyl-5-isobutyl-1H-pyrazol-3-on, 2-Propyl-4-butyl-5-isobutyl-1H-pyrazol-3-on, 2-Isopropyl-4-butyl-5-isobutyl-1H-pyrazol-3-on, 2-Butyl-4-butyl-5-isobutyl-1H-pyrazol-3-on, 2-Isobutyl-4-butyl-5-isobutyl-1H-pyrazol-3-on, 2-tert-Butyl-4-butyl-5-isobutyl-1H-pyrazol-3-on, 2-Phenyl-4-butyl-5-isobutyl-1H-pyrazol-3-on, 2-(4-Methylphenyl)-4-butyl-5-isobutyl-1H-pyrazol-3-on, 2-(2-Methylphenyl)-4-butyl-5-isobutyl-1H-pyrazol-3-on, 2-(3-Methylphenyl)-4-butyl-5-isobutyl-1H-pyrazol-3-on, 2-(4-Methoxy-4-butyl-5-isobutyl-1H-pyrazol-3-on, 2-Methyl-4-butyl-5-tert-butyl-1H-pyrazol-3-on, , 2-Ethyl-4-butyl-5-tert-butyl-1H-pyrazol-3-on, 2-Propyl-4-butyl-5-tert-butyl-1H-pyrazol-3-on, 2-Isopropyl-4-butyl-5-tert-butyl-1H-pyrazol-3-on, 2-Butyl-4-butyl-5-tert-butyl-1H-pyrazol-3-on, 2-Isobutyl-4-butyl-5-tert-butyl-1H-pyrazol-3-on, 2-tert-Butyl-4-butyl-5-tert-butyl-1H-pyrazol-3-on, 2-Phenyl-4-butyl-5-tert-butyl-1H-pyrazol-3-on, 2-(4-Methylphenyl)-4-butyl-5-tert-butyl-1H-pyrazol-3-on, 2-(2-Methylphenyl)-4-butyl-5-tert-butyl-1H-pyrazol-3-on, 2-(3-Methylphenyl)-4-butyl-5-tert-butyl-1H-pyrazol-3-on, 2-(4-Methoxy-4-butyl-5-phenyl-1H-pyrazol-3-on, 2-Methyl-4-butyl-5-phenyl-1H-pyrazol-3-on, , 2-Ethyl-4-butyl-5-phenyl-1H-pyrazol-3-on, 2-Propyl-4-butyl-5-phenyl-1H-pyrazol-3-on, 2-Isopropyl-4-butyl-5-phenyl-1H-pyrazol-3-on, 2-Butyl-4-butyl-5-phenyl-1H-pyrazol-3-on, 2-Isobutyl-4-butyl-5-phenyl-1H-pyrazol-3-on, 2-tert-Butyl-4-butyl-5-phenyl-1H-pyrazol-3-on, 2-Phenyl-4-butyl-5-phenyl-1H-pyrazol-3-on, 2-(4-Methylphenyl)-4-butyl-5-phenyl-1H-pyrazol-3-on, 2-(2-Methylphenyl)-4-butyl-5-phenyl-1H-pyrazol-3-on, 2-(3-Methylphenyl)-4-butyl-5-phenyl-1H-pyrazol-3-on, 2-(4-Methoxy-4-butyl-5-(2-Methylphenyl)-1H-pyrazol-3-on, 2-Methyl-4-butyl-5-(2-Methylphenyl)-1H-pyrazol-3-on, ,2-Ethyl-4-butyl-5-(2-Methylphenyl)-1H-pyrazol-3-on, 2-Propyl-4-butyl-5-(2-Methylphenyl)-1H-pyrazol-3-on, 2-Isopropyl-4-butyl-5-(2-Methylphenyl)-1H-pyrazol-3-on, 2-Butyl-4-butyl-5-(2-Methylphenyl)-1H-pyrazol-3-on, 2-Isobutyl-4-butyl-5-(2-Methylphenyl)-1H-pyrazol-3-on, 2-tert-Butyl-4-butyl-5-(2-Methyl-phenyl)-1H-pyrazol-3-on, 2-Phenyl-4-butyl-5-(2-Methylphenyl)-1H-pyrazol-3-on, 2-(4-Methylphenyl)-4-butyl-5-(2-Methylphenyl)-1H-pyrazol-3-on, 2-(2-Methylphenyl)-4-butyl-5-(2-Methylphenyl)-1H-pyrazol-3-on, 2-(3-Methylphenyl)-4-butyl-5-(2-Methylphenyl)-1H-pyrazol-3-on,
2-(4-Methoxy-4-butyl-5-(3-Methylphenyl)-1H-pyrazol-3-on, 2-Methyl-4-butyl-5-(3-Methyl-phenyl)-1H-pyrazol-3-on, ,2-Ethyl-4-butyl-5-(3-Methylphenyl)-1H-pyrazol-3-on, 2-Propyl-4-butyl-5-(3-Methylphenyl)-1H-pyrazol-3-on, 2-Isopropyl-4-butyl-5-(3-Methylphenyl)-1H-pyrazol-3-on, 2-Butyl-4-butyl-5-(3-Methylphenyl)-1H-pyrazol-3-on, 2-Isobutyl-4-butyl-5-(3-Methylphenyl)-1H-pyrazol-3-on, 2-tert-Butyl-4-butyl-5-(3-Methylphenyl)-1H-pyrazol-3-on, 2-Phenyl-4-butyl-5-(3-Methylphenyl)-1H-pyrazol-3-on, 2-(4-Methylphenyl)-4-butyl-5-(3-Methylphenyl)-1H-pyrazol-3-on, 2-(2-Methylphenyl)-4-butyl-5-(3-Methylphenyl)-1H-pyrazol-3-on, 2-(3-Methylphenyl)-4-butyl-5-(3-Methylphenyl)-1H-pyrazol-3-on, 2-(4-Methoxy-4-butyl-5-(3-Methylphenyl)-1H-pyrazol-3-on, 2-Methyl-4-butyl-5-(3-Methyl-phenyl)-1H-pyrazol-3-on, ,2-Ethyl-4-butyl-5-(3-Methylphenyl)-1H-pyrazol-3-on, 2-Propyl-4-butyl-5-(3-Methylphenyl)-1H-pyrazol-3-on, 2-Isopropyl-4-butyl-5-(3-Methylphenyl)-1H-pyrazol-3-on, 2-Butyl-4-butyl-5-(3-Methylphenyl)-1H-pyrazol-3-on,
2-Isobutyl-4-butyl-5-(3-Methylphenyl)-1H-pyrazol-3-on, 2-tert-Butyl-4-butyl-5-butyl-1H-pyrazol-3-on, 2-Phenyl-4-butyl-5-(3-Methylphenyl)-1H-pyrazol-3-on, 2-(4-Methylphenyl)-4-butyl-5-(3-Methylphenyl)-1H-pyrazol-3-on, 2-(2-Methylphenyl)-4-butyl-5-(3-Methylphenyl)-1H-pyrazol-3-on, 2-(3-Methylphenyl)-4-butyl-5-(3-Methylphenyl)-1H-pyrazol-3-on, 2-(4-Methoxy-4-butyl-5-butyl-1H-pyrazol-3-on, 2-Methyl-4-butyl-5-(4-Methylphenyl)-1H-pyrazol-3-on, 2-Ethyl-4-butyl-5-(4-Methylphenyl)-1H-pyrazol-3-on, 2-Propyl-4-butyl-5-(4-Methyl-phenyl)-1H-pyrazol-3-on, 2-Isopropyl-4-butyl-5-(4-Methylphenyl)-1H-pyrazol-3-on, 2-Butyl-4-butyl-5-(4-Methylphenyl)-1H-pyrazol-3-on, 2-Isobutyl-4-butyl-5-(4-Methylphenyl)-1H-pyrazol-3-on, 2-tert-Butyl-4-butyl-5-(4-Methylphenyl)-1H-pyrazol-3-on, 2-Phenyl-4-butyl-5-(4-Methylphenyl)-1H-pyrazol-3-on, 2-(4-Methylphenyl)-4-butyl-5-(4-Methylphenyl)-1H-pyrazol-3-on, 2-(2-Methylphenyl)-4-butyl-5-(4-Methylphenyl)-1H-pyrazol-3-on, 2-(3-Methyl-phenyl)-4-butyl-5-(4-Methylphenyl)-1H-pyrazol-3-on, 2-(4-Methoxy-4-butyl-5-(4-Methyl-phenyl)-1H-pyrazol-3-on, 2-Methyl-4-isobutyl-5-phenyl-1H-pyrazol-3-on, ,2-Ethyl-4-isobutyl-5-phenyl-1H-pyrazol-3-on, 2-Propyl-4-isobutyl-5-phenyl-1H-pyrazol-3-on, 2-Isopropyl-4-isobutyl-5-phenyl-1H-pyrazol-3-on, 2-Butyl-4-isobutyl-5-phenyl-1H-pyrazol-3-on, 2-Isobutyl-4-isobutyl-5-phenyl-1H-pyrazol-3-on, 2-tert-Butyl-4-isobutyl-5-phenyl-1H-pyrazol-3-on, 2-Phenyl-4-isobutyl-5-phenyl-1H-pyrazol-3-on, 2-(4-Methylphenyl)-4-isobutyl-5-phenyl-1H-pyrazol-3-on, 2-(2-Methylphenyl)-4-isobutyl-5-phenyl-1H-pyrazol-3-on, 2-(3-Methylphenyl)-4-isobutyl-5-phenyl-1H-pyrazol-3-on, 2-(4-Methoxy-4-isobutyl-5-phenyl-1H-pyrazol-3-on, 2-Methyl-4-isobutyl-5-methyl-1H-pyrazol-3-on, ,2-Ethyl-4-isobutyl-5-methyl-1H-pyrazol-3-on, 2-Propyl-4-isobutyl-5-methyl-1H-pyrazol-3-on, 2-Isopropyl-4-isobutyl-5-methyl-1H-pyrazol-3-on, 2-Butyl-4-isobutyl-5-methyl-1H-pyrazol-3-on, 2-Isobutyl-4-isobutyl-5-methyl-1H-pyrazol-3-on, 2-tert-Butyl-4-isobutyl-5-methyl-1H-pyrazol-3-on, 2-Phenyl-4-isobutyl-5-methyl-1H-pyrazol-3-on, 2-(4-Methylphenyl)-4-isobutyl-5-methyl-1H-pyrazol-3-on, 2-(2-Methylphenyl)-4-isobutyl-5-methyl-1H-pyrazol-3-on, 2-(3-Methylphenyl)-4-isobutyl-5-methyl -1H-pyrazol-3-on, 2-(4-Methoxy-4-isobutyl-5-methyl-1H-pyrazol-3-on, 2-methyl-4-isobutyl-5-ethyl-1H-pyrazol-3-on, ,2-ethyl-4-isobutyl-5-ethyl-1H-pyrazol-3-on, 2-propyl-4-isobutyl-5-ethyl-1H-pyrazol-3-on, 2-isopropyl-4-isobutyl-5-ethyl-1H-pyrazol-3-on, 2-butyl-4-isobutyl-5-ethyl-1H-pyrazol-3-on, 2-Isobutyl-4-isobutyl-5-ethyl-1H-pyrazol-3-on, 2-tert-butyl-4-isobutyl-5-ethyl-1H-pyrazol-3-on, 2-Phenyl-4-isobutyl-5-ethyl-1H-pyrazol-3-on, 2-(4-Methylphenyl)-4-isobutyl-5-ethyl-1H-pyrazol-3-on, 2-(2-Methylphenyl)-4-isobutyl-5-ethyl-1H-pyrazol-3-on, 2-(3-Methylphenyl)-4-isobutyl-5-ethyl-1H-pyrazol-3-on, 2-(4-Methoxy-4-isobutyl-5-ethyl-1H-pyrazol-3-on, 2-Methyl-4-isobutyl-5-propyl-1H-pyrazol-3-on, ,2-Ethyl-4-isobutyl-5-propyl-1H-pyrazol-3-on, 2-Propyl-4-isobutyl-5-propyl-1H-pyrazol-3-on, 2-Isopropyl-4-isobutyl-5-methyl-1H-pyrazol-3-on, 2-Butyl-4-isobutyl-5-propyl-1H-pyrazol-3-on, 2-Isobutyl-4-isobutyl-5-propyl-1H-pyrazol-3-on, 2-tert-Butyl-4-isobutyl-5-propyl-1H-pyrazol-3-on, 2-Phenyl-4-isobutyl-5-propyl-1H-pyrazol-3-on, 2-(4-Methylphenyl)-4-isobutyl-5-propyl-1H-pyrazol-3-on, 2-(2-Methylphenyl)-4-isobutyl-5-propyl-1H-pyrazol-3-on, 2-(3-Methylphenyl)-4-isobutyl-5-propyl-1H-pyrazol-3-on, 2-(4-Methoxy-4-isobutyl-5-propyl-1H-pyrazol-3-on, 2-Methyl-4-isobutyl-5-isopropyl-1H-pyrazol-3-on, ,2-Ethyl-4-isobutyl-5-isopropyl-1H-pyrazol-3-on, 2-Propyl-4-isobutyl-5-isopropyl-1H-pyrazol-3-on, 2-Isopropyl-4-isobutyl-5-isopropyl-1H-pyrazol-3-on, 2-Butyl-4-isobutyl-5-isopropyl-1H-pyrazol-3-on, 2-Isobutyl-4-isobutyl-5-isopropyl-1H-pyrazol-3-on,
2-tert-Butyl-4-isobutyl-5-isopropyl-1H-pyrazol-3-on, 2-Phenyl-4-isobutyl-5-isopropyl-1H-pyrazol-3-on, 2-(4-Methylphenyl)-4-isobutyl-5-isopropyl-1H-pyrazol-3-on, 2-(2-Methyl-phenyl)-4-isobutyl-5-isopropyl-1H-pyrazol-3-on, 2-(3-Methylphenyl)-4-isobutyl-5-isopropyl-1H-pyrazol-3-on, 2-(4-Methoxy-4-isobutyl-5-isopropyl-1H-pyrazol-3-on, 2-Methyl-4-isobutyl-5-butyl-1H-pyrazol-3-on, ,2-Ethyl-4-isobutyl-5-butyl-1H-pyrazol-3-on, 2-Propyl-4-isobutyl-5-butyl-1H-pyrazol-3-on, 2-Isopropyl-4-isobutyl-5-butyl-1H-pyrazol-3-on, 2-Butyl-4-isobutyl-5-butyl-1H-pyrazol-3-on, 2-Isobutyl-4-isobutyl-5-butyl-1H-pyrazol-3-on, 2-tert-Butyl-4-isobutyl-5-butyl-1H-pyrazol-3-on, 2-Phenyl-4-isobutyl-5-butyl-1H-pyrazol-3-on, 2-(4-Methylphenyl)-4-isobutyl-5-butyl-1H-pyrazol-3-on, 2-(2-Methylphenyl)-4-isobutyl-5-butyl-1H-pyrazol-3-on, 2-(3-Methylphenyl)-4-isobutyl-5-butyl-1H-pyrazol-3-on, 2-(4-Methoxy-4-isobutyl-5-butyl-1H-pyrazol-3-on, 2-Methyl-4-isobutyl-5-isobutyl-1H-pyrazol-3-on, ,2-Ethyl-4-isobutyl-5-isobutyl -1H-pyrazol-3-on, 2-Propyl-4-isobutyl-5-isobutyl-1H-pyrazol-3-on, 2-Isopropyl-4-isobutyl-5-isobutyl-1H-pyrazol-3-on, 2-Butyl-4-isobutyl-5-isobutyl-1H-pyrazol-3-on, 2-Isobutyl-4-isobutyl-5-isobutyl-1H-pyrazol-3-on, 2-tert-Butyl-4-isobutyl-5-isobutyl-1H-pyrazol-3-on, 2-Phenyl-4-isobutyl-5-isobutyl-1H-pyrazol-3-on,2-(4-Methylphenyl)-4-isobutyl-5-isobutyl-1H-pyrazol-3-on, 2-(2-Methylphenyl)-4-isobutyl-5-isobutyl-1H-pyrazol-3-on, 2-(3-Methylphenyl)-4-isobutyl-5-isobutyl-1H-pyrazol-3-on, 2-(4-Methoxy-4-isobutyl-5-isobutyl-1H-pyrazol-3-on, 2-Methyl-4-isobutyl-5-tert-butyl-1H-pyrazol-3-on, ,2-Ethyl-4-isobutyl-5-tert-butyl-1H-pyrazol-3-on, 2-Propyl-4-isobutyl-5-tert-butyl-1H-pyrazol-3-on, 2-Isopropyl-4-isobutyl-5-tert-butyl-1H-pyrazol-3-on, 2-Butyl-4-isobutyl-5-tert-butyl-1H-pyrazol-3-on, 2-Isobutyl-4-isobutyl-5-tert-butyl-1H-pyrazol-3-on, 2-tert-Butyl-4-isobutyl-5-tert-butyl-1H-pyrazol-3-on, 2-Phenyl-4-isobutyl-5-tert-butyl-1H-pyrazol-3-on, 2-(4-Methylphenyl)-4-isobutyl-5-tert-butyl-1H-pyrazol-3-on, 2-(2-Methylphenyl)-4-isobutyl-5-tert-butyl-1H-pyrazol-3-on, 2-(3-Methyl-phenyl)-4-isobutyl-5-tert-butyl-1H-pyrazol-3-on, 2-(4-Methoxy-4-isobutyl-5-phenyl-1H-pyrazol-3-on, 2-Methyl-4-isobutyl-5-phenyl-1H-pyrazol-3-on, ,2-Ethyl-4-isobutyl-5-phenyl-1H-pyrazol-3-on, 2-Propyl-4-isobutyl-5-phenyl-1H-pyrazol-3-on, 2-Isopropyl-4-isobutyl-5-phenyl-1H-pyrazol-3-on, 2-Butyl-4-isobutyl-5-phenyl-1H-pyrazol-3-on, 2-Isobutyl-4-isobutyl-5-phenyl-1H-pyrazol-3-on, 2-tert-Butyl-4-isobutyl-5-phenyl-1H-pyrazol-3-on, 2-Phenyl-4-isobutyl-5-phenyl-1H-pyrazol-3-on, 2-(4-Methylphenyl)-4-isobutyl-5-phenyl-1H-pyrazol-3-on, 2-(2-Methylphenyl)-4-isobutyl-5-phenyl-1H-pyrazol-3-on, 2-(3-Methylphenyl)-4-isobutyl-5-phenyl-1H-pyrazol-3-on, 2-(4-Methoxy-4-isobutyl-5-(2-Methylphenyl)-1H-pyrazol-3-on, 2-Methyl-4-isobutyl-5-(2-Methylphenyl)-1H-pyrazol-3-on, , 2-Ethyl-4-isobutyl-5-(2-Methylphenyl)-1H-pyrazol-3-on, 2-Propyl-4-isobutyl-5-(2-Methylphenyl)-1H-pyrazol-3-on, 2-Isopropyl-4-isobutyl-5-(2-Methylphenyl)-1H-pyrazol-3-on, 2-Butyl-4-isobutyl-5-(2-Methylphenyl)-1H-pyrazol-3-on, 2-Isobutyl-4-isobutyl-5-(2-Methylphenyl)-1H-pyrazol-3-on, 2-tert-Butyl-4-isobutyl-5-(2-Methylphenyl)-1H-pyrazol-3-on, 2-Phenyl-4-isobutyl-5-(2-Methylphenyl)-1H-pyrazol-3-on, 2-(4-Methylphenyl)-4-isobutyl-5-(2-Methylphenyl)-1H-pyrazol-3-on, 2-(2-Methylphenyl)-4-isobutyl-5-(2-Methylphenyl)-1H-pyrazol-3-on, 2-(3-Methylphenyl)-4-isobutyl-5-(2-Methylphenyl)-1H-pyrazol-3-on, 2-(4-Methoxy-4-isobutyl-5-(3-Methylphenyl)-1H-pyrazol-3-on, 2-Methyl-4-isobutyl-5-(3-Methylphenyl)-1H-pyrazol-3-on, 2-Ethyl-4-isobutyl-5-(3-Methylphenyl)-1H-pyrazol-3-on, 2-Propyl-4-isobutyl-5-(3-Methyl-phenyl)-1H-pyrazol-3-on, 2-Isopropyl-4-isobutyl-5-(3-Methylphenyl)-1H-pyrazol-3-on, 2-Butyl-4-isobutyl-5-(3-Methylphenyl)-1H-pyrazol-3-on, 2-Isobutyl-4-isobutyl-5-(3-Methyl-phenyl)-1H-pyrazol-3-on, 2-tert-Butyl-4-isobutyl-5-(3-Methylphenyl)-1H-pyrazol-3-on, 2-Phenyl-4-isobutyl-5-(3-Methylphenyl)-1H-pyrazol-3-on, 2-(4-Methylphenyl)-4-isobutyl-5-(3-Methylphenyl)-1H-pyrazol-3-on, 2-(2-Methylphenyl)-4-isobutyl-5-(3-Methylphenyl)-1H-pyrazol-3-on, 2-(3-Methylphenyl)-4-isobutyl-5-(3-Methylphenyl)-1H-pyrazol-3-on, 2-(4-Methoxy-4-isobutyl-5-(3-Methylphenyl)-1H-pyrazol-3-on, 2-Methyl-4-isobutyl-5-(3-Methyl-phenyl)-1H-pyrazol-3-on, ,2-Ethyl-4-isobutyl-5-(3-Methylphenyl)-1H-pyrazol-3-on, 2-Propyl-4-isobutyl-5-(3-Methylphenyl)-1H-pyrazol-3-on, 2-Isopropyl-4-isobutyl-5-(3-Methylphenyl)-1H-pyrazol-3-on, 2-Butyl-4-isobutyl-5-(3-Methylphenyl)-1H-pyrazol-3-on, 2-Isobutyl-4-isobutyl-5-(3-Methylphenyl)-1H-pyrazol-3-on, 2-tert-Butyl-4-isobutyl-5-butyl-1H-pyrazol-3-on, 2-Phenyl-4-isobutyl-5-(3-Methylphenyl)-1H-pyrazol-3-on, 2-(4-Methylphenyl)-4-isobutyl-5-(3-Methylphenyl)-1H-pyrazol-3-on, 2-(2-Methylphenyl)-4-isobutyl-5-(3-Methylphenyl)-1H-pyrazol-3-on, 2-(3-Methylphenyl)-4-isobutyl-5-(3-Methylphenyl)-1H-pyrazol-3-on, 2-(4-Methoxy-4-isobutyl-5-butyl-1H-pyrazol-3-on, 2-Methyl-4-isobutyl-5-(4-Methylphenyl)-1H-pyrazol-3-on, , 2-Ethyl-4-isobutyl-5-(4-Methylphenyl)-1H-pyrazol-3-on, 2-Propyl-4-isobutyl-5-(4-Methylphenyl)-1H-pyrazol-3-on, 2-Isopropyl-4-isobutyl-5-(4-Methylphenyl)-1H-pyrazol-3-on, 2-Butyl-4-isobutyl-5-(4-Methylphenyl)-1H-pyrazol-3-on, 2-Isobutyl-4-isobutyl-5-(4-Methylphenyl)-1H-pyrazol-3-on, 2-tert-Butyl-4-isobutyl-5-(4-Methylphenyl)-1H-pyrazol-3-on, 2-Phenyl-4-isobutyl-5-(4-Methylphenyl)-1H-pyrazol-3-on, 2-(4-Methylphenyl)-4-isobutyl-5-(4-Methylphenyl)-1H-pyrazol-3-on, 2-(2-Methylphenyl)-4-isobutyl-5-(4-Methylphenyl)-1H-pyrazol-3-on, 2-(3-Methylphenyl)-4-isobutyl-5-(4-Methylphenyl)-1H-pyrazol-3-on, 2-(4-Methoxy-4-isobutyl-5-(4-Methylphenyl)-1H-pyrazol-3-on, 2-Methyl-4-tert-butyl-5-phenyl-1H-pyrazol-3-on, ,2-Ethyl-4-tert-butyl5-phenyl-1H-pyrazol-3-on, 2-Propyl-4-tert-butyl-5-phenyl-1H-pyrazol-3-on, 2-Isopropyl-4-tert-butyl-5-phenyl-1H-pyrazol-3-on, 2-Butyl-4-tert-butyl-5-phenyl-1H-pyrazol-3-on, 2-Isobutyl-4-tert-butyl-5-phenyl-1H-pyrazol-3-on, 2-tert-Butyl-4-tert-butyl-5-phenyl-1H-pyrazol-3-on, 2-Phenyl-4-tert-butyl-5-phenyl-1H-pyrazol-3-on, 2-(4-Methylphenyl)-4-tert-butyl-5-phenyl-1H-pyrazol-3-on, 2-(2-Methylphenyl)-4-tert-butyl-5-phenyl-1H-pyrazol-3-on, 2-(3-Methylphenyl)-4-tert-butyl-5-phenyl-1H-pyrazol-3-on, 2-(4-Methoxy-4-tert-butyl-5-phenyl-1H-pyrazol-3-on, 2-Methyl-4-tert-butyl-5-methyl-1H-pyrazol-3-on, ,2-Ethyl-4-tert-butyl-5-methyl-1H-pyrazol-3-on, 2-Propyl-4-tert-butyl-5-methyl-1H-pyrazol-3-on, 2-Isopropyl-4-tert-butyl-5-methyl-1H-pyrazol-3-on, 2-Butyl-4-tert-butyl-5-methyl-1H-pyrazol-3-on, 2-Isobutyl-4-tert-butyl-5-methyl-1H-pyrazol-3-on, 2-tert-Butyl-4-tert-butyl-5-methyl-1H-pyrazol-3-on,2-Phenyl-4-tert-butyl-5-methyl-1H-pyrazol-3-on,2-(4-Methylphenyl)-4-tert-butyl-5-methyl-1H-pyrazol-3-on, 2-(2-Methylphenyl)-4-tert-butyl-5-methyl-1H-pyrazol-3-on, 2-(3-Methylphenyl)-4-tert-butyl-5-methyl-1H-pyrazol-3-on, 2-(4-Methoxy-4-tert-butyl-5-methyl-1H-pyrazol-3-on, 2-methyl-4-tert-butyl-5-ethyl-1H-pyrazol-3-on, 2-ethyl-4-tert-butyl-5-ethyl-1H-pyrazol-3-on, 2-propyl-4-tert-butyl-5-ethyl-1H-pyrazol-3-on, 2-isopropyl-4-tert-butyl-5-ethyl-1H-pyrazol-3-on, 2-butyl-4-tert-butyl-5-ethyl-1H-pyrazol-3-on, 2-Isobutyl-4-tert-butyl-5-ethyl-1H-pyrazol-3-on, 2-tert-butyl-4-tert-butyl-5-ethyl-1H-pyrazol-3-on, 2-Phenyl-4-tert-butyl-5-ethyl-1H-pyrazol-3-on, 2-(4-Methylphenyl)-4-tert-butyl-5-ethyl-1H-pyrazol-3-on, 2-(2-Methylphenyl)-4-tert-butyl-5-ethyl-1H-pyrazol-3-on, 2-(3-Methylphenyl)-4-tert-butyl-5-ethyl-1H-pyrazol-3-on, 2-(4-Methoxy-4-tert-butyl-5-ethyl-1H-pyrazol-3-on, 2-Methyl-4-tert-butyl-5-propyl-1H-pyrazol-3-on, ,2-Ethyl-4-tert-butyl-5-propyl-1H-pyrazol-3-on, 2-Propyl-4-tert-butyl-5-propyl-1H-pyrazol-3-on, 2-Isopropyl-4-tert-butyl-5-methyl-1H-pyrazol-3-on, 2-Butyl-4-tert-butyl-5-propyl-1H-pyrazol-3-on, 2-Isobutyl-4-tert-butyl-5-propyl-1H-pyrazol-3-on, 2-tert-Butyl-4-tert-butyl-5-propyl-1H-pyrazol-3-on, 2-Phenyl-4-tert-butyl-5-propyl-1H-pyrazol-3-on, 2-(4-Methylphenyl)-4-tert-butyl-5-propyl-1H-pyrazol-3-on, 2-(2-Methylphenyl)-4-tert-butyl-5-propyl-1H-pyrazol-3-on, 2-(3-Methylphenyl)-4-tert-butyl-5-propyl-1H-pyrazol-3-on, 2-(4-Methoxy-4-tert-butyl-5-propyl-1H-pyrazol-3-on, 2-Methyl-4-tert-butyl-5-isopropyl-1H-pyrazol-3-on, ,2-Ethyl-4-tert-butyl-5-isopropyl-1H-pyrazol-3-on, 2-Propyl-4-tert-butyl-5-isopropyl-1H-pyrazol-3-on, 2-Isopropyl-4-tert-butyl-5-isopropyl-1H-pyrazol-3-on, 2-Butyl-4-tert-butyl-5-isopropyl-1H-pyrazol-3-on, 2-Isobutyl-4-tert-butyl-5-isopropyl-1H-pyrazol-3-on, 2-tert-Butyl-4-tert-butyl-5-isopropyl-1H-pyrazol-3-on, 2-Phenyl-4-tert-butyl-5-isopropyl-1H-pyrazol-3-on, 2-(4-Methylphenyl)-4-tert-butyl-5-isopropyl-1H-pyrazol-3-on, 2-(2-Methylphenyl)-4-tert-butyl-5-isopropyl-1H-pyrazol-3-on, 2-(3-Methylphenyl)-4-tert-butyl-5-isopropyl-1H-pyrazol-3-on, 2-(4-Methoxy-4-tert-butyl-5-isopropyl-1H-pyrazol-3-on, 2-Methyl-4-tert-butyl-5-butyl-1H-pyrazol-3-on, ,2-Ethyl-4-tert-butyl-5-butyl-1H-pyrazol-3-on, 2-Propyl-4-tert-butyl-5-butyl-1H-pyrazol-3-on, 2-Isopropyl-4-tert-butyl-5-butyl-1H-pyrazol-3-on, 2-Butyl-4-tert-butyl-5-butyl-1H-pyrazol-3-on, 2-Isobutyl-4-tert-butyl-5-butyl-1H-pyrazol-3-on, 2-tert-Butyl-4-tert-butyl-5-butyl-1H-pyrazol-3-on, 2-Phenyl-4-tert-butyl-5-butyl-1H-pyrazol-3-on,2-(4-Methylphenyl)-4-tert-butyl-5-butyl-1H-pyrazol-3-on, 2-(2-Methylphenyl)-4-tert-butyl-5-butyl-1H-pyrazol-3-on, 2-(3-Methylphenyl)-4-tert-butyl-5-butyl-1H-pyrazol-3-on, 2-(4-Methoxy-4-tert-butyl-5-butyl-1H-pyrazol-3-on, 2-Methyl-4-tert-butyl-5-isobutyl-1H-pyrazol-3-on, ,2-Ethyl-4-tert-butyl-5-isobutyl-1H-pyrazol-3-on, 2-Propyl-4-tert-butyl-5-isobutyl-1H-pyrazol-3-on, 2-Isopropyl-4-tert-butyl-5-isobutyl-1H-pyrazol-3-on, 2-Butyl-4-tert-butyl-5-isobutyl-1H-pyrazol-3-on, 2-Isobutyl-4-tert-butyl-5-isobutyl-1H-pyrazol-3-on, 2-tert-Butyl-4-tert-butyl-5-isobutyl-1H-pyrazol-3-on, 2-Phenyl-4-tert-butyl-5-isobutyl-1H-pyrazol-3-on, 2-(4-Methylphenyl)-4-tert-butyl-5-isobutyl-1H-pyrazol-3-on, 2-(2-Methylphenyl)-4-tert-butyl-5-isobutyl-1H-pyrazol-3-on, 2-(3-Methylphenyl)-4-tert-butyl-5-isobutyl-1H-pyrazol-3-on, 2-(4-Methoxy-4-tert-butyl-5-isobutyl-1H-pyrazol-3-on, 2-Methyl-4-tert-butyl-5-tert-butyl-1H-pyrazol-3-on, ,2-Ethyl-4-tert-butyl-5-tert-butyl-1H-pyrazol-3-on, 2-Propyl-4-tert-butyl-5-tert-butyl-1H-pyrazol-3-on, 2-Isopropyl-4-tert-butyl-5-tert-butyl-1H-pyrazol-3-on, 2-Butyl-4-tert-butyl-5-tert-butyl-1H-pyrazol-3-on, 2-Isobutyl-4-tert-butyl-5-tert-butyl-1H-pyrazol-3-on, 2-tert-Butyl-4-tert-butyl-5-tert-butyl-1H-pyrazol-3-on, 2-Phenyl-4-tert-butyl-5-tert-butyl-1H-pyrazol-3-on, 2-(4-Methylphenyl)-4-tert-butyl-5-tert-butyl-1H-pyrazol-3-on, 2-(2-Methylphenyl)-4-tert-butyl-5-tert-butyl-1H-pyrazol-3-on, 2-(3-Methylphenyl)-4-tert-butyl-5-tert-butyl-1H-pyrazol-3-on, 2-(4-Methoxy-4-tert-butyl-5-phenyl-1H-pyrazol-3-on, 2-Methyl-4-tert-butyl-5-phenyl-1H-pyrazol-3-on, ,2-Ethyl-4-tert-butyl-5-phenyl-1H-pyrazol-3-on, 2-Propyl-4-tert-butyl-5-phenyl-1H-pyrazol-3-on, 2-Isopropyl-4-tert-butyl-5-phenyl-1H-pyrazol-3-on, 2-Butyl-4-tert-butyl-5-phenyl-1H-pyrazol-3-on, 2-Isobutyl-4-tert-butyl-5-phenyl-1H-pyrazol-3-on, 2-tert-Butyl-4-tert-butyl-5-phenyl-1H-pyrazol-3-on, 2-Phenyl-4-tert-butyl-5-phenyl-1H-pyrazol-3-on, 2-(4-Methylphenyl)-4-tert-butyl-5-phenyl-1H-pyrazol-3-on, 2-(2-Methylphenyl)-4-tert-butyl-5-phenyl-1H-pyrazol-3-on, 2-(3-Methylphenyl)-4-tert-butyl-5-phenyl-1H-pyrazol-3-on, 2-(4-Methoxy-4-tert-butyl-5-(2-Methylphenyl)-1H-pyrazol-3-on, 2-Methyl-4-tert-butyl-5-(2-Methylphenyl)-1H-pyrazol-3-on, 2-Ethyl-4-tert-butyl-5-(2-Methylphenyl)-1H-pyrazol-3-on, 2-Propyl-4-tert-butyl-5-(2-Methylphenyl)-1H-pyrazol-3-on, 2-Isopropyl-4-tert-butyl-5-(2-Methylphenyl)-1H-pyrazol-3-on, 2-Butyl-4-tert-butyl-5-(2-Methylphenyl)-1H-pyrazol-3-on, 2-Isobutyl-4-tert-butyl-5-(2-Methylphenyl)-1H-pyrazol-3-on, 2-tert-Butyl-4-tert-butyl-5-(2-Methylphenyl)-1H-pyrazol-3-on, 2-Phenyl-4-tert-butyl-5-(2-Methylphenyl)-1H-pyrazol-3-on, 2-(4-Methylphenyl)-4-tert-butyl-5-(2-Methylphenyl)-1H-pyrazol-3-on, 2-(2-Methylphenyl)-4-tert-butyl-5-(2-Methylphenyl)-1H-pyrazol-3-on, 2-(3-Methylphenyl)-4-tert-butyl-5-(2-Methylphenyl)-1H-pyrazol-3-on, 2-(4-Methoxy-4-tert-butyl-5-(3-Methylphenyl)-1H-pyrazol-3-on, 2-Methyl-4-tert-butyl-5-(3-Methylphenyl)-1H-pyrazol-3-on, ,2-Ethyl-4-tert-butyl-5-(3-Methylphenyl)-1H-pyrazol-3-on, 2-Propyl-4-tert-butyl-5-(3-Methylphenyl)-1H-pyrazol-3-on, 2-Isopropyl-4-tert-butyl-5-(3-Methylphenyl)-1H-pyrazol-3-on,2-Butyl-4-tert-butyl-5-(3-Methylphenyl)-1H-pyrazol-3-on, 2-Isobutyl-4-tert-butyl-5-(3-Methylphenyl)-1H-pyrazol-3-on, 2-tert-Butyl-4-tert-butyl-5-(3-Methylphenyl)-1H-pyrazol-3-on, 2-Phenyl-4-tert-butyl-5-(3-Methylphenyl)-1H-pyrazol-3-on, 2-(4-Methylphenyl)-4-tert-butyl-5-(3-Methylphenyl)-1H-pyrazol-3-on, 2-(2-Methylphenyl)-4-tert-butyl-5-(3-Methyl-phenyl)-1H-pyrazol-3-on, 2-(3-Methylphenyl)-4-tert-butyl-5-(3-Methylphenyl)-1H-pyrazol-3-on, 2-(4-Methoxy-4-tert-butyl-5-(3-Methylphenyl)-1H-pyrazol-3-on, 2-Methyl-4-tert-butyl-5-(3-Methylphenyl)-1H-pyrazol-3-on, ,2-Ethyl-4-tert-butyl-5-(3-Methylphenyl)-1H-pyrazol-3-on, 2-Propyl-4-tert-butyl-5-(3-Methylphenyl)-1H-pyrazol-3-on, 2-Isopropyl-4-tert-butyl-5-(3-Methylphenyl)-1H-pyrazol-3-on, 2-Butyl-4-tert-butyl-5-(3-Methylphenyl)-1H-pyrazol-3-on, 2-Isobutyl-4-tert-butyl-5-(3-Methylphenyl)-1H-pyrazol-3-on, 2-tert-Butyl-4-tert-butyl-5-butyl-1H-pyrazol-3-on, 2-Phenyl-4-tert-butyl-5-(3-Methylphenyl)-1H-pyrazol-3-on, 2-(4-Methyl-phenyl)-4-tert-butyl-5-(3-Methylphenyl)-1H-pyrazol-3-on, 2-(2-Methylphenyl)-4-tert-butyl-5-(3-Methylphenyl)-1H-pyrazol-3-on, 2-(3-Methylphenyl)-4-tert-butyl-5-(3-Methylphenyl)-1H-pyrazol-3-on, 2-(4-Methoxy-4-tert-butyl-5-butyl-1H-pyrazol-3-on, 2-Methyl-4-tert-butyl-5-(4-Methylphenyl)-1H-pyrazol-3-on, 2-Ethyl-4-tert-butyl-5-(4-Methylphenyl)-1H-pyrazol-3-on, 2-Propyl-4-tert-butyl-5-(4-Methylphenyl)-1H-pyrazol-3-on, 2-Isopropyl-4-tert-butyl-5-(4-Methylphenyl)-1H-pyrazol-3-on, 2-Butyl-4-tert-butyl-5-(4-Methylphenyl)-1H-pyrazol-3-on, 2-Isobutyl-4-tert-butyl-5-(4-Methylphenyl)-1H-pyrazol-3-on, 2-tert-Butyl-4-tert-butyl-5-(4-Methylphenyl)-1H-pyrazol-3-on, 2-Phenyl-4-tert-butyl-5-(4-Methylphenyl)-1H-pyrazol-3-on, 2-(4-Methylphenyl)-4-tert-butyl-5-(4-Methylphenyl)-1H-pyrazol-3-on, 2-(2-Methylphenyl)-4-tert-butyl-5-(4-Methylphenyl)-1H-pyrazol-3-on, 2-(3-Methylphenyl)-4-tert-butyl-5-(4-Methyl-phenyl)-1H-pyrazol-3-on, 2-(4-Methoxy-4-tert-butyl-5-(4-Methylphenyl)-1H-pyrazol-3-on, 2-Methyl-4-phenyl-5-phenyl-1H-pyrazol-3-on, 2-Ethyl-4-phenyl-5-phenyl-1H-pyrazol-3-on, 2-Propyl-4-phenyl-5-phenyl-1H-pyrazol-3-on, 2-Isopropyl-4-phenyl-5-phenyl-1H-pyrazol-3-on, 2-Butyl-4-phenyl-5-phenyl-1H-pyrazol-3-on, 2-Isobutyl-4-phenyl-5-phenyl-1H-pyrazol-3-on, 2-tert-Butyl-4-phenyl-5-phenyl-1H-pyrazol-3-on, 2-Phenyl-4-phenyl-5-phenyl-1H-pyrazol-3-on, 2-(4-Methylphenyl)-4-phenyl-5-phenyl-1H-pyrazol-3-on, 2-(2-Methylphenyl)-4-phenyl-5-phenyl-1H-pyrazol-3-on, 2-(3-Methylphenyl)-4-phenyl-5-phenyl-1H-pyrazol-3-on, 2-(4-Methoxy-4-phenyl-5-phenyl-1H-pyrazol-3-on, 2-Methyl-4-phenyl-5-methyl-1H-pyrazol-3-on, , 2-Ethyl-4-phenyl-5-methyl-1H-pyrazol-3-on, 2-Propyl-4-phenyl-5-methyl-1H-pyrazol-3-on, 2-Isopropyl-4-phenyl-5-methyl-1H-pyrazol-3-on, 2-Butyl-4-phenyl-5-methyl-1H-pyrazol-3-on, 2-Isobutyl-4-phenyl-5-methyl-1H-pyrazol-3-on, 2-tert-Butyl-4-phenyl-5-methyl-1H-pyrazol-3-on, 2-Phenyl-4-phenyl-5-methyl-1H-pyrazol-3-on, 2-(4-Methylphenyl)-4-phenyl-5-methyl-1H-pyrazol-3-on, 2-(2-Methylphenyl)-4-phenyl-5-methyl-1H-pyrazol-3-on, 2-(3-Methyl-phenyl)-4-phenyl-5-methyl-1H-pyrazol-3-on, 2-(4-Methoxy-4-phenyl-5-methyl-1H-pyrazol-3-on, 2-methyl-4-phenyl-5-ethyl-1H-pyrazol-3-on, ,2-ethyl-4-phenyl-5-ethyl-1H-pyrazol-3-on, 2-propyl-4-phenyl-5-ethyl-1H-pyrazol-3-on, 2-isopropyl-4-phenyl-5-ethyl-1H-pyrazol-3-on, 2-butyl-4-phenyl-5-ethyl-1H-pyrazol-3-on, 2-Isobutyl-4-phenyl-5-ethyl-1H-pyrazol-3-on, 2-tert-butyl-4-phenyl-5-ethyl-1H-pyrazol-3-on, 2-Phenyl-4-phenyl-5-ethyl-1H-pyrazol-3-on, 2-(4-Methylphenyl)-4-phenyl-5-ethyl-1H-pyrazol-3-on, 2-(2-Methylphenyl)-4-phenyl-5-ethyl-1H-pyrazol-3-on, 2-(3-Methylphenyl)-4-phenyl-5-ethyl-1H-pyrazol-3-on, 2-(4-Methoxy-4-phenyl-5-ethyl-1H-pyrazol-3-on, 2-Methyl-4-phenyl-5-propyl-1H-pyrazol-3-on, ,2-Ethyl-4-phenyl-5-propyl-1H-pyrazol-3-on, 2-Propyl-4-phenyl-5-propyl-1H-pyrazol-3-on, 2-Isopropyl-4-phenyl-5-methyl-1H-pyrazol-3-on, 2-Butyl-4-phenyl-5-propyl-1H-pyrazol-3-on, 2-Isobutyl-4-phenyl-5-propyl-1H-pyrazol-3-on, 2-tert-Butyl-4-phenyl-5-propyl-1H-pyrazol-3-on, 2-Phenyl-4-phenyl-5-propyl-1H-pyrazol-3-on, 2-(4-Methylphenyl)-4-phenyl-5-propyl-1H-pyrazol-3-on, 2-(2-Methylphenyl)-4-phenyl-5-propyl-1H-pyrazol-3-on, 2-(3-Methyl-phenyl)-4-phenyl-5-propyl-1H-pyrazol-3-on, 2-(4-Methoxy-4-phenyl-5-propyl-1H-pyrazol-3-on, 2-Methyl-4-phenyl-5-isopropyl-1H-pyrazol-3-on, ,2-Ethyl-4-phenyl-5-isopropyl-1H-pyrazol-3-on, 2-Propyl-4-phenyl-5-isopropyl-1H-pyrazol-3-on, 2-Isopropyl-4-phenyl-5-isopropyl-1H-pyrazol-3-on, 2-Butyl-4-phenyl-5-isopropyl-1H-pyrazol-3-on, 2-Isobutyl-4-phenyl-5-isopropyl-1H-pyrazol-3-on, 2-tert-Butyl-4-phenyl-5-isopropyl-1H-pyrazol-3-on, 2-Phenyl-4-phenyl-5-isopropyl-1H-pyrazol-3-on, 2-(4-Methylphenyl)-4-phenyl-5-isopropyl-1H-pyrazol-3-on, 2-(2-Methylphenyl)-4-phenyl-5-isopropyl-1H-pyrazol-3-on, 2-(3-Methylphenyl)-4-phenyl-5-isopropyl-1H-pyrazol-3-on, 2-(4-Methoxy-4-phenyl-5-isopropyl-1H-pyrazol-3-on, 2-Methyl-4-phenyl-5-butyl-1H-pyrazol-3-on, ,2-Ethyl-4-phenyl-5-butyl-1H-pyrazol-3-on, 2-Propyl-4-phenyl-5-butyl-1H-pyrazol-3-on, 2-Isopropyl-4-phenyl-5-butyl-1H-pyrazol-3-on, 2-Butyl-4-phenyl-5-butyl-1H-pyrazol-3-on, 2-Isobutyl-4-phenyl-5-butyl-1H-pyrazol-3-on, 2-tert-Butyl-4-phenyl-5-butyl-1H-pyrazol-3-on, 2-Phenyl-4-phenyl-5-butyl-1H-pyrazol-3-on, 2-(4-Methylphenyl)-4-phenyl-5-butyl-1H-pyrazol-3-on, 2-(2-Methylphenyl)-4-phenyl-5-butyl-1H-pyrazol-3-on, 2-(3-Methylphenyl)-4-phenyl-5-butyl-1H-pyrazol-3-on, 2-(4-Methoxy-4-phenyl-5-butyl-1H-pyrazol-3-on, 1-(2-pyridyl)pyrazol-2-ol,
2-Methyl-4-phenyl-5-isobutyl-1H-pyrazol-3-on, 2-Ethyl-4-phenyl-5-isobutyl-1H-pyrazol-3-on, 2-Propyl-4-phenyl-5-isobutyl-1H-pyrazol-3-on, 2-Isopropyl-4-phenyl-5-isobutyl-1H-pyrazol-3-on,
2-Butyl-4-phenyl-5-isobutyl-1H-pyrazol-3-on, 2-Isobutyl-4-phenyl-5-isobutyl-1H-pyrazol-3-on,
2-tert-Butyl-4-phenyl-5-isobutyl-1H-pyrazol-3-on, 2-Phenyl-4-phenyl-5-isobutyl-1H-pyrazol-3-on, 2-(4-Methylphenyl)-4-phenyl-5-isobutyl-1H-pyrazol-3-on, 2-(2-Methylphenyl)-4-phenyl-5-isobutyl-1H-pyrazol-3-on, 2-(3-Methylphenyl)-4-phenyl-5-isobutyl-1H-pyrazol-3-on, 2-(4-Methoxy-4-phenyl-5-isobutyl-1H-pyrazol-3-on, 2-Methyl-4-phenyl-5-tert-butyl-1H-pyrazol-3-on, ,2-Ethyl-4-phenyl-5-tert-butyl-1H-pyrazol-3-on, 2-Propyl-4-phenyl-5-tert-butyl-1H-pyrazol-3-on, 2-Isopropyl-4-phenyl-5-tert-butyl-1H-pyrazol-3-on, 2-Butyl-4-phenyl-5-tert-butyl-1H-pyrazol-3-on, 2-Isobutyl-4-phenyl-5-tert-butyl-1H-pyrazol-3-on, 2-tert-Butyl-4-phenyl-5-tert-butyl-1H-pyrazol-3-on, 2-Phenyl-4-phenyl-5-tert-butyl-1H-pyrazol-3-on, 2-(4-Methylphenyl)-4-phenyl-5-tert-butyl-1H-pyrazol-3-on, 2-(2-Methylphenyl)-4-phenyl-5-tert-butyl-1H-pyrazol-3-on, 2-(3-Methylphenyl)-4-phenyl-5-tert-butyl-1H-pyrazol-3-on, 2-(4-Methoxy-4-phenyl-5-phenyl-1H-pyrazol-3-on, 2-Methyl-4-phenyl-5-phenyl-1H-pyrazol-3-on, ,2-Ethyl-4-phenyl-5-phenyl-1H-pyrazol-3-on, 2-Propyl-4-phenyl-5-phenyl-1H-pyrazol-3-on,
2-Isopropyl-4-phenyl-5-phenyl-1H-pyrazol-3-on, 2-Butyl-4-phenyl-5-phenyl-1H-pyrazol-3-on, 2-Isobutyl-4-phenyl-5-phenyl-1H-pyrazol-3-on, 2-tert-Butyl-4-phenyl-5-phenyl-1H-pyrazol-3-on, 2-Phenyl-4-phenyl-5-phenyl-1H-pyrazol-3-on, 2-(4-Methylphenyl)-4-phenyl-5-phenyl-1H-pyrazol-3-on, 2-(2-Methylphenyl)-4-phenyl-5-phenyl-1H-pyrazol-3-on, 2-(3-Methylphenyl)-4-phenyl-5-phenyl-1H-pyrazol-3-on, 2-(4-Methoxy-4-phenyl-5-(2-Methyl-phenyl)-1H-pyrazol-3-on, 2-Methyl-4-phenyl-5-(2-Methylphenyl)-1H-pyrazol-3-on, 2-Ethyl-4-phenyl-5-(2-Methylphenyl)-1H-pyrazol-3-on, 2-Propyl-4-phenyl-5-(2-Methylphenyl)-1H-pyrazol-3-on, 2-Isopropyl-4-phenyl-5-(2-Methylphenyl)-1H-pyrazol-3-on, 2-Butyl-4-phenyl-5-(2-Methylphenyl)-1H-pyrazol-3-on, 2-Isobutyl-4-phenyl-5-(2-Methylphenyl)-1H-pyrazol-3-on, 2-tert-Butyl-4-phenyl-5-(2-Methylphenyl)-1H-pyrazol-3-on, 2-Phenyl-4-phenyl-5-(2-Methylphenyl)-1H-pyrazol-3-on, 2-(4-Methylphenyl)-4-phenyl-5-(2-Methylphenyl)-1H-pyrazol-3-on, 2-(2-Methylphenyl)-4-phenyl-5-(2-Methylphenyl)-1H-pyrazol-3-on, 2-(3-Methylphenyl)-4-phenyl-5-(2-Methylphenyl)-1H-pyrazol-3-on, 2-(4-Methoxy-4-phenyl-5-(3-Methylphenyl)-1H-pyrazol-3-on, 2-Methyl-4-phenyl-5-(3-Methylphenyl)-1H-pyrazol-3-on, 2-Ethyl-4-phenyl-5-(3-Methylphenyl)-1H-pyrazol-3-on, 2-Propyl-4-phenyl-5-(3-Methylphenyl)-1H-pyrazol-3-on, 2-Isopropyl-4-phenyl-5-(3-Methylphenyl)-1H-pyrazol-3-on, 2-Butyl-4-phenyl-5-(3-Methylphenyl)-1H-pyrazol-3-on, 2-Isobutyl-4-phenyl-5-(3-Methylphenyl)-1H-pyrazol-3-on, 2-tert-Butyl-4-phenyl-5-(3-Methylphenyl)-1H-pyrazol-3-on, 2-Phenyl-4-phenyl-5-(3-Methylphenyl)-1H-pyrazol-3-on, 2-(4-Methylphenyl)-4-phenyl-5-(3-Methylphenyl)-1H-pyrazol-3-on, 2-(2-Methylphenyl)-4-phenyl-5-(3-Methylphenyl)-1H-pyrazol-3-on, 2-(3-Methylphenyl)-4-phenyl-5-(3-Methylphenyl)-1H-pyrazol-3-on, 2-(4-Methoxy-4-phenyl-5-(3-Methylphenyl)-1H-pyrazol-3-on, 2-Methyl-4-phenyl-5-(3-Methylphenyl)-1H-pyrazol-3-on, , 2-Ethyl-4-phenyl-5-(3-Methylphenyl)-1H-pyrazol-3-on, 2-Propyl-4-phenyl-5-(3-Methyl-phenyl)-1H-pyrazol-3-on, 2-Isopropyl-4-phenyl-5-(3-Methylphenyl)-1H-pyrazol-3-on, 2-Butyl-4-phenyl-5-(3-Methylphenyl)-1H-pyrazol-3-on, 2-Isobutyl-4-phenyl-5-(3-Methyl-phenyl)-1H-pyrazol-3-on, 2-tert-Butyl-4-phenyl-5-butyl-1H-pyrazol-3-on, 2-Phenyl-4-phenyl-5-(3-Methylphenyl)-1H-pyrazol-3-on, 2-(4-Methylphenyl)-4-phenyl-5-(3-Methylphenyl)-1H-pyrazol-3-on, 2-(2-Methylphenyl)-4-phenyl-5-(3-Methylphenyl)-1H-pyrazol-3-on, 2-(3-Methylphenyl)-4-phenyl-5-(3-Methylphenyl)-1H-pyrazol-3-on, 2-(4-Methoxy-4-phenyl-5-butyl-1H-pyrazol-3-on,
2-Methyl-4-phenyl-5-(4-Methylphenyl)-1H-pyrazol-3-on, 2-Ethyl-4-phenyl-5-(4-Methyl-phenyl)-1H-pyrazol-3-on, 2-Propyl-4-phenyl-5-(4-Methylphenyl)-1H-pyrazol-3-on, 2-Isopropyl-4-phenyl-5-(4-Methylphenyl)-1H-pyrazol-3-on, 2-Butyl-4-phenyl-5-(4-Methyl-phenyl)-1H-pyrazol-3-on, 2-Isobutyl-4-phenyl-5-(4-Methylphenyl)-1H-pyrazol-3-on, 2-tert-Butyl-4-phenyl-5-(4-Methylphenyl)-1H-pyrazol-3-on, 2-Phenyl-4-phenyl-5-(4-Methylphenyl) -1H-pyrazol-3-on, 2-(4-Methylphenyl)-4-phenyl-5-(4-Methylphenyl)-1H-pyrazol-3-on, 2-(2-Methylphenyl)-4-phenyl-5-(4-Methylphenyl)-1H-pyrazol-3-on, 2-(3-Methylphenyl)-4-phenyl-5-(4-Methylphenyl)-1H-pyrazol-3-on, 2-(4-Methoxy-4-phenyl-5-(4-Methylphenyl)-1H-pyrazol-3-on, 2-Methyl-4-(4-Methylphenyl)-5-phenyl-1H-pyrazol-3-on, ,2-Ethyl-4-(4-Methyl-phenyl)5-phenyl-1H-pyrazol-3-on, 2-Propyl-4-(4-Methylphenyl)-5-phenyl-1H-pyrazol-3-on, 2-Isopropyl-4-(4-Methylphenyl)-5-phenyl-1H-pyrazol-3-on, 2-Butyl-4-(4-Methylphenyl)-5-phenyl-1H-pyrazol-3-on, 2-Isobutyl-4-(4-Methylphenyl)-5-phenyl-1H-pyrazol-3-on, 2-tert-Butyl-4-(4-Methylphenyl)-5-phenyl-1H-pyrazol-3-on, 2-Phenyl-4-(4-Methylphenyl)-5-phenyl-1H-pyrazol-3-on, 2-(4-Methylphenyl)-4-(4-Methylphenyl)-5-phenyl-1H-pyrazol-3-on, 2-(2-Methylphenyl)-4-(4-Methylphenyl)-5-phenyl-1H-pyrazol-3-on, 2-(3-Methylphenyl)-4-(4-Methylphenyl)-5-phenyl-1H-pyrazol-3-on, 2-(4-Methoxy-4-(4-Methylphenyl)-5-phenyl-1H-pyrazol-3-on,
2-Methyl-4-(4-Methylphenyl)-5-methyl-1H-pyrazol-3-on, 2-Ethyl-4-(4-Methylphenyl)-5-methyl-1H-pyrazol-3-on, 2-propyl-4-(4-Methylphenyl)-5-methyl-1H-pyrazol-3-on, 2-Isopropyl-4-(4-Methylphenyl)-5-methyl-1H-pyrazol-3-on, 2-Butyl-4-(4-Methylphenyl)-5-methyl-1H-pyrazol-3-on, 2-Isobutyl-4-(4-Methylphenyl)-5-methyl-1H-pyrazol-3-on, 2-tert-Butyl-4-(4-Methyl-phenyl)-5-methyl-1H-pyrazol-3-on, 2-Phenyl-4-(4-Methylphenyl)-5-methyl-1H-pyrazol-3-on, 2-(4-Methylphenyl)-4-(4-Methylphenyl)-5-methyl-1H-pyrazol-3-on, 2-(2-Methylphenyl)-4-(4-Methylphenyl)-5-methyl-1H-pyrazol-3-on, 2-(3-Methylphenyl)-4-(4-Methylphenyl)-5-methyl-1H-pyrazol-3-on, 2-(4-Methoxy-4-(4-Methylphenyl)-5-methyl-1H-pyrazol-3-on, 2-methyl-4-(4-Methylphenyl)-5-ethyl-1H-pyrazol-3-on, ,2-ethyl-4-(4-Methylphenyl)-5-ethyl-1H-pyrazol-3-on, 2-propyl-4-(4-Methylphenyl)-5-ethyl-1H-pyrazol-3-on, 2-isopropyl-4-(4-Methylphenyl)-5-ethyl-1H-pyrazol-3-on, 2-butyl-4-(4-Methylphenyl)-5-ethyl-1H-pyrazol-3-on, 2-Isobutyl-4-(4-Methylphenyl)-5-ethyl-1H-pyrazol-3-on, 2-tert-butyl-4-(4-Methylphenyl)-5-ethyl-1H-pyrazol-3-on, 2-Phenyl-4-(4-Methylphenyl)-5-ethyl-1H-pyrazol-3-on, 2-(4-Methylphenyl)-4-(4-Methylphenyl)-5-ethyl-1H-pyrazol-3-on, 2-(2-Methylphenyl)-4-(4-Methylphenyl)-5-ethyl-1H-pyrazol-3-on, 2-(3-Methylphenyl)-4-(4-Methylphenyl)-5-ethyl-1H-pyrazol-3-on, 2-(4-Methoxy-4-(4-Methylphenyl)-5- ethyl -1 H-pyrazol-3-on, 2-Methyl-4-(4-Methylphenyl)-5-propyl-1 H-pyrazol-3-on, 2-Ethyl-4-(4-Methylphenyl)-5- propyl -1 H-pyrazol-3-on, 2-Propyl-4-(4-Methylphenyl)-5- propyl -1 H-pyrazol-3-on, 2-Isopropyl-4-(4-Methylphenyl)-5- methyl -1H-pyrazol-3-on, 2-Butyl-4-(4-Methylphenyl)-5- propyl -1 H-pyrazol-3-on, 2-Isobutyl- 4-(4-Methylphenyl)-5- propyl -1 H-pyrazol-3-on,
2-tert-Butyl-4-(4-Methylphenyl)-5- propyl -1 H-pyrazol-3-on, 2-Phenyl-4-(4-Methylphenyl)-5-propyl -1 H-pyrazol-3-on, 2-(4-Methylphenyl)-4-(4-Methylphenyl)-5- propyl -1 H-pyrazol-3-on, 2-(2-Methylphenyl)-4-(4-Methylphenyl)-5- propyl -1 H-pyrazol-3-on, 2-(3-Methylphenyl)-4-(4-Methylphenyl)-5- propyl -1 H-pyrazol-3-on, 2-(4-Methoxy-4-(4-Methylphenyl)-5- propyl -1H-pyrazol-3-on, 2-Methyl-4-(4-Methylphenyl)-5-isopropyl-1 H-pyrazol-3-on, 2-Ethyl-4-(4-Methylphenyl)-5- isopropyl -1 H-pyrazol-3-on, 2-Propyl-4-(4-Methylphenyl)-5- isopropyl -1 H-pyrazol-3-on, 2-Isopropyl-4-(4-Methylphenyl)-5- isopropyl -1 H-pyrazol-3-on, 2-Butyl-4-(4-Methylphenyl)-5- isopropyl -1 H-pyrazol-3-on, 2-Isobutyl- 4-(4-Methylphenyl)-5- isopropyl -1H-pyrazol-3-on, 2-tert-Butyl-4-(4-Methylphenyl)-5- isopropyl -1 H-pyrazol-3-on, 2-Phenyl-4-(4-Methylphenyl)-5- isopropyl -1 H-pyrazol-3-on, 2-(4-Methylphenyl)-4-(4-Methylphenyl)-5- isopropyl -1 H-pyrazol-3-on, 2-(2-Methylphenyl)-4-(4-Methylphenyl)-5- isopropyl -1 H-pyrazol-3-on, 2-(3-Methylphenyl)-4-(4-Methylphenyl)-5- isopropyl -1 H-pyrazol-3-on, 2-(4-Methoxy-4-(4-Methylphenyl)-5- isopropyl -1 H-pyrazol-3-on, 2-Methyl-4-(4-Methylphenyl)-5- butyl -1H-pyrazol-3-on, 2-Ethyl-4-(4-Methylphenyl)-5- butyl-1 H-pyrazol-3-on, 2-Propyl-4-(4-Methylphenyl)-5- butyl -1 H-pyrazol-3-on, 2-Isopropyl-4-(4-Methylphenyl)-5- butyl -1 H-pyrazol-3-on, 2-Butyl-4-(4-Methylphenyl)-5- butyl -1 H-pyrazol-3-on, 2-Isobutyl- 4-(4-Methylphenyl)-5- butyl-1 H-pyrazol-3-on, 2-tert-Butyl-4-(4-Methylphenyl)-5- butyl -1 H-pyrazol-3-on, 2-Phenyl-4-(4-Methylphenyl)-5- butyl -1 H-pyrazol-3-on, 2-(4-Methylphenyl)-4-(4-Methylphenyl)-5- butyl -1H-pyrazol-3-on, 2-(2-Methylphenyl)-4-(4-Methylphenyl)-5- butyl -1 H-pyrazol-3-on, 2-(3-Methylphenyl)-4-(4-Methylphenyl)-5- butyl -1 H-pyrazol-3-on, 2-(4-Methoxy-4-(4-Methylphenyl)-5- butyl -1 H-pyrazol-3-on, 2-Methyl-4-(4-Methylphenyl)-5- isobutyl -1 H-pyrazol-3-on,2-Ethyl-4-(4-Methylphenyl)-5- isobutyl -1 H-pyrazol-3-on, 2-Propyl-4-(4-Methylphenyl)-5-isobutyl -1 H-pyrazol-3-on, 2-Isopropyl-4-(4-Methylphenyl)-5- isobutyl -1 H-pyrazol-3-on,
2-Butyl-4-(4-Methylphenyl)-5- isobutyl -1 H-pyrazol-3-on, 2-Isobutyl- 4-(4-Methylphenyl)-5-isobutyl -1 H-pyrazol-3-on, 2-tert-Butyl-4-(4-Methylphenyl)-5- isobutyl -1 H-pyrazol-3-on, 2-Phenyl-4-(4-Methylphenyl)-5- isobutyl -1 H-pyrazol-3-on,
2-(4-Methylphenyl)-4-(4-Methylphenyl)-5- isobutyl -1 H-pyrazol-3-on, 2-(2-Methylphenyl)-4-(4-Methylphenyl)-5- isobutyl -1 H-pyrazol-3-on,
2-(3-Methylphenyl)-4-(4-Methylphenyl)-5- isobutyl -1H-pyrazol-3-on, 2-(4-Methoxy-4-(4-Methylphenyl)-5- isobutyl -1H-pyrazol-3-on, 2-Methyl-4-(4-Methylphenyl)-5- tert-butyl -1H-pyrazol-3-on, 2-Ethyl-4-(4-Methylphenyl)-5- tert-butyl -1H-pyrazol-3-on, 2-Propyl-4-(4-Methylphenyl)-5- tert-butyl -1H-pyrazol-3-on, 2-Isopropyl-4-(4-Methylphenyl)-5-tert-butyl -1H-pyrazol-3-on,
2-Butyl-4-(4-Methylphenyl)-5- tert-butyl -1H-pyrazol-3-on, 2-Isobutyl-4-(4-Methylphenyl)-5-tert-butyl -1H-pyrazol-3-on,
2-tert-Butyl-4-(4-Methylphenyl)-5- tert-butyl -1H-pyrazol-3-on, 2-Phenyl-4-(4-Methylphenyl)-5-tert-butyl -1H-pyrazol-3-on, 2-(4-Methylphenyl)-4-(4-Methylphenyl)-5- tert-butyl -1H-pyrazol-3-on, 2-(2-Methylphenyl)-4-(4-Methylphenyl)-5- tert-butyl -1H-pyrazol-3-on, 2-(3-Methylphenyl)-4-(4-Methylphenyl)-5- tert-butyl -1H-pyrazol-3-on, 2-(4-Methoxy-4-(4-Methylphenyl)-5- phenyl-1H-pyrazol-3-on, 2-Methyl-4-(4-Methylphenyl)-5- phenyl -1H-pyrazol-3-on, 2-Ethyl-4-(4-Methylphenyl)-5- phenyl -1H-pyrazol-3-on, 2-Propyl-4-(4-Methylphenyl)-5- phenyl -1H-pyrazol-3-on, 2-Isopropyl-4-(4-Methylphenyl)-5-phenyl -1H-pyrazol-3-on, 2-Butyl-4-(4-Methylphenyl)-5- phenyl -1H-pyrazol-3-on, 2-Isobutyl-4-(4-Methylphenyl)-5- phenyl -1H-pyrazol-3-on, 2-tert-Butyl-4-(4-Methylphenyl)-5- phenyl -1H-pyrazol-3-on, 2-Phenyl-4-(4-Methylphenyl)-5- phenyl -1H-pyrazol-3-on, 2-(4-Methylphenyl)-4-(4-Methylphenyl)-5- phenyl-1H-pyrazol-3-on, 2-(2-Methylphenyl)-4-(4-Methylphenyl)-5- phenyl -1H-pyrazol-3-on, 2-(3-Methylphenyl)-4-(4-Methylphenyl)-5- phenyl -1H-pyrazol-3-on, 2-(4-Methoxy-4-(4-Methylphenyl)-5- (2-Methylphenyl)-1H-pyrazol-3-on, 2-Methyl-4-(4-Methylphenyl)-5- (2-Methylphenyl) -1H-pyrazol-3-on, 2-Ethyl-4-(4-Methylphenyl)-5- (2-Methylphenyl) -1H-pyrazol-3-on, 2-Propyl-4-(4-Methylphenyl)-5- (2-Methylphenyl) -1H-pyrazol-3-on, 2-Isopropyl-4-(4-Methylphenyl)-5- (2-Methylphenyl) -1H-pyrazol-3-on, 2-Butyl-4-(4-Methylphenyl)-5- (2-Methylphenyl) -1H-pyrazol-3-on,2-Isobutyl- 4-(4-Methylphenyl)-5- (2-Methylphenyl) -1H-pyrazol-3-on, 2-tert-Butyl-4-(4-Methylphenyl)-5- (2-Methylphenyl) -1H-pyrazol-3-on, 2-Phenyl-4-(4-Methylphenyl)-5- (2-Methylphenyl) -1H-pyrazol-3-on, 2-(4-Methylphenyl)-4-(4-Methylphenyl)-5- (2-Methylphenyl) -1H-pyrazol-3-on, 2-(2-Methylphenyl)-4-(4-Methylphenyl)-5- (2-Methylphenyl) -1H-pyrazol-3-on, 2-(3-Methylphenyl)-4-(4-Methylphenyl)-5- (2-Methylphenyl)-1H-pyrazol-3-on, 2-(4-Methoxy-4-(4-Methylphenyl)-5- (3-Methylphenyl) -1H-pyrazol-3-on, 2-Methyl-4-(4-Methylphenyl)-5- (3-Methylphenyl) -1H-pyrazol-3-on, 2-Ethyl-4-(4-Methylphenyl)-5- (3-Methylphenyl) -1H-pyrazol-3-on, 2-Propyl-4-(4-Methylphenyl)-5- (3-Methylphenyl) -1H-pyrazol-3-on, 2-Isopropyl-4-(4-Methylphenyl)-5-(3-Methylphenyl) -1H-pyrazol-3-on, 2-Butyl-4-(4-Methylphenyl)-5- (3-Methylphenyl) -1H-pyrazol-3-on, 2-Isobutyl-4-(4-Methylphenyl)-5-(3-Methylphenyl) -1H-pyrazol-3-on, 2-tert-Butyl-4-(4-Methylphenyl)-5- (3-Methylphenyl) -1H-pyrazol-3-on, 2-Phenyl-4-(4-Methylphenyl)-5- (3-Methylphenyl) -1H-pyrazol-3-on, 2-(4-Methylphenyl)-4-(4-Methylphenyl)-5- (3-Methylphenyl) -1H-pyrazol-3-on, 2-(2-Methylphenyl)-4-(4-Methylphenyl)-5- (3-Methylphenyl) -1H-pyrazol-3-on, 2-(3-Methylphenyl)-4-(4-Methylphenyl)-5- (3-Methylphenyl) -1H-pyrazol-3-on, 2-(4-Methoxy-4-(4-Methylphenyl)-5- (3-Methylphenyl) -1H-pyrazol-3-on, 2-Methyl-4-(4-Methylphenyl)-5- (3-Methylphenyl) -1H-pyrazol-3-on, 2-Ethyl-4-(4-Methylphenyl)-5- (3-Methylphenyl) -1H-pyrazol-3-on, 2-Propyl-4-(4-Methylphenyl)-5- (3-Methylphenyl) -1H-pyrazol-3-on, 2-Isopropyl-4-(4-Methylphenyl)-5-(3-Methylphenyl) -1H-pyrazol-3-on, 2-Butyl-4-(4-Methylphenyl)-5- (3-Methylphenyl) -1H-pyrazol-3-on, 2-Isobutyl-4-(4-Methylphenyl)-5- (3-Methylphenyl) -1H-pyrazol-3-on, 2-tert-Butyl-4-(4-Methylphenyl)-5- butyl -1H-pyrazol-3-on, 2-Phenyl-4-(4-Methylphenyl)-5- (3-Methylphenyl)-1H-pyrazol-3-on, 2-(4-Methylphenyl)-4-(4-Methylphenyl)-5- (3-Methylphenyl) -1H-pyrazol-3-on, 2-(2-Methylphenyl)-4-(4-Methylphenyl)-5- (3-Methylphenyl) -1H-pyrazol-3-on, 2-(3-Methylphenyl)-4-(4-Methylphenyl)-5- (3-Methylphenyl) -1H-pyrazol-3-on, 2-(4-Methoxy-4-(4-Methylphenyl)-5- butyl -1H-pyrazol-3-on, 2-Methyl-4-(4-Methylphenyl)-5- (4-Methylphenyl)-1H-pyrazol-3-on, , 2-Ethyl-4-(4-Methylphenyl)-5- (4-Methylphenyl) -1H-pyrazol-3-on, 2-Propyl-4-(4-Methylphenyl)-5- (4-Methylphenyl) -1H-pyrazol-3-on, 2-Isopropyl-4-(4-Methylphenyl)-5- (4-Methylphenyl) -1H-pyrazol-3-on, 2-Butyl-4-(4-Methylphenyl)-5- (4-Methylphenyl) -1H-pyrazol-3-on, 2-Isobutyl- 4-(4-Methylphenyl)-5- (4-Methylphenyl) -1H-pyrazol-3-on, 2-tert-Butyl-4-(4-Methylphenyl)-5- (4-Methylphenyl) -1H-pyrazol-3-on, 2-Phenyl-4-(4-Methylphenyl)-5- (4-Methylphenyl) -1H-pyrazol-3-on, 2-(4-Methylphenyl)-4-(4-Methylphenyl)-5- (4-Methylphenyl) -1H-pyrazol-3-on, 2-(2-Methylphenyl)-4-(4-Methylphenyl)-5- (4-Methylphenyl) -1H-pyrazol-3-on, 2-(3-Methylphenyl)-4-(4-Methylphenyl)-5- (4-Methylphenyl) -1H-pyrazol-3-on, 2-(4-Methoxy)-4-(4-Methylphenyl)-5- (4-Methylphenyl)-1H-pyrazol-3-on,
1-Methyl-2-phenyl-4-amino-5-methyl-pyrazol-3-on, 1-Ethyl-2-phenyl-4-amino-5-methyl-pyrazol-3-on, 1-Propyl-2-phenyl-4-amino-5-methyl-pyrazol-3-on, 1-Isopropyl-2-phenyl-4-amino-5-methyl-pyrazol-3-on, 1-Butyl-2-phenyl-4-amino-5-methyl-pyrazol-3-on, 1-Isobutyl-2-phenyl-4-amino-5-methyl-pyrazol-3-on, 1-tert-Butyl-2-phenyl-4-amino-5-methyl-pyrazol-3-on, 1-Phenyl-2-phenyl-4-amino-5-methyl-pyrazol-3-on, 1-(2-Methylphenyl)-2-phenyl-4-amino-5-methyl-pyrazol-3-on, 1-(3-Methylphenyl)-2-phenyl-4-amino-5-methyl-pyrazol-3-on, 1-(4-Methylphenyl)-2-phenyl-4-amino-5-methyl-pyrazol-3-on, 1-Benzyl-2-phenyl-4-amino-5-methyl-pyrazol-3-on, 1-(2-Methoxyphenyl)-2-phenyl-4-amino-5-methyl-pyrazol-3-on, 1-(4-Methoxyphenyl)-2-phenyl-4-amino-5-methyl-pyrazol-3-on, 1-Cyclohexyl-2-phenyl-4-amino-5-methyl-pyrazol-3-on, 1-Cyclopentyl-2-phenyl-4-amino-5-methyl-pyrazol-3-on, 1-Methyl-2-phenyl-4-formylamido-5-methyl-pyrazol-3-on, 1-Ethyl-2-phenyl-4-formylamido-5-methyl-pyrazol-3-on, 1-Propyl-2-phenyl-4-formylamido-5-methyl-pyrazol-3-on, 1-Isopropyl-2-phenyl-4-formylamido-5-methyl-pyrazol-3-on, 1-Butyl-2-phenyl-4-formylamido-5-methyl-pyrazol-3-on, 1-Isobutyl-2-phenyl-4-formylamido-5-methyl-pyrazol-3-on, 1-tert-Butyl-2-phenyl-4-formylamido-5-methyl-pyrazol-3-on, 1-Phenyl-2-phenyl-4-formylamido-5-methyl-pyrazol-3-on, 1-(2-Methylphenyl)-2-phenyl-4-formylamido-5-methyl-pyrazol-3-on, 1-(3-Methylphenyl)-2-phenyl-4-formylamido-5-methyl-pyrazol-3-on, 1-(4-Methylphenyl)-2-phenyl-4-formylamido-5-methyl-pyrazol-3-on, 1-Benzyl-2-phenyl-4-formylamido-5-methyl-pyrazol-3-on, 1-(2-Methoxy-phenyl)-2-phenyl-4-formylamido-5-methyl-pyrazol-3-on, 1-(4-Methoxyphenyl)-2-phenyl-4-formylamido-5-methyl-pyrazol-3-on, 1-Cyclohexyl-2-phenyl-4-formylamido-5-methyl-pyrazol-3-on, 1-Cyclopentyl-2-phenyl-4-formylamido-5-methyl-pyrazol-3-on, 1-Methyl-2-phenyl-4-acetylamido-5-methyl-pyrazol-3-on, 1-Ethyl-2-phenyl-4-acetylamido-5-methyl-pyrazol-3-on, 1-Propyl-2-phenyl-4-acetylamido-5-methyl-pyrazol-3-on, 1-Isopropyl-2-phenyl-4-acetylamido-5-methyl-pyrazol-3-on, 1-Butyl-2-phenyl-4-acetylamido-5-methyl-pyrazol-3-on, 1-Isobutyl-2-phenyl-4-acetylamido-5-methyl-pyrazol-3-on, 1-tert-Butyl-2-phenyl-4-acetylamido-5-methyl-pyrazol-3-on, 1-Phenyl-2-phenyl-4-acetylamido-5-methyl-pyrazol-3-on, 1-(2-Methylphenyl)-2-phenyl-4-acetylamido-5-methyl-pyrazol-3-on, 1-(3-Methylphenyl)-2-phenyl-4-acetylamido-5-methyl-pyrazol-3-on, 1-(4-Methylphenyl)-2-phenyl-4-acetylamido-5-methyl-pyrazol-3-on, 1-Benzyl-2-phenyl-4-acetylamido-5-methyl-pyrazol-3-on, 1-(2-Methoxyphenyl)-2-phenyl-4-acetylamido-5-methyl-pyrazol-3-on, 1-(4-Methoxyphenyl)-2-phenyl-4-acetylamido-5-methyl-pyrazol-3-on, 1-Cyclohexyl-2-phenyl-4-acetylamido-5-methyl-pyrazol-3-on, 1-Cyclopentyl-2-phenyl-4-acetylamido-5-methyl-pyrazol-3-on, 2-Methyl-1-phenyl-4-amino-5-methyl-pyrazol-3-on, 2-Ethyl-1-phenyl-4-amino-5-methyl-pyrazol-3-on, 2-Propyl-1-phenyl-4-amino-5-methyl-pyrazol-3-on, 2-Isopropyl-1-phenyl-4-amino-5-methyl-pyrazol-3-on, 2-Butyl-1-phenyl-4-amino-5-methyl-pyrazol-3-on, 2-Isobutyl-1-phenyl-4-amino-5-methyl-pyrazol-3-on, 2-tert-Butyl-1-phenyl-4-amino-5-methyl-pyrazol-3-on, 2-Phenyl-1-phenyl-4-amino-5-methyl-pyrazol-3-on, 2-(o-methylphenyl)-1-phenyl-4-amino-5-methyl-pyrazol-3-on, 2-(3-Methylphenyl)-1-phenyl-4-amino-5-methyl-pyrazol-3-on, 2-(4-Methylphenyl)-1-phenyl-4-amino-5-methyl-pyrazol-3-on, 2-Benzyl-1-phenyl-4-amino-5-methyl-pyrazol-3-on, 2-(o-methoxyphenyl)-1-phenyl-4-amino-5-methyl-pyrazol-3-on, 2-(4-Methoxyphenyl)-1-phenyl-4-amino-5-methyl-pyrazol-3-on, 2-Cyclohexyl-1-phenyl-4-amino-5-methyl-pyrazol-3-on, 2-Cyclopentyl-1-phenyl-4-amino-5-methyl-pyrazol-3-on, 2-Methyl-1-phenyl-4-formylamido-5-methyl-pyrazol-3-on, 2-Ethyl-1-phenyl-4-formylamido-5-methyl-pyrazol-3-on, 2-Propyl-1-phenyl-4-formylamido-5-methyl-pyrazol-3-on, 2-Isopropyl-1-phenyl-4-formylamido-5-methyl-pyrazol-3-on, 2-Butyl-1-phenyl-4-formylamido-5-methyl-pyrazol-3-on, 2-Isobutyl-1-phenyl-4-formylamido-5-methyl-pyrazol-3-on, 2-tert-Butyl-1-phenyl-4-formylamido-5-methyl-pyrazol-3-on, 2-Phenyl-1-phenyl-4-formylamido-5-methyl-pyrazol-3-on, 2-(o-methylphenyl)-1-phenyl-4-formylamido-5-methyl-pyrazol-3-on, 2-(3-Methylphenyl)-1-phenyl-4-formylamido-5-methyl-pyrazol-3-on, 2-(4-Methylphenyl)-1-phenyl-4-formylamido-5-methyl-pyrazol-3-on, 2-Benzyl-1-phenyl-4-formyl-amido-5-methyl-pyrazol-3-on, 2-(o-methoxyphenyl)-1-phenyl-4-formylamido-5-methyl-pyrazol-3-on, 2-(4-Methoxyphenyl)-1-phenyl-4-formylamido-5-methyl-pyrazol-3-on, 2-Cyclohexyl-1-phenyl-4-formylamido-5-methyl-pyrazol-3-on, 2-Cyclopentyl-1-phenyl-4-formylamido-5-methyl-pyrazol-3-on, 2-Methyl-1-phenyl-4-acetylamido-5-methyl-pyrazol-3-on, 2-Ethyl-1-phenyl-4-acetylamido-5-methyl-pyrazol-3-on, 2-Propyl-1-phenyl-4-acetylamido-5-methyl-pyrazol-3-on, 2-Isopropyl-1-phenyl-4-acetylamido-5-methyl-pyrazol-3-on, 2-Butyl-1-phenyl-4-acetylamido-5-methyl-pyrazol-3-on, 2-Isobutyl-1-phenyl-4-acetylamido-5-methyl-pyrazol-3-on, 2-tert-Butyl-1-phenyl-4-acetylamido-5-methyl-pyrazol-3-on, 2-Phenyl-1-phenyl-4-acetylamido-5-methyl-pyrazol-3-on, 2-(o-methylphenyl)-1-phenyl-4-acetylamido-5-methyl-pyrazol-3-on, 2-(3-Methylphenyl)-1-phenyl-4-acetylamido-5-methyl-pyrazol-3-on, 2-(4-Methylphenyl)-1-phenyl-4-acetylamido-5-methyl-pyrazol-3-on, 2-Benzyl-1-phenyl-4-acetylamido-5-methyl-pyrazol-3-on, 2-(o-methoxyphenyl)-1-phenyl-4-acetylamido-5-methyl-pyrazol-3-on, 2-(4-Methoxyphenyl)-1-phenyl-4-acetylamido-5-methyl-pyrazol-3-on, 2-Cyclohexyl-1-phenyl-4-acetylamido-5-methyl-pyrazol-3-on, 2-Cyclopentyl-1-phenyl-4-acetylamido-5-methyl-pyrazol-3-on, 2-Methyl-1-phenyl-4-amino-5-ethyl-pyrazol-3-on, 2-Ethyl-1-phenyl-4-amino-5-ethyl-pyrazol-3-on, 2-Propyl-1-phenyl-4-amino-5-ethyl-pyrazol-3-on, 2-Isopropyl-1-phenyl-4-amino-5-ethyl-pyrazol-3-on, 2-Butyl-1-phenyl-4-amino-5-ethyl-pyrazol-3-on, 2-Isobutyl-1-phenyl-4-amino-5-ethyl-pyrazol-3-on, 2-tert-Butyl-1-phenyl-4-amino-5-ethyl-pyrazol-3-on, 2-Phenyl-1-phenyl-4-amino-5-ethyl-pyrazol-3-on, 2-(o-methylphenyl)-1-phenyl-4-amino-5-ethyl-pyrazol-3-on, 2-(3-Methylphenyl)-1-phenyl-4-amino-5-ethyl-pyrazol-3-on, 2-(4-Methylphenyl)-1-phenyl-4-amino-5-ethyl-pyrazol-3-on, 2-Benzyl-1-phenyl-4-amino-5-ethyl-pyrazol-3-on, 2-(o-methoxyphenyl)-1-phenyl-4-amino-5-ethyl-pyrazol-3-on, 2-(4-Methoxyphenyl)-1-phenyl-4-amino-5-ethyl-pyrazol-3-on, 2-Cyclohexyl-1-phenyl-4-amino-5-ethyl-pyrazol-3-on, 2-Cyclopentyl-1-phenyl-4-amino-5-ethyl-pyrazol-3-on, 2-Methyl-1-phenyl-4-formylamido-5-ethyl-pyrazol-3-on, 2-Ethyl-1-phenyl-4-formylamido-5-ethyl-pyrazol-3-on, 2-Propyl-1-phenyl-4-formylamido-5-ethyl-pyrazol-3-on, 2-Isopropyl-1-phenyl-4-formylamido-5-ethyl-pyrazol-3-on, 2-Butyl-1-phenyl-4-formylamido-5-ethyl-pyrazol-3-on, 2-Isobutyl-1-phenyl-4-formylamido-5-ethyl-pyrazol-3-on, 2-tert-Butyl-1-phenyl-4-formylamido-5-ethyl-pyrazol-3-on, 2-Phenyl-1-phenyl-4-formylamido-5-ethyl-pyrazol-3-on, 2-(o-methylphenyl)-1-phenyl-4-formylamido-5-ethyl-pyrazol-3-on, 2-(3-Methylphenyl)-1-phenyl-4-formylamido-5-ethyl-pyrazol-3-on, 2-(4-Methylphenyl)-1-phenyl-4-formylamido-5-ethyl-pyrazol-3-on, 2-Benzyl-1-phenyl-4-formylamido-5-ethyl-pyrazol-3-on, 2-(o-methoxyphenyl)-1-phenyl-4-formylamido-5-ethyl-pyrazol-3-on, 2-(4-Methoxyphenyl)-1-phenyl-4-formylamido-5-ethyl-pyrazol-3-on, 2-Cyclohexyl-1-phenyl-4-formylamido-5-ethyl-pyrazol-3-on, 2-Cyclopentyl-1-phenyl-4-formylamido-5-ethyl-pyrazol-3-on, 2-Methyl-1-phenyl-4-acetylamido-5-ethyl-pyrazol-3-on, 2-Ethyl-1-phenyl-4-acetylamido-5-ethyl-pyrazol-3-on, 2-Propyl-1-phenyl-4-acetylamido-5-ethyl-pyrazol-3-on, ,2-Isopropyl-1-phenyl-4-acetylamido-5-ethyl-pyrazol-3-on 2-Butyl-1-phenyl-4-acetylamido-5-ethyl-pyrazol-3-on, 2-Isobutyl-1-phenyl-4-acetylamido-5-ethyl-pyrazol-3-on, 2-tert-Butyl-1-phenyl-4-acetylamido-5-ethyl-pyrazol-3-on, 2-Phenyl-1-phenyl-4-acetylamido-5-ethyl-pyrazol-3-on, 2-(o-methylphenyl)-1-phenyl-4-acetylamido-5-ethyl-pyrazol-3-on, 2-(3-Methylphenyl)-1-phenyl-4-acetylamido-5-ethyl-pyrazol-3-on, 2-(4-Methylphenyl)-1-phenyl-4-acetylamido-5-ethyl-pyrazol-3-on, 2-Benzyl-1-phenyl-4-acetylamido-5-ethyl-pyrazol-3-on, 2-(o-methoxyphenyl)-1-phenyl-4-acetylamido-5-ethyl-pyrazol-3-on, 2-(4-Methoxyphenyl)-1-phenyl-4-acetylamido-5-ethyl-pyrazol-3-on, 2-Cyclohexyl-1-phenyl-4-acetylamido-5-ethyl-pyrazol-3-on, 2-Cyclopentyl-1-phenyl-4-acetylamido-5-ethyl-pyrazol-3-on, 2-Methyl-1-phenyl-4-amino-5-tert-butyl-pyrazol-3-on, 2-Ethyl-1-phenyl-4-amino-5-tert-butyl-pyrazol-3-on, 2-Propyl-1-phenyl-4-amino-5-tert-butyl-pyrazol-3-on, 2-Isopropyl-1-phenyl-4-amino-5-tert-butyl-pyrazol-3-on, 2-Butyl-1-phenyl-4-amino-5-tert-butyl-pyrazol-3-on, 2-Isobutyl-1-phenyl-4-amino-5-tert-butyl-pyrazol-3-on, 2-tert-Butyl-1-phenyl-4-amino-5-tert-butyl-pyrazol-3-on, 2-Phenyl-1-phenyl-4-amino-5-tert-butyl-pyrazol-3-on, 2-(o-methylphenyl)-1-phenyl-4-amino-5-tert-butyl-pyrazol-3-on, 2-(3-Methylphenyl)-1-phenyl-4-amino-5-tert-butyl-pyrazol-3-on, 2-(4-Methylphenyl)-1-phenyl-4-amino-5-tert-butyl-pyrazol-3-on, 2-Benzyl-1-phenyl-4-amino-5-tert-butyl-pyrazol-3-on, 2-(o-methoxyphenyl)-1-phenyl-4-amino-5-tert-butyl-pyrazol-3-on, 2-(4-Methoxyphenyl)-1-phenyl-4-amino-5-tert-butyl-pyrazol-3-on, 2-Cyclohexyl-1-phenyl-4-amino-5-tert-butyl-pyrazol-3-on, 2-Cyclopentyl-1-phenyl-4-amino-5-tert-butyl-pyrazol-3-on, 2-Methyl-1-phenyl-4-formylamido-5-tert-butyl-pyrazol-3-on, 2-Ethyl-1-phenyl-4-formylamido-5-tert-butyl-pyrazol-3-on, 2-Propyl-1-phenyl-4-formylamido-5-tert-butyl-pyrazol-3-on, 2-Isopropyl-1-phenyl-4-formylamido-5-tert-butyl-pyrazol-3-on, 2-Butyl-1-phenyl-4-formylamido-5-tert-butyl-pyrazol-3-on, 2-Isobutyl-1-phenyl-4-formylamido-5-tert-butyl-pyrazol-3-on, 2-tert-Butyl-1-phenyl-4-formylamido-5-tert-butyl-pyrazol-3-on, 2-Phenyl-1-phenyl-4-formylamido-5-tert-butyl-pyrazol-3-on, 2-(o-methylphenyl)-1-phenyl-4-formylamido-5-tert-butyl-pyrazol-3-on, 2-(3-Methylphenyl)-1-phenyl-4-formylamido-5-tert-butyl-pyrazol-3-on, 2-(4-Methylphenyl)-1-phenyl-4-formylamido-5-tert-butyl-pyrazol-3-on, 2-Benzyl-1-phenyl-4-formylamido-5-tert-butyl-pyrazol-3-on, 2-(o-methoxyphenyl)-1-phenyl-4-formylamido-5-tert-butyl-pyrazol-3-on, 2-(4-Methoxyphenyl)-1-phenyl-4-formylamido-5-tert-butyl-pyrazol-3-on, 2-Cyclohexyl-1-phenyl-4-formylamido-5-tert-butyl-pyrazol-3-on, 2-Cyclopentyl-1-phenyl-4-formylamido-5-tert-butyl-pyrazol-3-on, 2-Methyl-1-phenyl-4-acetylamido-5-tert-butyl-pyrazol-3-on, 2-Ethyl-1-phenyl-4-acetylamido-5-tert-butyl-pyrazol-3-on, 2-Propyl-1-phenyl-4-acetylamido-5-tert-butyl-pyrazol-3-on, 2-Isopropyl-1-phenyl-4-acetylamido-5-tert-butyl-pyrazol-3-on, 2-Butyl-1-phenyl-4-acetylamido-5-tert-butyl-pyrazol-3-on, 2-Isobutyl-1-phenyl-4-acetylamido-5-tert-butyl-pyrazol-3-on, 2-tert-Butyl-1-phenyl-4-acetylamido-5-tert-butyl-pyrazol-3-on, 2-Phenyl-1-phenyl-4-acetylamido-5-tert-butyl-pyrazol-3-on, 2-(o-methylphenyl)-1-phenyl-4-acetylamido-5-tert-butyl-pyrazol-3-on, 2-(3-Methylphenyl)-1-phenyl-4-acetylamido-5-tert-butyl-pyrazol-3-on, 2-(4-Methylphenyl)-1-phenyl-4-acetylamido-5-tert-butyl-pyrazol-3-on, 2-Benzyl-1-phenyl-4-acetylamido-5-tert-butyl-pyrazol-3-on, 2-(o-methoxyphenyl)-1-phenyl-4-acetylamido-5-tert-butyl-pyrazol-3-on, 2-(4-Methoxyphenyl)-1-phenyl-4-acetylamido-5-tert-butyl-pyrazol-3-on, 2-Cyclohexyl-1-phenyl-4-acetylamido-5-tert-butyl-pyrazol-3-on, 2-Cyclopentyl-1-phenyl-4-acetylamido-5-tert-butyl-pyrazol-3-on, 2-Methyl-1-phenyl-4-amino-5-phenyl-pyrazol-3-on, 2-Ethyl-1-phenyl-4-amino-5-phenyl-pyrazol-3-on, 2-Propyl-1-phenyl-4-amino-5-phenyl-pyrazol-3-on, 2-Isopropyl-1-phenyl-4-amino-5-phenyl-pyrazol-3-on, 2-Butyl-1-phenyl-4-amino-5-phenyl-pyrazol-3-on, 2-Isobutyl-1-phenyl-4-amino-5-phenyl-pyrazol-3-on, 2-tert-Butyl-1-phenyl-4-amino-5-phenyl-pyrazol-3-on, 2-Phenyl-1-phenyl-4-amino-5-phenyl-pyrazol-3-on, 2-(o-methylphenyl)-1-phenyl-4-amino-5-phenyl-pyrazol-3-on, 2-(3-Methylphenyl)-1-phenyl-4-amino-5-phenyl-pyrazol-3-on, 2-(4-Methylphenyl)-1-phenyl-4-amino-5-phenyl-pyrazol-3-on, 2-Benzyl-1-phenyl-4-amino-5-phenyl-pyrazol-3-on, 2-(o-methoxyphenyl)-1-phenyl-4-amino-5-phenyl-pyrazol-3-on, 2-(4-Methoxyphenyl)-1-phenyl-4-amino-5-phenyl-pyrazol-3-on, 2-Cyclohexyl-1-phenyl-4-amino-5-phenyl-pyrazol-3-on, 2-Cyclopentyl-1-phenyl-4-amino-5-phenyl-pyrazol-3-on, 2-Methyl-1-phenyl-4-formylamido-5-phenyl-pyrazol-3-on, 2-Ethyl-1-phenyl-4-formylamido-5-phenyl-pyrazol-3-on, 2-Propyl-1-phenyl-4-formylamido-5-phenyl-pyrazol-3-on, 2-Isopropyl-1-phenyl-4-formylamido-5-phenyl-pyrazol-3-on, 2-Butyl-1-phenyl-4-formylamido-5-phenyl-pyrazol-3-on, 2-Isobutyl-1-phenyl-4-formylamido-5-phenyl-pyrazol-3-on, 2-tert-Butyl-1-phenyl-4-formylamido-5-phenyl-pyrazol-3-on, 2-Phenyl-1-phenyl-4-formylamido-5-phenyl-pyrazol-3-on, 2-(o-methylphenyl)-1-phenyl-4-formylamido-5-phenyl-pyrazol-3-on, 2-(3-Methylphenyl)-1-phenyl-4-formylamido-5-phenyl-pyrazol-3-on, 2-(4-Methylphenyl)-1-phenyl-4-formylamido-5-phenyl-pyrazol-3-on, 2-Benzyl-1-phenyl-4-formylamido-5-phenyl-pyrazol-3-on, 2-(o-methoxyphenyl)-1-phenyl-4-formylamido-5-phenyl-pyrazol-3-on, 2-(4-Methoxyphenyl)-1-phenyl-4-formylamido-5-phenyl-pyrazol-3-on, 2-Cyclohexyl-1-phenyl-4-formylamido-5-phenyl-pyrazol-3-on, 2-Cyclopentyl-1-phenyl-4-formylamido-5-phenyl-pyrazol-3-on, 2-Methyl-1-phenyl-4-acetylamido-5-phenyl-pyrazol-3-on, 2-Ethyl-1-phenyl-4-acetylamido-5-phenyl-pyrazol-3-on, 2-Propyl-1-phenyl-4-acetylamido-5-phenyl-pyrazol-3-on, 2-Isopropyl-1-phenyl-4-acetylamido-5-phenyl-pyrazol-3-on, 2-Butyl-1-phenyl-4-acetylamido-5-phenyl-pyrazol-3-on, 2-Isobutyl-1-phenyl-4-acetylamido-5-phenyl-pyrazol-3-on, 2-tert-Butyl-1-phenyl-4-acetylamido-5-phenyl-pyrazol-3-on, 2-Phenyl-1-phenyl-4-acetylamido-5-phenyl-pyrazol-3-on, 2-(o-methylphenyl)-1-phenyl-4-acetylamido-5-phenyl-pyrazol-3-on, 2-(3-Methylphenyl)-1-phenyl-4-acetylamido-5-phenyl-pyrazol-3-on, 2-(4-Methylphenyl)-1-phenyl-4-acetylamido-5-phenyl-pyrazol-3-on, 2-Benzyl-1-phenyl-4-acetylamido-5-phenyl-pyrazol-3-on, 2-(o-methoxyphenyl)-1-phenyl-4-acetylamido-5-phenyl-pyrazol-3-on, 2-(4-Methoxyphenyl)-1-phenyl-4-acetylamido-5-phenyl-pyrazol-3-on, 2-Cyclohexyl-1-phenyl-4-acetylamido-5-phenyl-pyrazol-3-on oder 2-Cyclopentyl-1-phenyl-4-acetylamido-5-phenyl-pyrazol-3-on.

Sofern nicht näher spezifiziert steht Butyl für iso-Butyl oder n-Butyl. Insbesonders sind solche Verbindungen bevorzugt, die keinen Pyridyl-Substituenten aufweisen.

Insbesonders sind 2-Isopropylpyrazol-3-ol, 1- Methylpyrazol-4-ol, 5-Methyl-2-phenyl-4H-pyrazol-3-one, 1,3,5-trimethylpyrazol-4-ol, 1-Isopropyl-3,5-dimethyl-pyrazol-4-ol, 1-(4-hydroxy-1,5-dimethyl-pyrazol-3-yl)ethanone, 1-Benzylpyrazol-4-ol oder eine beliebige Mischung aus der zwei oder mehr der zuvor genannten Verbindungen bevorzugt.

Bei polymerisationsfähigen Verbindungen handelt es sich bevorzugt um mono-, di- oder triethylenisch ungesättigte C₃- bis C₈-Carbonsäuren, mono-, di- oder triethylenisch ungesättigte C₃- bis C₈ Aldehyde, mono-, di- oder triethylenisch ungesättigte C₃- bis C₈-Carbonsäureester mit 1 bis 20 Kohlenstoffatomen in den Estergruppen, mono-, di- oder triethylenisch ungesättigte C₃- bis C₈-Carbonsäureamide, mono-, di- oder triethylenisch ungesättigte C₃- bis C₈-Nitrile, mono-, di- oder triethylenisch ungesättigte C₃- bis C₈-Carbonsäureanhydride, Vinylester von gesättigten C₂- bis C₂₀-Carbonsäuren, Vinylether von gesättigten C₁-bis C₁₀- Alkoholen, Vinylaromaten, Vinylheteroaromaten, Vinyllactame mit 3 bis 10 Kohlenstoffatomen im Ring, offenkettige N-Vinylamidverbindungen und N-Vinylaminverbindungen, Vinylhalogenide, aliphatische, gegebenenfalls halogenierte Kohlenwasserstoffe mit 2 bis 8 Kohlenstoffatomen und 1 oder 2 olefinischen Doppelbindungen, Vinylidene oder beliebige Mischungen aus zwei oder mehr der zuvor genannten Verbindungen

Mono-, di- oder triethylenisch ungesättigte C₃- bis C₈-Carbonsäuren sind bevorzugt (Meth)acrylsäure, Dimethacrylsäure, Ethacrylsäure, Citraconsäure, Methylenmalonsäure, Crotonsäure, Fumarsäure, Mesaconsäure, Itaconsäure, Maleinsäure.

Mono ungesättigte C₃- bis C₈-Aldehyde sind bevorzugt (Meth)acrolein oder Crotonaldehyd.

Mono-, di- oder triethylenisch ungesättigte C₃- bis C₈-Carbonsäureester mit 1 bis 20 Kohlenstoffatome in den Estergruppen sind bevorzugt (Meth)acrylsäureester mit C₁- bis C₂₀-Alkyl, Dimethacrylsäureester mit C₁- bis C₂₀-Alkyl, Ethacrylsäureester mit C₁- bis C₂₀ Alkyl, Citraconsäureester mit C₁- bis C₂₀ Alkyl, Methylenmalonsäureester mit C₁- bis C₂₀-Alkyl, Crotonsäureester mit C₁- bis C₂₀-Alkyl, Fumarsäureester mit C₁- bis C₂₀-Alkyl, Mesaconsäureester mit C₁- bis C₂₀-Alkyl, Itaconsäureester mit C₁- bis C₂₀-Alkyl, Maleinsäureester mit C₁- bis C₂₀-Alkyl. Bei mehrprotonigen Säuren können alle Säuregruppen oder nur ein Teil der Säuregruppen verestert sein.

Mono-, di- oder triethylenisch ungesättigte C₃- bis C₈-Carbonsäureamide sind bevorzugt (Meth)acrylsäureamid, Dimethacrylsäureamid, Ethacrylsäureamid, Citraconsäureamid, Methylenmalonsäureamid, Crotonsäureamid, Fumarsäureamid, Mesaconsäureamid, Itaconsäureamid, Maleinsäureamid. Der Stickstoff der Amidgruppe kann als Substituenten zweimal H, zweimal unabhängig voneinander C₁- bis C₂₀-Alkyl, einmal H und einem C₁- bis C₂₀-Alkyl oder einen Zyklus mit 4 bis 5 Kohlenstoffatomen im Zyklus, wobei der Stickstoff an der Zyklusbildung beteiligt ist, aufweisen.

Ein monoethylenisch ungesättigtes C₃- Nitril ist bevorzugt (Meth)acrylnitril.
Mono-, di- oder triethylenisch ungesättigte C₃- bis C₈-Carbonsäureanhydride sind beispielsweise (Meth)acrylsäureanhydrid, Itaconsäureanhydrid, Maleinsäureanhydrid.
Vinylester von gesättigten C₂- bis C₂₀-Carbonsäuren sind bevorzugt Vinyllaurat, Vinylstearat, Vinylpropionat, Versaticsäurevinylester oder Vinylacetat.

Vinylether von gesättigten C₁-bis C₁₀- Alkoholen sind bevorzugt Methylvinylether, Ethylvinylether, Butylvinylether, 4-Hydroxybutylvinylehter, Vinylisobutylether oder Dodecylvinylether.

Vinylaromaten sind bevorzugt Vinyltoluol, ortho- oder para-Methylsytrol, ortho-Butylstyrol, para-n-Butylstyrol, para-n-Decylstyrol, Styrol oder Divinylbenzol.
Vinylheteroaromaten sind bevorzugt 2-Vinylpyridin, N-Vinylimidazol, N-Vinylpiperidon, N-Vinyl-2-methylimidazol oder N-Vinyl-4-methylimidazol.

Vinyllactame mit 3 bis 10 Kohlenstoffatomen im Ring sind bevorzugt N-Vinylcaprolactam, N-Vinylpyrrolidon, Lauolactam, oxygenierte Purine wie Xanthin oder dessen Derivate wie 3-Methylxanthin, Hypoxanthin, Guanin, Theophyllin, Coffein, Adenin oder Theobromin.

Offenkettige N-Vinylamidverbindungen sind bevorzugt N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid oder N-Vinylbutyramid,

Offenkettige N-Vinylaminverbindugnen sind bevorzugt N-Vinyl-N-dimethylamin, N-Vinyl-N-methlethylamin oder N-Vinyl-N-diethylamin.

Vinylhalogenide sind bevorzugt Chlor, Fluor oder Brom substituierte ethylenisch ungesättigte Verbindungen, bevorzugterweise Vinylchlorid, Vinylfluorid oder Vinylidenchlorid.

Aliphatische, gegebenenfalls halogenierte Kohlenwasserstoffe mit 2 bis 8 Kohlenstoffatomen und 1 oder 2 olefinischen Doppelbindungen sind bevorzugt Ethylen, Propen, 1-Buten, 2-Buten, Isobuten, Butadien, Isopren oder Chloropren.

Ein bevorzugtes Vinyliden ist Vinylidencyanid.

Weitere polymerisationsfähige Verbindungen sind bevorzugt Vinylphosphorsäuren, Vinylessigsäure, Allylessigsäure, N-Vinylcarbazol, Hydroxymethylvinylketon, N,N-Divinylethylen-harnstoff, Vinylencarbonat, Tetrafluorethylen, Hexafluorpropen, Nitroethylen, α-Chloracrylester, α-Cyanoacrylester, Methylenmalonester, α-Cyansorbinsäureester, Cyclopentadien, Cyclopenten, Cyclohexen, Cyclododecen oder eine Mischung aus zwei oder aller zuvor genannten polymerisationsfähigen Verbindungen.

Besonders bevorzugt handelt es sich bei einer polymerisationsfähigen Verbindung um (Meth)acrylsäure oder (Meth)acrylsäureester mit C₁- bis C₈-Alkyl. Insbesondere handelt es sich bei einer polymerisationsfähigen Verbindung um (Meth)acrylsäure, Methyl(meth)acrylat, Ethyl(meth)acrylat, n-Butyl(meth)acrylat oder 2-Ethylhexyl(meth)acrylat.

Die Gesamtmenge der Verbindungen der allgemeinen Formel (I), (II), (III) und (IV) in der erfindungsgemäßen Mischung beträgt bevorzugt 0,1 bis 1000 Gew. ppm bezogen auf das Gesamtgewicht der polymerisationsfähigen Verbindungen. Besonders bevorzugt beträgt die Gesamtmenge der Verbindungen der allgemeinen Formel (I), (II), (III) und (IV) in der erfindungsgemäßen Mischung 1 bis 900 Gew. ppm, ganz besonders bevorzugt 10 bis 800 Gew. ppm und insbesondere 10 bis 500 Gew. ppm.

Enthält die erfindungsgemäße Mischung neben einer oder mehreren polymerisationsfähigen Verbindungen und einer oder mehrerer Verbindungen der allgemeinen Formel (I), (II), (III) und/oder (IV) noch andere Komponenten, beträgt die Gesamtmenge der polymerisationsfähigen Verbindungen in der erfindungsgemäßen Mischung bevorzugt mindestens 5 Gew.% bezogen auf das Gesamtgewicht der Mischung, weiter bevorzugt mindestens 20 Gew. %, weiter bevorzugt mindestens 30 Gew.%, weiter bevorzugt mindestens 40 Gew.%, weiter bevorzugt mindestens 50 Gew.%, weiter bevorzugt mindestens 60 Gew.%, weiter bevorzugt mindestens 70 Gew.%, weiter bevorzugt mindestens 80 Gew.% , weiter bevorzugt mindestens 90 Gew.% und besonders bevorzugt mindestens 95 Gew.%.

Andere Komponenten können beispielsweise Lösungsmittel wie organische Lösungsmittel, Wasser oder Lösungsmittelgemische, eingelöste Gase wie Luft, Nebenkomponenten, Costabilisatoren oder beliebige Mischungen von mehreren dieser Komponenten sein.

Bei Nebenkomponenten handelt es sich beispielsweise um Nebenprodukte, die bei der Herstellung der polymerisationsfähigen Verbindungen und/oder im Rahmen ihrer Aufreinigung entstanden sind und während der Aufreinigung nicht vollständig abgetrennt wurden. Bei Nebenkomponenten kann es sich beispielsweise auch um unterschiedliche Verschmutzungen handeln, die während Herstellung, Aufreinigung und/oder Lagerung in die polymerisationsfähigen Verbindungen eingetragen wurden. Nebenkomponenten können auf gleichem Wege über die Verbindungen der allgemeinen Formel (I), (II), (III) und/oder (IV) in die Mischungen eingetragen werden.

(Meth)acrylsäure kann abhängig von der Herstellung und Aufreinigung eine oder mehrere Nebenkomponenten enthalten. Nebenkomponenten, die enthalten sein können, sind beispielsweise Wasser, Di(meth)acrylsäure, Essigsäure, Propionsäure, einen oder mehrerer Aldehyde wie Acetaldehyd, Acrolein, Propionaldehyd, Furfural-2, Furfural-3, Cumaron oder Benzaldehyd, Protoanemonin, Maleinsäure, Maleinsäureanhydrid, Allylacrylat oder eine beliebige Mischung aus zwei oder mehr der zuvor genannten Nebenkomponenten. Der Gehalt einer Nebenkomponente liegt in der Regel im Bereich von der erfassbaren Nachweisgrenze bis 20000 Gew. ppm bezogen auf die Menge an (Meth)acrylsäure.

(Meth)acrylsäureester können abhängig von ihrer Herstellung und Aufreinigung eine oder mehrere Nebenkomponenten enthalten. Nebenkomponenten, die enthalten sein können, sind beispielsweise Wasser, (Meth)acrylsäure, Alkohole der jeweiligen (Meth)acrylsäureester, Propionat-Ester mit den Alkoholen der jeweiligen (Meth)acrylsäureestern, Acetat-Ester mit den Alkoholen der jeweiligen (Meth)Acrylsäureestern, Ether der jeweiligen (Meth)acrylsäureester-Alkohole oder eine beliebige Mischung aus zwei oder mehr der zuvor genannten Nebenkomponenten. Der Gehalt einer Nebenkomponente liegt in der Regel im Bereich von der erfassbaren Nachweisegrenze bis 20000 Gew. ppm bezogen auf die Menge an (Meth)acrylsäureester.

Die erfindungsgemäße Mischung wird bevorzugt unter einer sauerstoffhaltigen Atomsphäre gelagert und/oder transportiert. Die sauerstoffhaltige Atomsphäre weist bevorzugt einen Sauerstoffgehalt von 5 bis 30 vol.% Sauerstoff, insbesondere einen Sauerstoffgehalt von 5 bis 21 vol.%, auf.

Um eine Sauerstoffsättigung der erfindungsgemäßen Mischung schnellstmöglich zu erreichen und/oder eine kontinuierliche Sauerstoffsättigung sicher zu stellen, kann es bevorzugt sein, dass ein sauerstoffhaltiges Gas in die Mischung eingebracht wird.

Das sauerstoffhaltige Gas kann beispielsweise über eine oder mehrere Düsen, deren Öffnungen in die Mischung hineinragen, in die Mischung eingebracht werden.

Bei einem sauerstoffhaltigen Gas handelt es sich beispielsweise um Luft, Stickstoff-LuftGemische oder von Luft unterschiedliche Sauerstoff-Stickstoffgemische mit einem Sauerstoffgehalt von 5 bis 30 Vol. Prozent. Neben Sauerstoff und Stickstoff können Sauerstoff-Stickstoffgemische auch andere Gase wie Kohlenstoffdioxid, Kohlenstoffmonoxid, Wasserstoff, Methan, ein oder mehrere Edelgase oder eine Mischung aus zwei oder mehr der zuvor genannten Gase enthalten.

Die erfindungsgemäße Mischung kann dadurch hergestellt werden, dass man eine oder mehrere Verbindungen der allgemeinen Formel (I), (II), (III) und/oder (IV) mit einer oder mehreren polymerisationsfähigen Verbindungen vermischt. Es kann dazu zweckdienlich sein, dass ein Konzentrat von Verbindungen der allgemeinen Formel (I), (II), (III) und/oder (IV) in einer oder mehrerer polymerisationsfähigen Verbindungen und/oder in einem geeigneten Lösungsmittel hergestellt wird und das so erhaltene Konzentrat dann mit polymerisationsfähigen Verbindungen verdünnt wird. Bei der Wahl eins geeigneten Lösungsmittels, das auch ein Lösungsmittelgemisch sein kann, orientiert sich der Fachmann anhand allgemeiner, zweckdienlicher Überlegungen, wie Löslichkeit, Mischbarkeit und potentiell auftretenden Nebeneffekten. Dem Konzentrat können dabei eine oder mehrere polymerisationsfähige Verbindungen zugeführt werden, oder das Konzentrat kann einer oder mehrerer vorgelegter polymerisationsfähiger Verbindungen zugeführt werden.

Die erfindungsgemäße Mischung enthält bevorzugt einen Costabilisator. Ein Costabilisator kann als chemische Einzelverbindungen oder als Gemisch von Verbindungen in der erfindungsgemäßen Mischung enthalten sein. Ein Costabilisator trägt dazu bei, die radikalische Polymerisation der polymerisationsfähigen Verbindungen zu unterbinden, beziehungsweise zu verlangsamen. Der Costabilisator kann dabei synergistisch mit den in der Mischung enthaltenden Verbindungen der allgemeinen Formel (I), (II), (III) und/oder (IV) zusammenwirken, um die radikalische Polymerisation der polymerisationsfähigen Verbindungen zu unterbinden, beziehungsweise zu verlangsamen. Der Costabilisator kann der erfindungsmäßen Mischung vor und/oder währender der Lagerung und/oder Transport zugegeben werden. Der Costabilisator kann dabei gemeinsam oder separat von den Verbindungen der allgemeinen Formel (I), (II), (III) und/oder (IV) den polymerisationsfähigen Verbindungen zugegeben werden. Der Costabilisator kann aber auch bereits bei der Herstellung und/oder Aufreinigung den polymerisationsfähigen Verbindungen zugegeben worden sein, wobei Verbindungen der allgemeinen Formel (I), (II), (III) und/oder (IV) dann anschließend der vorliegenden Mischung zugegeben werden. Es ist bevorzugt, dass die Gesamtmenge an Costabilisator in den erfindungsgemäßen Mischungen 0,1 bis 5000 Gew. ppm bezogen auf die Gesamtmenge der polymerisationsfähigen Verbindungen in der Mischung beträgt. Es ist weiter bevorzugt, dass die Gesamtmenge an Costabilisator 1 bis 4000 Gew. ppm, insbesondere 5 bis 2500 Gew. ppm und besonders bevorzugt 50 bis 750 Gew. ppm bezogen auf die Gesamtmenge der polymerisationsfähigen Verbindungen in der Mischung beträgt. Die Menge an Costabilisator kann durch Zugabe oder Entfernen angepasst werden. Methoden zur Entfernung des Costabilisators sind dem Fachmann bekannt (siehe beispielsweise US 6046357). Geeignete Methoden umfassen beispielsweise Destillation, Kristallisation, lonenaustausch, Extraktion, umgekehrte Osmose und/oder Membranverfahren. Bei der Wahl der geeigneten Methode oder Kombination von Methoden orientiert sich der Fachmann an seinem allgemeinen Fachwissen. Werden Verbindungen der allgemeinen Formel (I), (II), (III) und/oder (IV) mit abgetrennt, sind diese ggf. wieder zu ergänzen. Gegebenenfalls kann es auch notwendig sein, den Gehalt an Verbindungen der allgemeinen Formel (I), (II), (III) und/oder (IV) zu erhöhen, um die gewünschte Polymerisationsstabilisierung zu erzielen, wenn der Anteil an Costabilisator in der Mischung reduziert bzw. abgetrennt wird.

Bei dem Costabilisator handelt es sich beispielsweise um Metallsalze bestehend aus der Gruppe von Kupfer-, Mangan- und Cer-Salzen, Phenothiazine, phenolische Verbindungen, N-Oxyle, Phenylendiamine, Nitrosoverbindungen, Harnstoffe, Thioharnstoffe, oder um Mischungen aus zwei oder mehr der zuvor genannten Substanzklassen. Bevorzugt handelt es sich bei dem Costabilisator um Phenotiazine, phenolische Verbindungen, N-Oxyle oder um Mischungen aus zwei oder mehr der zuvor genannten Substanzklassen.

Bei Kupfersalzen handelt es sich um Kupfer (I) und/oder Kupfer (II) Salze, die eine ausreichende Löslichkeit in den polymerisationsfähigen Verbindungen und in der erfindungsgemäßen Mischung aufweisen. Geeignete Kupfersalze sind beispielsweise Kupfer(II)phenolat, Kupfer(II)acetylacetonat, Kupfer(II)gluconat, Kupfer(II)tartrat, Kupfer(II)acetat, Kupfer(II)formiat, Kupfer(II)nitrart, Kupfer(II)hydroxid, Kupfer(II)sulfat, Kupfer(II)carbonat, Kupfer(II)naphthenat, Kupfer(II)acrylat, Kupfer(II)halogenide wie Kupfer(II)chlorid, Kupfer(II)salicylat, Kupfer(II)sulfonat, Kupfer(II)propionat, Kupfer(II)octanat, Kupfer(I)chlorid, Kupfer(I)iodid, Kupder(I)bromid, Kupfer(I)cyanid, Kupfer(I)acetat, Kupfer(I)oxid, Kupfer(I)sulfat, komplexe Kupfersalze wie Cu(NH₃)₄+ mit entsprechenden Gegenionen, ligandenstabilisierte Kupferkomplexe wie sie in der DE 10 2012 223 695 A1 offenbart sind, oder beliebige Mischungen aus zwei oder mehr der zuvor genannten Kupfersalze. Die entsprechenden Kupfersalze können auch in Form ihrer Hydrate eingesetzt werden.

Bei Mangansalzen handelt es sich bevorzugt um Mangan (II) Salze, die eine ausreichende Löslichkeit in den polymerisationsfähigen Verbindungen und in der erfindungsgemäßen Mischung aufweisen. Geeignete Mangansalze sind beispielsweise Mangan(II)phenolat, Mangan(II)acetylacetonat, Mangan(II)gluconat, Mangan(II)tartrat, Mangan(II)acetat, Mangan(II)formiat, Mangan(II)nitrart, Mangan(II)hydroxid, Mangan(II)sulfat, Mangan(II)carbonat, Mangan(II)naphthenat, Mangan(II)aciylat, Mangan(II)halogenide wie Mangan(II)chlorid, Mangan(II)salicylat, Mangan(II)sulfonat, Mangan (II)propionat, Mangan(II)octanat, ligandenstabilisierte Mangankomplexe wie sie in der DE 10 2012 223 695 A1 offenbart sind, oder beliebige Mischungen aus zwei oder mehr der zuvor genannten Mangansalze. Die entsprechenden Mangansalze können auch in Form ihrer Hydrate eingesetzt werden.

Bei Cersalzen handelt es sich bevorzugt um Cer(III) Salze, die eine ausreichende Löslichkeit in den polymerisationsfähigen Verbindungen und in der erfindungsgemäßen Mischung aufweisen. Geeignete Cersalze sind beispielsweise Cer(III)phenolat, Cer(III)acetylacetonat, Cer(III)gluconat, Cer(III)tartrat, Cer(III)acetat, Cer(III)formiat, Cer(III)nitrart, Cer(III)hydroxid, Cer(III)sulfat, Cer(III)carbonat, Cer(III)naphthenat, Cer(III)acrylat, CerIII)halogenide wie Cer(III)chlorid, Cer(III)salicylat, Cer(III)sulfonat, Cer(III)propionat, Cer(III)octanat, ligandenstabilisierte Cerkomplexe wie sie in der DE 10 2012 223 695 A1 offenbart sind, oder beliebige Mischungen aus zwei oder mehr der zuvor genannten Cersalze. Die entsprechenden Cersalze können auch in Form ihrer Hydrate eingesetzt werden.

Phenotiazine sind beispielsweise Bis-(a-methylbenzyl)phenothiazin, 3,7-Dioctylphenothiazin, Bis-(a-dimethylbenzyl)phenothiazin oder eine beliebige Mischung von zwei oder mehr der zuvor genannten Verbindungen.

Phenolische Verbindungen sind beispielsweise Hydrochinon, Hydrochinonmonomethylether, wie para-Methoxyphenol (MEHQ), Pyrogallol, Catechol, Resorcin, Phenol, Kresol, 2,4-Dimethyl-6-tert-butylphenol, 2,6-Di-tert-butyl-para-kresol oder eine beliebige Mischung von zwei oder mehr der zuvor genannten Verbindungen.

N-Oxyle sind beispielsweise Di-tert-butylnitroxid, 2,2,6,6-Tetramethyl-4-hydroxypiperidyl-1-oxyl, 2,2,6,6-Tetramethylpiperidyl-1-oxyl, 2,2,6,6-Tetramethylpiperidinoxyl, 4-Hydroxy-2,2,6,6-tetramethylpiperidinoxyl, 4,4',4"-Tris-1-(2,2,6,6-tetramethylpiperidinoxyl)phosphite oder eine beliebige Mischung von zwei oder mehr der zuvor genannten Verbindungen.

Ein weiterer Gegenstand der vorliegenden Erfindung ist die Verwendung einer oder mehrerer Verbindungen der allgemeinen Formel (I), (II), (III), (IV) oder einer beliebigen Mischung dieser Verbindungen als Lager- und/oder Transportstabilisator von einer oder mehrerer polymerisationsfähigen Verbindungen. Auf die bereits beschriebenen bevorzugten Ausführungsformen wird in vollem Umfang Bezug genommen. Damit sind besonders bevorzugte, insbesonders bevorzugte oder durch ähnliche Formulierungen als bevorzugt gekennzeichnete Ausführungsformen miterfasst.

Weiterhin ist ein Verfahren zur Stabilisierung von einer oder mehrerer polymerisationsfähigen Verbindungen bei Lagerung und/oder Transport unter Verwendung einer oder mehrerer Verbindungen der allgemeinen Formel (I), (II), (III), (IV) oder einer beliebigen Mischung dieser Verbindungen Gegenstand der vorliegenden Erfindung. Auf die bereits beschriebenen bevorzugten Ausführungsformen wird in vollem Umfang Bezug genommen. Damit sind besonders bevorzugte, insbesonders bevorzugte oder durch ähnliche Formulierungen als bevorzugt gekennzeichnete Ausführungsformen miterfasst.

Das erfindungsgemäße Verfahren umfasst das Bereitstellen einer Mischung, die eine oder mehrere Verbindungen der allgemeinen Formel (I), (II), (III), (IV) oder eine beliebige Mischung dieser und eine oder mehrere polymerisationsfähige Verbindungen enthält, wobei die Mischung mit Sauerstoff zu 50 bis 100%, bevorzugt zu 90 bis 100 % und besonders bevorzugt zu 95 bis 100% gesättigt ist. Der Grad der Sauerstoffsättigung bezieht auf den Grad der Sauerstoffsättigung der im Gleichgewicht erreicht wird, wenn die Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.

Ein weiterer Gegenstand der vorliegenden Erfindung ist ein Verfahren zur Polymerisation von einer oder mehreren polymerisationsfähigen Verbindungen in Gegenwart einer oder mehrerer Verbindungen der allgemeinen Formel (I), (II), (III), (IV) oder einer beliebige Mischung dieser, wobei die Reaktionsmischung zu 0 bis 50 %, bevorzugt zu 0 bis 10 %, besonders bevorzugt zu 0 bis 5 %, weiter bevorzugt zu 0 bis 1 % und insbesonders bevorzugt zu 0 bis 0,1 % mit Sauerstoff gesättigt ist. Der Grad der Sauerstoffsättigung bezieht auf den Grad der Sauerstoffsättigung der erreicht wird, wenn die Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.

Ist die Sauerstoffsättigung der Reaktionsmischung zu hoch, kann die Sauerstoffsättigung beispielsweise durch Einleiten von Stickstoff in die Reaktionsmischung und/oder in die Atmosphäre oberhalb der Reaktionsmischung reduziert werden. Der eingeleitete Stickstoff sollte dabei weitgehend frei von Sauerstoff sein, beispielsweise einen Sauerstoffgehalt von weniger als 1 vol.%, bevorzugt weniger als 0,1 vol.% aufweisen.

Auf die bereits beschriebenen bevorzugten Ausführungsformen wird in vollem Umfang Bezug genommen. Damit sind besonders bevorzugte, insbesonders bevorzugte oder durch ähnliche Formulierungen als bevorzugt gekennzeichnete Ausführungsformen miterfasst.

Es ist bevorzugt, dass die Polymerisation bei einer Temperatur von 50 bis 150°C und weiter bevorzugt bei einer Temperatur von 70 bis 120°C durchgeführt wird.

Es ist bevorzugt, dass die Polymerisation unter einer Stickstoffatomsphäre durchgeführt wird. Eine Stickstoffatomsphäre weist einen Stickstoffgehalt von 85 bis 100 vol.%, bevorzugt 90 bis 100 vol.% und besonders bevorzugt 99 bis 100 vol.% auf. Der Sauerstoffgehalt der Stickstoffatomsphäre ist unter 5 vol.%.

Es ist bevorzugt, dass die Polymerisation in Gegenwart eines Polymerisationsstarters durchgeführt wird. Als Polymerisationsstarter eignen sich die dem Fachmann bekannten Polymerisationsstarter wie sie beispielsweise in The Chemistry of Radical Polymerisation, 2nd Edition, 2005, Seite 49 bis 166 beschrieben sind. Der Polymerisationsstarter wird in wirksamen Mengen eingesetzt.

Durch das erfindungsgemäße Verfahren ist es möglich, eine oder mehrere polymerisationsfähige Verbindungen, die mit einer oder mehrerer Verbindungen der allgemeinen Formel (I), (II), (III), (IV) oder einer beliebigen Mischung dieser lager- und/oder transportstabilisiert sind zu polymerisieren, ohne die Notwendigkeit die Verbindungen der allgemeinen Formel (I), (II), (III), (IV) oder die beliebige Mischung dieser zuvor von den polymerisationsfähigen Verbindungen abzutrennen. Die Reaktionsmischung kann somit stabilisiert im Reaktor zur Polymerisation vorgelegt werden. Durch Absenken der Sauerstoffsättigung in der Reaktionsmischung, bevorzugt durch Einleiten von Stickstoff in den Reaktorraum oberhalb der Reaktionsmischung und/oder durch Einleiten von Stickstoff in die Reaktionsmischung kann die Stabilisierungswirkung der Verbindungen der allgemeinen Formel (I), (II), (III), (IV) oder der beliebigen Mischung dieser reduziert werden, dass die Reaktionsmischung dann polymerisiert werden kann. Die Zugabe des Polymerisationsstarters kann vor, während oder nach dem Absenken der Sauerstoffsättigung erfolgen. Aus Sicherheitsgründen ist es bevorzugt, wenn der Polymerisationsstarter vor dem Absenken der Sauerstoffsättigung zugegeben wird.

Der Gegenstand der vorliegenden Erfindung umfasst insbesonders die nachfolgenden Ausführungsformen.
1. Mischung enthaltend eine oder mehrerer Verbindungen der allgemeinen Formel (I) 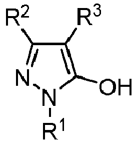 wobei
   R¹ H, C₁- bis C₂₀-Alkyl, Alkanoyl mit C₁- bis C₈-Alkyl oder ein aromatischer Rest ist,
   R² H, C₁- bis C₂₀-Alkyl, Carboxylester mit C₁- bis C₈-Alkyl, Alkynoyl mit C₁- bis C₈-Alkyl oder ein aromatischer Rest ist,
   R³ H, C₁- bis C₂₀-Alkyl, Carboxylester mit C₁- bis C₈-Alkyl, Alkanoyl mit C₁- bis C₈-Alkyl, ein aromatischer Rest oder -CH₂-CHR⁴-C(O)OR⁵ ist, wobei R⁴ H oder Methyl ist und R⁵ H, C₁-bis C₂₀ Alkyl oder ein aromatischer Rest ist,
   wobei R² und R³ nicht gleichzeitig Methyl sind, wenn R¹ Phenyl ist,
   und/oder eine oder mehrere Verbindungen der allgemeinen Formel (II) 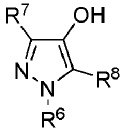
   wobei
   R⁶ H, C₁- bis C₂₀-Alkyl, Alkanoyl mit C₁- bis C₈-Alkyl oder ein aromatischer Rest ist,
   R⁷ H, C₁- bis C₂₀-Alkyl, Carboxylester mit C₁- bis C₈-Alkyl, Alkanoyl mit C₁- bis C₈-Alkyl oder ein aromatischer Rest ist,
   R⁸ H, C₁- bis C₂₀-Alkyl, Carboxylester mit C₁- bis C₈-Alkyl, Alkanoyl mit C₁- bis C₈-Alkyl, ein aromatischer Rest oder -CH₂-CHR^{4I}-C(O)OR^{5I} ist, wobei R^{4I} H oder Methyl ist und R^{5I} H, C₁-bis C₂₀-Alkyl oder ein aromatischer Rest ist,
   wobei
   R⁶ und R⁷ nicht gleichzeitig Phenyl sind, wenn R⁸ Phenyl oder Methyl ist,
   R⁶ und R⁸ nicht gleichzeitig Phenyl sind, wenn R⁷ H oder Methyl ist,
   R⁶ nicht Phenyl und R⁸ nicht Methyl ist, wenn R⁷ H oder Methyl ist,
   und/oder eine oder mehrere Verbindungen der allgemeinen Formel (III) 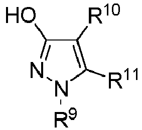
   wobei
   R⁹ H, C₁- bis C₂₀-Alkyl, Alkanoyl mit C₁- bis C₈-Alkyl, oder ein aromatischer Rest ist,
   R¹⁰ H, C₁- bis C₂₀-Alkyl, Alkanoyl mit C₁- bis C₈-Alkyl, ein aromatischer Rest oder - C(O)O-R^{5II} ist, wobei R^{5II} H, C₁-bis C₂₀ Alkyl oder ein aromatischer Rest ist,
   R¹¹ H, C₁-bis C₂₀-Alkyl, Alkanoyl mit C₁- bis C₈-Alkyl, ein aromatischer Rest oder-C(O)O-R^{5III} ist, wobei R^{5III} H, C₁- bis C₂₀-Alkyl oder ein aromatischer Rest ist,
   und/oder eine oder mehrere Verbindungen der allgemeinen Formel (IV) 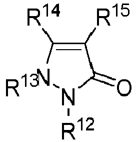
   wobei
   R¹² H, C₁- bis C₂₀-Alkyl, Alkanoyl mit C₁- bis C₈-Alkyl oder ein aromatischer Rest ist,
   R¹³ H, C₁- bis C₂₀-Alkyl, Alkanoyl mit C₁- bis C₈-Alkyl, ein aromatischer Rest oder-C(O)O-R^{5IV} ist, wobei R^{51V} H, C₁- bis C₂₀-Alkyl oder ein aromatischer Rest ist.
   R¹⁴ H, C₁- bis C₂₀-Alkyl, Alkanoyl mit C₁- bis C₈-Alkyl, Carboxylester mit C₁- bis C₈-Alkyl oder ein aromatischer Rest ist,
   R¹⁵ H, NH₂, NH(CO)R¹⁶, C₁- bis C₂₀-Alkyl, Alkanoyl mit C₁-bis C₈-Alkyl, Carboxylester mit C₁- bis C₈-Alkyl oder ein aromatischer Rest ist, wobei R¹⁶ H, C₁- bis C₂₀-Alkyl oder ein aromatischer Rest ist
   und eine oder mehrere polymerisationsfähige Verbindungen.
2. Mischung nach Ausführungsform 1, wobei
   R¹ H, C₁- bis C₈-Alkyl, Alkanoyl mit C₁- bis C₆-Alkyl oder ein aromatischer Rest ist,
   R² H, C₁- bis C₈-Alkyl, Alkanoyl mit C₁- bis C₆-Alkyl oder ein aromatischer Rest ist,
   R³ H, C₁- bis C₈-Alkyl, Alkanoyl mit C₁- bis C₆-Alkyl, ein aromatischer Rest oder -CH₂-CHR⁴-C(O)OR⁵ ist, wobei R⁴ H oder Methyl ist und R⁵ H, C₁-bis C₈ Alkyl oder ein aromatischer Rest ist,
   wobei R² und R³ nicht gleichzeitig Methyl sind, wenn R¹ Phenyl ist.
3. Mischung nach Ausführungsform 1, wobei
   R⁶ H, C₁- bis C₈-Alkyl, Alkanoyl mit C₁- bis C₆-Alkyl oder ein aromatischer Rest ist,
   R⁷ H, C₁- bis C₈-Alkyl, Alkanoyl mit C₁- bis C₆-Alkyl oder ein aromatischer Rest ist,
   R⁸ H, C₁- bis C₈-Alkyl, Alkanoyl mit C₁- bis C₆-Alkyl, ein aromatischer Rest oder -CH₂-CHR^{4I}-C(O)OR^{5I} ist, wobei R^{4I} H oder Methyl ist und R^{5I} H, C₁-bis C₈-Alkyl oder ein aromatischer Rest ist,
   wobei
   R⁶ und R⁷ nicht gleichzeitig Phenyl sind, wenn R⁸ Phenyl oder Methyl ist,
   R⁶ und R⁸ nicht gleichzeitig Phenyl sind, wenn R⁷ H oder Methyl ist,
   R⁶ nicht Phenyl und R⁸ nicht Methyl ist, wenn R⁷ H oder Methyl ist.
4. Mischung nach Ausführungsform 1, wobei
   R⁹ H, C₁- bis C₈-Alkyl, Alkanoyl mit C₁- bis C₆-Alkyl oder ein aromatischer Rest ist,
   R¹⁰ H, C₁- bis C₈-Alkyl, Alkanoyl mit C₁- bis C₆-Alkyl, ein aromatischer Rest oder - C(O)O-R^{5II} ist, wobei R^{5II} H, C₁-bis C₈ Alkyl oder ein aromatischer Rest ist,
   R¹¹ H, C₁-bis C₈-Alkyl, Alkanoyl mit C₁- bis C₆-Alkyl, ein aromatischer Rest oder-C(O)O-R^{5III} ist, wobei R^{5III} H, C₁- bis C₈-Alkyl oder ein aromatischer Rest ist.
5. Mischung nach Ausführungsform 1, wobei
   R¹² H, C₁- bis C₈-Alkyl, Alkanoyl mit C₁- bis C₆-Alkyl oder ein aromatischer Rest ist,
   R¹³ H, C₁- bis C₈-Alkyl, Alkanoyl mit C₁- bis C₆-Alkyl, ein aromatischer Rest oder - C(O)O-R^{5IV} ist, wobei R^{5IV} H, C₁- bis C₈-Alkyl oder ein aromatischer Rest ist.
   R¹⁴ H, C₁- bis C₈-Alkyl, Alkanoyl mit C₁- bis C₆-Alkyl oder ein aromatischer Rest ist,
   R¹⁵ H, C₁- bis C₈-Alkyl, Alkanoyl mit C₁- bis C₆-Alkyl oder ein aromatischer Rest ist.
6. Mischung nach Ausführungsform 1, wobei
   R¹ H, C₁- bis C₆-Alkyl, Alkanoyl mit C₁- bis C₆-Alkyl oder ein aromatischer Rest ist,
   R² H, C₁- bis C₆-Alkyl, Alkanoyl mit C₁- bis C₆-Alkyl oder ein aromatischer Rest ist,
   R³ H, C₁- bis C₆-Alkyl, Alkanoyl mit C₁- bis C₆-Alkyl, ein aromatischer Rest oder -CH₂-CHR⁴-C(O)OR⁵ ist, wobei R⁴ H oder Methyl ist und R⁵ H, C₁-bis C₆ Alkyl oder ein aromatischer Rest ist,
   wobei R² und R³ nicht gleichzeitig Methyl sind, wenn R¹ Phenyl ist.
7. Mischung nach Ausführungsform 1, wobei
   R⁶ H, C₁- bis C₆-Alkyl, Alkanoyl mit C₁- bis C₆-Alkyl, oder ein aromatischer Rest ist,
   R⁷ H, C₁- bis C₆-Alkyl, Alkanoyl mit C₁- bis C₆-Alkyl oder ein aromatischer Rest ist,
   R⁸ H, C₁- bis C₆-Alkyl, Alkanoyl mit C₁- bis C₆-Alkyl, ein aromatischer Rest oder-CH₂-CHR^{4I}-C(O)OR^{5I} ist, wobei R^{4I} H oder Methyl ist und R^{5I} H, C₁-bis C₆-Alkyl oder ein aromatischer Rest ist,
   wobei
   R⁶ und R⁷ nicht gleichzeitig Phenyl sind, wenn R⁸ Phenyl oder Methyl ist,
   R⁶ und R⁸ nicht gleichzeitig Phenyl sind, wenn R⁷ H oder Methyl ist,
   R⁶ nicht Phenyl und R⁸ nicht Methyl ist, wenn R⁷ H oder Methyl ist.
8. Mischung nach Ausführungsform 1, wobei
   R⁹ H, C₁- bis C₆-Alkyl, Alkanoyl mit C₁- bis C₆-Alkyl oder ein aromatischer Rest ist,
   R¹⁰ H, C₁- bis C₆-Alkyl, Alkanoyl mit C₁- bis C₆-Alkyl, ein aromatischer Rest oder-C(O)O-R^{5II} ist, wobei R^{5II} H, C₁-bis C₆ Alkyl oder ein aromatischer Rest ist,
   R¹¹ H, C₁-bis C₆-Alkyl, Alkanoyl mit C₁- bis C₆-Alkyl, ein aromatischer Rest oder-C(O)O-R^{5III} ist, wobei R^{5III} H, C₁- bis C₆-Alkyl oder ein aromatischer Rest ist.
9. Mischung nach Ausführungsform 1, wobei
   R¹² H, C₁- bis C₆-Alkyl, Alkanoyl mit C₁- bis C₆-Alkyl oder ein aromatischer Rest ist,
   R¹³ H, C₁- bis C₆-Alkyl, Alkanoyl mit C₁- bis C₆-Alkyl, ein aromatischer Rest oder-C(O)O-R^{5IV} ist, wobei R^{5IV} H, C₁- bis C₆-Alkyl oder ein aromatischer Rest ist.
   R¹⁴ H, C₁- bis C₆-Alkyl, Alkanoyl mit C₁- bis C₆-Alkyl oder ein aromatischer Rest ist,
   R¹⁵ H, C₁- bis C₆-Alkyl, NH₂, NH(CO)R¹⁶, Alkanoyl mit C₁- bis C₆-Alkyl oder ein aromatischer Rest ist, wobei R¹⁶ H, C₁-bis C₆-Alkyl oder ein aromatischer Rest ist
10. Mischung enthaltend 2-Isopropylpyrazol3-ol, 1-Methylpyrazol-4-ol, 5-Methyl-2-phenyl-4H-pyrazok-3-on, 1,3,5-Trimethylpyrazol-4-ol, 1-Isopropyl-3,5-dimethyl-pyrazol-4-ol, 1-(4-Hydroxy-1,5-dimethyl-pyrazol-3-yl)ethanon, 1-Benzylpyrazol-4-ol oder eine belieibe Mischung aus zwei oder mehr der zuvor genannten Verbindungen und eine oder mehrere polymerisationsfähige Verbindungen.
11. Mischung nach einer der Ausführungsformen 1 bis 10, wobei
   es sich bei einer polymerisationsfähigen Verbindung um (Meth)acrylsäure oder um (Meth)acrylsäureester mit C₁- bis C₈-Alkyl, insbesondere um (Meth)acrylsäure, Methyl(meth)acrylat, Ethyl(meth)acrylat, n-Butyl(meth)acrylat oder 2-Ethylhexyl(meth)acrylat handelt.
12. Mischung nach einer der Ausführungsformen 1 bis 11, wobei die Gesamtmenge der Verbindungen der allgemeinen Formel (I), (II), (III) und (IV) in der Mischung 0,1 bis 1000 Gew. ppm, bevorzugt 1 bis 900 Gew. ppm, besonders bevorzugt 10 bis 800 Gew. ppm und insbesondere 10 bis 500 Gew. ppm beträgt, bezogen auf das Gesamtgewicht der polymerisationsfähigen Verbindungen.
13. Mischung nach einer der Ausführungsformen 1 bis 12, wobei die Gesamtmenge der polymerisationsfähigen Verbindungen in der Mischung mindestens 5 Gew.% bezogen auf das Gesamtgewicht der Mischung, weiter bevorzugt mindestens 20 Gew. %, weiter bevorzugt mindestens 30 Gew.%, weiter bevorzugt mindestens 40 Gew.%, weiter bevorzugt mindestens 50 Gew.%, weiter bevorzugt mindestens 60 Gew.%, weiter bevorzugt mindestens 70 Gew.%, weiter bevorzugt mindestens 80 Gew.% , weiter bevorzugt mindestens 90 Gew.% und besonders bevorzugt mindestens 95 Gew.% beträgt.
14. Mischung nach einer der Ausführungsformen 1 bis 13, wobei es sich bei der polymerisationsfähigen Verbindung um (Meth)acrylsäure handelt, wobei die Mischung eine oder mehrere der nachfolgenden Komponenten aufweist:
   a. Wasser 20 bis 15000 Gew. ppm bezogen auf die Menge an (Meth)acrylsäure
   b. Di(meth)acrylsäure 100 bis 20000 Gew. ppm bezogen auf die Menge an (Meth)acrylsäure
   c. Essigsäure 100 bis 2000 Gew. ppm bezogen auf die Menge an (Meth)acrylsäure
   d. Propionsäure 100 bis 2000 Gew. ppm bezogen auf die Menge an (Meth)acrylsäure
   e. ein oder mehrere Aldehyde wie Acetaldehyd, Acrolein, Propionaldehyd, Furfural-2, Furfural-3, Cumaron oder Benzaldehyd, wobei die Menge pro Aldehyd 0 bis 1 Gew. ppm bezogen auf die Menge an (Meth)acrylsäure beträgt
   f. Protoanemonin 0 bis 2 Gew. ppm bezogen auf die Menge an (Meth)acrylsäure
   g. Maleinsäure 0 bis 1 Gew. ppm bezogen auf die Menge an (Meth)acrylsäure
   h. Maleinsäureanhydrid 0 bis 1 Gew. ppm bezogen auf die Menge an (Meth)acrylsäure
   i. Allylacrylat 0 bis 30 Gew. ppm, insbesonders 0 bis 1 Gew. ppm bezogen auf die Menge an (Meth)acrylsäure.
15. Mischung nach einer der Ausführungsformen 1 bis 13, wobei es sich bei einer polymerisationsfähigen Verbindung um einen (Meth)acrylsäureester gemäß Ausführungsform 11 handelt, wobei die Mischung eine oder mehrere der nachfolgenden Komponenten aufweist:
   a. Wasser 1 bis 2000 Gew. ppm bezogen auf die Menge an (Meth)acrylsäureester, bevorzugt 1 bis 500 Gew. ppm
   b. (Meth)acrylsäure 1 bis 2000 Gew. ppm bezogen auf die Menge an (Meth)acrylsäureester, bevorzugt 1 bis 100 Gew. ppm
   c. Alkohole der jeweiligen (Meth)acrylsäureester 0,01 bis 2000 Gew. ppm, bevorzugt 0,1 bis 1000 Gew. ppm bezogen auf die Menge an (Meth)acrylsäureester
   d. Propionat-Ester mit den Alkoholen der jeweiligen (Meth)acrylsäureester 0,1 bis 2000 Gew. ppm, bevorzugt 10 bis 1000 Gew. ppm bezogen auf die Menge an (Meth)acrylsäureester
   e. Acetat-Ester mit den Alkoholen der jeweiligen (Meth)acrylsäureester 1 bis 2000 Gew. ppm, bevorzugt 5 bis 2000 Gew. ppm bezogen auf die Menge an (Meth)acrylsäureester
   f. Ether der jeweiligen (Meth)acrylsäureester-Alkohole 0 bis 2000 Gew. ppm bezogen auf die Menge an (Meth)acrylsäureester.
16. Mischung nach einer der Ausführungsformen 1 bis 15, wobei die Mischung unter einer sauerstoffhaltigen Atomsphäre gelagert und/oder transportiert wird.
17. Mischung nach Ausführungsform 16, wobei die sauerstoffhaltige Atomsphäre einen Sauerstoffgehalt von 5 bis 30 vol.% Sauerstoff und insbesondere einen Sauerstoffgehalt von 5 bis 21 vol.% aufweist.
18. Mischung nach Ausführungsform 16 oder 17, wobei die Mischung für 10 Sekunden oder mehr, bevorzugt für 1 Minute oder mehr, weiter bevorzugt für 10 Minuten oder mehr und besonders bevorzugt für 60 Minuten oder mehr unter einer sauerstoffhalten Atmosphäre gelagert und/oder transportiert wird.
19. Mischung nach einer der Ausführungsformen 1 bis 18, wobei die Mischung einen Costabilisator enthält.
20. Mischung nach Ausführungsform 19, wobei die in der Mischung enthaltene Gesamtmenge an Costabilisator 0,1 bis 5000 Gew. ppm bezogen auf die Gesamtmenge der in der Mischung enthaltenen polymerisationsfähigen Verbindungen beträgt, bevorzugt, 1 bis 4000 Gew. ppm, insbesondere 5 bis 2500 Gew. ppm und weiter bevorzugt 50 bis 750 Gew. ppm.
21. Mischung nach einer der Ausführungsformen 19 bis 20, wobei es sich bei dem Costabilisator um Metallsalze bestehend aus der Gruppe von Kupfer-, Mangan- und Cer-Salzen, Phenothiazine, phenolische Verbindungen, N-Oxyle, Phenylendiamine, Nitrosoverbindungen, Harnstoffe, Thioharnstoffe, oder um Mischungen aus zwei oder mehr der zuvor genannten Substanzklassen handelt.
22. Verwendung einer oder mehrerer Verbindungen der allgemeinen Formel (I), (II), (III), (IV) wie in einer der Ausführungsformen 1 bis 10 definiert oder einer beliebigen Mischung dieser, zur Lager- und/oder Transportstabilisierung einer oder mehrerer polymerisationsfähigen Verbindungen.
23. Verwendung nach Ausführungsform 22, wobei es sich bei einer polymerisationsfähigen Verbindung um (Meth)acrylsäure oder um (Meth)acrylsäureester mit C₁- bis C₈-Alkyl, insbesondere um (Meth)acrylsäure, Methyl(meth)acrylat, Ethyl(meth)acrylat, n-Butyl(meth)acrylat oder 2-Ethylhexyl(meth)acrylat handelt.
24. Verwendung nach einer der Ausführungsformen 22 oder 23, wobei die Verbindungen der allgemeinen Formel (I), (II), (III) und/oder (IV) in einer Gesamtmenge von 0,1 bis 1000 Gew. ppm, bevorzugt 1 bis 900 Gew. ppm, besonders bevorzugt 10 bis 800 Gew. ppm und insbesondere 10 bis 500 Gew. ppm, bezogen auf das Gesamtgewicht der polymerisationsfähigen Verbindungen eingesetzt werden.
25. Verwendung nach einer der Ausführungsformen 22 bis 24, wobei es sich bei einer polymerisationsfähigen Verbindung um (Meth)acrylsäure handelt und wobei eine oder mehrere der nachfolgenden Komponenten enthalten sind:
   a. Wasser 20 bis 15000 Gew. ppm bezogen auf die Menge an (Meth)acrylsäure
   b. Di(meth)acrylsäure 100 bis 20000 Gew. ppm bezogen auf die Menge an (Meth)acrylsäure
   c. Essigsäure 100 bis 2000 Gew. ppm bezogen auf die Menge an (Meth)acrylsäure
   d. Propionsäure 100 bis 2000 Gew. ppm bezogen auf die Menge an (Meth)acrylsäure
   e. ein oder mehrere Aldehyde wie Acetaldehyd, Acrolein, Propionaldehyd, Furfural-2, Furfural-3, Cumaron oder Benzaldehyd, wobei die Menge pro Aldehyde 0 bis 1 Gew. ppm bezogen auf die Menge an (Meth)acrylsäure beträgt
   f. Protoanemonin 0 bis 2 Gew. ppm bezogen auf die Menge an (Meth)acrylsäure
   g. Maleinsäure 0 bis 1 Gew. ppm bezogen auf die Menge an (Meth)acrylsäure
   h. Maleinsäureanhydrid 0 bis 1 Gew. ppm bezogen auf die Menge an (Meth)acrylsäure
   i. Allylacrylat 0 bis 30 Gew. ppm, insbesonders 0 bis 1 Gew. ppm bezogen auf die Menge an (Meth)acrylsäure.
26. Verwendung nach einer der Ausführungsformen 22 bis 24, wobei es sich bei einer polymerisationsfähigen Verbindung um einen (Meth)acrylsäureester gemäß Ausführungsform 23 handelt und wobei eine oder mehrere der nachfolgenden Komponenten enthalten sind:
   a. Wasser 1 bis 2000 Gew. ppm bezogen auf die Menge an (Meth)acrylsäureester, bevorzugt 1 bis 500 Gew. ppm
   b. (Meth)acrylsäure 1 bis 2000 Gew. ppm bezogen auf die Menge an (Meth)acrylsäureester, bevorzugt 1 bis 100 Gew. ppm
   c. Alkohole der jeweiligen (Meth)acrylsäureester 0,01 bis 2000 Gew. ppm, bevorzugt 0,1 bis 1000 Gew. ppm bezogen auf die Menge an (Meth)acrylsäureester
   d. Propionat-Ester mit den Alkoholen der jeweiligen (Meth)acrylsäureester 0,1 bis 2000 Gew. ppm, bevorzugt 10 bis 1000 Gew. ppm bezogen auf die Menge an (Meth)acrylsäureester
   e. Acetat-Ester mit den Alkoholen der jeweiligen (Meth)acrylsäureester 1 bis 2000 Gew. ppm, bevorzugt 5 bis 2000 Gew. ppm bezogen auf die Menge an (Meth)acrylsäureester
   f. Ether der jeweiligen (Meth)acrylsäureester-Alkohole 0 bis 2000 Gew. ppm bezogen auf die Menge an (Meth)acrylsäureester.
27. Verfahren zur Polymerisation von einer oder mehreren polymerisationsfähigen Verbindungen in Gegenwart einer oder mehrerer Verbindungen der allgemeinen Formel (I), (II), (III), (IV) wie in einer der Ausführungsformen 1 bis 10 definiert oder einer beliebige Mischung dieser, wobei die Reaktionsmischung zu 0 bis 50 %, bevorzugt zu 0 bis 10 %, besonders bevorzugt zu 0 bis 5 % weiter bevorzugt 0 bis 1% und insbesonders bevorzugt zu 0 bis 0,1 % mit Sauerstoff gesättigt ist, wobei der Grad der Sauerstoffsättigung sich auf den Grad der Sauerstoffsättigung bezieht der im Gleichgewicht erreicht wird, wenn die vorliegende Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.
28. Verfahren nach Ausführungsform 27, wobei die Gesamtmenge der Verbindungen der allgemeinen Formel (I), (II), (III) und (IV) in der Reaktionsmischung 0,1 bis 1000 Gew. ppm, bevorzugt 1 bis 900 Gew. ppm, besonders bevorzugt 10 bis 800 Gew. ppm und insbesondere 10 bis 500 Gew. ppm beträgt, bezogen auf das Gesamtgewicht der polymerisationsfähigen Verbindungen.
29. Verfahren nach einer der Ausführungsformen 27 bis 28, wobei die Polymerisation bei einer Temperatur von 50 bis 150 °C und bevorzugt 70 bis 120 °C durchgeführt wird.
30. Verfahren nach einer der Ausführungsformen 27 bis 29, wobei die Polymerisation unter einer Stickstoffatomsphäre mit einem Stickstoffgehalt von 85 bis 100 vol.% und einem Sauerstoffgehalt von weniger als 5 vol.%, bevorzugt mit einem Stickstoffgehalt von 90 bis 100 vol.% und besonders bevorzugt mit einem Stickstoffgehalt von 99 bis 100 vol.% durchgeführt wird.
31. Verfahren nach einer der Ausführungsformen 27 bis 30, wobei es sich bei einer polymerisationsfähigen Verbindung um (Meth)acrylsäure oder um (Meth)acrylsäureester mit C₁- bis C₈-Alkyl, insbesondere um (Meth)acrylsäure, Methyl(meth)acrylat, Ethyl(meth)acrylat, n-Butyl(meth)acrylat oder 2-Ethylhexyl(meth)acrylat handelt.
32. Verfahren nach einer der Ausführungsformen 27 bis 30, wobei es sich bei einer polymerisationsfähigen Verbindung um (Meth)acrylsäure handelt und wobei eine oder mehrere der nachfolgenden Komponenten enthalten sind:
   a. Wasser 20 bis 15000 Gew. ppm bezogen auf die Menge an (Meth)acrylsäure
   b. Di(meth)acrylsäure 100 bis 20000 Gew. ppm bezogen auf die Menge an (Meth)acrylsäure
   c. Essigsäure 100 bis 2000 Gew. ppm bezogen auf die Menge an (Meth)acrylsäure
   d. Propionsäure 100 bis 2000 Gew. ppm bezogen auf die Menge an (Meth)acrylsäure
   e. ein oder mehrere Aldehyde wie Acetaldehyd, Acrolein, Propionaldehyd, Furfural-2, Furfural-3, Cumaron oder Benzaldehyd, wobei die Menge pro Aldehyd 0 bis 1 Gew. ppm bezogen auf die Menge an (Meth)acrylsäure beträgt
   f. Protoanemonin 0 bis 2 Gew. ppm bezogen auf die Menge an (Meth)acrylsäure
   g. Maleinsäure 0 bis 1 Gew. ppm bezogen auf die Menge an (Meth)acrylsäure
   h. Maleinsäureanhydrid 0 bis 1 Gew. ppm bezogen auf die Menge an (Meth)acrylsäure
   i. Allylacrylat 0 bis 30 Gew. ppm, insbesonders 0 bis 1 Gew. ppm bezogen auf die Menge an (Meth)acrylsäure.
33. Verfahren nach einer der Ausführungsformen 27 bis 30, wobei es sich bei einer polymerisationsfähigen Verbindung um einen (Meth)acrylsäureester gemäß Ausführungsform 31 handelt und wobei eine oder mehrere der nachfolgenden Komponenten enthalten sind:
   a. Wasser 1 bis 2000 Gew. ppm bezogen auf die Menge an (Meth)acrylsäureester, bevorzugt 1 bis 500 Gew. ppm
   b. (Meth)acrylsäure 1 bis 2000 Gew. ppm bezogen auf die Menge an (Meth)acrylsäureester, bevorzugt 1 bis 100 Gew. ppm
   c. Alkohole der jeweiligen (Meth)acrylsäureester 0,01 bis 2000 Gew. ppm, bevorzugt 0,1 bis 1000 Gew. ppm bezogen auf die Menge an (Meth)acrylsäureester
   d. Propionat-Ester mit den Alkoholen der jeweiligen (Meth)acrylsäureester 0,1 bis 2000 Gew. ppm, bevorzugt 10 bis 1000 Gew. ppm bezogen auf die Menge an (Meth)acrylsäureester
   e. Acetat-Ester mit den Alkoholen der jeweiligen (Meth)acrylsäureester 1 bis 2000 Gew. ppm, bevorzugt 5 bis 2000 Gew. ppm bezogen auf die Menge an (Meth)acrylsäureester
   f. Ether der jeweiligen (Meth)acrylsäureester-Alkohole 0 bis 2000 Gew. ppm bezogen auf die Menge an (Meth)acrylsäureester.
34. Verfahren zur Stabilisierung von einer oder mehrerer polymerisationsfähigen Verbindungen unter Verwendung einer oder mehrerer Verbindungen der allgemeinen Formel (I), (II), (III), (IV) wie einer der Ausführungsformen 1 bis 10 definiert, oder einer beliebigen Mischung dieser.
35. Verfahren nach Ausführungsform 34, wobei die eine oder mehrere polymerisationsfähigen Verbindungen bei Lagerung und/oder Transport stabilisiert werden.
36. Verfahren nach einer der Ausführungsformen 34 bis 35, wobei es sich bei einer polymerisationsfähigen Verbindung um (Meth)acrylsäure oder um (Meth)acrylsäureester mit C₁- bis C₈-Alkyl, insbesondere um (Meth)acrylsäure, Methyl(meth)acrylat, Ethyl(meth)acrylat, n-Butyl(meth)acrylat oder 2-Ethylhexyl(meth)acrylat handelt.
37. Verfahren nach einer der Ausführungsformen 34 bis 35, wobei es sich bei einer polymerisationsfähigen Verbindung um (Meth)acrylsäure handelt und wobei eine oder mehrere der nachfolgenden Komponenten enthalten sind:
   a. Wasser 20 bis 15000 Gew. ppm bezogen auf die Menge an (Meth)acrylsäure
   b. Di(meth)acrylsäure 100 bis 20000 Gew. ppm bezogen auf die Menge an (Meth)acrylsäure
   c. Essigsäure 100 bis 2000 Gew. ppm bezogen auf die Menge an (Meth)acrylsäure
   d. Propionsäure 100 bis 2000 Gew. ppm bezogen auf die Menge an (Meth)acrylsäure
   e. ein oder mehrere Aldehyde wie Acetaldehyd, Acrolein, Propionaldehyd, Furfural-2, Furfural-3, Cumaron oder Benzaldehyd, wobei die Menge pro Aldehyd 0 bis 1 Gew. ppm bezogen auf die Menge an (Meth)acrylsäure beträgt
   f. Protoanemonin 0 bis 2 Gew. ppm bezogen auf die Menge an (Meth)acrylsäure
   g. Maleinsäure 0 bis 1 Gew. ppm bezogen auf die Menge an (Meth)acrylsäure
   h. Maleinsäureanhydrid 0 bis 1 Gew. ppm bezogen auf die Menge an (Meth)acrylsäure
   i. Allylacrylat 0 bis 30 Gew. ppm, insbesonders 0 bis 1 Gew. ppm bezogen auf die Menge an (Meth)acrylsäure.
38. Verfahren nach einer der Ausführungsformen 34 bis 35, wobei es sich bei einer polymerisationsfähigen Verbindung um einen (Meth)acrylsäureester gemäß Ausführungsform 36 handelt und wobei eine oder mehrere der nachfolgenden Komponenten enthalten sind:
   a. Wasser 1 bis 2000 Gew. ppm bezogen auf die Menge an (Meth)acrylsäureester, bevorzugt 1 bis 500 Gew. ppm
   b. (Meth)acrylsäure 1 bis 2000 Gew. ppm bezogen auf die Menge an (Meth)acrylsäureester, bevorzugt 1 bis 100 Gew. ppm
   c. Alkohole der jeweiligen (Meth)acrylsäureester 0,01 bis 2000 Gew. ppm, bevorzugt 0,1 bis 1000 Gew. ppm bezogen auf die Menge an (Meth)acrylsäureester
   d. Propionat-Ester mit den Alkoholen der jeweiligen (Meth)acrylsäureester 0,1 bis 2000 Gew. ppm, bevorzugt 10 bis 1000 Gew. ppm bezogen auf die Menge an (Meth)acrylsäureester
   e. Acetat-Ester mit den Alkoholen der jeweiligen (Meth)acrylsäureester 1 bis 2000 Gew. ppm, bevorzugt 5 bis 2000 Gew. ppm bezogen auf die Menge an (Meth)acrylsäureester
   f. Ether der jeweiligen (Meth)acrylsäureester-Alkohole 0 bis 2000 Gew. ppm bezogen auf die Menge an (Meth)acrylsäureester.
39. Verfahren nach einer der Ausführungsformen 34 bis 38, wobei das Verfahren das Bereitstellen einer Mischung, die eine oder mehrere Verbindungen der allgemeinen Formel (I), (II), (III), (IV) oder eine beliebige Mischung dieser und eine oder mehrere polymerisationsfähige Verbindungen enthält umfasst, wobei die Mischung mit Sauerstoff zu 50 bis 100%, bevorzugt zu 90 bis 100% und besonders bevorzugt zu 95 bis 100 % gesättigt ist, wobei der Grad der Sauerstoffsättigung sich auf den Grad der Sauerstoffsättigung bezieht der im Gleichgewicht erreicht wird, wenn die vorliegende Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.
40. Verfahren nach Ausführungsform 39, wobei die Gesamtmenge der polymerisationsfähigen Verbindungen in der Mischung mindestens 5 Gew.% bezogen auf das Gesamtgewicht der Mischung, weiter bevorzugt mindestens 20 Gew. %, weiter bevorzugt mindestens 30 Gew.%, weiter bevorzugt mindestens 40 Gew.%, weiter bevorzugt mindestens 50 Gew.%, weiter bevorzugt mindestens 60 Gew.%, weiter bevorzugt mindestens 70 Gew.%, weiter bevorzugt mindestens 80 Gew.% , weiter bevorzugt mindestens 90 Gew.% und besonders bevorzugt mindestens 95 Gew.% beträgt.
41. Verfahren nach einer der Ausführungsformen 34 bis 40, wobei die Verbindungen der allgemeinen Formel (I), (II), (III) und/oder (IV) in einer Gesamtmenge von 0,1 bis 1000 Gew. ppm, bevorzugt 1 bis 900 Gew. ppm, besonders bevorzugt 10 bis 800 Gew. ppm und insbesondere 10 bis 500 Gew. ppm, bezogen auf das Gesamtgewicht der polymerisationsfähigen Verbindungen eingesetzt werden.

### Beispiele:

Die nachfolgenden Beispiele schränken den Gegenstand der vorliegenden Erfindung nicht ein.

| Einsatzstoff | Hersteller | Reinheit |
|---|---|---|
| Acrylsäure | BASF SE | ≥99% |
| Toluol | BASF SE | >99,7 % |
| 2,2'-Azobis(4-methoxy-2,4-dimethylvalerontril) | FUJIFILM Wako Chemicals Europe GmbH | n.a. |
| 4-Methoxyphenol | Sigma-Aldrich | ≥98% |
| Ethylacrylat | BASF SE | ≥99,7% |

Die in den Versuchen eingesetzte Acrylsäure wurde frisch destilliert, um den darin enthaltenen Stabilisator zu entfernen. Die destillierte Acrylsäure weist eine Reinheit von größer 99,5 Gew. % auf. In der destillierten Acrylsäure enthaltene Nebenkomponenten sind Essigsäure (0,0726 Gew.%), Diacrylsäure (0,0497 Gew.%) und Propionsäure (0,0184 Gew. %).

Gewichtsangaben in den Versuchsbeschreibungen, wie Gewichtsprozent (Gew. %) oder Gew. ppm, die auf Acrylsäure bezogen sind, beziehen sich auf die Masse der jeweils eingesetzten Acrylsäure incl. der darin enthaltenen Nebenkomponenten.

Versuche zur Polymerisationsinhibierung unter Luft oder Stickstoff:
Für die Vergleichsversuche wurde eine 40 Gew. ppm 4-Methoxyphenol (MEHQ) enthaltende Acrylsäurelösung hergestellt. Die Angabe Gew. ppm bezieht sich auf die Masse der eingesetzten Acrylsäure.

Für weitere Vergleichsversuche wurden Mischungen von Acrylsäure mit Verbindungen, die von den Verbindungen der allgemeinen Formel (I), (II), (III) oder (IV) unterschiedlich sind, hergestellt, wobei die Molmenge der eingesetzten Verbindungen der Molmenge an MEHQ in den Vergleichsversuchen entspricht.

Für die erfindungsgemäßen Versuche wurden Mischungen von Acrylsäure mit Verbindungen der allgemeinen Formel (I), (II), (III) und/oder (IV) hergestellt, wobei die Molmenge an Verbindungen der allgemeinen Formel (I), (II), (III) und/oder (IV) in den Mischungen der Molmenge an MEHQ in den Vergleichsversuchen entspricht.

### Exemplarische Verdünnungsreihe:

Von einer Acrylsäurelösung mit einem Gehalt von 1000 Gew. ppm MEHQ bezogen auf die Masse der eingesetzten Acrylsäure wurden 2,5 g entnommen und mit Acrylsäure auf einen Gehalt von 500 Gew. ppm MEHQ bezogen auf die Masse der Acrylsäure verdünnt.
Von der so erhaltenen Acrylsäurelösung wurden 2,5 g entnommen und mit Acrylsäure auf einen Gehalt von 250 Gew. ppm MEHQ bezogen auf die Masse der Acrylsäure verdünnt. Von der so erhaltenen Acrylsäurelösung wurden 2,5 g entnommen und mit Acrylsäure auf einen Gehalt von 125 Gew. ppm MEHQ bezogen auf die Masse der Acrylsäure verdünnt. Von der so erhaltenen Acrylsäurelösung wurden 2,5 g entnommen und mit Acrylsäure auf einen Gehalt von 80 Gew. ppm MEHQ bezogen auf die Masse der Acrylsäure verdünnt. Von der so erhaltenen Acrylsäurelösung wurden 2,5 g entnommen und mit Acrylsäure auf einen Gehalt von 40 Gew. ppm MEHQ bezogen auf die Masse der Acrylsäure verdünnt.

Als Starterlösung wurde eine frisch angesetzte Toluol-Lösung von 2,2'-Azobis(4-methoxy-2,4-dimethylvalerontril) verwendet. Der Gehalt an 2,2'-Azobis(4-methoxy-2,4-dimehtylvalerontril) in der Starterlösung beträgt 6420 Gew. ppm bezogen auf die Masse an Toluol in der Starterlösung.

### Tests zur Polymerisationsinhibierung unter Luft:

Unter Luft wurden 2.2 g Acrylsäurelösung enthaltend 0,000715 mmol des jeweiligen Polymerisationsstabilisators vorgelegt. Die vorgelegte Acrylsäurelösung wurde mit 0,22 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß.

Das Reaktionsgefäß enthaltend die Mischung wurde anschließend in einem auf 40°C erwärmten Heizblock platziert.

Nachdem die Mischung die Temperatur von 40°C erreicht hat wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 42 Sekunden dauernder Temperaturanstieg um 145°C auf eine maximale Temperatur von 185°C gemessen. Die maximale Temperatur lag nach 9188 Sekunden vor, nachdem die Mischung auf 40°C temperiert war. Zur Berechnung der Effizienz der Polymerisationsstabilisatoren in Bezug auf MEHQ entspricht der Wert von 9188 Sekunden einer Effizienz von 100 Sekunden.

Für eine erfindungsgemäße Mischung mit 5-Methyl-2-phenyl-4H-pyrazol-3-on als Polymerisationsstabilisator wurde ein 48 Sekunden dauernder Temperaturanstieg um 125°C auf eine maximale Temperatur von 165°C beobachtet. Die maximale Temperatur lag nach 7874 Sekunden vor, nachdem die Mischung auf 40°C temperiert war. Im Vergleich zu MEHQ weist 5-Methyl-2-phenyl-4H-pyrazol-3-on als Polymerisationsstabilisator somit eine Effizienz von 86 Prozent auf.

### Tests zur Polymerisationsinhibierung unter Stickstoff:

Unter Luft wurden 2,2 g Acrylsäurelösung enthaltend 0,000715 mmol des jeweiligen Polymerisationsstabilisators vorgelegt. Die vorgelegte Acrylsäurelösung wurde mit 0,22 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß. In das Reaktionsgefäß wurde oberhalb der vorgelegten Mischung Stickstoff unter Druckausgleich eingeleitet. Der Stickstoffstrom beträgt 3 bis 4 l/h. Die Einleitdauer beträgt 20 Sekunden. Der Stickstoff weist eine Reinheit von größer 99,9 % auf.

Das Reaktionsgefäß enthaltend die Mischung wurde in einem Heizblock platziert. Die Mischung wurde anschließend im Heizblock innerhalb von 4 Minuten auf 40°C erwärmt.

Nachdem die Mischung die Temperatur von 40°C erreicht hat, wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 54 Sekunden dauernder Temperaturanstieg um 133°C auf eine maximale Temperatur von 173°C gemessen. Die maximale Temperatur lag nach 3978 Sekunden vor, nachdem die Mischung auf 40 °C temperiert war. Zur Berechnung der Effizienz der Polymerisationsstabilisatoren in Bezug auf MEHQ entspricht der Wert von 3978 Sekunden einer Effizienz von 100 %.

Für eine erfindungsgemäße Mischung mit 5-Methyl-2-phenyl-4H-pyrazol-3-on als Polymerisationsstabilisator wurde ein 66 Sekunden dauernder Temperaturanstieg um 119°C auf eine maximale Temperatur von 159 °C beobachtet. Die maximale Temperatur lag nach 1056 Sekunden vor, nachdem die Mischung auf 40°C temperiert war.
Im Vergleich zu MEHQ weist 5-Methyl-2-phenyl-4H-pyrazol-3-on als Polymerisationsstabilisator somit eine Effizienz von 27 Prozent auf.

Ergebnisse der Tests in Acrylsäure mit nicht erfindungsgemäßen Verbindungen:

| Struktur | IUPAC-Namen | Relative Stabilisierung von Acrylsäure unter Luft bezogen auf MeHQ (100%) | Relative Stabilisierung von Acrylsäure unter Stickstoff bezogen auf MeHQ (100%)) |
|---|---|---|---|
| 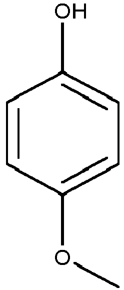 | 4-Methoxyphenol | 100 | 100 |
| 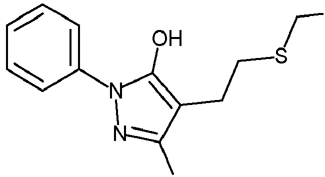 | 4-(2-Ethylsulfanylethyl)-5-methyl-2-phenyl-pyrazol-3-ol | 71 | 64 |
| 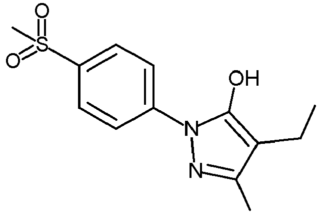 | 4-(2-Ethylsulfanylethyl)-5-methyl-2-(4-methylsulfonylphenyl)pyrazol-3-ol | 68 | 50 |
| 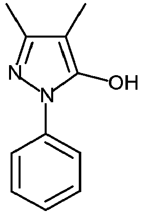 | 4,5-Dimethyl-2-phenyl-pyrazol-3-ol | 57 | 71 |
| 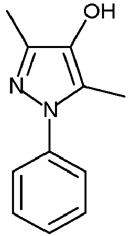 | 3,5-Dimethyl-1-phenyl-pyrazol-4-ol | 134 | 154 |
| 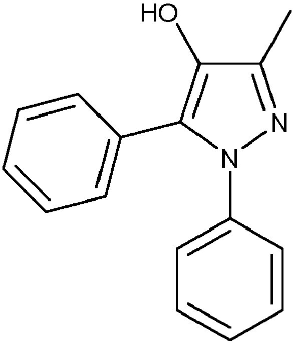 | 3-Methyl-1,5-diphenyl-pyrazol-4-ol | 97 | 161 |

Ergebnisse der Tests in Acrylsäure mit erfindungsgemäßen Verbindungen:

| Struktur | IUPAC-Name | Relative Stabilisierung von Acrylsäure unter Luft bezogen auf MeHQ (100%) | Relative Stabilisierung von Acrylsäure unter Stickstoff bezogen auf MeHQ (100%) |
|---|---|---|---|
| 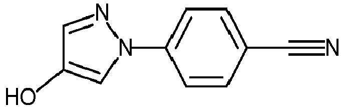 | 4-(4-Hydroxypyrazol-1-yl)benzonitrile | 85 | 33 |
| 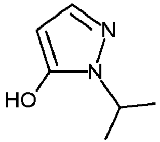 | 2-Isoopro-pylpyrazol-3-ol | 83 | 72 |
| 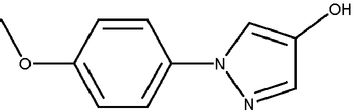 | 1-(4-Methoxyphenyl)pyrazol-4-ol | 95 | 43 |
| 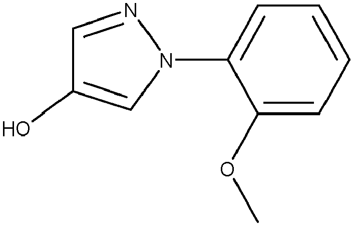 | 1-(2-Methoxyphenyl)-pyrazol-4-ol | 96 | 51 |
| 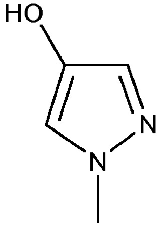 | 1-Methylpyrazol-4-ol | 93 | 53 |
| 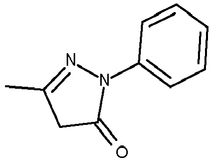 | 5-Methyl-2-phenyl-4H-pyrazol-3-one | 86 | 27 |
| 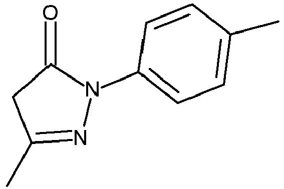 | 5-Methyl-2-(p-tolyl)-4H-pyrazol-3-one | 103 | 39 |
| 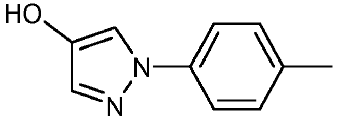 | 1-(p-Tolyl)pyrazol-4-ol | 89 | 51 |
| 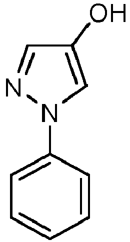 | 1-Phenylpyrazol-4-ol | 85 | 94 |
| 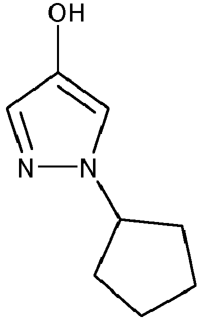 | 1-Cyclo-pentylpyrazol-4-ol | 144 | 104 |
| 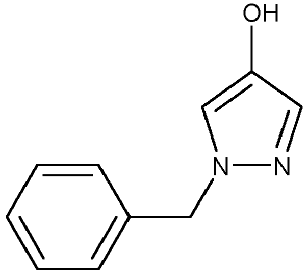 | 1-Benzylpyrazol-4-ol | 91 | 46 |
| 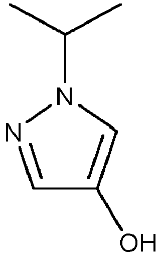 | 1-Isopropyl-pyrazol-4-ol | 89 | 80 |
| 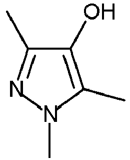 | 1,3,5-trimethyl-pyrazol-4-ol | 102 | 87 |
| 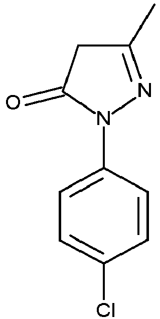 | 2-(4-Chlorophenyl)-5-methyl-4H-pyrazol-3-one | 78 | 12 |
| 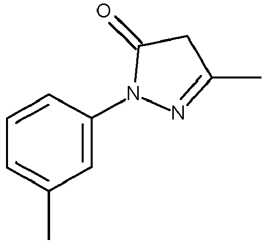 | 5-Methyl-2-(m-tolyl)-4H-pyrazol-3-one | 77 | 15 |
| 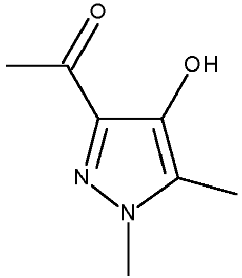 | 1-(4-hydroxy-1,5-dimethyl-pyrazol-3-yl)ethanone | 97% | 34% |
| 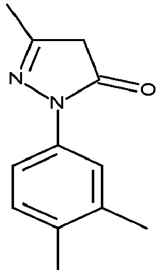 | 2-(3,4-Dimethylphenyl)-5-methyl-4H-pyrazol-3-one | 83 | 25 |
| 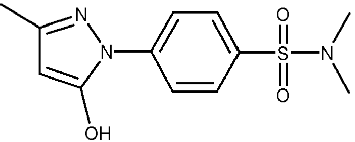 | 4-(5-Hydroxy-3-methyl-pyrazol-1-yl)-N,N-dimethyl-benzenesulfonamid | 75 | 13 |
| 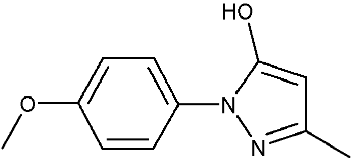 | 2-(4-methoxyphenyl)-5-methyl-pyrazol-3-ol | 84 | 16 |
| 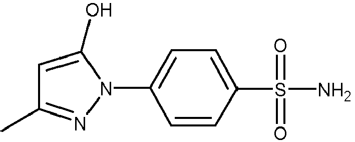 | 4-(5-Hydroxy-3-methyl-pyrazol-1-yl)-benzenesulfonamide | 78 | 11 |
| 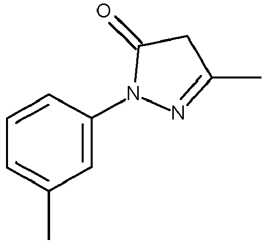 | 5-Methyl-2-(m-tolyl)-4H-pyrazol-3-one | 77 | 15 |
| 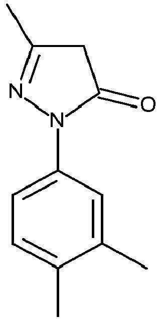 | 2-(3,4-Dimethylphenyl)-5-methyl-4H-pyrazol-3-one | 82 | 25 |
| 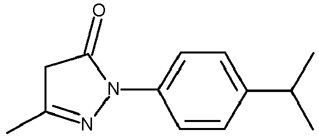 | 2-(4-Isopropylphenyl)-5-methyl-4H-pyrazol-3-one | 81 | 24 |
| 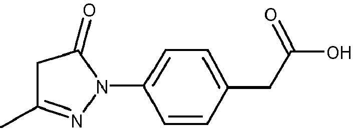 | 2-[4-(3-Methyl-5-oxo-4 H-pyrazol-1-yl)phenyl]acetic acid | 82 | 20 |
| 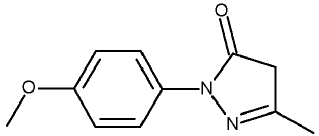 | 2-(4-Methoxyphenyl)-5-methyl-4H-pyrazol-3-one | 75 | 13 |
| 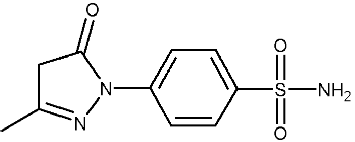 | 4-(3-Methyl-5-oxo-4 H-pyrazol-1-yl)benzenesulfona mide | 78 | 11 |
| 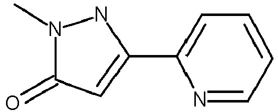 | 2-Methyl-5-(2-pyridyl)-1H-pyrazol-3-one | 79 | 18 |
| 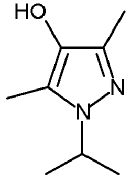 | 1-Isopropyl-3,5-dimethyl-pyrazol-4-ol | 238 | 122 |

### Polymerisationsversuche:

Unter einer Stickstoffatmosphäre wurden in einem Reaktionsgefäß 450 g deionisiertes Wasser vorgelegt. Die vorgelegte Reaktionsmischung wurde unter Rühren auf 95°C erhitzt. Nach Erreichen der Temperatur von 95°C erfolgte die Zuführung von drei Strömen unter Beibehaltung der Temperatur.
Strom 1: Zuführung über 5 h, 500 g Acrylsäure
Strom 2: Zuführung über 4,75 h, 15 g Natriumhypophosphite in 35 g deionisiertem Wasser
Strom 3: Zuführung über 5,25 h, 5 g Natriumpersulfat in 66,4 g deionisiertem Wasser

Nach Zugabe der drei Ströme wurde die Reaktionsmischung für eine weitere Stunde bei 95°C gerührt. Die Reaktionsmischung wurde anschließend auf Raumtemperatur abgekühlt und mit 80 g deionisiertem Wasser versetzt.

Erfindungsgemäß war die Acrylsäure von Strom 1 mit 280 Gew. ppm Edaravon (5-Methyl-2-phenyl-4H-pyrazol-3-on) bezogen auf die Masse der Acrylsäure stabilisiert. Im Vergleichsversuch war die Acrylsäure von Strom 1 mit 200 Gew. ppm MEHQ bezogen auf die Masse der Acrylsäure stabilisiert.

Die erhaltenen Polymere wurden mittels GPC analysiert (Kalibrierung mit Na-PAA-Standard, Elutionsmittel 0,01 mol/l Phosphatpuffer, pH=7,4 in dest. Wasser mit 0,01 M NaN3).

| | Mw (g/mol) |
|---|---|
| Polymer (Edaravon) | 9560 |
| Polymer (MEHQ) | 9400 |

### Tests in Acrylaten:

### Versuche zur Polymerisationsinhibierung unter Luft oder Stickstoff:

Für die Versuche zur Polymerisationsinhibierung unter Luft oder Stickstoff wurde entstabilisiertes Ethylacrylat verwendet. Hierfür wurde Ethylacrylat, das 10 bis 20 Gew. ppm MEHQ bezogen auf die Menge an Ethylacrylat enthält drucklos über Aluminiumoxid Typ 90 aktiv neutral filtriert. Pro 100 ml stabilisiertem Ethylacrylat wurden 20 g Aluminiumoxid Typ 90 aktiv neutral verwendet. Nach der Filtration war mittels HPLC-Analytik kein MEHQ mehr im Ethylacrylat nachweisbar.

Für die Vergleichsversuche wurde eine 13 Gew. ppm MEHQ enthaltende Ethylacrylatlösung hergestellt. Die Angabe Gew. ppm bezieht sich auf die Masse des in der Lösung enthaltenen Ethylacrylats.

Für weitere Vergleichsversuche wurden Mischungen von Ethylacrylat mit Verbindungen die von den Verbindungen der allgemeinen Formel (I), (II), (III) oder (IV) unterschiedlich sind hergestellt, wobei die Molmenge der eingesetzten Verbindungen der Molmenge an MEHQ in den Vergleichsversuchen entspricht.

Für die erfindungsgemäßen Versuche wurden Mischungen von Ethylacrylat mit Verbindungen der allgemeinen Formel (I), (II), (III) und/oder (IV) hergestellt, wobei die Molmenge an Verbindungen der allgemeinen Formel (I), (II), (III) und/oder (IV) in den Mischungen der Molmenge an MEHQ in den Vergleichsversuchen entspricht.

### Exemplarische Verdünnungsreihe:

Von einer Ethylacrylatlösung mit einem Gehalt von 500 Gew. ppm MEHQ bezogen auf die Masse des in der Lösung enthaltenen Ethylacrylats wurde 1,0 g entnommen und mit Ethylacrylat auf einen Gehalt von 240 Gew. ppm MEHQ bezogen auf die Masse des Ethylacrylats in der Lösung verdünnt.

Von der so erhaltenen Ethylacrylatlösung wurden 1,5 g entnommen und mit Ethylacrylat auf einen Gehalt von 80 Gew. ppm MEHQ bezogen auf die Masse des Ethylacrylats in der Lösung verdünnt.

Von der so erhaltenen Ethylacrylatlösung wurden 1.5 g entnommen und mit Ethylacrylat auf einen Gehalt von 30 Gew. ppm MEHQ bezogen auf die Masse des Ethylacrylats in der Lösung verdünnt.

Von der so erhaltenen Ethylacrylatlösung wurden 2,0 g entnommen und mit Ethylacrylat auf einen Gehalt von 13 Gew. ppm MEHQ bezogen auf die Masse des Ethylacrylats in der Lösung verdünnt.

Als Starterlösung wurde eine frisch angesetzte Toluol-Lösung von 2,2'-Azobis(4-methoxy-2,4-dimehtylvalerontril) verwendet. Der Gehalt an 2,2'-Azobis(4-methoxy-2,4-dimehtyl-valerontril) in der Starterlösung beträgt 6420 Gew. ppm bezogen auf die Masse an Toluol in der Starterlösung.

### Tests zur Polymerisationsinhibierung unter Luft:

Unter Luft wurden 2.0 g Ethylacrylatlösung enthaltend 0,00021 mmol des jeweiligen Polymerisationsstabilisators vorgelegt. Die vorgelegte Ethylacrylatlösung wurde mit 0,17 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß.

Das Reaktionsgefäß enthaltend die Mischung wurde in einem auf 40°C erwärmten Heizblock platziert.
Nachdem die Mischung die Temperatur von 40°C erreicht hat wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 1200 Sekunden dauernder Temperaturanstieg um 8°C auf eine maximale Temperatur von 48°C gemessen. Die maximale Temperatur lag nach 29084 Sekunden vor, nachdem die Mischung auf 40°C temperiert war. Zur Berechnung der Effizienz der Polymerisationsstabilisatoren in Bezug auf MEHQ entspricht der Wert von 29084 Sekunden einer Effizienz von 100 %.

Für eine erfindungsgemäße Mischung mit 5-Methyl-2-phenyl-4H-pyrazol-3-on als Polymerisationsstabilisator wurde ein 1200 Sekunden dauernder Temperaturanstieg um 6°C auf eine maximale Temperatur von 46°C beobachtet. Die maximale Temperatur lag nach 28470 Sekunden vor, nachdem die Mischung auf 40°C temperiert war.
Im Vergleich zu MEHQ weist 5-Methyl-2-phenyl-4H-pyrazol-3-on als Polymerisationsstabilisator somit eine Effizienz von 98 Prozent auf.

### Tests zur Polymerisationsinhibierung unter Stickstoff:

Unter Luft wurden 2.0 g Ethylacrylatlösung enthaltend 000021 mmol des jeweiligen Polymerisationsstabilisators vorgelegt. Die vorgelegte Ethylacrylatlösung wurde mit 0,17 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß. In das Reaktionsgefäß wurde oberhalb der vorgelegten Mischung Stickstoff unter Druckausgleich eingeleitet. Der Stickstoffstrom beträgt 3 bis 4 l/h. Die Einleitdauer beträgt 20 Sekunden. Der Stickstoff weist eine Reinheit von größer 99,9 % auf.

Das Reaktionsgefäß enthaltend die Mischung wurde in einem Heizblock platziert. Die Mischung wurde anschließend im Heizblock innerhalb von 4 Minuten auf 40°C erwärmt. Nachdem die Mischung die Temperatur von 40°C erreicht hat wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 240 Sekunden dauernder Temperaturanstieg um 73°C auf eine maximale Temperatur von 113°C gemessen. Die maximale Temperatur lag nach 460 Sekunden vor, nachdem die Mischung auf 40°C temperiert war. Zur Berechnung der Effizienz der Polymerisationsstabilisatoren in Bezug auf MEHQ entspricht der Wert von 460 Sekunden einer Effizienz von 100 Sekunden.

Für eine erfindungsgemäße Mischung mit 5-Methyl-2-phenyl-4H-pyrazol-3-on als Polymerisationsstabilisator wurde ein 240 Sekunden dauernder Temperaturanstieg um 80°C auf eine maximale Temperatur von 120°C beobachtet. Die maximale Temperatur lag nach 426 Sekunden vor, nachdem die Mischung auf 40°C temperiert war.
Im Vergleich zu MEHQ weist 5-Methyl-2-phenyl-4H-pyrazol-3-on als Polymerisationsstabilisator somit eine Effizienz von 93 Prozent auf.

Ergebnisse der Tests in Ethylacrylat mit erfindungsgemäßen Verbindungen:

| Struktur | IUPAC-Name | Relative Stabilisierung von Ethylacrylat unter Luft bezogen auf MeHQ (100%) | Relative Stabilisierung von Ethylacrylat unter Stickstoff bezogen auf MeHQ (100%) |
|---|---|---|---|
| 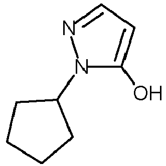 | 2-Cyclo-pentylpyrazol-3-ol | 97 | 45 |
| 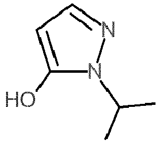 | 2-Isopropyl-pyrazol-3-ol | 95 | 69 |
| 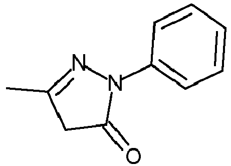 | 5-Methyl-2-phenyl-4H-pyrazol-3-one | 86 | 45 |
| 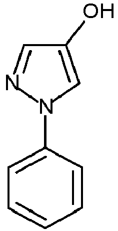 | 1-Phenylpyrazol-4-ol | 105 | 51 |
| 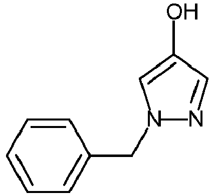 | 1-benzylpyrazol-4-ol | 107 | 58 |
| 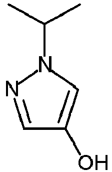 | 1-Isopropylpyrazol-4-ol | 106 | 101 |
| 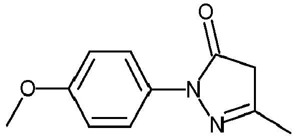 | 2-(4-Methoxyphenyl)-5-methyl-4H-pyrazol-3-one | 89 | 46 |
| 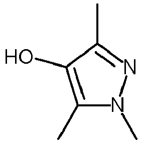 | 1,3,5-Trimethylpyrazol-4-ol | 117 | 97 |
| 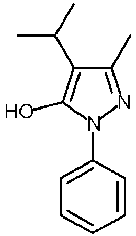 | 4-Isopropyl-5-methyl-2-phenyl-pyrazol-3-ol | 151 | 63 |
| 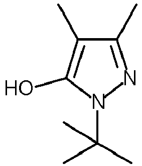 | 2-tert-Butyl-4,5-dimethyl-pyrazol-3-ol | 106 | 74 |
| 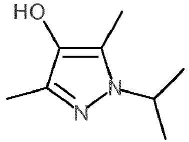 | 1-Isopropyl-3,5-dimethyl-pyrazol-4-ol | 122 | 86 |

### Polymerisationsversuche mit Acrylaten:

### Homopolymerisation von n-Butylacrylat:

### Vergleichsversuch:

Unter einer Stickstoffatmosphäre wurden in einem Reaktionsgefäß 210 g n-Butylacetat, 14,7 g n-Butylacrylat (stabilisiert mit 15 Gew. ppm MEHQ bezogen auf die Menge des n-Butylacrylats) und 0,08 g Azobisisobutyronitril (AIBN) vorgelegt. Die vorgelegte Reaktionsmischung wurde unter Rühren auf 80°C erhitzt. Nach 15 Minuten nach Erreichen der Temperatur von 80°C erfolgte die Zuführung von zwei Strömen unter Beibehaltung der Temperatur.
Strom 1: Zuführung über 2 h, 195,3 g n- Butylacrylat (stabilisiert mit 15 Gew. ppm MEHQ bezogen auf die Menge des n-Butylacrylats) und 100,5 g n-Butylacetat
Strom 2: Zuführung über 3 h, 0,98 g AIBN und 74,1 g n-Butylacetat
   Die Reaktionsmischung wurde nach Zugabe der Ströme für 2h bei 80°C gerührt und anschließend auf Raumtemperatur abgekühlt.

### Erfindungsgemäß:

Unter einer Stickstoffatmosphäre wurden in einem Reaktionsgefäß 210 g n-Butylacetat, 14,7 g n-Butylacrylat (stabilisiert mit 21 Gew. ppm 5-Methyl-2-phenyl-4H-pyrazol-3-on bezogen auf die Menge des n-Butylacrylats) und 0,08 g AIBN vorgelegt. Die vorgelegte Reaktionsmischung wurde unter Rühren auf 80°C erhitzt. Nach 15 Minuten nach Erreichen der Temperatur von 80°C erfolgte die Zuführung von zwei Strömen unter Beibehaltung der Temperatur.
Strom 1: Zuführung über 2 h, 195,3 g n- Butylacrylat (stabilisiert mit 21 Gew. ppm 5-Methyl-2-phenyl-4H-pyrazol-3-on bezogen auf die Menge des n-Butylacrylats) und 100,5 g n-Butylacetat
Strom 2: Zuführung über 3 h, 0,98 g AIBN und 74,1 g n-Butylacetat
   Die Reaktionsmischung wurde nach Zugabe der Ströme für 2h bei 80°C gerührt und anschließend auf Raumtemperatur abgekühlt.

Die erhaltenen Polymere wurden mittels GPC analysiert (Kalibrierung Polystyrolstandard, Elutionsmittel: THF + 0,1 Vol. % Trifluoressigsäure).

| | Mw (g/mol) |
|---|---|
| Polymer (erfindungsgemäß) | 164000 |
| Polymer (MEHQ) | 163000 |

### Emulsionspolymerisation von n-Butylacrylat, Styrol und Methacrylsäure:

### Vergleichsversuch:

Unter einer Stickstoffatmosphäre wurden in einem Reaktionsgefäß 250 g VE Wasser und 4 g Kaliumperoxodisulfat vorgelegt. Das vorgelegte Reaktionsgemisch wurde unter Rühren auf 60°C erwärmt. Bei 60°C wurden unter Rühren 8 g von Zulauf 1 dem Reaktionsgemisch zugeführt. 5 Minuten nach Zugabe wurden bei 60°C und unter Rühren der Rest von Zulauf 1 und Zulauf 2 dem Reaktionsgemisch zugeführt. Der Rest von Zulauf 1 wurde über eine Zeit von 160 Minuten, der Zulauf 2 wurde über eine Zeit von 150 Minuten dem Reaktionsgemisch zugeführt.
Zulauf 1: 4 g Natriumhydrogensulfit, 100 g VE Wasser
Zulauf 2: 400 g Styrol (stabilisiert mit 20 Gew. ppm MEHQ bezogen auf die Menge Styrol), 400 g n-Butylacrylat (stabilisiert mit 15 Gew. ppm MEHQ bezogen auf die Menge n-Butylacrylat), 15 g Methacrylsäure (stabilisiert mit 200 Gew. ppm MEHQ bezogen auf die Menge Methacrylsäure), 20 g Disponil FES 147 und 450 g VE Wasser.

Nach Beendigung der Zuführung von Zulauf 1 wurde die Temperatur des Reaktionsgemisches auf 65°C erhöht und das Reaktionsgemisch für 1 h bei dieser Temperatur gehalten. Die erhaltene Dispersion wurde auf Umgebungstemperatur abgekühlt, mit wässriger Ammoniaklösung (25 Gew. %) auf pH 7 eingestellt und durch ein Sieb filtriert.

### Erfindungsgemäß:

Unter einer Stickstoffatmosphäre wurden in einem Reaktionsgefäß 250 g VE Wasser und 4 g Kaliumperoxodisulfat vorgelegt. Das vorgelegte Reaktionsgemisch wurde unter Rühren auf 60°C erwärmt. Bei 60°C wurden unter Rühren 8 g von Zulauf 1 dem Reaktionsgemisch zugeführt. 5 Minuten nach Zugabe wurden bei 60°C und unter Rühren der Rest von Zulauf 1 und Zulauf 2 dem Reaktionsgemisch zugeführt. Der Rest von Zulauf 1 wurde über eine Zeit von 160 Minuten, der Zulauf 2 wurde über eine Zeit von 150 Minuten dem Reaktionsgemisch zugeführt.
Zulauf 1: 4 g Natriumhydrogensulfit, 100 g VE Wasser
Zulauf 2: 400 g Styrol (stabilisiert mit 28 Gew. ppm Edaravon bezogen auf die Menge Styrol), 400 g n-Butylacrylat (stabilisiert mit 21 Gew. ppm Edaravon bezogen auf die Menge n-Butylacrylat), 15 g Methacrylsäure (stabilisiert mit 280 Gew. ppm Edaravon bezogen auf die Menge Methacrylsäure), 20 g Disponil FES 147 und 450 g VE Wasser.

Nach Beendigung der Zuführung von Zulauf 1 wurde die Temperatur des Reaktionsgemisches auf 65°C erhöht und das Reaktionsgemisch für 1 h bei dieser Temperatur gehalten. Die erhaltene Dispersion wurde auf Umgebungstemperatur abgekühlt, mit wässriger Ammoniaklösung (25 Gew. %) auf pH 7 eingestellt und durch ein Sieb filtriert.

| | Mittlerer Teilchendurchmesser (nm) | Glastemperatur (°C) | Mw (g/mol) | Koagulat (Gew.%) | Restmonomerengehalt (Gew. ppm) |
|---|---|---|---|---|---|
| Vergleichsversuch | 163 | 19 | 820000 | 0,25 | n-Butylacrylat: 300 Styrol: < 20 |
| Erfindungsgemäß | 168 | 21 | 850000 | 0,4 | n-Butylacrylat: 500 Styrol: < 20 |

### Der mittlere Teilchendurchmesser (z-Average) wurde wie folgt bestimmt:

Die Proben wurden mit einem Zetasizer Nano der Firma Malvern analysiert. Sie wurden mit Milliporewasser (pH4) auf eine Messkonzentration von 0,0005% verdünnt und dann 5µm filtriert bestimmt. Die Messungen erfolgten bei 25°C.

### Die Glastemperatur wurde wie folgt ermittelt:

Die Polymerdispersionen wurden bei 80°C/Vakuum getrocknet. Die Glastemperatur wurde mittels DSC bestimmt. Vor der Messung wurden die Proben von 140°C aus schnell abgekühlt und anschließend mit 20 K/min von -50°C bis 140°C vermessen.

Die Molmassenbestimmung erfolgte mittels GPC (Kalibrierung mit Polystyrol-Standard, Elutionsmittel: THF + 0.1 Vol. % Trifluoressigsäure)

Der Restmonomergehalt wurde mittels Head-Space GC bestimmt.

Die Koagulatmenge (aggregierte Polymerkolloide in der Apparatur und im Filtrationsrückstand) wurde gewogen.

## Patentansprüche

1. Mischung enthaltend eine oder mehrere Verbindungen der allgemeinen Formel (I) 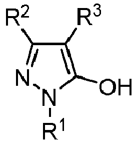 wobei
R¹ H, C₁- bis C₂₀-Alkyl, Alkanoyl mit C₁- bis C₈-Alkyl oder ein aromatischer Rest ist,
R² H, C₁- bis C₂₀-Alkyl, Carboxylester mit C₁- bis C₈-Alkyl, Alkanoyl mit C₁- bis C₈-Alkyl oder ein aromatischer Rest ist,
R³ H, C₁- bis C₂₀-Alkyl, Carboxylester mit C₁- bis C₈-Alkyl, Alkanoyl mit C₁- bis C₈-Alkyl, ein aromatischer Rest oder -CH₂-CHR⁴-C(O)OR⁵ ist, wobei R⁴ H oder Methyl ist und R⁵ H, C₁-bis C₂₀ Alkyl oder ein aromatischer Rest ist,
wobei R² und R³ nicht gleichzeitig Methyl sind, wenn R¹ Phenyl ist,
und/oder eine oder mehrere Verbindungen der allgemeinen Formel (II) 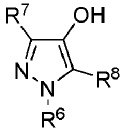
wobei
R⁶ H, C₁- bis C₂₀-Alkyl, Alkanoyl mit C₁- bis C₈-Alkyl oder ein aromatischer Rest ist,
R⁷ H, C₁- bis C₂₀-Alkyl, Carboxylester mit C₁- bis C₈-Alkyl, Alkanoyl mit C₁- bis C₈-Alkyl oder ein aromatischer Rest ist,
R⁸ H, C₁- bis C₂₀-Alkyl, Carboxylester mit C₁- bis C₈-Alkyl, Alkanoyl mit C₁- bis C₈-Alkyl, ein aromatischer Rest oder -CH₂-CHR^{4I}-C(O)OR^{5I} ist, wobei R^{4I} H oder Methyl ist und R^{5I} H, C₁-bis C₂₀-Alkyl oder ein aromatischer Rest ist,
wobei
R⁶ und R⁷ nicht gleichzeitig Phenyl sind, wenn R⁸ Phenyl oder Methyl ist,
R⁶ und R⁸ nicht gleichzeitig Phenyl sind, wenn R⁷ H oder Methyl ist,
R⁶ nicht Phenyl und R⁸ nicht Methyl ist, wenn R⁷ H oder Methyl ist,
und/oder eine oder mehrere Verbindungen der allgemeinen Formel (III) 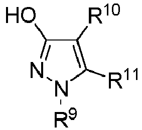
wobei
R⁹ H, C₁- bis C₂₀-Alkyl, Alkanoyl mit C₁- bis C₈-Alkyl oder ein aromatischer Rest ist,
R¹⁰ H, C₁- bis C₂₀-Alkyl, Alkanoyl mit C₁- bis C₈-Alkyl, ein aromatischer Rest oder - C(O)O-R^{5II} ist, wobei R^{5II} H, C₁-bis C₂₀ Alkyl oder ein aromatischer Rest ist,
R¹¹ H, C₁-bis C₂₀-Alkyl, Alkanoyl mit C₁- bis C₈-Alkyl, ein aromatischer Rest oder-C(O)O-R^{5III} ist, wobei R^{5III} H, C₁- bis C₂₀-Alkyl oder ein aromatischer Rest ist, und/oder eine oder mehrere Verbindungen der allgemeinen Formel (IV) 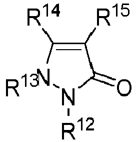
wobei
R¹² H, C₁- bis C₂₀-Alkyl, Alkanoyl mit C₁- bis C₈-Alkyl oder ein aromatischer Rest ist,
R¹³ H, C₁- bis C₂₀-Alkyl, Alkanoyl mit C₁- bis C₈-Alkyl, ein aromatischer Rest oder-C(O)O-R^{5IV} ist, wobei R^{5IV} H, C₁- bis C₂₀-Alkyl oder ein aromatischer Rest ist.
R¹⁴ H, C₁- bis C₂₀-Alkyl, Alkanoyl mit C₁- bis C₈-Alkyl, Caroxylester mit C₁- bis C₈-Alkyl oder ein aromatischer Rest ist,
R¹⁵ H, NH₂, NH(CO)R¹⁶, C₁- bis C₂₀-Alkyl, Alkanoyl mit C₁- bis C₈-Alkyl, Carboxylester mit C₁- bis C₈-Alkyl oder ein aromatischer Rest ist, wobei R¹⁶ H, C₁- bis C₂₀-Alkyl oder ein aromatischer Rest ist
und eine oder mehrere polymerisationsfähige Verbindungen.

2. Mischung nach Anspruch 1, wobei
R¹ H, C₁- bis C₈-Alkyl, Alkanoyl mit C₁- bis C₆-Alkyl oder ein aromatischer Rest ist,
R² H, C₁- bis C₈-Alkyl, Alkanoyl mit C₁- bis C₆-Alkyl oder ein aromatischer Rest ist,
R³ H, C₁- bis C₈-Alkyl, Alkanoyl mit C₁- bis C₆-Alkyl, ein aromatischer Rest oder -CH₂-CHR⁴-C(O)OR⁵ ist, wobei R⁴ H oder Methyl ist und R⁵ H, C₁-bis C₈ Alkyl oder ein aromatischer Rest ist,
wobei R² und R³ nicht gleichzeitig Methyl sind, wenn R¹ Phenyl ist.

3. Mischung nach Anspruch 1, wobei
R⁶ H, C₁- bis C₈-Alkyl, Alkanoyl mit C₁- bis C₆-Alkyl oder ein aromatischer Rest ist,
R⁷ H, C₁- bis C₈-Alkyl, Alkanoyl mit C₁- bis C₆-Alkyl oder ein aromatischer Rest ist,
R⁸ H, C₁- bis C₈-Alkyl, Alkanoyl mit C₁- bis C₆-Alkyl, ein aromatischer Rest oder -CH₂-CHR^{4I}-C(O)OR^{5I} ist, wobei R^{4I} H oder Methyl ist und R^{5I} H, C₁-bis C₈-Alkyl oder ein aromatischer Rest ist,
wobei
R⁶ und R⁷ nicht gleichzeitig Phenyl sind, wenn R⁸ Phenyl oder Methyl ist,
R⁶ und R⁸ nicht gleichzeitig Phenyl sind, wenn R⁷ H oder Methyl ist,
R⁶ nicht Phenyl und R⁸ nicht Methyl ist, wenn R⁷ H oder Methyl ist.

4. Mischung nach Anspruch 1, wobei
R⁹ H, C₁- bis C₈-Alkyl, Alkanoyl mit C₁- bis C₆-Alkyl oder ein aromatischer Rest ist,
R¹⁰ H, C₁- bis C₈-Alkyl, Alkanoyl mit C₁- bis C₆-Alkyl, ein aromatischer Rest oder-C(O)O-R^{5II} ist, wobei R^{5II} H, C₁-bis C₈ Alkyl oder ein aromatischer Rest ist,
R¹¹ H, C₁-bis C₈-Alkyl, Alkanoyl mit C₁- bis C₆-Alkyl, ein aromatischer Rest oder-C(O)O-R^{5III} ist, wobei R^{5III} H, C₁- bis C₈-Alkyl oder ein aromatischer Rest ist.

5. Mischung nach Anspruch 1, wobei
R¹² H, C₁- bis C₈-Alkyl, Alkanoyl mit C₁- bis C₆-Alkyl oder ein aromatischer Rest ist,
R¹³ H, C₁- bis C₈-Alkyl, Alkanoyl mit C₁- bis C₆-Alkyl, ein aromatischer Rest oder - C(O)O-R^{5IV} ist, wobei R^{5IV} H, C₁- bis C₈-Alkyl oder ein aromatischer Rest ist.
R¹⁴ H, C₁- bis C₈-Alkyl, Alkanoyl mit C₁- bis C₆-Alkyl, oder ein aromatischer Rest ist,
R¹⁵ H, C₁- bis C₈-Alkyl, Alkanoyl mit C₁- bis C₆-Alkyl oder ein aromatischer Rest ist.

6. Mischung nach einem der Ansprüche 1 bis 5, wobei es sich bei einer polymerisationsfähigen Verbindung um (Meth)acrylsäure oder um (Meth)acrylsäureester mit C₁- bis C₈-Alkyl handelt.

7. Mischung nach einem der Ansprüche 1 bis 6, wobei die Gesamtmenge der Verbindungen der allgemeinen Formel (I), (II), (III) und (IV) in der Mischung 0,1 bis 1000 Gew. ppm, bezogen auf das Gesamtgewicht der polymerisationsfähigen Verbindungen beträgt.

8. Mischung nach einem der Ansprüche 1 bis 7, wobei die Gesamtmenge der polymerisationsfähigen Verbindungen in der Mischung mindestens 5 Gew.% bezogen auf das Gesamtgewicht der Mischung beträgt.

9. Mischung nach einem der Ansprüche 1 bis 8, wobei die Mischung unter einer sauerstoffhaltigen Atomsphäre gelagert und/oder transportiert wird.

10. Mischung nach einem der Ansprüche 1 bis 9, wobei die Mischung einen Costabilisator enthält.

11. Verwendung einer oder mehrerer Verbindungen der allgemeinen Formel (I), (II), (III), (IV) wie in einem der Ansprüche 1 bis 5 definiert oder einer beliebigen Mischung dieser, zur Lager- und/oder Transportstabilisierung einer oder mehrerer polymerisationsfähigen Verbindungen.

12. Verfahren zur Polymerisation von einer oder mehreren polymerisationsfähigen Verbindungen in Gegenwart einer oder mehrerer Verbindungen der allgemeinen Formel (I), (II), (III), (IV) wie in einer der Ausführungsformen 1 bis 9 definiert oder einer beliebige Mischung dieser, wobei die Reaktionsmischung zu 0 bis 50 % mit Sauerstoff gesättigt ist, wobei der Grad der Sauerstoffsättigung sich auf den Grad der Sauerstoffsättigung bezieht der im Gleichgewicht erreicht wird, wenn die vorliegende Mischung unter Standardbedingungen unter einer sauerstoffhaltigen Atmosphäre mit einem Sauerstoffanteil von 5 bis 30 vol.% gelagert und/oder transportiert wird.

13. Verfahren zur Stabilisierung von einer oder mehrerer polymerisationsfähigen Verbindungen unter Verwendung einer oder mehrerer Verbindungen der allgemeinen Formel (I), (II), (III), (IV) wie einer der Ausführungsformen 1 bis 9 definiert, oder einer beliebigen Mischung dieser.
